(19) Europäisches Patentamt
European Patent Office
Office européen des brevets

(11) EP 4 079 733 A1

(12) **EUROPEAN PATENT APPLICATION**
published in accordance with Art. 153(4) EPC

(43) Date of publication:
**26.10.2022 Bulletin 2022/43**

(21) Application number: **20903847.0**

(22) Date of filing: **18.12.2020**

(51) International Patent Classification (IPC):
***C07D 487/04*** *(2006.01)* ***A61P 35/00*** *(2006.01)*
***A61K 31/41*** *(2006.01)*

(52) Cooperative Patent Classification (CPC):
**A61K 31/41; A61K 31/437; A61K 31/53; A61P 35/00; C07D 471/04; C07D 487/04**

(86) International application number:
**PCT/CN2020/137618**

(87) International publication number:
**WO 2021/121390 (24.06.2021 Gazette 2021/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.12.2019 CN 201911329611**
**20.04.2020 CN 202010312893**
**28.08.2020 CN 202010882490**

(71) Applicant: **Evopoint Biosciences Co., Ltd.**
**Suzhou, Jiangsu 215000 (CN)**

(72) Inventors:
- **HU, Yonghan**
  **Suzhou, Jiangsu 215000 (CN)**
- **WU, Dongdong**
  **Suzhou, Jiangsu 215000 (CN)**
- **PENG, Wei**
  **Suzhou, Jiangsu 215000 (CN)**
- **ZHANG, Xiuchun**
  **Suzhou, Jiangsu 215000 (CN)**
- **WU, Yuchuan**
  **Suzhou, Jiangsu 215000 (CN)**

(74) Representative: **Wilson, Justin Scott**
**Withers & Rogers LLP**
**2 London Bridge**
**London SE1 9RA (GB)**

(54) **HETEROCYCLIC COMPOUND, AND PHARMACEUTICAL COMPOSITION THEREOF, PREPARATION METHOD THEREFOR, INTERMEDIATE THEREOF AND APPLICATION THEREOF**

(57) A heterocyclic compound, and a pharmaceutical composition thereof, a preparation method therefor, an intermediate thereof and an application thereof. The structure of the heterocyclic compound is as shown in formula (I) below. The compound has CDK7 inhibitory activity, and can be used to treat tumors and other diseases.

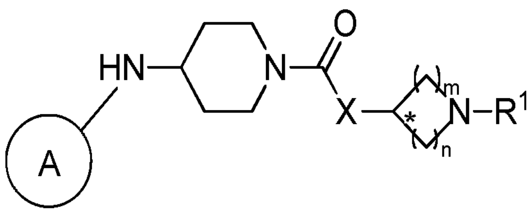

I

EP 4 079 733 A1

## Description

### CROSS-REFERENCE TO RELATED APPLICATIONS

The present application claims priority to the Chinese Patent Application 201911329611.X filed on Dec. 20, 2019, the Chinese Patent Application 202010312893.9 filed on Apr. 20, 2020, and the Chinese Patent Application 202010882490.8 filed on Aug. 28, 2020, which are incorporated herein by reference in entirety.

### TECHNICAL FIELD

The present application relates to a heterocyclic compound and a pharmaceutical composition, a preparation method, an intermediate and use thereof.

### BACKGROUND

Cyclin-dependent kinases (CDKs) belong to the serine/threonine kinase family, and the monomers thereof are not active. They must be bound to corresponding cyclins to form active heterodimer complexes to exert a regulatory effect, and thus phosphorylation of corresponding substrates can be catalyzed, and cells can be directly or indirectly regulated to complete the cell cycle, thereby causing cell growth and proliferation. It has now been found that the human genome encodes 21 CDKs and more than 15 cyclins. CDKs can be divided into two major classes according to their functions: CDKs that control cell cycle and CDKs that control cell transcription. Among these, CDK 1/2/4/6 are primarily associated with the cell cycle, while CDK7/8/9/10 are mainly associated with the transcriptional mechanism of the genetic information within the cells (Asghar U, Witkiewicz A K, Turner N C, et al., The history and future of targeting cyclin-dependent kinases in cancer therapy. Nat Rev Drug Discov, 2015 (2): 130-146).

CDK7 is an important member of the CDK family, and it regulates the cell cycle mainly in two indirect ways. One is that CDK7, together with cyclin H and Mat1, constitutes CAK (CDKs activating kinase), and further phosphorylates CDK1/2, thereby activating their function in the cell cycle (Yee A, Nichols MA, Wu L, Hall FL, Kobayashi R, Xiong Y. Molecular cloning of CDK7-associated human MAT1, a cyclin-dependent kinase-activating kinase (CAK) assembly factor. Cancer Res. 1995; 55: 6058-6062). Another way is that CDK7, as a subunit component of the universal transcription factor TFIIH, phosphorylates the carboxy-terminal domain (CTD) of the large subunit of RNA polymerase II (RNAP II), and regulates the gene transcription process in cells (Kelso TW, Baumgart K, Eickhoff J, Albert T, Antrecht C, Lemcke S et al. Cyclin-dependent kinase 7 controls mRNA synthesis by affecting stability of preinitiation complexes, leading to altered gene expression, cell cycle progression, and survival of tumor cells. Mol Cell Biol. 2014; 34: 3675-3688). Because of its dual function of CAK and CTD phosphorylation, CDK7 plays an important role in cell proliferation, cell cycle and transcription.

In recent years, inhibition of CDK7 has gradually become a promising therapeutic strategy for a variety of cancers. Inhibition of CDK7 can inhibit expression of key oncogenes such as c-Myc (Chipumuro E, Marco E, Christensen CL, Kwiatkowski N, Zhang T, Hatheway CM, et al. CDK7 inhibition suppresses super-enhancer-linked oncogenic transcription in MYCN-driven cancer. Cell. 2014, 159: 1126-39). Preclinical research data show that small molecule inhibitors that inhibit CDK7 have good anti-cancer effect in hormone receptor positive and triple negative breast cancers (Wang Y, Zhang T, Kwiatkowski N, Abraham BJ, Lee TI, Xie S, et al. CDK7dependent transcriptional addiction in triple-negative breast cancer. Cell. 2015; 163: 174-86), and in cancers driven by transcription factors, such as small cell lung cancer (SCLC) (Christensen CL, Kwiatkowski N, Abraham BJ, Carretero J, Al-Shahrour F, Zhang T, et al. Targeting transcriptional addictions in small cell lung cancer with a covalent CDK7 inhibitor. Cancer Cell 2014; 26: 909-22). There is currently a lack of effective treatment approaches for these cancers, and therefore there are significant unmet medical needs. Also, CDK7 inhibitors may be effective against cancers that have become resistant to current therapies because they have a different mechanism of action. Therefore, the development of CDK7 inhibitors would likely be an effective means for treating these malignant tumors.

CDK7 inhibitors that have been reported include THZ1, SY-1365 (a compound in clinic trials from Syros), CT7001 (a compound in clinic trials from Carrick), and the like. Their structures are as follows:

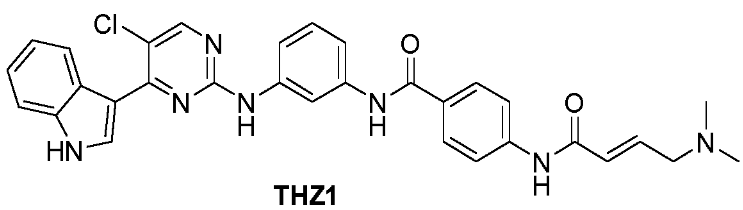

THZ1

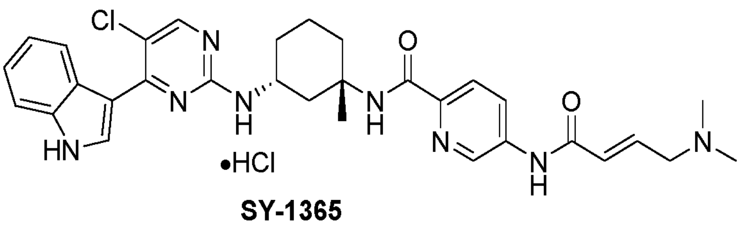

**SY-1365**

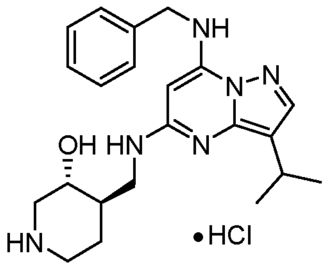

**CT7001**

Patent application WO2019099298A1 of the Lilly Company discloses two CDK7 inhibitors in Example 1 and Example 3, and the structures of the compounds are as follows:

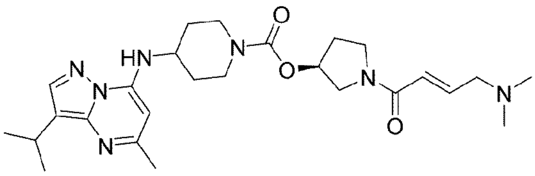

WO2019099298 Example 1

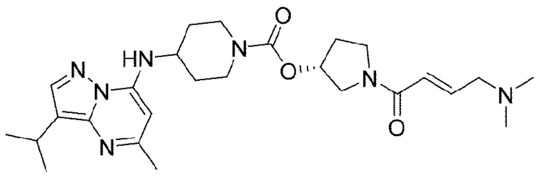

WO2019099298 Example 3

## SUMMARY

The technical problem to be solved by the present invention is that the existing CDK7 inhibitors are relatively homogeneous in structure, and thus the present application provides a heterocyclic compound with a new structure and a pharmaceutical composition, a preparation method, an intermediate and use thereof. The heterocyclic compound disclosed herein has good inhibition effect on CDK7, and can be used for treating diseases such as tumors.

The present invention provides a compound of formula I:

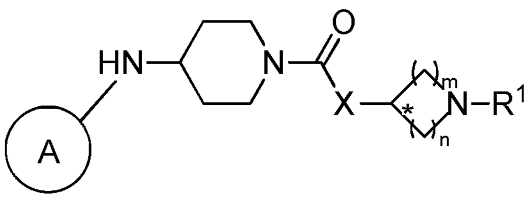

**I**

or a tautomer, a stereoisomer or an isotopic derivative thereof, or a pharmaceutically acceptable salt of any of the foregoing (namely the aforementioned compound of formula I, tautomer, stereoisomer or isotopic derivative), or a crystalline form or a solvate of any of the foregoing (namely the aforementioned compound of formula I, tautomer, stereoisomer, isotopic derivative or pharmaceutically acceptable salt); wherein, ring A is

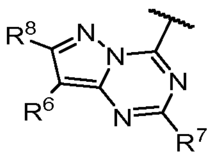, 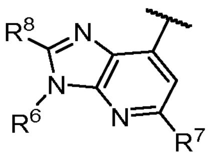, 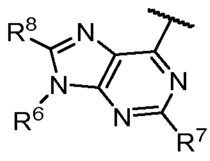,

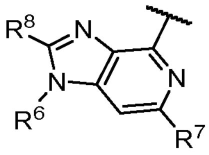, 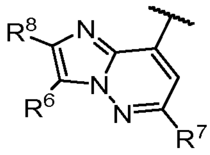, 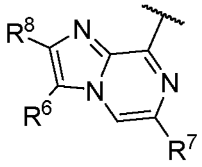 or 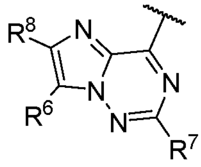;

X is O or $NR^a$;
$R^a$ is hydrogen, methyl or ethyl;
m is 1 or 2;
n is 1, 2 or 3;

$R^1$ is

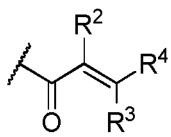 (e.g., 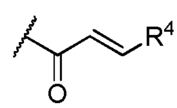) or 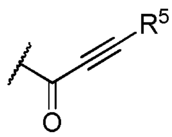;

$R^2$ is hydrogen or $C_{1-6}$ alkyl;
$R^3$ is hydrogen or $C_{1-6}$ alkyl;
$R^4$ is hydrogen, halogen, $C_{1-6}$ alkyl or $-CH_2-NR^{4a}R^{4b}$ (e.g., $-CH_2-N(CH_3)_2$);
$R^{4a}$ and $R^{4b}$ are each independently hydrogen or $C_{1-6}$ alkyl (e.g., methyl); or, $R^{4a}$ and $R^{4b}$, together with a nitrogen atom attached thereto, form a substituted or unsubstituted 4-6 membered heterocycloalkyl, wherein the 4-6 membered heterocycloalkyl has 0, 1 or 2 additional heteroatoms independently selected from N, O and S, and the substituted 4-6 membered heterocycloalkyl means that the 4-6 membered heterocycloalkyl is substituted with 1, 2, 3 or $4R^b$;
each $R^b$ is independently halogen, hydroxy or $C_{1-4}$ alkyl;
$R^5$ is hydrogen or $C_{1-6}$ alkyl (e.g., methyl);
each $R^6$ is independently hydrogen, cyclopropyl or $C_{1-6}$ alkyl (e.g., methyl, ethyl, *n*-propyl or isopropyl, preferably isopropyl);
each $R^7$ is independently hydrogen, a substituted or unsubstituted $C_{1-6}$ alkyl (the $C_{1-6}$ alkyl is, e.g., methyl, ethyl or isopropyl; further, the substituted or unsubstituted $C_{1-6}$ alkyl is, e.g., methyl, ethyl, isopropyl or $-CH_2OH$), a substituted or unsubstituted phenyl, a substituted or unsubstituted 5-6 membered heteroaryl (e.g., the 5-6 membered heteroaryl is, e.g., pyrazolyl, such as

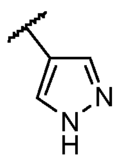

; further, the substituted or unsubstituted 5-6 membered heteroaryl is, e.g.,

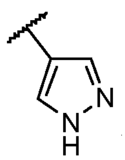

), a substituted or unsubstituted $C_{3-6}$ cycloalkyl (e.g., cyclopropyl), $-OR^{7a}$ (e.g.,

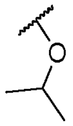

or

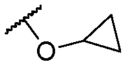)

or $-NR^{7b}R^{7c}$ (e.g.,

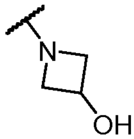, 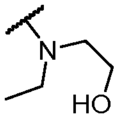, 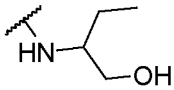, 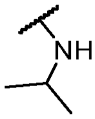, 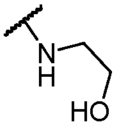,

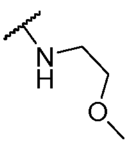, 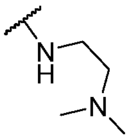, 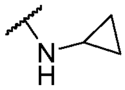, 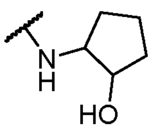, 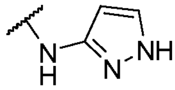 or 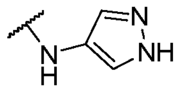,

wherein

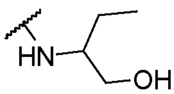

is, e.g.,

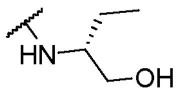

), wherein the substituted $C_{1-6}$ alkyl, the substituted phenyl, the substituted $C_{3-6}$ cycloalkyl and the substituted 5-6 membered heteroaryl mean that the $C_{1-6}$ alkyl, the phenyl, the $C_{3-6}$ cycloalkyl and the 5-6 membered heteroaryl are each independently substituted with 1, 2, 3 or 4 $R^{7d}$ (e.g., substituted with 1 $R^{7d}$);
each $R^{7d}$ is independently hydroxy, halogen, $C_{1-4}$ alkyl, $-NR^{a1}R^{a2}$ (e.g., $-N(CH_3)_2$) or $C_{1-4}$ alkoxy (e.g., methoxy);
$R^{7a}$ is hydrogen, a substituted or unsubstituted $C_{1-6}$ alkyl (the $C_{1-6}$ alkyl is, e.g., isopropyl; further, the substituted or unsubstituted $C_{1-6}$ alkyl is isopropyl), a substituted or unsubstituted $C_{3-6}$ cycloalkyl (the $C_{3-6}$ cycloalkyl is, e.g., cyclopropyl; further, the substituted or unsubstituted $C_{3-6}$ cycloalkyl is, e.g., cyclopropyl), a substituted or unsubstituted 4-6 membered heterocycloalkyl or a substituted or unsubstituted 5-6 membered heteroaryl, wherein the substituted $C_{1-6}$ alkyl, the substituted $C_{3-6}$ cycloalkyl, the substituted 4-6 membered heterocycloalkyl and the substituted 5-6 membered heteroaryl mean that the $C_{1-6}$ alkyl, the $C_{3-6}$ cycloalkyl, the 4-6 membered heterocycloalkyl and the 5-6 membered heteroaryl are each independently substituted with 1, 2. 3 or 4 $R^c$ (e.g., substituted with 1 $R^c$);
each $R^c$ is independently hydroxy, halogen, $C_{1-4}$ alkyl, $-NR^{c1}R^{c2}$ (e.g., $-N(CH_3)_2$) or $C_{1-4}$ alkoxy (e.g., methoxy);
$R^{7b}$ and $R^{7c}$ are each independently hydrogen, a substituted or unsubstituted $C_{1-4}$ alkyl (the $C_{1-4}$ alkyl is, e.g., ethyl, isopropyl or *sec*-butyl; further, the substituted or unsubstituted $C_{1-4}$ alkyl is, e.g., ethyl, isopropyl,

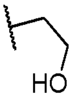, 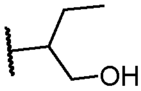, 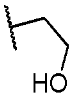, 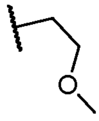 or 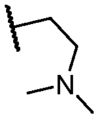,

wherein

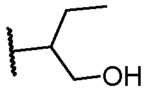

is, e.g.,

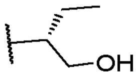

), a substituted or unsubstituted $C_{3-6}$ cycloalkyl (the $C_{3-6}$ cycloalkyl is, e.g., cyclopropyl or cyclopentyl; further, the substituted or unsubstituted $C_{3-6}$ cycloalkyl is, e.g., cyclopropyl or

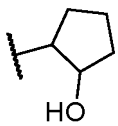,

wherein

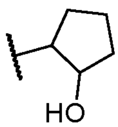

is, e.g.,

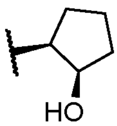

), a substituted or unsubstituted 4-6 membered heterocycloalkyl, or a substituted or unsubstituted 5-6 membered heteroaryl (the 5-6 membered heteroaryl is, e.g., pyrazolyl, such as

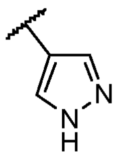

or

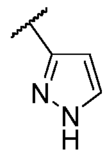
;

further, the substituted or unsubstituted 5-6 membered heteroaryl is, e.g.,

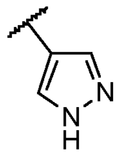 or 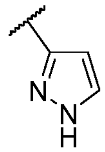

), wherein the substituted $C_{1-4}$ alkyl, the substituted $C_{3-6}$ cycloalkyl, the substituted 4-6 membered heterocycloalkyl and the substituted 5-6 membered heteroaryl mean that the $C_{1-4}$ alkyl, the $C_{3-6}$ cycloalkyl, the 4-6 membered heterocycloalkyl and the 5-6 membered heteroaryl are each independently substituted with 1, 2, 3, or 4 $R^d$ (e.g., substituted with 1 $R^d$);

or, $R^{7b}$ and $R^{7c}$, together with a nitrogen atom attached thereto, form a substituted or unsubstituted 4-6 membered heterocycloalkyl (the 4-6 membered heterocycloalkyl is, e.g., azetidinyl; further, the substituted or unsubstituted 4-6 membered heterocycloalkyl is, e.g.,

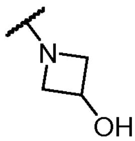

), wherein the 4-6 membered heterocycloalkyl has 0, 1 or 2 additional heteroatoms independently selected from N, O and S, and the substituted 4-6 membered heterocycloalkyl means that the 4-6 membered heterocycloalkyl is substituted with 1, 2, 3 or 4 $R^e$ (e.g., substituted with 1 $R^e$);

each $R^d$ is independently hydroxy, halogen, $C_{1-4}$ alkyl, $-NR^{d1}R^{d2}$ (e.g., $-N(CH_3)_2$) or $C_{1-4}$ alkoxy (e.g., methoxy);

each $R^e$ is independently hydroxy, halogen, $C_{1-4}$ alkyl, $-NR^{e1}R^{e2}$ or $C_{1-4}$ alkoxy (e.g., methoxy);

each $R^{a1}$, each $R^{a2}$, each $R^{c1}$, each $R^{c2}$ , each $R^{d1}$, each $R^{d2}$, each $R^{e1}$ and each $R^{e2}$ are each independently hydrogen or $C_{1-4}$ alkyl (e.g., methyl);

each $R^8$ is independently hydrogen or $C_{1-4}$ alkyl (e.g., methyl);

when a carbon atom marked by * has chirality, the carbon atom is in S configuration, R configuration, or a mixture thereof;

the number of heteroatoms in the 4-6 membered heterocycloalkyl and the number of heteroatoms in the 5-6 membered heteroaryl are independently 1, 2 or 3, and each heteroatom is independently selected from N, O and S.

In some embodiments, each $R^7$ is independently hydrogen, a substituted or unsubstituted $C_{1-6}$ alkyl, a substituted or unsubstituted phenyl, a substituted or unsubstituted 5-6 membered heteroaryl, $-OR^{7a}$ or $-NR^{7b}R^{7c}$, wherein the substituted $C_{1-6}$ alkyl, the substituted phenyl and the substituted 5-6 membered heteroaryl mean that the $C_{1-6}$ alkyl, the phenyl and the 5-6 membered heteroaryl are each independently substituted with 1, 2, 3 or 4 $R^{7d}$.

In some embodiments, the compound of formula I is defined as follows:

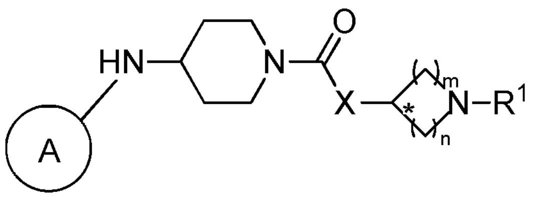

**I**

wherein, ring A is

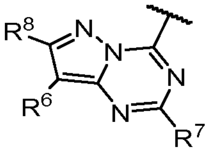, 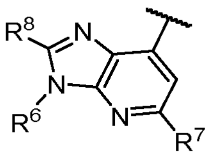, 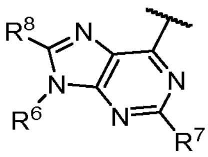,

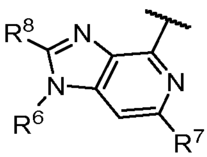 or 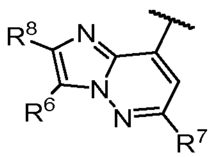;

X is O or $NR^a$;
$R^a$ is hydrogen, methyl or ethyl;
m is 1 or 2;
n is 1, 2 or 3;
$R^1$ is

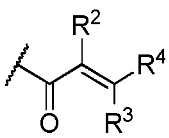 (e.g., 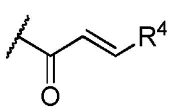) or 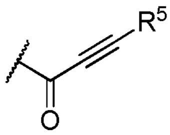;

$R^2$ is hydrogen or $C_{1-6}$ alkyl;
$R^3$ is hydrogen or $C_{1-6}$ alkyl;
$R^4$ is hydrogen, halogen, $C_{1-6}$ alkyl or $-CH_2-NR^{4a}R^{4b}$ (e.g., $-CH_2-N(CH_3)_2$);
$R^{4a}$ and $R^{4b}$ are each independently hydrogen or $C_{1-6}$ alkyl (e.g., methyl); or, $R^{4a}$ and $R^{4b}$, together with a nitrogen atom attached thereto, form a substituted or unsubstituted 4-6 membered heterocycloalkyl, wherein the 4-6 membered heterocycloalkyl has 0, 1 or 2 additional heteroatoms independently selected from N, O and S, and the substituted 4-6 membered heterocycloalkyl means that the 4-6 membered heterocycloalkyl is substituted with 1, 2, 3 or 4 $R^b$;
each $R^b$ is independently halogen, hydroxy or $C_{1-4}$ alkyl;
$R^5$ is hydrogen or $C_{1-6}$ alkyl (e.g., methyl);
each $R^6$ is independently hydrogen, cyclopropyl or $C_{1-6}$ alkyl (e.g., methyl, ethyl, *n*-propyl or isopropyl, preferably isopropyl);
each $R^7$ is independently hydrogen, $C_{1-6}$ alkyl, phenyl, 5-6 membered heteroaryl (e.g., pyrazolyl, such as

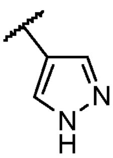

), $-OR^{7a}$ or $-NR^{7b}R^{7c}$ (e.g.,

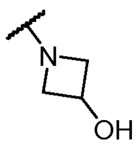, 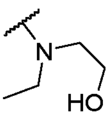, 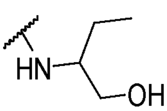

or

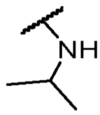,

wherein

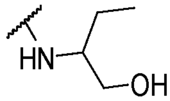

is, e.g.,

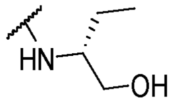

), wherein the number of heteroatoms in the 5-6 membered heteroaryl is 1, 2 or 3, and each heteroatom is independently selected from N, O and S;

$R^{7a}$ is hydrogen or a substituted or unsubstituted $C_{1-6}$ alkyl, wherein the substituted $C_{1-6}$ alkyl means that the $C_{1-6}$ alkyl is substituted with 1, 2, 3 or 4 $R^c$;

each $R^c$ is independently hydroxy, halogen, $C_{1-4}$ alkyl or $C_{1-4}$ alkoxy;

$R^{7b}$ and $R^{7c}$ are each independently hydrogen or a substituted or unsubstituted $C_{1-4}$ alkyl (the substituted or unsubstituted $C_{1-4}$ alkyl is, e.g., ethyl, isopropyl,

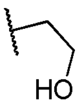 or 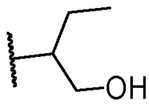,

wherein

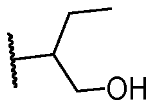

is, e.g.,

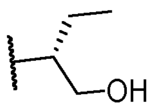

), wherein the substituted $C_{1-4}$ alkyl means that the $C_{1-4}$ alkyl is substituted with 1, 2, 3 or 4 $R^d$;

or, $R^{7b}$ and $R^{7c}$, together with a nitrogen atom attached thereto, form a substituted or unsubstituted 4-6 membered heterocycloalkyl (the 4-6 membered heterocycloalkyl is, e.g., azetidinyl), wherein the 4-6 membered heterocycloalkyl has 0, 1 or 2 additional heteroatoms independently selected from N, O and S, and the substituted 4-6 membered heterocycloalkyl means that the 4-6 membered heterocycloalkyl is substituted with 1, 2, 3 or 4 $R^e$;

each $R^d$ is independently hydroxy, halogen, $C_{1-4}$ alkyl or $C_{1-4}$ alkoxy;

each $R^e$ is independently hydroxy, halogen, $C_{1-4}$ alkyl or $C_{1-4}$ alkoxy;

each $R^8$ is independently hydrogen or $C_{1-4}$ alkyl (e.g., methyl);

when a carbon atom marked by * has chirality, the carbon atom is in S configuration, R configuration, or a mixture thereof.

In some embodiments, in the compound of formula I, ring A is

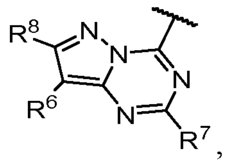

and the other variables are defined as in any of the embodiments of the present invention.

In some embodiments, in the compound of formula I, ring A is

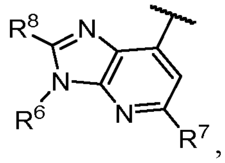

and the other variables are defined as in any of the embodiments of the present invention.

In some embodiments, in the compound of formula I, ring A is

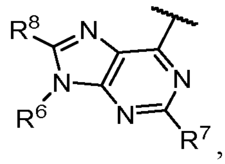

and the other variables are defined as in any of the embodiments of the present invention.

In some embodiments, in the compound of formula I, ring A is

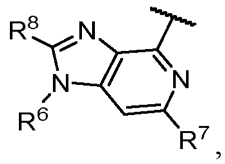

and the other variables are defined as in any of the embodiments of the present invention.

In some embodiments, in the compound of formula I, ring A is

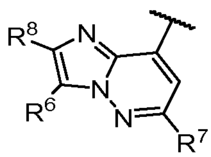

, and the other variables are defined as in any of the embodiments of the present invention.

In some embodiments, in the compound of formula I, ring A is

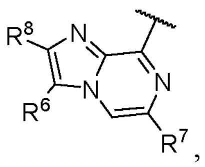

and the other variables are defined as in any of the embodiments of the present invention.

In some embodiments, in the compound of formula I, ring A is

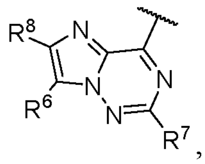

and the other variables are defined as in any of the embodiments of the present invention.

In some embodiments, in the compound of formula I, ring A is preferably

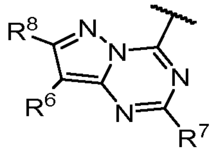 or 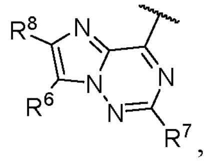

and the other variables are defined as in any of the embodiments of the present invention.

In some embodiments, in the compound of formula I, $R^a$ is hydrogen, and the other variables are defined as in any of the embodiments of the present invention.

In some embodiments, in the compound of formula I described in any of the embodiments above, n is 1, and the other variables are defined as in any of the embodiments of the present invention.

In some embodiments, in the compound of formula I, $R^2$ is hydrogen, and the other variables are defined as in any of the embodiments of the present invention.

In some embodiments, in the compound of formula I, $R^3$ is hydrogen, and the other variables are defined as in any of the embodiments of the present invention.

In some embodiments, in the compound of formula I, $R^4$ is $-CH_2-NR^{4a}R^{4b}$, and the other variables are defined as in any of the embodiments of the present invention.

In some embodiments, in the compound of formula I, $R^{4a}$ is $C_{1-6}$ alkyl, and the other variables are defined as in any of the embodiments of the present invention.

In some embodiments, in the compound of formula I, $R^{4b}$ is $C_{1-6}$ alkyl, and the other variables are defined as in any of the embodiments of the present invention.

In some embodiments, in the compound of formula I, $R^4$ is $-CH_2-N(CH_3)_2$, and the other variables are defined as in any of the embodiments of the present invention.

In some embodiments, in the compound of formula I, $R^5$ is $C_{1-6}$ alkyl, and the other variables are defined as in any of the embodiments of the present invention.

In some embodiments, in the compound of formula I, each $R^{7d}$ is independently hydroxy, $-NR^{a1}R^{a2}$ or $C_{1-4}$ alkoxy, and the other variables are defined as in any of the embodiments of the present invention.

In some embodiments, in the compound of formula I, $R^{7d}$ is hydroxy.

In some embodiments, in the compound of formula I, $R^{a1}$ and $R^{a2}$ are $C_{1-4}$ alkyl, and the other variables are defined as in any of the embodiments of the present invention.

In some embodiments, in the compound of formula I, each $R^c$ is independently hydroxy, $-NR^{c1}R^{c2}$ or $C_{1-4}$ alkoxy, and the other variables are defined as in any of the embodiments of the present invention.

In some embodiments, in the compound of formula I, $R^{c1}$ and $R^{c2}$ are $C_{1-4}$ alkyl, and the other variables are defined as in any of the embodiments of the present invention.

In some embodiments, in the compound of formula I, $R^c$ is hydroxy, and the other variables are defined as in any of the embodiments of the present invention.

In some embodiments, in the compound of formula I, each $R^d$ is independently hydroxy, $-NR^{d1}R^{d2}$ or $C_{1-4}$ alkoxy, and the other variables are defined as in any of the embodiments of the present invention.

In some embodiments, in the compound of formula I, $R^{d1}$ and $R^{d2}$ are $C_{1-4}$ alkyl, and the other variables are defined as in any of the embodiments of the present invention.

In some embodiments, in the compound of formula I, $R^d$ is hydroxy, and the other variables are defined as in any of the embodiments of the present invention.

In some embodiments, in the compound of formula I, each $R^e$ is independently hydroxy, $-NR^{e1}R^{e2}$ or $C_{1-4}$ alkoxy, and the other variables are defined as in any of the embodiments of the present invention.

In some embodiments, in the compound of formula I, $R^{e1}$ and $R^{e2}$ are $C_{1-4}$ alkyl, and the other variables are defined as in any of the embodiments of the present invention.

In some embodiments, in the compound of formula I, $R^e$ is hydroxy, and the other variables are defined as in

any of the embodiments of the present invention.

In some embodiments, in the compound of formula I, each $R^6$ is independently hydrogen or $C_{1-6}$ alkyl, and the other variables are defined as in any of the embodiments of the present invention.

In some embodiments, in the compound of formula I, each $R^7$ is independently hydrogen, a substituted or unsubstituted $C_{1-6}$ alkyl, a substituted or unsubstituted 5-6 membered heteroaryl, $-OR^{7a}$ or $-NR^{7b}R^{7c}$, wherein the substituted $C_{1-6}$ alkyl and the substituted 5-6 membered heteroaryl mean that the $C_{1-6}$ alkyl and the 5-6 membered heteroaryl are each independently substituted with 1, 2, 3 or 4 $R^{7d}$ (e.g., substituted with 1 $R^{7d}$), and the other variables are defined as in any of the embodiments of the present invention.

In some embodiments, in the compound of formula I, $R^{7a}$ is hydrogen, a substituted or unsubstituted $C_{1-6}$ alkyl, or a substituted or unsubstituted $C_{3-6}$ cycloalkyl, wherein the substituted $C_{1-6}$ alkyl and the substituted $C_{3-6}$ cycloalkyl mean that the $C_{1-6}$ alkyl and the $C_{3-6}$ cycloalkyl are each independently substituted with 1, 2, 3 or 4 $R^c$ (e.g., substituted with 1 $R^c$), and the other variables are defined as in any of the embodiments of the present invention.

In some embodiments, in the compound of formula I, $R^{7b}$ and $R^{7c}$ are each independently hydrogen, a substituted or unsubstituted $C_{1-4}$ alkyl, a substituted or unsubstituted $C_{3-6}$ cycloalkyl, or a substituted or unsubstituted 5-6 membered heteroaryl, wherein the substituted $C_{1-4}$ alkyl, the substituted $C_{3-6}$ cycloalkyl and the substituted 5-6 membered heteroaryl mean that the $C_{1-4}$ alkyl, the $C_{3-6}$ cycloalkyl and the 5-6 membered heteroaryl are each independently substituted with 1, 2, 3 or 4 $R^d$ (e.g., substituted with 1 $R^d$), and the other variables are as defined in any embodiment of the present invention.

In some embodiments, in the compound of formula I, one of $R^{7b}$ and $R^{7c}$ is hydrogen, and the other variables are as defined in any embodiment of the present invention.

In some embodiments, in the compound of formula I, each $R^7$ is independently hydrogen, $C_{1-6}$ alkyl, $C_{3-6}$ cycloalkyl, 5-6 membered heteroaryl or $-NR^{7b}R^{7c}$, and the other variables are as defined in any embodiment of the present invention.

In some embodiments, in the compound of formula I in which ring A is

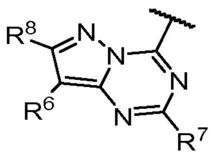
,

$R^6$, $R^7$ and $R^8$ are defined as follows:

$R^6$ is $C_{1-6}$ alkyl (e.g., isopropyl);
$R^7$ is a substituted or unsubstituted $C_{1-6}$ alkyl (the $C_{1-6}$ alkyl is, e.g., methyl; further, the substituted or unsubstituted $C_{1-6}$ alkyl is, e.g., methyl), $-OR^{7a}$ (e.g.,

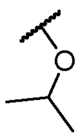 or 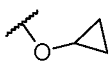),

or $-NR^{7b}R^{7c}$ (e.g.,

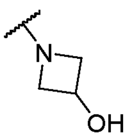, 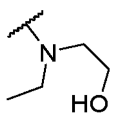, 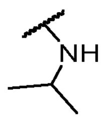 or 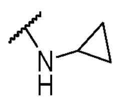

), wherein the substituted $C_{1-6}$ alkyl means that the $C_{1-6}$ alkyl is substituted with 1, 2, 3 or 4 $R^{7d}$ (e.g., substituted with 1 $R^{7d}$);
$R^{7a}$ is hydrogen, a substituted or unsubstituted $C_{1-6}$ alkyl (the $C_{1-6}$ alkyl is, e.g., isopropyl; further, the substituted or unsubstituted $C_{1-6}$ alkyl is, e.g., isopropyl), or a substituted or unsubstituted $C_{3-6}$ cycloalkyl (the $C_{3-6}$ cycloalkyl is, e.g., cyclopropyl; further, the substituted or unsubstituted $C_{3-6}$ cycloalkyl is, e.g., cyclopropyl), wherein the substituted $C_{1-6}$ alkyl and the substituted $C_{3-6}$ cycloalkyl mean that the $C_{1-6}$ alkyl and the $C_{3-6}$ cycloalkyl are each

independently substituted with 1, 2. 3 or 4 $R^c$ (e.g., substituted with 1 $R^c$);
$R^{7b}$ and $R^{7c}$ are each independently hydrogen, a substituted or unsubstituted $C_{1-4}$ alkyl (the $C_{1-4}$ alkyl is, e.g., ethyl or isopropyl; further, the substituted or unsubstituted $C_{1-4}$ alkyl is, e.g., ethyl, isopropyl or

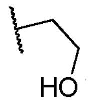

), or a substituted or unsubstituted $C_{3-6}$ cycloalkyl (the $C_{3-6}$ cycloalkyl is, e.g., cyclopropyl; further, the substituted or unsubstituted $C_{3-6}$ cycloalkyl is, e.g., cyclopropyl), wherein the substituted $C_{1-4}$ alkyl and the substituted $C_{3-6}$ cycloalkyl mean that the $C_{1-4}$ alkyl and the $C_{3-6}$ cycloalkyl are substituted with 1, 2, 3 or 4 $R^d$ (e.g., substituted with 1 $R^d$);
or, $R^{7b}$ and $R^{7c}$, together with a nitrogen atom attached thereto, form a substituted or unsubstituted 4-6 membered heterocycloalkyl (the 4-6 membered heterocycloalkyl is, e.g., azetidinyl; further, the substituted or unsubstituted 4-6 membered heterocycloalkyl is, e.g.,

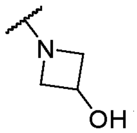

), wherein the 4-6 membered heterocycloalkyl has 0, 1 or 2 additional heteroatoms independently selected from N, O and S, and the substituted 4-6 membered heterocycloalkyl means that the 4-6 membered heterocycloalkyl is substituted with 1, 2, 3 or 4 $R^e$ (e.g., substituted with 1 $R^e$);
$R^8$ is hydrogen;
the other variables are defined as in any of the embodiments of the present invention.
In some embodiments, in the compound of formula I in which ring A is

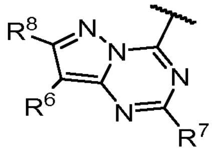,

$R^6$, $R^7$ and $R^8$ are defined as follows:
$R^6$ is $C_{1-6}$ alkyl (e.g., isopropyl);
$R^7$ is $C_{1-6}$ alkyl (e.g., methyl) or $-NR^{7b}R^{7c}$ (e.g.,

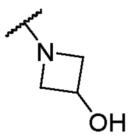 or 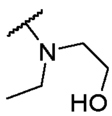);

$R^{7b}$ and $R^{7c}$ are each independently hydrogen or a substituted or unsubstituted $C_{1-4}$ alkyl, wherein the substituted $C_{1-4}$ alkyl means that the $C_{1-4}$ alkyl is substituted with 1, 2, 3 or 4 $R^d$;
or, $R^{7b}$ and $R^{7c}$, together with a nitrogen atom attached thereto, form a substituted or unsubstituted 4-6 membered heterocycloalkyl, wherein the 4-6 membered heterocycloalkyl has 0, 1 or 2 additional heteroatoms independently selected from N, O and S, and the substituted 4-6 membered heterocycloalkyl means that the 4-6 membered heterocycloalkyl is substituted with 1, 2, 3 or 4 $R^e$;
$R^8$ is hydrogen;
the other variables are defined as in any of the embodiments of the present invention.

In some embodiments, in the compound of formula I in which ring A is

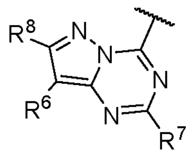

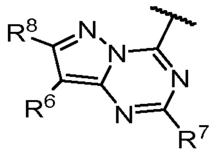

may be

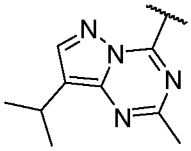 , 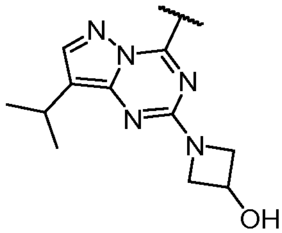 , 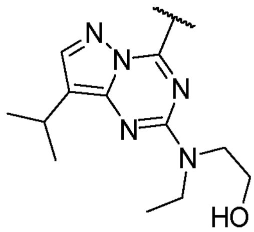 ,

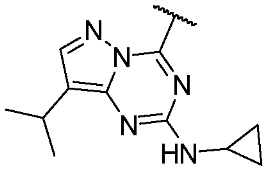 , 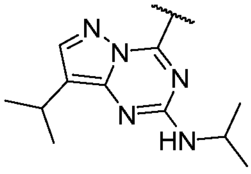 , 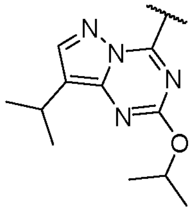 or 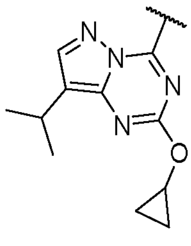 ,

and the other variables are defined as in any of the embodiments of the present invention.

In some embodiments, in the compound of formula I in which ring A is

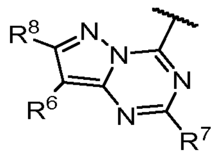

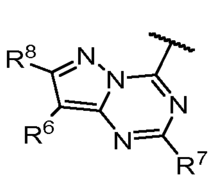 may be 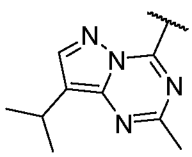 , 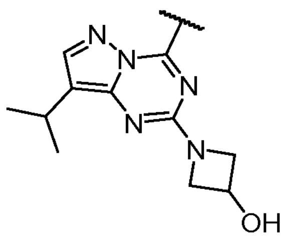 or 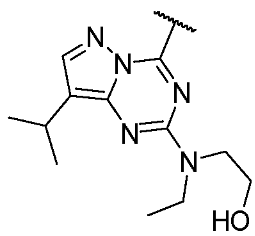 ,

and the other variables are defined as in any of the embodiments of the present invention.

In some embodiments, in the compound of formula I in which ring A is

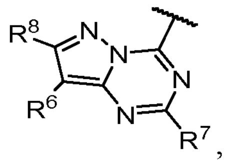

$R^6$, $R^7$ and $R^8$ are defined preferably as follows:

$R^6$ is $C_{1-6}$ alkyl (e.g., isopropyl);
$R^7$ is $C_{1-6}$ alkyl (e.g., methyl);
$R^8$ is hydrogen;
the other variables are defined as in any of the embodiments of the present invention.

In some embodiments, in the compound of formula I in which ring A is

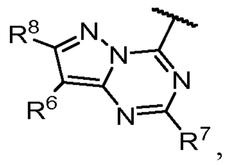

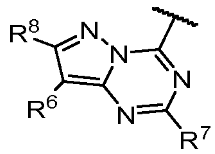

is preferably

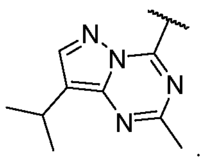

In some embodiments, in the compound of formula I in which ring A is

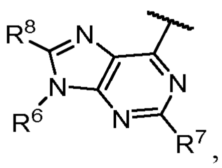

$R^6$, $R^7$ and $R^8$ are defined as follows:

$R^6$ is $C_{1-6}$ alkyl (e.g., ethyl or isopropyl);
$R^7$ is hydrogen, a substituted or unsubstituted $C_{1-6}$ alkyl (the $C_{1-6}$ alkyl is, e.g., methyl or ethyl; further, the substituted or unsubstituted $C_{1-6}$ alkyl is, e.g., methyl, ethyl or $-CH_2OH$),
a substituted or unsubstituted 5-6 membered heteroaryl (the 5-6 membered heteroaryl is, e.g., pyrazolyl, such as

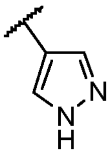 ;

further, the substituted or unsubstituted 5-6 membered heteroaryl is, e.g.,

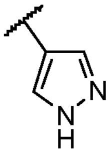

) or $-NR^{7b}R^{7c}$ (e.g.,

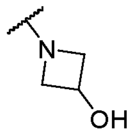, 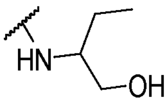, 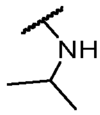 or 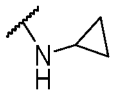,

wherein

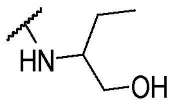

is, e.g.,

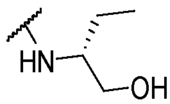

), wherein the substituted $C_{1-6}$ alkyl and the substituted 5-6 membered heteroaryl mean that the $C_{1-6}$ alkyl and the 5-6 membered heteroaryl are each independently substituted with 1, 2, 3 or 4 $R^{7d}$ (e.g., substituted with 1 $R^{7d}$); $R^{7b}$ and $R^{7c}$ are each independently hydrogen, a substituted or unsubstituted $C_{1-4}$ alkyl (the $C_{1-4}$ alkyl is, e.g., isopropyl or *sec*-butyl; further, the substituted or unsubstituted $C_{1-4}$ alkyl is, e.g., isopropyl or

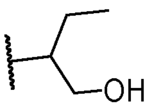,

wherein

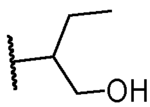

is, e.g.,

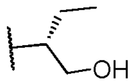

), or a substituted or unsubstituted $C_{3-6}$ cycloalkyl (the $C_{3-6}$ cycloalkyl is, e.g., cyclopropyl; further, the substituted or unsubstituted $C_{3-6}$ cycloalkyl is, e.g., cyclopropyl), wherein the substituted $C_{1-4}$ alkyl and the substituted $C_{3-6}$ cycloalkyl mean that the $C_{1-4}$ alkyl and the $C_{3-6}$ cycloalkyl are each independently substituted with 1, 2, 3 or 4 $R^d$ (e.g., substituted with 1 $R^d$); preferably, one of $R^{7b}$ and $R^{7c}$ is hydrogen;

or, $R^{7b}$ and $R^{7c}$, together with a nitrogen atom attached thereto, form a substituted or unsubstituted 4-6 membered heterocycloalkyl (the 4-6 membered heterocycloalkyl is, e.g., azetidinyl; further, the substituted or unsubstituted 4-6 membered heterocycloalkyl is, e.g.,

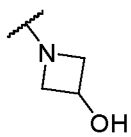

), wherein the 4-6 membered heterocycloalkyl has 0, 1 or 2 additional heteroatoms independently selected from N, O and S, and the substituted 4-6 membered heterocycloalkyl means that the 4-6 membered heterocycloalkyl is substituted with 1, 2, 3 or 4 $R^e$ (e.g., substituted with 1 $R^e$);

$R^8$ is hydrogen or $C_{1-4}$ alkyl (e.g., methyl), preferably hydrogen;

the other variables are defined as in any of the embodiments of the present invention.

In some embodiments, in the compound of formula I in which ring A is

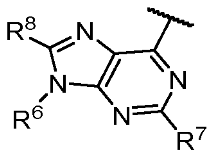
,

$R^6$, $R^7$ and $R^8$ are defined as follows:

$R^6$ is $C_{1-6}$ alkyl (e.g., ethyl or isopropyl);

$R^7$ is hydrogen, $C_{1-6}$ alkyl (e.g., methyl), 5-6 membered heteroaryl (e.g., pyrazolyl, such as

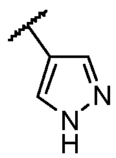
)

or $-NR^{7b}R^{7c}$ (e.g.,

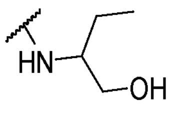
or
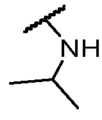
,

wherein

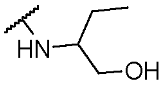

is, e.g.,

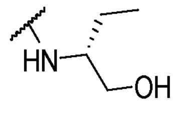);

$R^{7a}$ is hydrogen or a substituted or unsubstituted $C_{1-6}$ alkyl, wherein the substituted $C_{1-6}$ alkyl means that the $C_{1-6}$ alkyl is substituted with 1, 2, 3 or 4 $R^c$;

$R^{7b}$ and $R^{7c}$ are each independently hydrogen or a substituted or unsubstituted $C_{1-4}$ alkyl, wherein the substituted $C_{1-4}$ alkyl means that the $C_{1-4}$ alkyl is substituted with 1, 2, 3 or 4 $R^d$;

or, $R^{7b}$ and $R^{7c}$, together with a nitrogen atom attached thereto, form a substituted or unsubstituted 4-6 membered heterocycloalkyl, wherein the 4-6 membered heterocycloalkyl has 0, 1 or 2 additional heteroatoms independently selected from N, O and S, and the substituted 4-6 membered heterocycloalkyl means that the 4-6 membered heterocycloalkyl is substituted with 1, 2, 3 or 4 $R^e$;

$R^8$ is hydrogen or $C_{1-4}$ alkyl;

the other variables are defined as in any of the embodiments of the present invention.

In some embodiments, in the compound of formula I in which ring A is

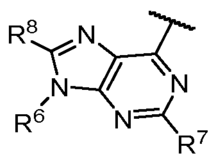,

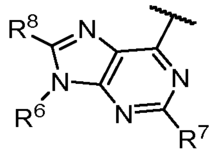

may be

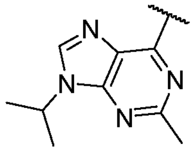, 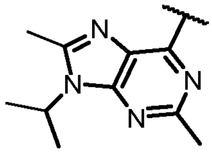, 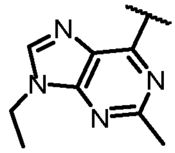, 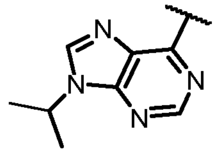,

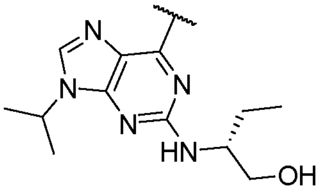, 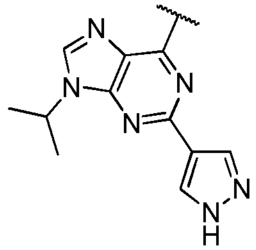, 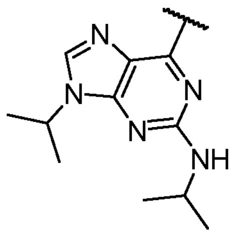, 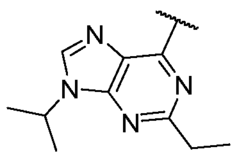,

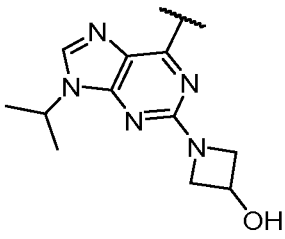, 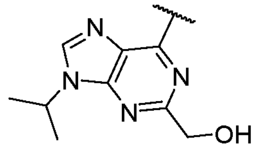 or 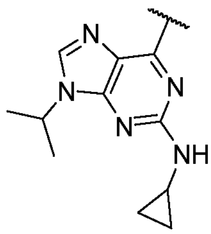,

and the other variables are defined as in any of the embodiments of the present invention.

In some embodiments, in the compound of formula I in which ring A is

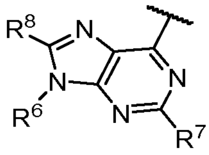,

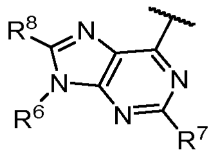

may be

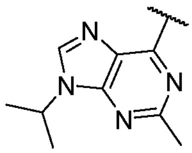, 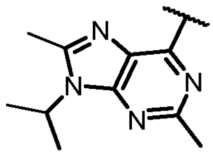, 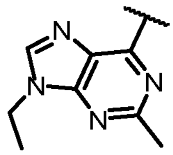, 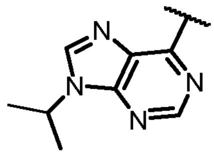,

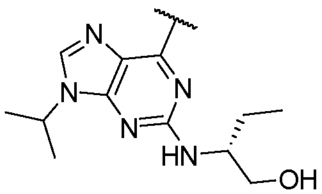, 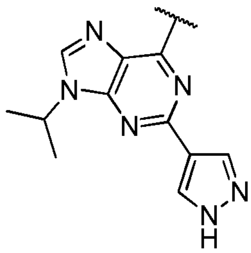 or 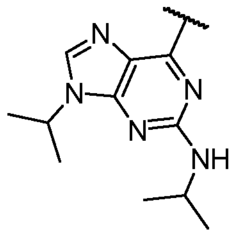,

and the other variables are defined as in any of the embodiments of the present invention.

In some embodiments, in the compound of formula I in which ring A is

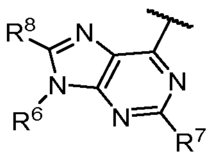,

$R^6$, $R^7$ and $R^8$ are defined preferably as follows:

$R^6$ is $C_{1-6}$ alkyl (e.g., isopropyl);
$R^7$ is $C_{1-6}$ alkyl (e.g., methyl);

$R^8$ is hydrogen;
the other variables are defined as in any of the embodiments of the present invention.

In some embodiments, in the compound of formula I in which ring A is

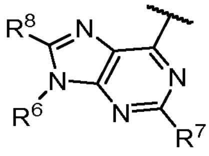

,

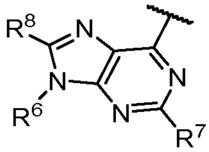

is preferably

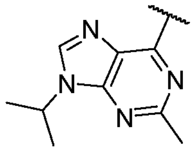

.

In some embodiments, in the compound of formula I in which ring A is

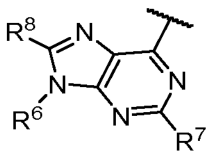

,

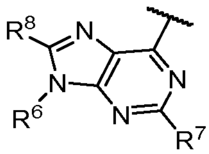

is preferably

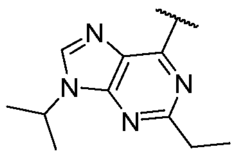

.

In some embodiments, in the compound of formula I in which ring A is

; 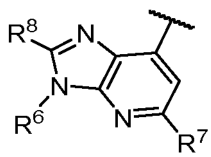

,

$R^6$, $R^7$ and $R^8$ are defined as follows:

$R^6$ is $C_{1-6}$ alkyl (e.g., ethyl or isopropyl), preferably isopropyl;

$R^7$ is hydrogen, a substituted or unsubstituted $C_{1-6}$ alkyl (e.g., the $C_{1-6}$ alkyl is, e.g., methyl or isopropyl; further, the substituted or unsubstituted $C_{1-6}$ alkyl is, e.g., methyl or isopropyl) or $-NR^{7b}R^{7c}$ (e.g.,

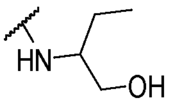, 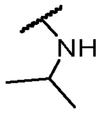, 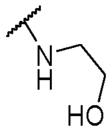, 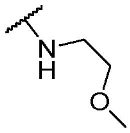, 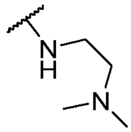 or 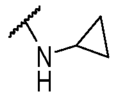,

wherein

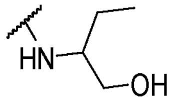

is, e.g.,

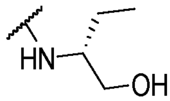

), wherein the substituted $C_{1-6}$ alkyl means that the $C_{1-6}$ alkyl is substituted with 1, 2, 3 or 4 $R^{7d}$ (e.g., substituted with 1 $R^{7d}$);

$R^{7b}$ and $R^{7c}$ are each independently hydrogen, a substituted or unsubstituted $C_{1-4}$ alkyl (the $C_{1-4}$ alkyl is, e.g., ethyl, isopropyl or *sec*-butyl; further, the substituted or unsubstituted $C_{1-4}$ alkyl is, e.g., ethyl, isopropyl,

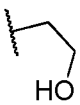, 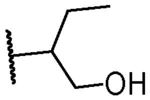, 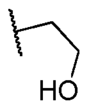, 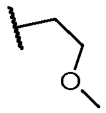 or 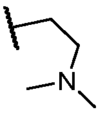,

wherein

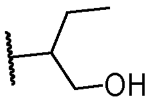

is, e.g.,

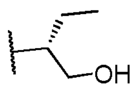

), or a substituted or unsubstituted $C_{3-6}$ cycloalkyl (the $C_{3-6}$ cycloalkyl is, e.g., cyclopropyl; further, the substituted or unsubstituted $C_{3-6}$ cycloalkyl is, e.g., cyclopropyl), wherein the substituted $C_{1-4}$ alkyl and the substituted $C_{3-6}$ cycloalkyl mean that the $C_{1-4}$ alkyl and the $C_{3-6}$ cycloalkyl are each independently substituted with 1, 2, 3 or 4 $R^d$ (e.g., substituted with 1 $R^d$); preferably, one of $R^{7b}$ and $R^{7c}$ is hydrogen;

$R^8$ is hydrogen or $C_{1-4}$ alkyl (e.g., methyl), preferably hydrogen;

the other variables are defined as in any of the embodiments of the present invention.

In some embodiments, in the compound of formula I in which ring A is

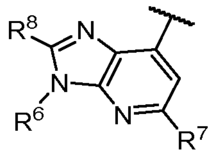,

$R^6$, $R^7$ and $R^8$ are defined as follows:

$R^6$ is $C_{1-6}$ alkyl (e.g., ethyl or isopropyl);
$R^7$ is hydrogen or $C_{1-6}$ alkyl (e.g., methyl);
$R^8$ is hydrogen or $C_{1-4}$ alkyl (e.g., methyl);
the other variables are defined as in any of the embodiments of the present invention.

In some embodiments, in the compound of formula I in which ring A is

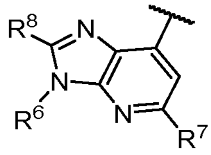,

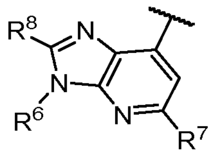

may be

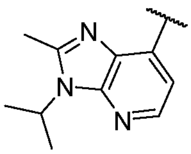, 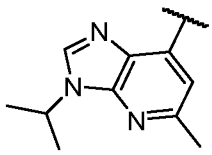, 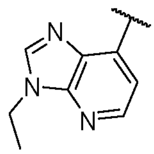, 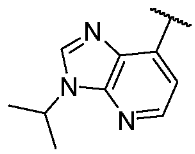,

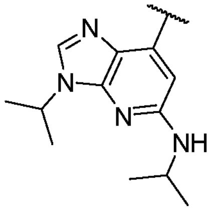, 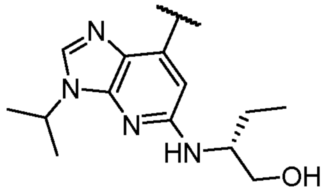, 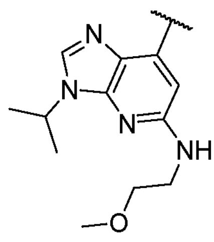, 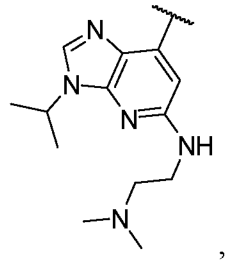,

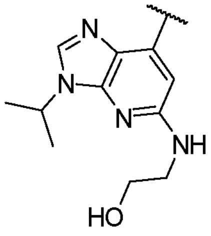, 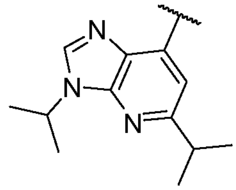 or 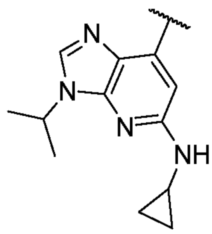.

In some embodiments, in the compound of formula I in which ring A is

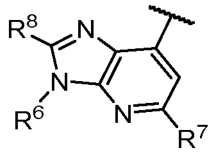,

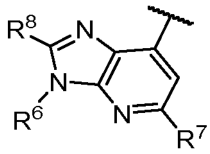

may be

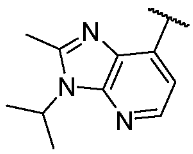, 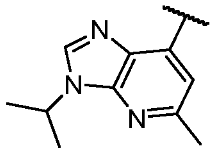, 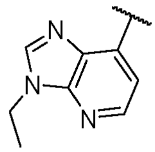 or 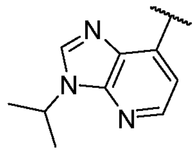,

and the other variables are defined as in any of the embodiments of the present invention.

In some embodiments, in the compound of formula I in which ring A is

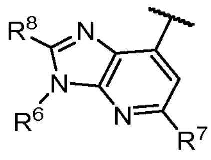,

$R^6$, $R^7$ and $R^8$ are defined preferably as follows:

$R^6$ is $C_{1-6}$ alkyl;
$R^7$ is $C_{1-6}$ alkyl;
$R^8$ is hydrogen;
the other variables are defined as in any of the embodiments of the present invention.

In some embodiments, in the compound of formula I in which ring A is

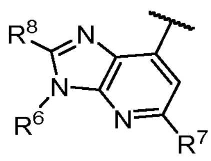,

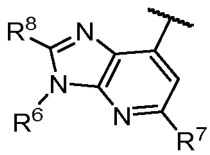

is preferably

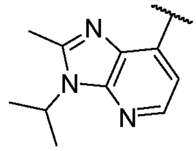
.

In some embodiments, in the compound of formula I in which ring A is

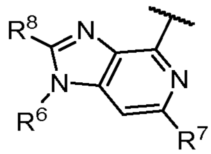
,

$R^6$, $R^7$ and $R^8$ are defined as follows:

$R^6$ is $C_{1-6}$ alkyl (e.g., isopropyl);
$R^7$ is $C_{1-6}$ alkyl (e.g., methyl);
$R^8$ is hydrogen;
the other variables are defined as in any of the embodiments of the present invention.

In some embodiments, in the compound of formula I in which ring A is

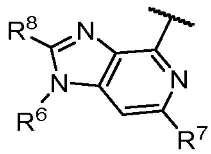
,

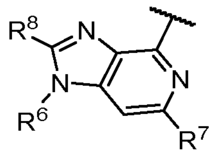

may be

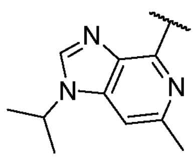
,

and the other variables are defined as in any of the embodiments of the present invention.

In some embodiments, in the compound of formula I in which ring A is

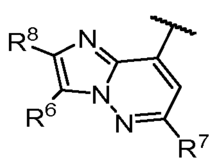
,

$R^6$, $R^7$ and $R^8$ are defined as follows:

$R^6$ is $C_{1-6}$ alkyl (e.g., isopropyl);
$R^7$ is a substituted or unsubstituted $C_{1-6}$ alkyl (the $C_{1-6}$ alkyl is, e.g., methyl; further, the substituted or unsubstituted $C_{1-6}$ alkyl is, e.g., methyl) or $-NR^{7b}R^{7c}$ (e.g.,

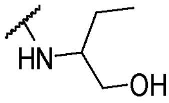,

such as

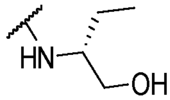

), wherein the substituted $C_{1-6}$ alkyl means that the $C_{1-6}$ alkyl is substituted with 1, 2, 3 or 4 $R^{7d}$ (e.g., substituted with 1 $R^{7d}$);
$R^{7b}$ and $R^{7c}$ are each independently hydrogen or a substituted or unsubstituted $C_{1-4}$ alkyl (the $C_{1-4}$ alkyl is, e.g., isobutyl; further, the substituted or unsubstituted $C_{1-4}$ alkyl is, e.g.,

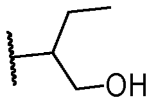,

such as

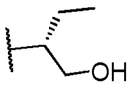

), wherein the substituted $C_{1-4}$ alkyl means that the $C_{1-4}$ alkyl is substituted with 1, 2, 3 or 4 $R^d$ (e.g., substituted with 1 $R^d$); preferably, one of $R^{7b}$ and $R^{7c}$ is hydrogen;
$R^8$ is hydrogen;
the other variables are defined as in any of the embodiments of the present invention.

In some embodiments, in the compound of formula I in which ring A is

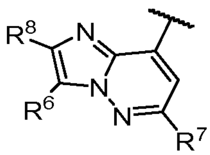,

$R^6$, $R^7$ and $R^8$ are defined as follows:

$R^6$ is $C_{1-6}$ alkyl (e.g., isopropyl);
$R^7$ is $C_{1-6}$ alkyl (e.g., methyl);
$R^8$ is hydrogen;
the other variables are defined as in any of the embodiments of the present invention.

In some embodiments, in the compound of formula I in which ring A is

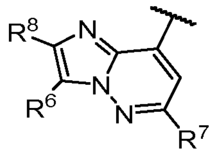
,

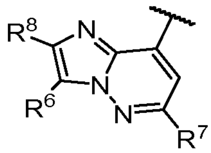

may be

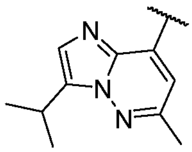
or
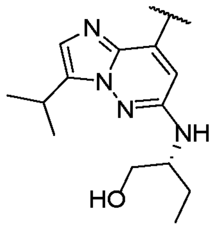
,

and the other variables are defined as in any of the embodiments of the present invention.

In some embodiments, in the compound of formula I in which ring A is

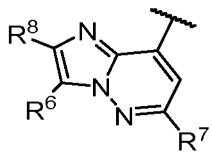
,

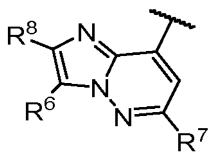

may be

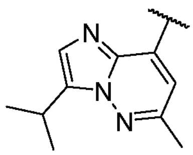
,

and the other variables are defined as in any of the embodiments of the present invention.

In some embodiments, in the compound of formula I in which ring A is

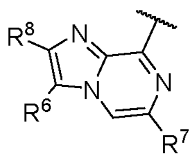
,

$R^6$, $R^7$ and $R^8$ are defined as follows:

$R^6$ is $C_{1-6}$ alkyl (e.g., isopropyl);
$R^7$ is $C_{1-6}$ alkyl (e.g., methyl) or $-NR^{7b}R^{7c}$ (e.g.,

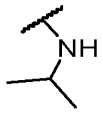 or 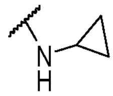 );

$R^{7b}$ and $R^{7c}$ are each independently hydrogen, $C_{1-4}$ alkyl or $C_{3-6}$ cycloalkyl (e.g., the $C_{3-6}$ cycloalkyl is, e.g., cyclopropyl); preferably, one of $R^{7b}$ and $R^{7c}$ is hydrogen;
$R^8$ is hydrogen;
the other variables are defined as in any of the embodiments of the present invention.

In some embodiments, in the compound of formula I in which ring A is

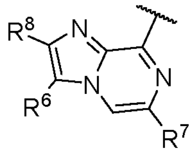,

$R^6$, $R^7$ and $R^8$ are defined as follows:

$R^6$ is $C_{1-6}$ alkyl (e.g., isopropyl);
$R^7$ is $C_{1-6}$ alkyl (e.g., methyl);
$R^8$ is hydrogen;
the other variables are defined as in any of the embodiments of the present invention.

In some embodiments, in the compound of formula I in which ring A is

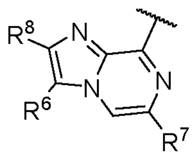,

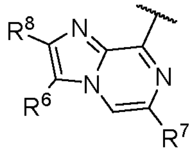

may be

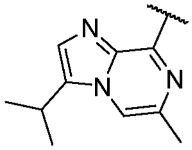, 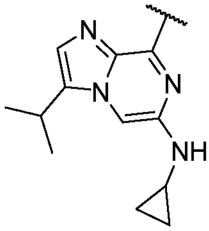, 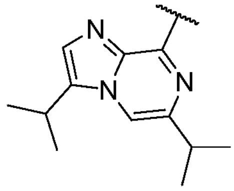

or

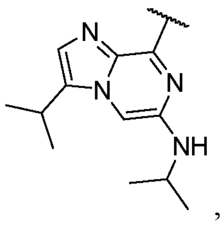

and the other variables are defined as in any of the embodiments of the present invention.

In some embodiments, in the compound of formula I in which ring A is

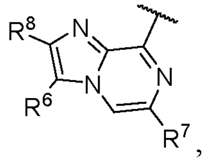

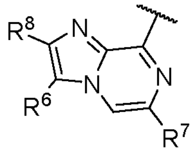

may be

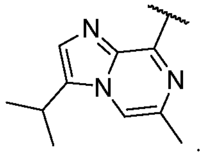

In some embodiments, in the compound of formula I in which ring A is

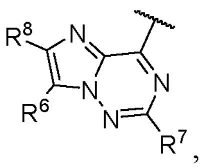

$R^6$, $R^7$ and $R^8$ are defined as follows:

$R^6$ is $C_{1-6}$ alkyl (e.g., isopropyl);

$R^7$ is a substituted or unsubstituted $C_{1-6}$ alkyl (the $C_{1-6}$ alkyl is, e.g., methyl; further, the substituted or unsubstituted $C_{1-6}$ alkyl is, e.g., methyl), $-OR^{7a}$ (e.g.,

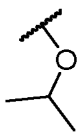

), or $-NR^{7b}R^{7c}$ (e.g.,

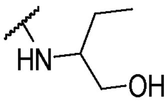, 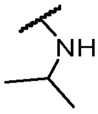, 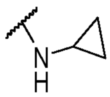, 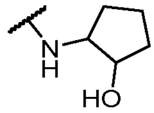, 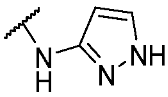 or 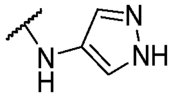,

wherein

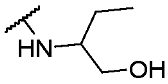

is, e.g.,

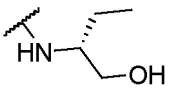

), wherein the substituted $C_{1-6}$ alkyl means that the $C_{1-6}$ alkyl is substituted with 1, 2, 3 or 4 $R^{7d}$ (e.g., substituted with 1 $R^{7d}$);

$R^{7a}$ is hydrogen or a substituted or unsubstituted $C_{1-6}$ alkyl (the $C_{1-6}$ alkyl is, e.g., isopropyl; further, the substituted or unsubstituted $C_{1-6}$ alkyl is, e.g., isopropyl), wherein the substituted $C_{1-6}$ alkyl means that the $C_{1-6}$ alkyl is substituted with 1, 2, 3 or 4 $R^c$ (e.g., substituted with 1 $R^c$);

$R^{7b}$ and $R^{7c}$ are each independently hydrogen, a substituted or unsubstituted $C_{1-4}$ alkyl (the $C_{1-4}$ alkyl is, e.g., isopropyl or *sec*-butyl; further, the substituted or unsubstituted $C_{1-4}$ alkyl is, e.g., isopropyl or

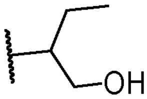,

wherein

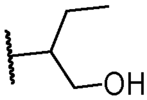

is, e.g.,

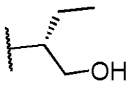

), a substituted or unsubstituted $C_{3-6}$ cycloalkyl (the $C_{3-6}$ cycloalkyl is, e.g., cyclopropyl or cyclopentyl; further, the substituted or unsubstituted $C_{3-6}$ cycloalkyl is, e.g., cyclopropyl or

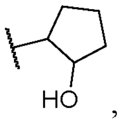,

wherein

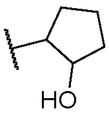

is, e.g.,

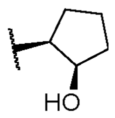

), or a substituted or unsubstituted 5-6 membered heteroaryl (the 5-6 membered heteroaryl is, e.g., pyrazolyl, such as

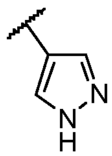

or

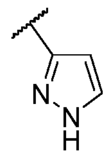

; further, the substituted or unsubstituted 5-6 membered heteroaryl is, e.g.,

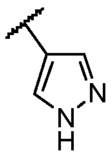

or

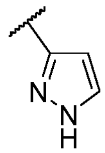

), wherein the substituted $C_{1-4}$ alkyl, the substituted $C_{3-6}$ cycloalkyl and the substituted 5-6 membered heteroaryl mean that the $C_{1-4}$ alkyl, the $C_{3-6}$ cycloalkyl and the 5-6 membered heteroaryl are each independently substituted with 1, 2, 3, or 4 $R^d$ (e.g., substituted with 1 $R^d$); preferably, one of $R^{7b}$ and $R^{7c}$ is hydrogen;
$R^8$ is hydrogen;
the other variables are defined as in any of the embodiments of the present invention.

In some embodiments, in the compound of formula I in which ring A is

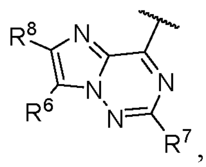

,

$R^6$, $R^7$ and $R^8$ are defined as follows:

$R^6$ is $C_{1-6}$ alkyl (e.g., isopropyl);
$R^7$ is $C_{1-6}$ alkyl (e.g., methyl);
$R^8$ is hydrogen;
the other variables are defined as in any of the embodiments of the present invention.

In some embodiments, in the compound of formula I in which ring A is

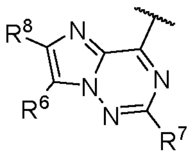,

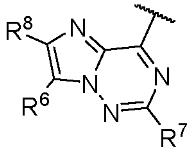

may be

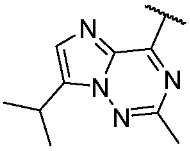, 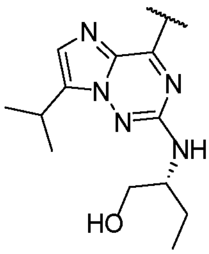, 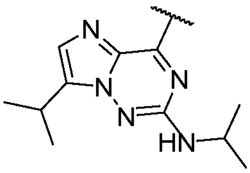,

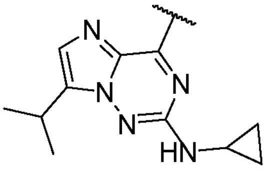, 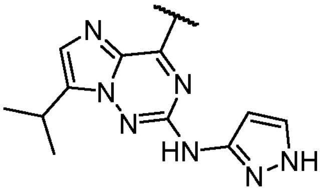, 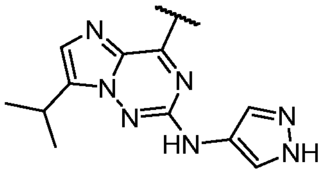,

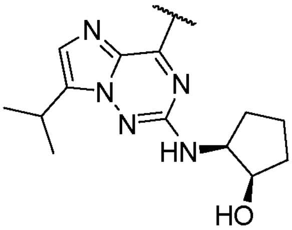, 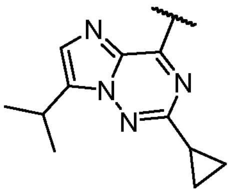, 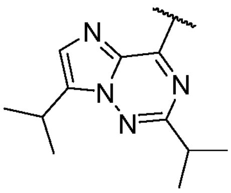 or 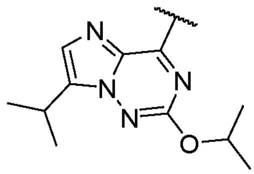;

, and the other variables are defined as in any of the embodiments of the present invention.

In some embodiments, in the compound of formula I in which ring A is

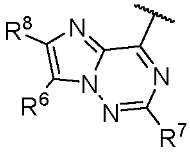,

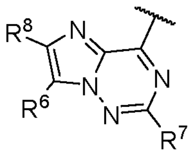

is preferably

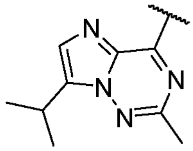

,

and the other variables are defined as in any of the embodiments of the present invention.

In some embodiments, in the compound of formula I, X is O, and the other variables are defined as in any of the embodiments of the present invention.

In some embodiments, in the compound of formula I, m is 2, n is 1, and the other variables are defined as in any of the embodiments of the present invention.

In some embodiments, in the compound of formula I, $R^1$ is

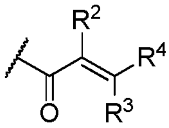

;

, and the other variables are defined as in any of the embodiments of the present invention.

In some embodiments, in the compound of formula I, each $R^6$ is independently $C_{1-6}$ alkyl, preferably isopropyl, and the other variables are defined as in any of the embodiments of the present invention.

In some embodiments, in the compound of formula I, each $R^7$ is independently $C_{1-6}$ alkyl, and the other variables are defined as in any of the embodiments of the present invention.

In some embodiments, in the compound of formula I, $R^8$ is hydrogen, and the other variables are defined as in any of the embodiments of the present invention.

In some embodiments, in the compound of formula I, the groups are defined as follows:

wherein, ring A is

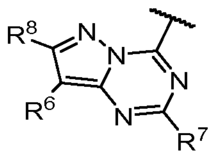
,
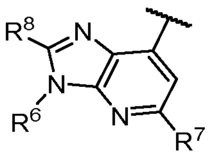
,
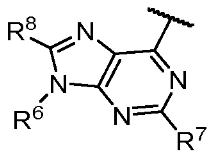
,

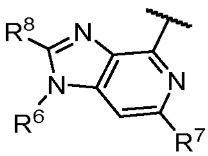
or
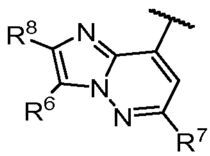
;

X is O;
m is 2;
n is 1;
$R^1$ is

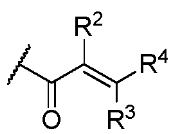

;

$R^2$ is hydrogen;
$R^3$ is hydrogen;
$R^4$ is $-CH_2-NR^{4a}R^{4b}$ (e.g., $-CH_2-N(CH_3)_2$);
$R^{4a}$ and $R^{4b}$ are each independently hydrogen or $C_{1-6}$ alkyl (e.g., methyl);
each $R^6$ is independently $C_{1-6}$ alkyl (e.g., methyl, ethyl, *n*-propyl or isopropyl, preferably isopropyl);
each $R^7$ is independently $C_{1-6}$ alkyl;
$R^8$ is hydrogen;
a carbon atom marked by * is in *S* configuration, *R* configuration, or a mixture thereof.

In some embodiments, the compound of formula I has any of the following structures:

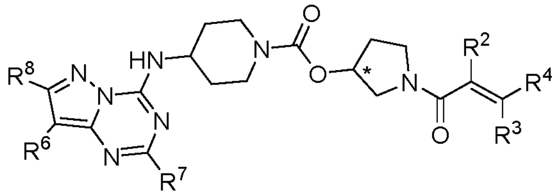
,
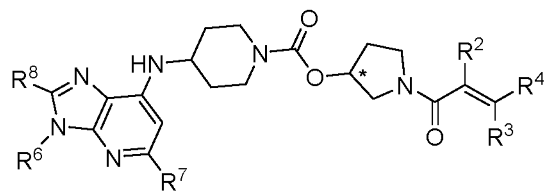
,
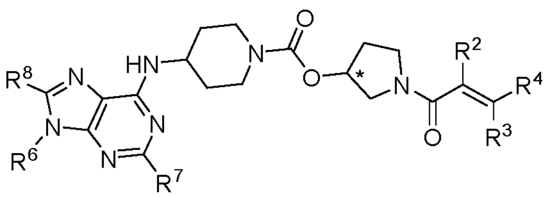
,
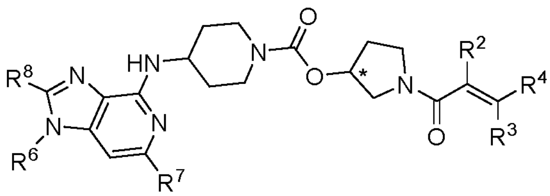
,
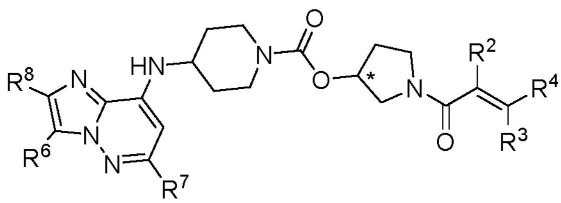
,
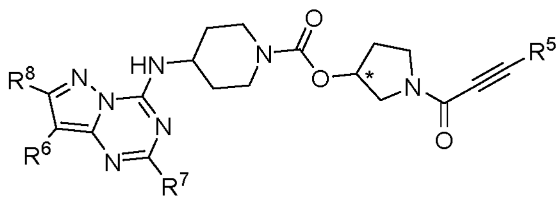
,
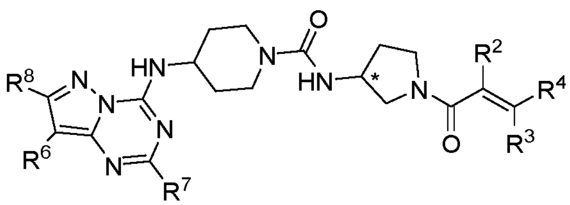
,
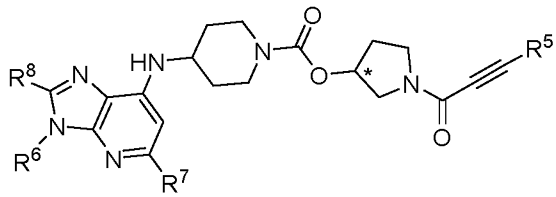
,
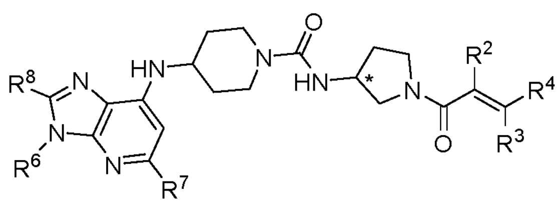
,
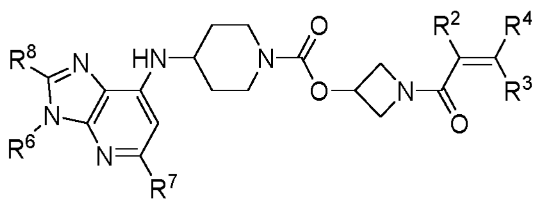
,
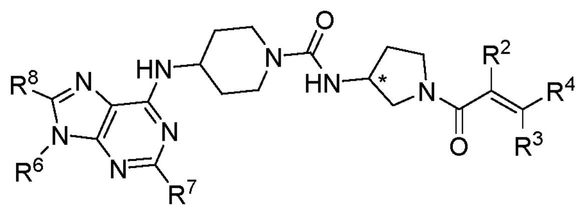
;

wherein, $R^2$, $R^3$, $R^4$, $R^5$, $R^6$, $R^7$, $R^8$ and * are defined as in any of the aforementioned embodiments.

In some embodiments, the compound of formula I has any of the following structures:

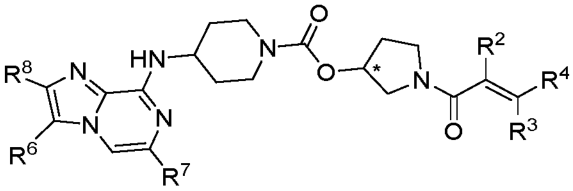
,
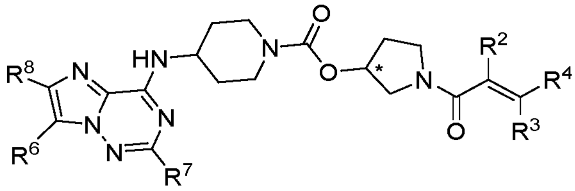
,
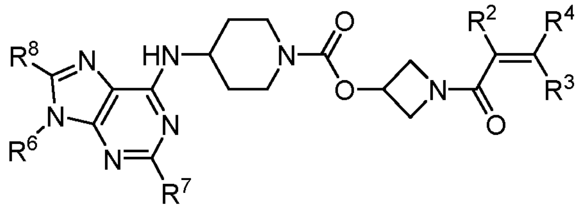
;

wherein, $R^2$, $R^3$, $R^4$, $R^5$, $R^6$, $R^7$, $R^8$ and * are defined as in any of the aforementioned embodiments.

In some embodiments, the compound of formula I has any of the following structures:

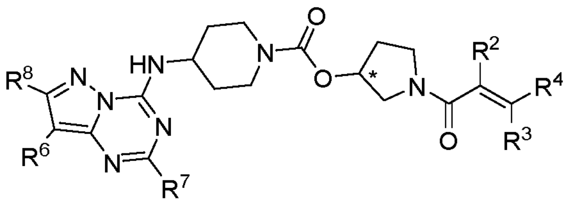
,
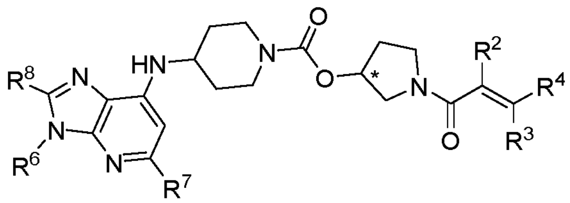
,
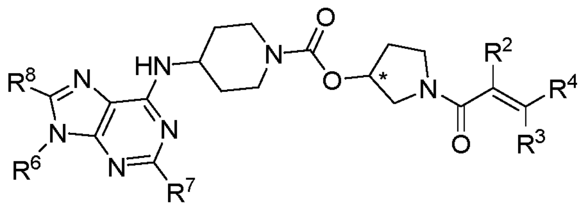
;

wherein, $R^2$, $R^3$, $R^4$, $R^6$, $R^7$, $R^8$ and * are defined as in any of the aforementioned embodiments.

In some embodiments, in the compound of formula I, in consideration of the inhibitory activity against CDK7, ring A is preferably

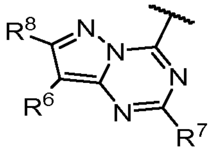
or
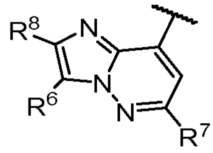
,

and further preferably

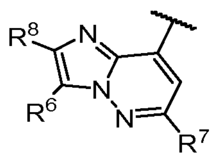
,

and the other variables are defined as in any of the embodiments of the present invention.

In some embodiments, in the compound of formula I, in consideration of the inhibitory activity against HCC70 cells, ring A is preferably

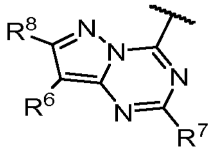 or 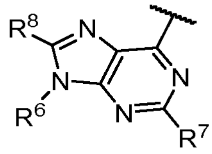,

and further preferably

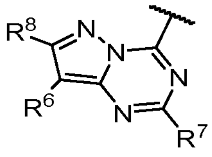,

and the other variables are defined as in any of the embodiments of the present invention.

In some embodiments, in the compound of formula I, in consideration of the inhibitory activity against OVCAR3 cells, ring A is preferably

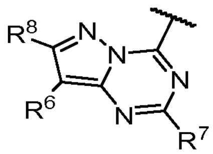

, and the other variables are defined as in any of the embodiments of the present invention.

In some embodiments, in the compound of formula I as described in any of the aforementioned embodiments, a carbon atom marked by * is in S configuration.

In some embodiments, in the compound of formula I as described in any of the aforementioned embodiments, a carbon atom marked by * is in R configuration.

In some embodiments, the compound of formula I has any of the following structures:

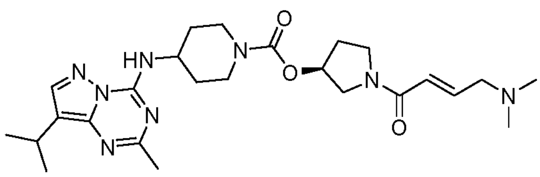, 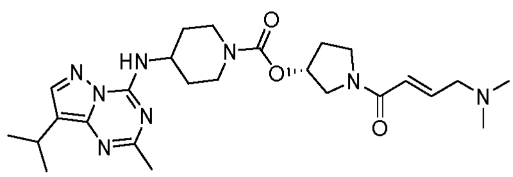,

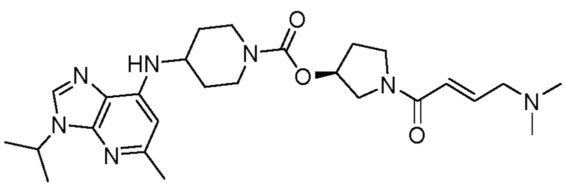, 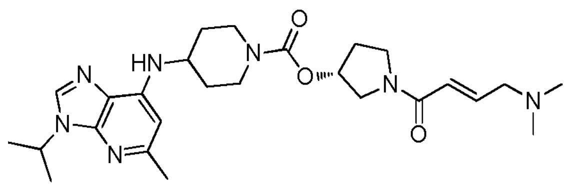,

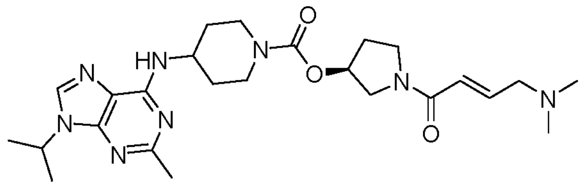,
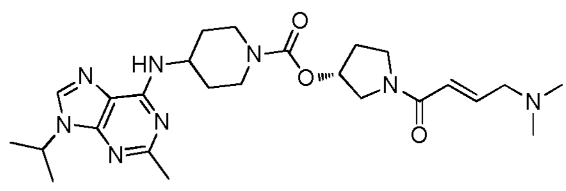,
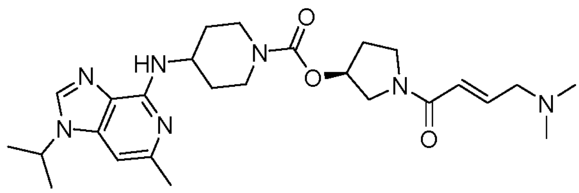,
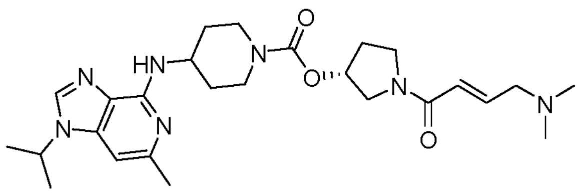,
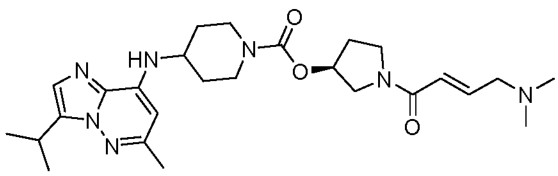,
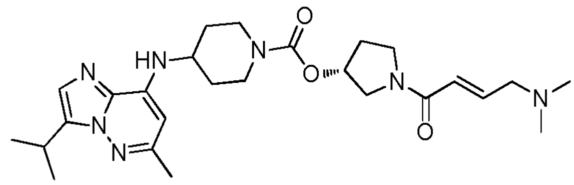,
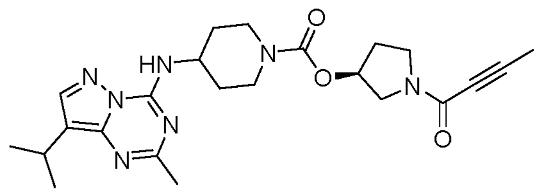,
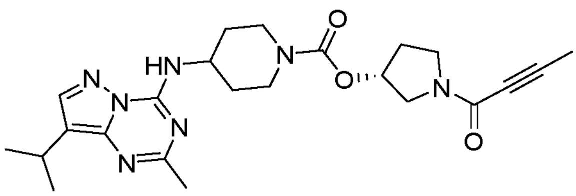,
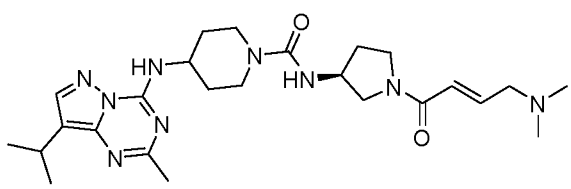,
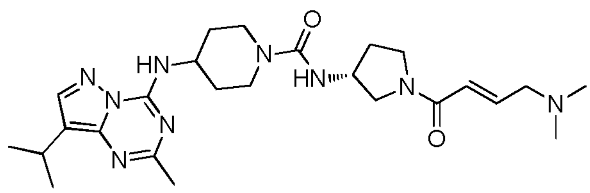,
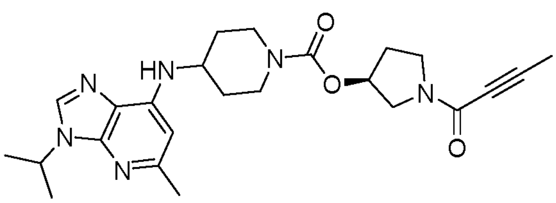,
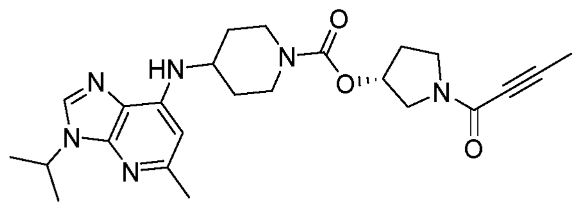,
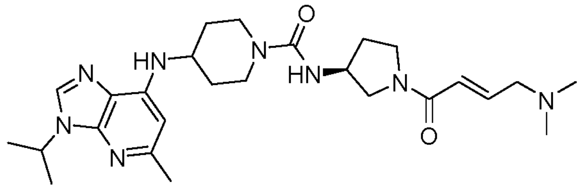,
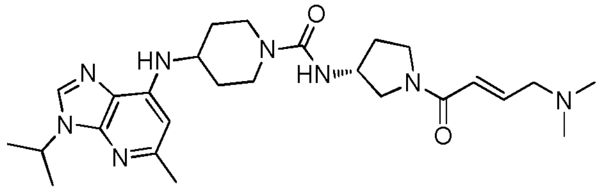,

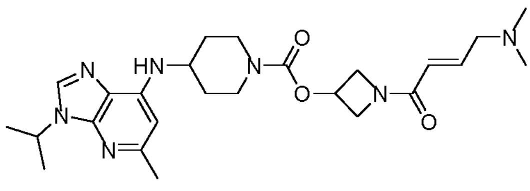

,

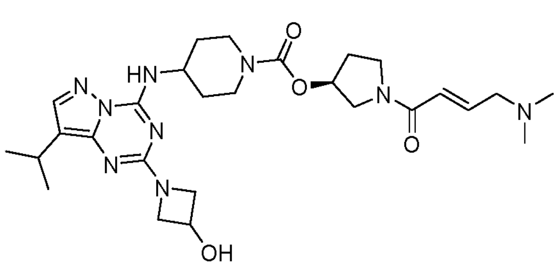

,

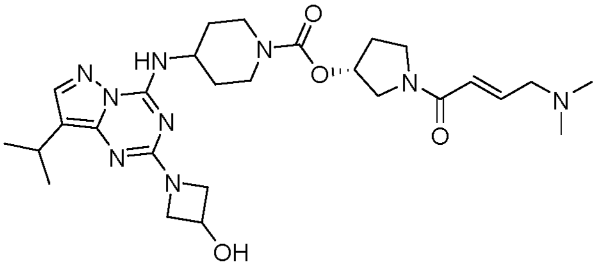

,

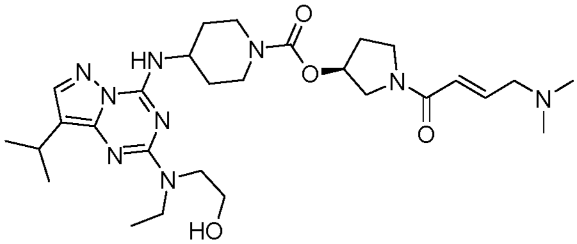

,

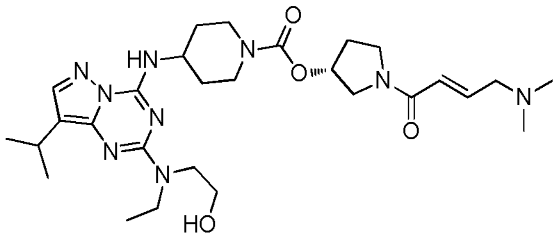

,

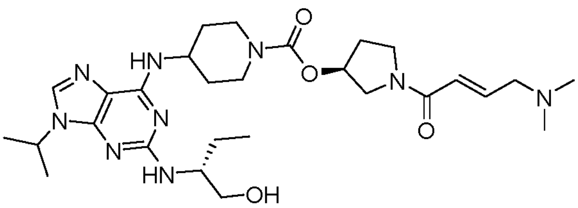

,

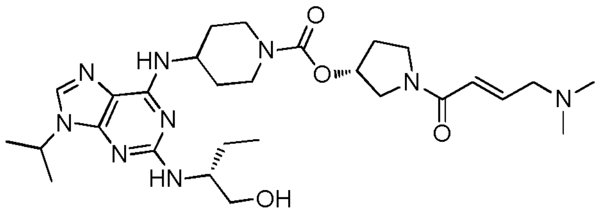

,

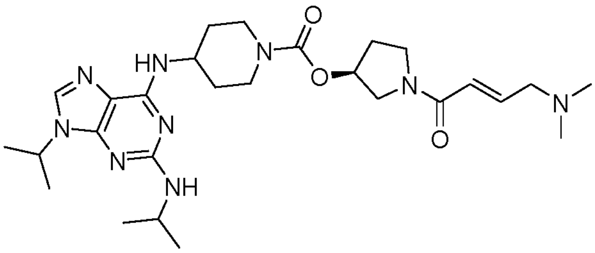

,

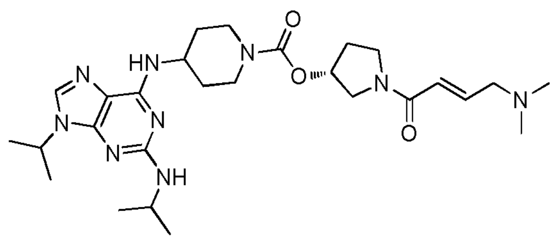

,

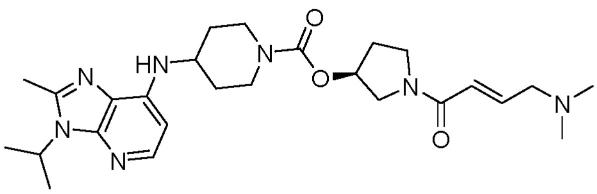

,

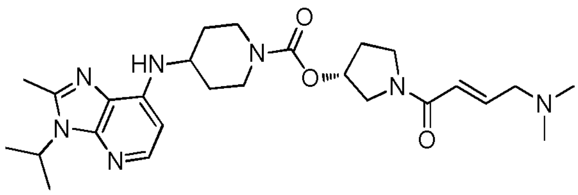

,

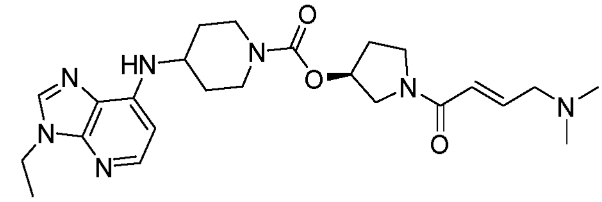
,
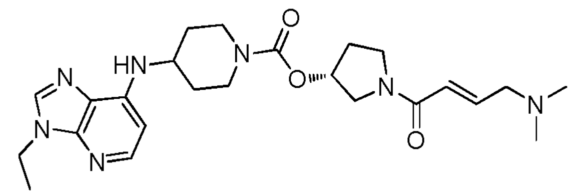
,

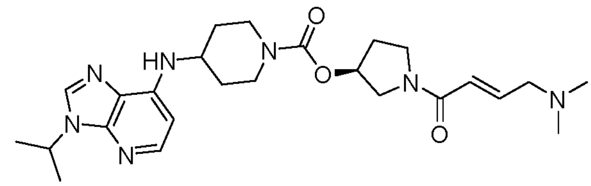
,
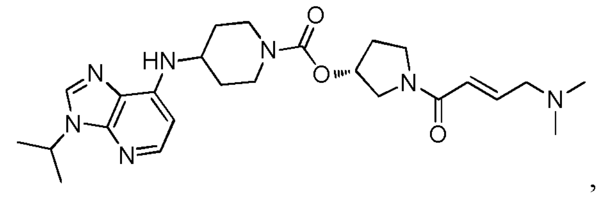
,

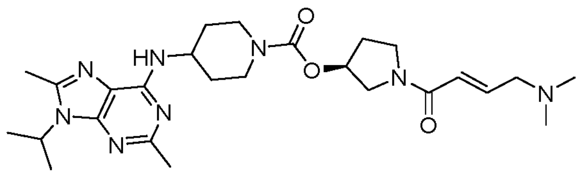
,
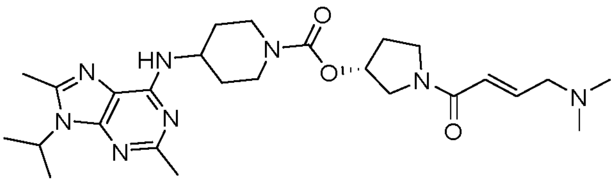
,

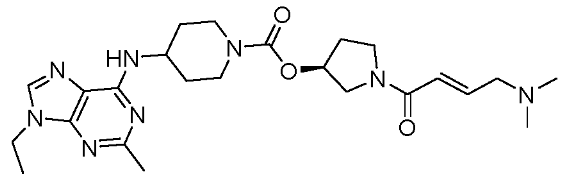
,
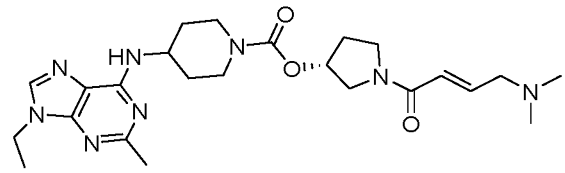
,

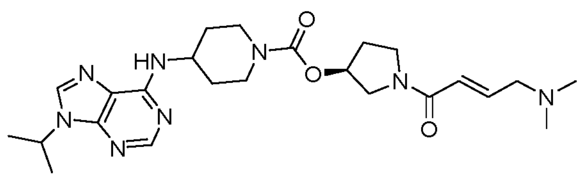
,
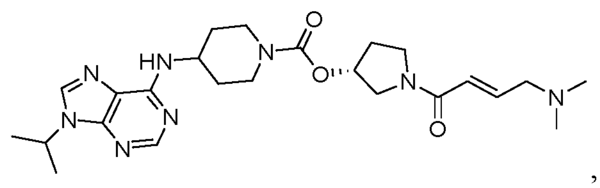
,

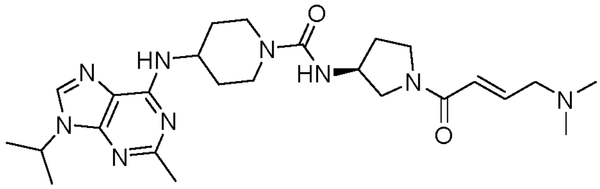
,
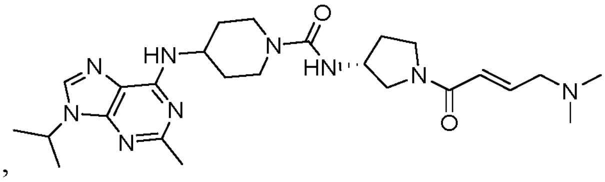
,

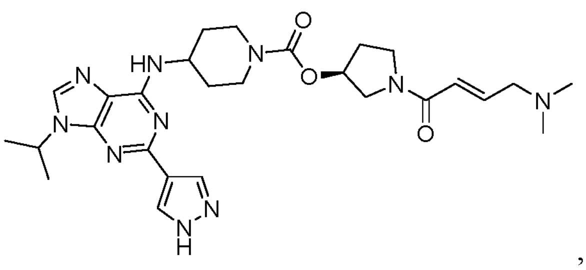
,
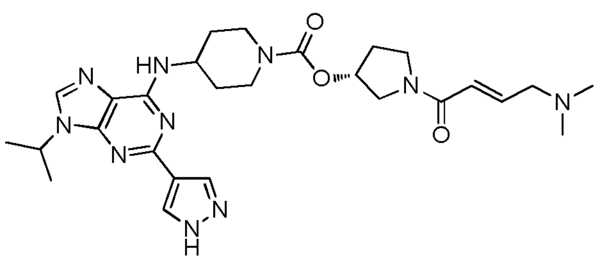
,

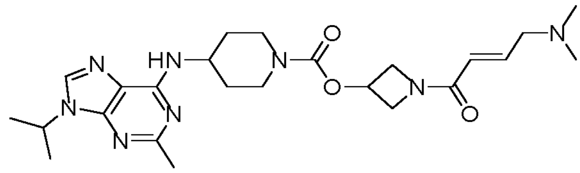
,
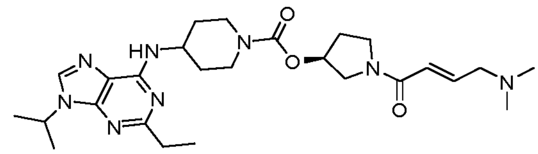
,

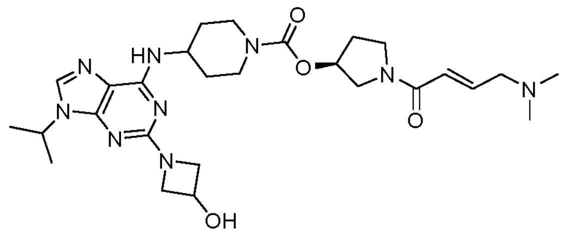
,
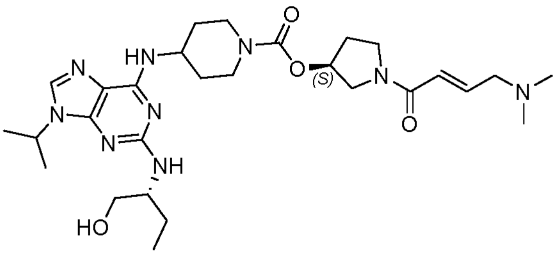
,

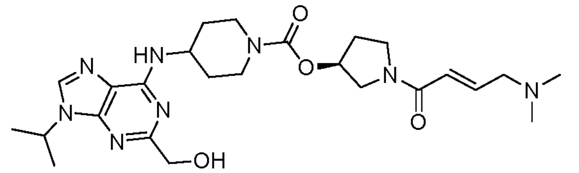
,
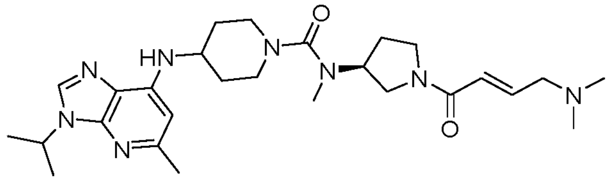
,

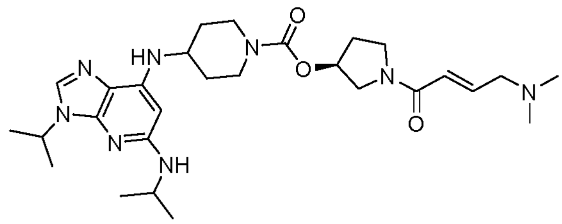
,
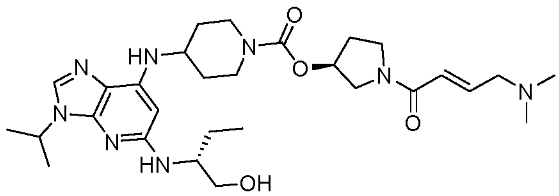
,

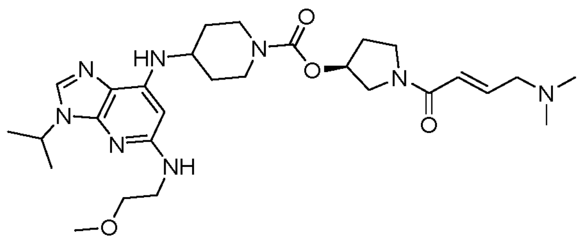
,
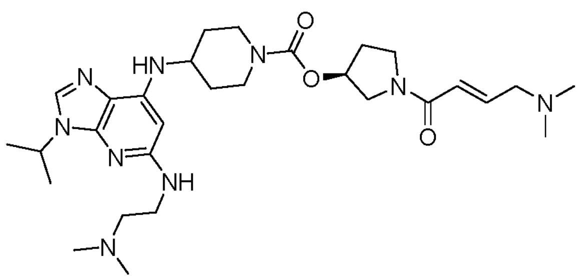
,

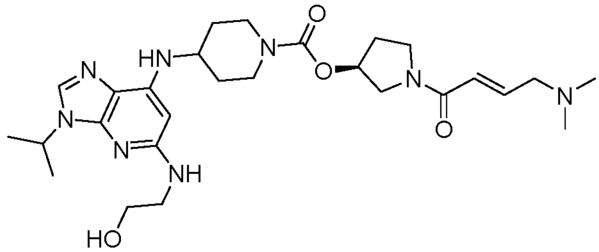
,
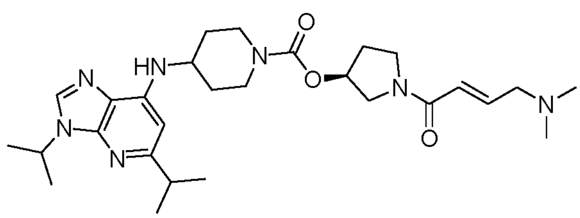
,

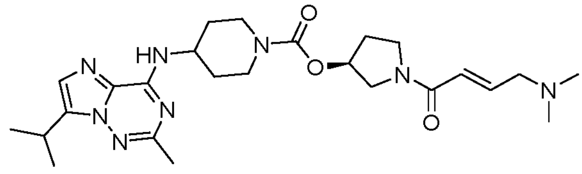,

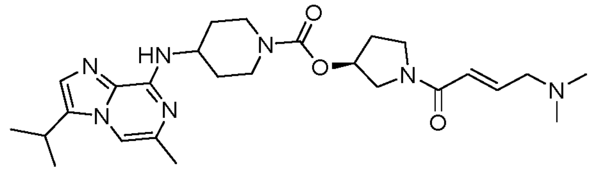,

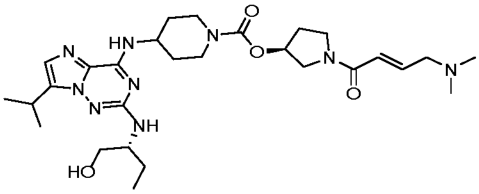,

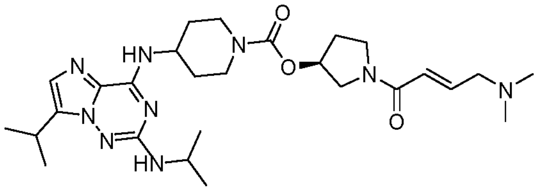,

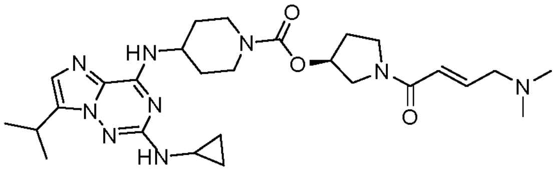,

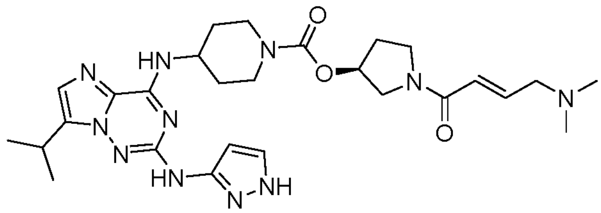,

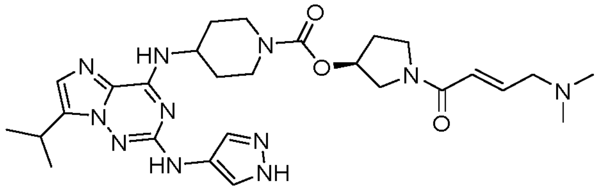,

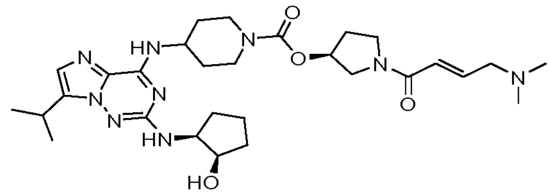,

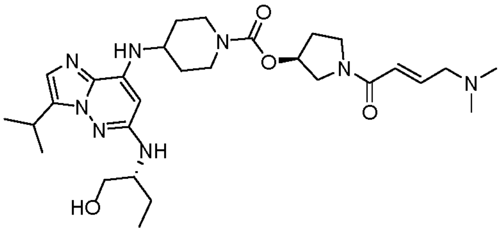,

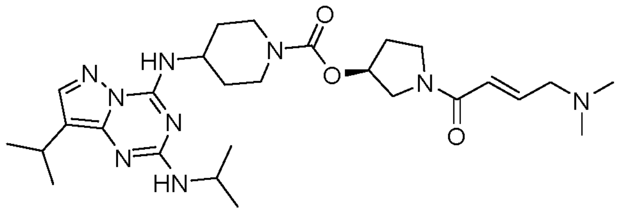,

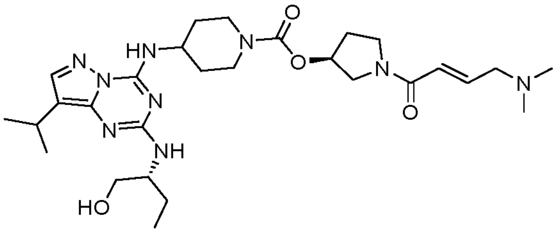,

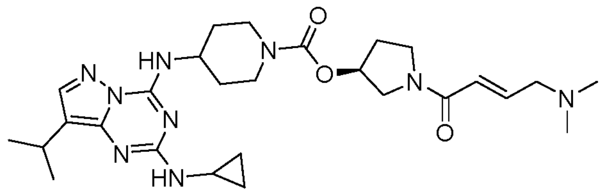,

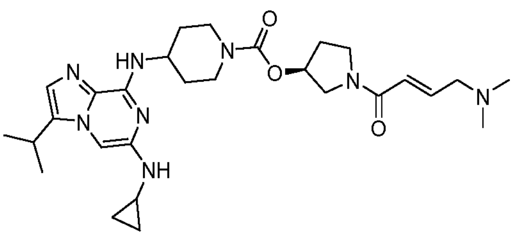,

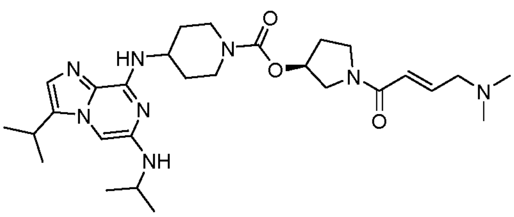,

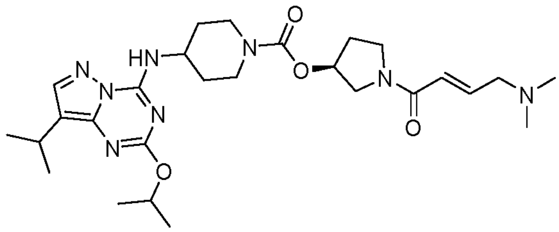, 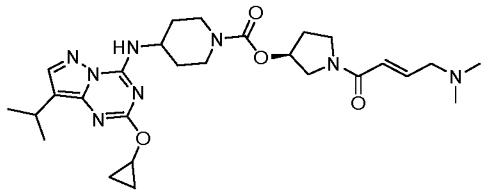,

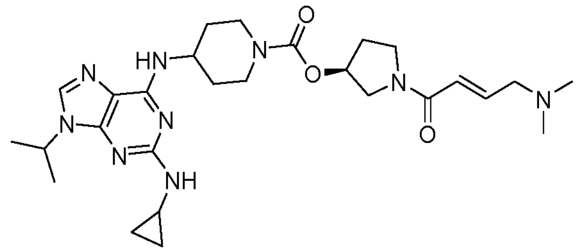, 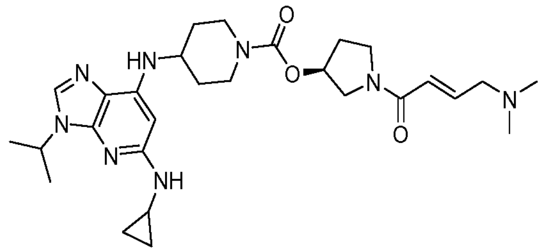,

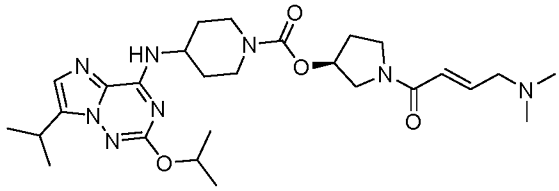, 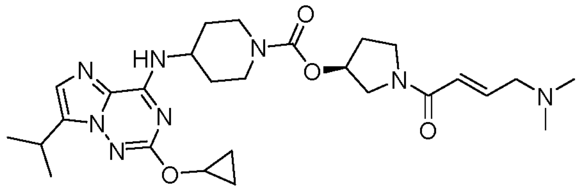,

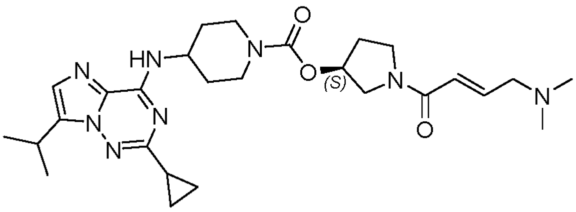, 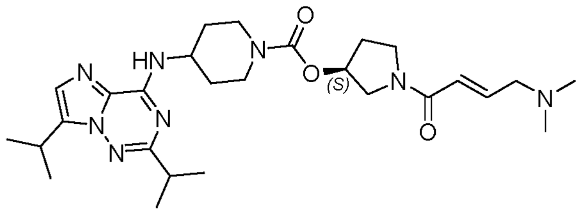,

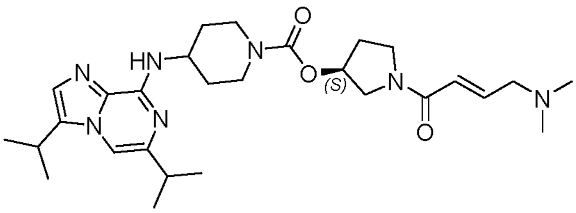.

The present invention also provides a method for preparing the compound of formula I described above, which comprises: subjecting a compound of formula II and

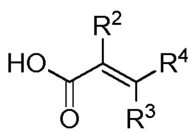

or

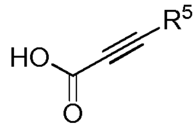

to a condensation reaction shown below in an organic solvent (e.g., DMF and/or THF) in the presence of a condensing agent (e.g., one or more of HATU, PyBOP and T3P) and a base (e.g., TEA and/or DIPEA) to obtain the compound of formula I, wherein ring A, X, m, n, $R^1$, $R^2$, $R^3$, $R^4$, $R^5$ and * are defined as above;

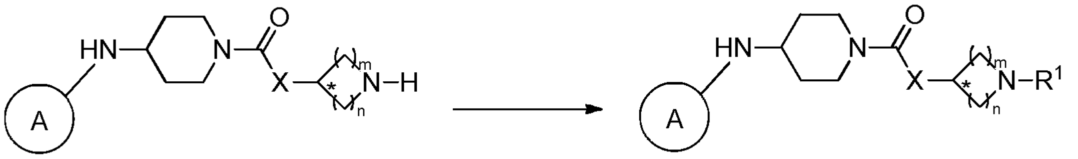

II I

In some embodiments, a method for preparing the compound of formula II may comprise: subjecting a compound of formula III to a Boc deprotection reaction shown below in an organic solvent (e.g., DCM and/or dioxane) in the presence of an acid (e.g., HCl and/or TFA) to obtain the compound of formula II, wherein ring A, X, m, n and * are defined as above;

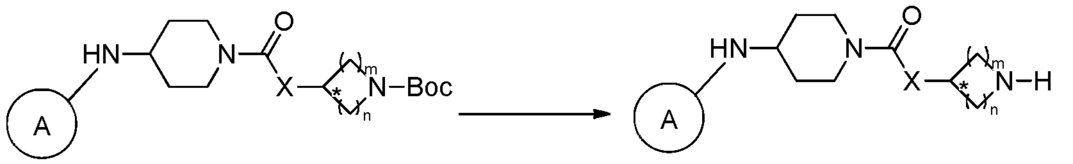

III II

In some embodiments, a method for preparing the compound of formula III may comprise: subjecting a compound of formula IV-1 and a compound of formula IV-2 to a condensation reaction shown below in an organic solvent (e.g., one or more of THF, DCM and $CH_3CN$) in the presence of a base (e.g., TEA and/or DIPEA) to obtain the compound of formula III, wherein LG is a leaving group (e.g., chloro or *p*-nitrophenyl), and ring A, X, m, n and * are defined as above;

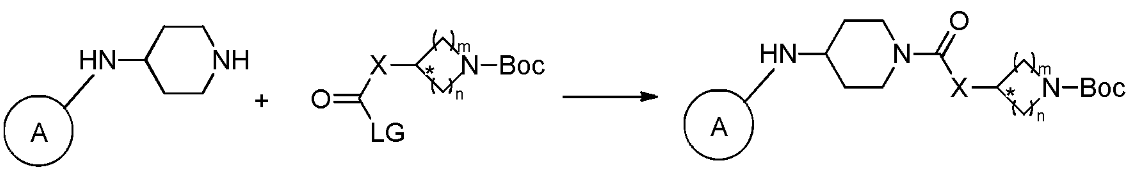

IV-1 IV-2 III

In some embodiments, a method for preparing the compound of formula IV-1 may comprise: subjecting a compound of formula V to a Boc deprotection reaction shown below in an organic solvent (e.g., DCM and/or dioxane) in the presence of an acid (e.g., HCl and/or TFA) to obtain the compound of formula IV-1, wherein ring A is defined as above;

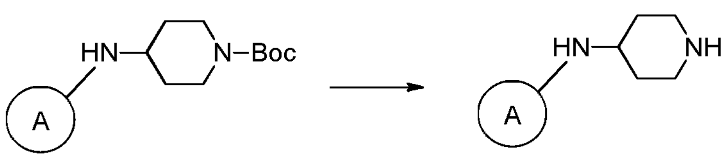

V IV-1

The present invention also provides a compound or a tautomer, a stereoisomer or an isotopic derivative thereof,

or a pharmaceutically acceptable salt of any of the foregoing, or a crystalline form or a solvate of any of the foregoing, wherein the compound is selected from any of the following structures:

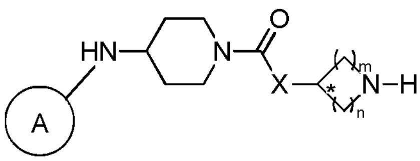

II,

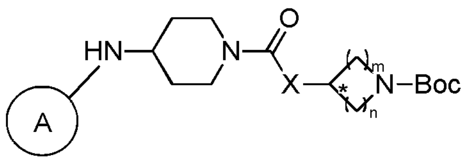

III,

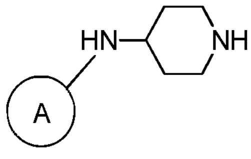

IV-1,

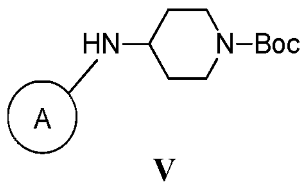

V;

wherein ring A, X, m, n and * are defined as above.

The present invention also provides a pharmaceutical composition, which comprises:

(i) the compound of formula I or the tautomer, the stereoisomer or the isotopic derivative thereof, or the pharmaceutically acceptable salt of any of the foregoing, or the crystalline form or the solvate of any of the foregoing; and
(ii) at least one pharmaceutical adjuvant.

The present invention also provides use of the compound of formula I or the tautomer, the stereoisomer or the isotopic derivative thereof, or the pharmaceutically acceptable salt of any of the foregoing, or the crystalline form or the solvate of any of the foregoing in preparing a CDK7 inhibitor.

The present invention also provides use of the compound of formula I or the tautomer, the stereoisomer or the isotopic derivative thereof, or the pharmaceutically acceptable salt of any of the foregoing, or the crystalline form or the solvate of any of the foregoing in preparing a medicament.

In some embodiments, the medicament is a medicament for preventing and/or treating a CDK7-mediated disease, e.g., a tumor, such as breast cancer, ovarian cancer, small cell lung cancer, acute myeloid leukemia, acute lymphocytic leukemia, bladder cancer, colon cancer, prostate cancer, epithelial sarcoma and soft tissue sarcoma.

In some embodiments, the medicament is a medicament for preventing and/or treating a tumor, such as breast cancer, ovarian cancer, small cell lung cancer, acute myeloid leukemia or acute lymphocytic leukemia.

The present invention also provides a method for preventing and/or treating a CDK7-mediated disease, which comprises administering to a subject in need thereof a therapeutically effective amount of the compound of formula I or the tautomer, the stereoisomer or the isotopic derivative thereof, or the pharmaceutically acceptable salt of any of the foregoing, or the crystalline form or the solvate of any of the foregoing.

In some embodiments, the CDK7-mediated disease may be a tumor, such as breast cancer, ovarian cancer, small cell lung cancer, acute myeloid leukemia, acute lymphocytic leukemia, bladder cancer, colon cancer, prostate cancer, epithelial sarcoma and soft tissue sarcoma.

The present invention also provides a method for preventing and/or treating a tumor, which comprises administering to a subject in need thereof a therapeutically effective amount of the compound of formula I or the tautomer, the stereoisomer or the isotopic derivative thereof, or the pharmaceutically acceptable salt of any of the foregoing, or the crystalline form or the solvate of any of the foregoing.

In some embodiments, the tumor can be breast cancer, ovarian cancer, small cell lung cancer, acute myeloid leukemia, acute lymphocytic leukemia, bladder cancer, colon cancer, prostate cancer, epithelial sarcoma and soft tissue sarcoma.

Unless otherwise stated, terms used herein have the following definitions, and definitions of terms not referred to hereinafter are the same as those generally understood by those skilled in the art to which the present invention pertains.

The term "tautomer" refers to functional isomers resulting from the rapid movement of an atom in a molecule between two positions. For example, acetone and 1-propen-2-ol can be mutually converted when hydrogen atom rapidly migrates between oxygen and $\alpha$-carbon atoms.

The term "stereoisomer" refers to isomers of a molecule having the same order of atoms or radicals but different

spatial arrangement, such as *cis-trans* isomer, optical isomer or atropisomer. Such stereoisomers can be separated, purified and enriched by asymmetric synthesis or chiral resolution (including but not limited to thin layer chromatography, centrifugal partition chromatography, column chromatography, gas chromatography, and high pressure liquid chromatography), and can also be obtained by chiral resolution via bonding (such as chemical bonding) or salification (such as physical bonding) with other chiral compounds. Optical isomers include enantiomers and diastereoisomers.

The term "isotopic derivative" refers to a compound in which one or more atoms are replaced by one or more atoms having a specific atomic mass or mass number. Examples of isotopes that can be incorporated into the compound include, but are not limited to, isotopes of hydrogen, carbon, nitrogen, oxygen, fluorine, sulfur, and chlorine (such as $^{2}H$, $^{3}H$, $^{13}C$, $^{14}C$, $^{15}N$, $^{18}O$, $^{17}O$, $^{18}F$, $^{35}S$ and $^{36}Cl$). An isotopic compound can generally be prepared by replacing a non-isotopically labeled reagent with an isotopically labeled reagent according to the methods described herein. Typical examples of isotopic derivatives include deuterated compounds.

The term "pharmaceutically acceptable salt" refers to a salt prepared with the compound and a relatively non-toxic and pharmaceutically acceptable acid or base. When the compound contains a relatively acidic functional group, a base addition salt can be obtained by contacting a neutral form of the compound with a sufficient amount of a pharmaceutically acceptable base in a pure solution or a suitable inert solvent. Pharmaceutically acceptable base addition salts include, but are not limited to: lithium salt, sodium salt, potassium salt, calcium salt, aluminum salt, magnesium salt, zinc salt, bismuth salt, ammonium salt, and diethanolamine salt. When the compound disclosed herein contains a relatively basic functional group, an acid addition salt can be obtained by contacting a neutral form of the compound with a sufficient amount of a pharmaceutically acceptable acid in a pure solution or a suitable inert solvent. The pharmaceutically acceptable acid includes inorganic acids, including, but not limited to: hydrochloric acid, hydrobromic acid, hydroiodic acid, nitric acid, carbonic acid, phosphoric acid, phosphorous acid, and sulfuric acid. The pharmaceutically acceptable acids include organic acids, including, but not limited to: acetic acid, propionic acid, oxalic acid, isobutyric acid, maleic acid, malonic acid, benzoic acid, succinic acid, suberic acid, boletic acid, lactic acid, mandelic acid, phthalic acid, benzenesulfonic acid, *p*-toluenesulfonic acid, citric acid, salicylic acid, tartaric acid, methanesulfonic acid, isonicotinic acid, acidic citric acid, oleic acid, tannic acid, pantothenic acid, bitartrate, ascorbic acid, gentisic acid, fumaric acid, gluconic acid, saccharic acid, formic acid, ethanesulfonic acid, pamoic acid (i.e., 4,4'-methylene-bis(3-hydroxy-2-naphthoic acid)), amino acids (such as glutamic acid and arginine), and the like. When a compound contains both relatively acidic functional group and relatively basic functional group, it can be converted to either a base addition salt or an acid addition salt. For details, see Berge et al., "Pharmaceutical Salts", Journal of Pharmaceutical Science 66:1-19 (1977), or Handbook of Pharmaceutical Salts: Properties, Selection, and Use (P. Heinrich Stahl and Camille G. Wermuth, ed., Wiley-VCH, 2002).

The term "crystalline form" means that ions or molecules in it are arranged strictly periodically in a three-dimensional space in a certain manner and have a periodic recurring pattern at certain intervals; due to the difference in above periodic arrangements, there may be multiple crystalline forms, i.e., polymorphism.

The term "solvate" refers to a substance formed by combining a molecule with a stoichiometric or non-stoichiometric amount of a solvent. Solvent molecules in the solvate may be present in ordered or unordered arrangements. The solvents include, but are not limited to, water, methanol, ethanol, and the like.

The term "halogen" refers to fluorine, chlorine, bromine or iodine.

The term "alkyl" refers to a linear or branched saturated monovalent hydrocarbyl having a specified number of carbon atoms. For example, $C_{1-4}$ alkyl refers to alkyl having 1-4 carbon atoms. Examples of alkyl include, but are not limited to, methyl, ethyl, *n*-propyl, isopropyl, *n*-butyl, isobutyl, *sec*-butyl, *tert*-butyl, and pentyl. In some embodiments, $C_{1-4}$ alkyl can be methyl, ethyl, *n*-propyl, isopropyl, *n*-butyl, isobutyl, *sec*-butyl, or *tert*-butyl. In some embodiments, $C_{1-6}$ alkyl can be $C_{1-4}$ alkyl, such as methyl, ethyl, *n*-propyl, isopropyl, *n*-butyl, isobutyl, *sec*-butyl, or *tert*-butyl.

The term "alkoxy" refers to -O-$R^X$, wherein $R^X$ is the alkyl defined above. In some embodiments, $C_{1-4}$ alkoxy can be methoxy, ethoxy, *n*-propoxy, isopropoxy, *n*-butoxy, isobutoxy, *sec*-butoxy, or *tert*-butoxy.

The term "cycloalkyl" refers to saturated monocyclic or polycyclic (e.g., fused, spiro or bridged) hydrocarbyl formed by carbon atoms. In some embodiments, cycloalkyl is a monocyclic group. In some embodiments, $C_{3-6}$ cycloalkyl can be cyclopropyl, cyclobutyl, cyclopentyl, or cyclohexyl.

The term "heterocycloalkyl" refers to a non-aromatic, saturated or partially unsaturated, and monocyclic or polycyclic (e.g., fused, spiro or bridged) cyclic group formed by carbon atoms and at least one heteroatom selected from N, O and S. In some embodiments, heterocycloalkyl is a saturated cyclic group. In some embodiments, heterocycloalkyl is a monocyclic group. In some embodiments, heterocycloalkyl is a saturated monocyclic group. Heterocycloalkyl can be connected to the rest of the molecule via a heteroatom or a carbon atom in the ring. Examples of heterocycloalkyl include, but are not limited to, 1-piperidinyl, 2-piperidinyl, 3-piperidinyl, 4-morpholinyl, 3-morpholinyl, tetrahydrofuran-2-yl, tetrahydrothiophen-2-yl, tetrahydrothiophen-3-yl, 1-piperazinyl, and 2-piperazinyl. 4-6 membered heterocycloalkyl can be 4, 5 or 6 membered heterocycloalkyl. The 4 membered heterocycloalkyl is, e.g., azetidinyl. The 5 membered heterocycloalkyl is, e.g., tetrahydrofuranyl, tetrahydropyrrolyl or tetrahydrothienyl. The 6 membered heterocycloalkyl is, e.g., piperidinyl, morpholinyl or piperazinyl. In some cases, in -$NR^gR^h$ or similar groups, if it is defined that $R^g$ and $R^h$,

together with a nitrogen atom attached thereto, form a substituted or unsubstituted 4-6 membered heterocycloalkyl and the 4-6 membered heterocycloalkyl has 0, 1 or 2 additional heteroatoms independently selected from N, O and S, the meaning of "additional" is clear, and namely it refers to other heteroatoms besides the N atom in the $-\underline{N}R^gR^h$ (where the nitrogen atom is underlined). For example, if

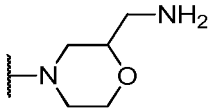

is formed by $-NR^gR^h$, the additional heteroatom is 1 oxygen atom; if

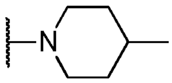

is formed by $-NR^gR^h$, there's 0 additional heteroatom.

The term "heteroaryl" refers to an aromatic cyclic group formed by carbon atoms and at least one heteroatom selected from N, O and S. 5 membered heteroaryl is, e.g., furanyl, thienyl, pyrrolyl, pyrazolyl, oxazolyl, thiazolyl, imidazolyl or triazolyl; 6-membered heteroaryl is, e.g., pyrazinyl, pyridazinyl, pyridinyl or pyrimidinyl.

As used herein, "x-y membered" for describing a cyclic group means that the number of atoms in the ring is x-y. For example, cyclopropyl is 3 membered, tetrahydropyrrolyl is 5 membered, and piperidinyl is 6 membered.

" 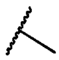 "

used herein in the structural formulas describing groups means that the corresponding group is attached via that site to other fragments and groups of the compound. For example, in

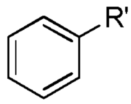

, when R' is

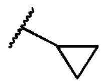 , 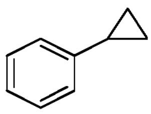

is formed.

When any variable (e.g., R) occurs more than once in the constitution or structure of a compound, the variable is independently defined in each case. Thus, for example, if a group is substituted with 0-2 R, the group can be optionally substituted with up to two R, and the definition of R in each case is independent. Furthermore, a combination of a substituent and/or a variable is permissible only if the combination can result in a stable compound. For example, if in

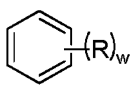

,

w is 0, 1 or 2, and each R is independently methyl or fluoro,

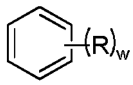

includes

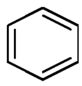, 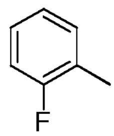 or 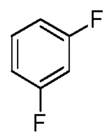 .

The term "pharmaceutical adjuvant" refers to excipients and additives used in the manufacture of pharmaceutical products and in the formulation of pharmaceutical formulas, and it refers to all substances contained in the pharmaceutical preparation except for the active ingredient. See Volume 4 of Pharmacopoeia of The People's Republic of China (2015 Edition), or Handbook of Pharmaceutical Excipients (Raymond C Rowe, 2009 Sixth Edition).

The term "treat" "treating" or "treatment" refers to therapeutic therapy. When specific conditions are involved, "treat" "treating" or "treatment" refers to: (1) relieving one or more biological manifestations of a disease or disorder, (2) interfering with (a) one or more points in a biological cascade that causes or results in a disorder or (b) one or more biological manifestations of a disorder, (3) ameliorating one or more symptoms, effects or side effects associated with a disorder, or ameliorating one or more symptoms, effects or side effects associated with a disorder or treatment thereof, or (4) slowing the progression of one or more biological manifestations of a disease or disorder.

The term "therapeutically effective amount" refers to an amount of a compound that, when administered to a patient, is sufficient to effectively treat or prevent a disease or disorder described herein. The "therapeutically effective amount" will vary depending on the compound, the disorder and the severity thereof, and the age of the patient to be treated, but can be adjusted as desired by those skilled in the art.

The term "subject" refers to any animal, preferably a mammal, most preferably a human, that is about to receive administration of or has received administration of a compound or composition. The term "mammal" includes any mammal. Examples of mammals include, but are not limited to, cows, horses, sheep, pigs, cats, dogs, mice, rats, rabbits, guinea pigs, monkeys, humans, and the like, with humans being most preferred.

All patents and publications referred to herein are incorporated by reference in their entirety.

The biological activity of the compounds disclosed herein can be assessed by using any conventionally known method. Appropriate detection methods are well known in the art. For example, affinity activity, agonistic activity and/or antagonistic activity of the compound disclosed herein for a dopamine receptor, pharmacokinetic activity and/or liver microsome stability of the compound disclosed herein, and the like can be detected by an appropriate conventional method. The detection methods provided by the present invention are presented as examples only and do not limit the present invention. The compounds disclosed herein are active in at least one of the detection methods provided by the present invention.

The above preferred conditions may be combined arbitrarily to obtain preferred embodiments of the present invention without departing from the general knowledge in the art.

The reagents and starting materials used in the present invention are commercially available.

The abbreviations referred to herein have the following meanings: PE represents petroleum ether; EA represents ethyl acetate; ACN represents acetonitrile; Sphos represents 2-dicyclohexylphosphino-2',6'-dimethoxybiphenyl; $Pd_2(dba)_3$ represents tris(dibenzylideneacetone) dipalladium; HATU represents 2-(7-azabenzotriazol)-*N*,*N*,*N'*,*N'*-tetramethyluronium hexafluorophosphate; LDA represents lithium diisopropylamide; THF represents tetrahydrofuran; PyBOP represents benzotriazol-1-yl-oxytripyrrolidinophosphonium hexafluorophosphate; DBU represents 1,8-diazabicycloundec-7-ene; TEA represents triethylamine; DIPEA represents *N*,*N*-diisopropylethylamine; DCM represents dichloromethane; DMF represents *N*,*N*-dimethylformamide; DMSO represents dimethyl sulfoxide; TFA represents trifluoroacetic acid; DMAP represents 4-dimethylaminopyridine; BINAP represents 2,2'-bis(diphenylphosphino)-1,1'-binaphthyl; T3P represents 1-propylphosphoric anhydride; pd(dppf)$Cl_2$ represents [1,1'-bis(diphenylphosphino) ferrocene]dichloropalladium(II); NBS represents *N*-bromosuccinimide; NCS represents *N*-chlorosuccinimide; Xantphos represents 4,5-bis(diphenylphosphino)-9,9-dimethylxanthene; DIAD represents diisopropyl azodicarboxylate; Sphos Pd G3 represents (2-dicyclohexylphosphino-2',6'-dimethoxybiphenyl)[2-(2'-amino-1,1'-biphenyl)]palladium (II) mesylate.

The compound disclosed herein has CDK7 inhibitory activity, and it can be used as a reference substance for *in vitro* test of inhibitory activity of a compound against CDK7, and can also be used for treating diseases such as a tumor.

## BRIEF DESCRIPTION OF THE DRAWINGS

FIG. 1 shows change in the body weight of experimental animals versus time of administration in Efficacy Example 6.

FIG. 2 shows the growth curve of tumor in Efficacy Example 6.

## DETAILED DESCRIPTION

The present invention is further illustrated by the following examples, which are not intended to limit the present invention. Experimental procedures without specified conditions in the following examples were performed in accordance with conventional procedures and conditions, or in accordance with instructions.

**Example 1 SZ-015095:** (*S*,*E*)-1-(4-(dimethylamino)but-2-enoyl)pyrrolidin-3-yl 4-((3-isopropyl-5-methyl-3*H*-imidazo[4,5-*b*]pyridin-7-yl)amino)piperidine-1-carboxylate

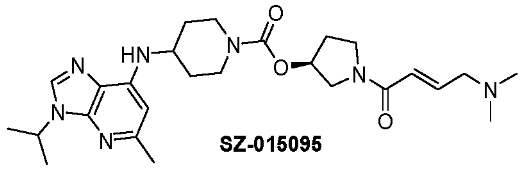

**SZ-015095**

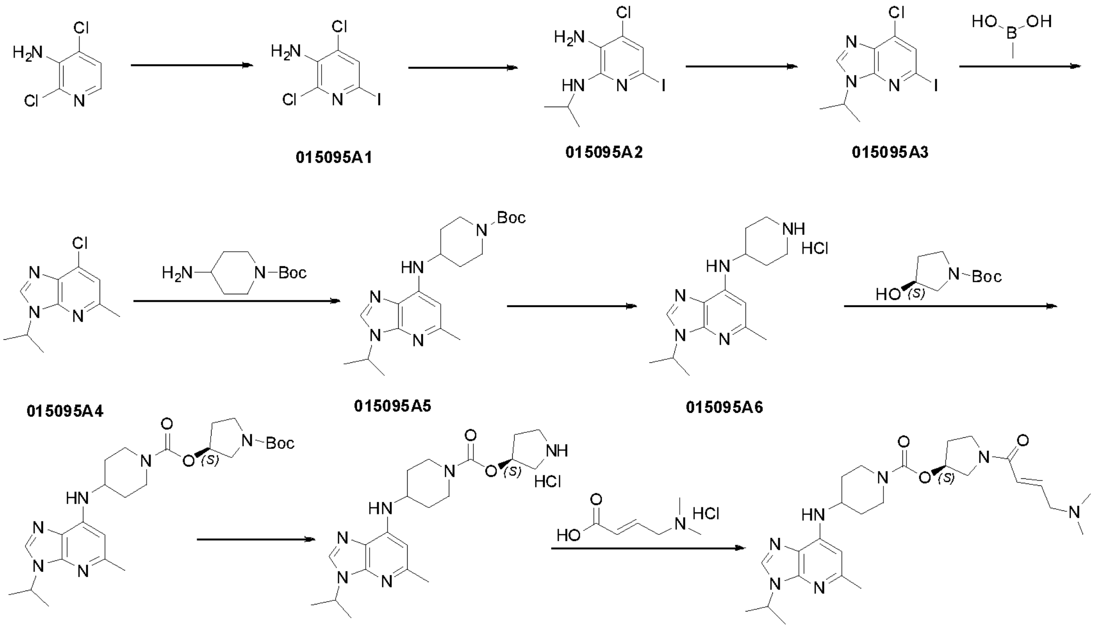

015095A1 015095A2 015095A3 015095A4 015095A5 015095A6 015095A7 015095A8 SZ-015095

**Step 1: 015095A1**

Trifluoroacetic acid (1.9 g, 16.6 mmol) and *N*-iodosuccinimide (13.6 g, 60.7 mmol) were added to a solution of 3-amino-2,4-dichloropyridine (9.0 g, 55.2 mmol) in acetonitrile (100 mL) at room temperature, and the reaction solution was stirred at 40°C overnight. After the reaction was completed, sodium thiosulfate solution (50 mL) was added to the reaction solution, and the resulting reaction solution was extracted with ethyl acetate (30 mL × 3). The organic phases were combined, dried over anhydrous sodium sulfate and filtered, and then the residue obtained after concentration was purified by column chromatography to give **015095A1** (13.7 g, 87% yield, a yellow solid).

By LCMS $(M+H)^+ m/z$, the calculation value was 288.9, and the measurement value was 288.9 $[M+H]^+$.

**Step 2: 015095A2**

Compound **015095A1**(5.0 g, 17.3 mmol) was dissolved in *n*-butanol (30 mL), and isopropylamine (5.1 g, 86.5 mmol) was added. The reaction solution was stirred at 180°C for 18 h. After the reaction was completed, the reaction solution was concentrated. Water (10 mL) was added to the residue, and ethyl acetate (30 mL × 3) was added for extraction. The organic phases were combined, dried over anhydrous sodium sulfate and filtered, and then the residue obtained after concentration was purified and concentrated by column chromatography to give **015095A2** (3.8 g, 70% yield, a red solid).

$^1$H NMR ($CDCl_3$, 400MHz): δ 6.98 (s, 1H), 4.22-4.15 (m, 1H), 1.24-1.22 (d, J=8Hz, 6H). By LCMS $(M+H)^+$*m/z*, the calculation value was 312.0, and the measurement value was 312.1 $[M+H]^+$.

**Step 3: 015095A3**

Triethyl orthoformate (1.92 g, 12.9 mmol) was added to a solution of **015095A2** (1.35 g, 4.33 mmol) in formic acid (60 mL) at room temperature, and the reaction solution was stirred at 100°C for 4 h. After the reaction was completed, the reaction solution was cooled to room temperature and concentrated to remove the solvent. The residue was diluted with saturated aqueous sodium bicarbonate solution (150 mL) and stirred for 30 min, and ethyl acetate (150 mL) was added. The mixed solution was filtered, and the filter cake was washed with ethyl acetate to give filtrate. The aqueous phase was then separated out and extracted with ethyl acetate (50 mL × 2), and the organic phases were combined, washed with saturated brine, dried over anhydrous sodium sulfate, filtered, and concentrated. The residue was purified and concentrated by column chromatography to give **015095A3** (710 mg, 51% yield, a red solid).

By LCMS $(M+H)^+$*m/z,* the calculation value was 322.0, and the measurement value was 321.9 $[M+H]^+$.

**Step 4: 015095A4**

Compound **015095A3** (2.3 g, 7.16 mmol), methylboronic acid (1.29 g, 21.5 mmol), potassium carbonate (4.94 g, 35.8 mmol) and bis(triphenylphosphine)palladium(II) dichloride (503 mg, 0.176 mmol) were dissolved in DMF at room temperature. The reaction solution was stirred at 100°C overnight. After the reaction was completed, the reaction solution was then concentrated. The residue was purified by silica gel column chromatography to give **015095A4** (1.0 g, 67% yield, a yellow solid).

$^1$H NMR ($CDCl_3$, 400MHz): δ8.10 (s, 1H), 7.16 (s, 1H), 5.04-4.94 (m, 1H), 1.65-1.63 d, *J* = 8 Hz, 6H). By LCMS $(M+H)^+$*m/z,* the calculation value was 210.1, and the measurement value was 210.2 $[M+H]^+$.

**Step 5: 015095A5**

Compound **015095A4** (400 mg, 1.89 mmol), *N*-Boc-4-aminopiperidine (758 mg, 3.79 mmol), potassium phosphate (803 mg, 3.79 mmol), Sphos (77.5 mg, 0.189 mmol) and $Pd_2(dba)_3$ (173 mg, 0.189 mmol) were dissolved in toluene at room temperature. The reaction solution was stirred at 110°C overnight. After the reaction was completed, the reaction solution was then concentrated. The residue was purified by silica gel column chromatography to give **015095A5** (604 mg, 85% yield, a yellow solid).

$^1$H NMR ($CDCl_3$, 400MHz): δ7.78 (s, 1H), 6.21(s, 1H), 5.09-5.07 (m, 1H), 4.96-4.91 (m, 1H), 4.13-4.06(m, 2H), 3.70-3.68 (m, 2H), 3.01-2.95 (m, 2H), 2.53 (s, 3H), 2.09-2.04 (m, 2H), 1.61 (d, *J* = 8 Hz, 6H), 1.48 (s, 9H). By LCMS $(M+H)^+$*m/z,* the calculation value was 374.2, and the measurement value was 374.3 $[M+H]^+$.

**Step 6: 015097A6**

The crude product of **015095A5** (400 mg, 1.07 mmol) obtained in the previous step was dissolved in 5 N dioxane hydrochloride solution (5 mL) at room temperature, and the reaction solution was stirred at room temperature for 1 h. After the reaction was completed, the reaction solution was concentrated to give a crude product of **015095A6** (315 mg, > 100%, a yellow solid), which was directly used in the next step without purification.

By LCMS $(M+H)^+$*m/z*, the calculation value was 274.2, and the measurement value was 274.2 $[M+H]^+$.

**Step 7: 015095A7**

Triethylamine (288 mg, 2.85 mmol) and triphosgene (423 mg, 1.45 mmol) were added to a solution of (*S*)-1-*N*-*tert*-butoxycarbonyl-3-hydroxypyrrolidine (213 mg, 1.14 mmol) in tetrahydrofuran (10 mL) at 0 °C, and the reaction solution was stirred at room temperature for 45 min. The reaction solution was concentrated to remove the solvent, and the residue was dissolved in dichloromethane (10 mL). The aforementioned dichloromethane solution was added to a solution of a crude product of **015095A6** (315 mg, 0.95 mmol) obtained in the previous step in dichloromethane (12 mL) and triethylamine (0.4 mL) at 0 °C, and the reaction solution was stirred at room temperature overnight. After the reaction was completed, water (20 mL) was added to the reaction solution, and dichloromethane (15 mL × 3) was added for extraction. The organic phases were combined, washed with saturated brine and concentrated. The residue was purified by silica gel column chromatography to give **015095A7** (101 mg, 19.3% yield, a yellow solid).

By LCMS $(M+H)^+$*m/z*, the calculation value was 487.3, and the measurement value was 487.4 $[M+H]^+$.

**Step 8: 015095A8**

**015095A7** (101 mg, 0.207 mmol) was dissolved in 5 N dioxane hydrochloride solution (10 mL) at room temperature, and the reaction solution was stirred at room temperature for 1 h. After the reaction was completed, the reaction solution was concentrated to give a crude product of **015095A8** (65 mg, 81%, a yellow oil), which was directly used in the next step without purification.

By LCMS $(M+H)^+$*m/z*, the calculation value was 387.2, and the measurement value was 387.3 $[M+H]^+$.

**Step 9: SZ-015095**

*Trans*-4-dimethylaminocrotonic acid hydrochloride (29.9 mg, 0.180 mmol), **015095A8** (35 mg, 0.09 mmol), triethylamine (0.2 mL) and HATU (41.2 mg, 0.108 mmol) were added to *N*,*N*-dimethylformamide (5 mL), and the reaction solution was stirred at room temperature for 1 h. After the reaction was completed, water (40 mL) was added to the reaction solution, and ethyl acetate (15 mL × 3) was added for extraction. The organic phases were combined, washed with saturated brine and concentrated. The residue was purified by preparative high performance liquid chromatography to give **SZ-015095** (5.4 mg, 12% yield, a white solid).

By LCMS $(M+H)^+$*m/z,* the calculation value was 498.3, and the measurement value was 498.3 $[M+H]^+$.

$^1H$ NMR ($CDCl_3$, 400MHz): $\delta$ 7.78 (s, 1H), 6.98-6.91 (m, 1H), 6.33-6.21 (m, 2H), 5.31 (s, 1H), 5.15-5.08 (m, 1H), 5.13-5.11 (m, 1H), 4.96-4.89 (m, 1H), 4.15-4.00 (m, 2H), 3.80-3.56 (m, 5H), 3.11-3.02 (m, 4H), 2.54 (s, 3H), 2.27-2.26 (m, 6H), 2.21-2.17 (m, 1H), 2.11-2.04 (m, 3H), 1.58-1.56 (m, 6H), 1.49-1.48 (m, 2H).

**Example 2 SZ-015256:** (*S*,*E*)-1-(4-(dimethylamino)but-2-enoyl)pyrrolidin-3-yl 4-((8-isopropyl-2-methylpyrazolo[1,5-*a*][1,3,5]triazin-4-yl)amino)piperidine-1-carboxylate

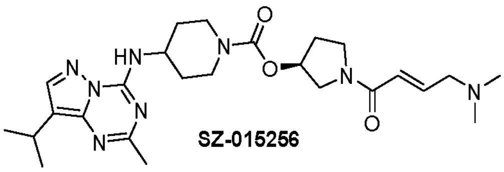

SZ-015256

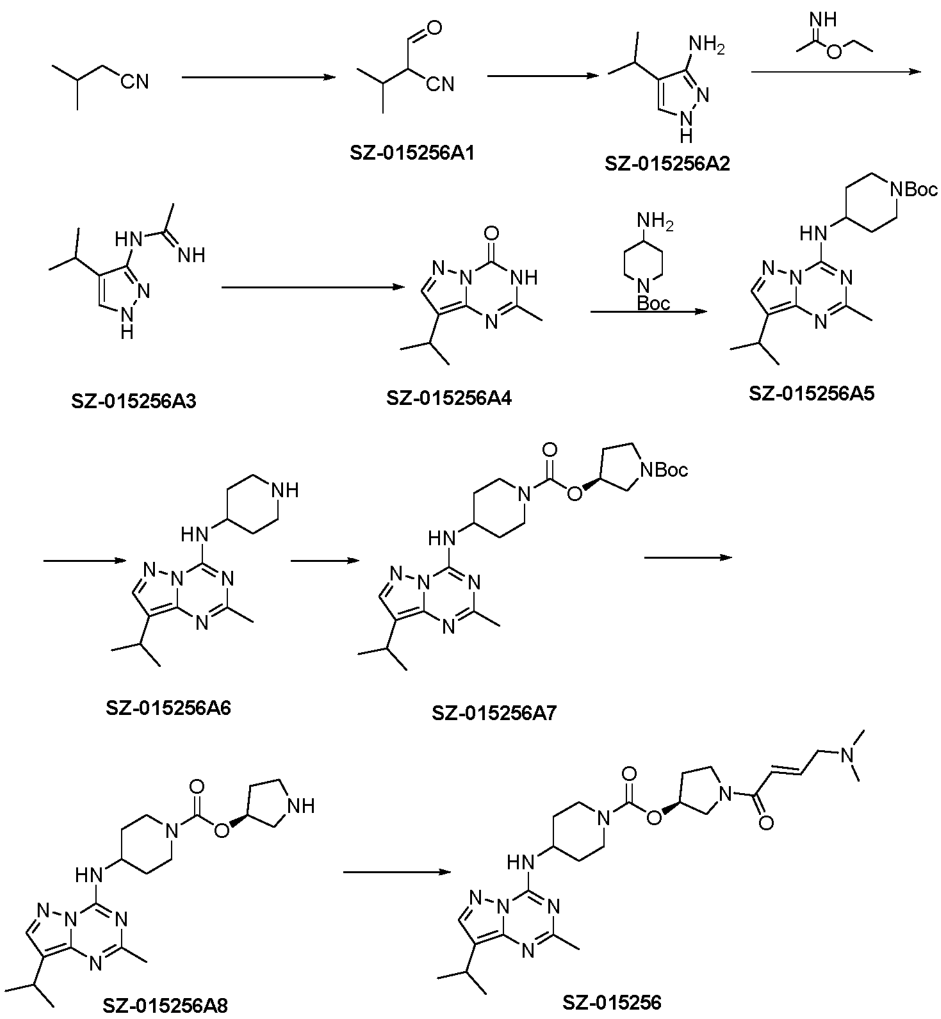

SZ-015256A1 SZ-015256A2 SZ-015256A3 SZ-015256A4 SZ-015256A5 SZ-015256A6 SZ-015256A7 SZ-015256A8 SZ-015256

Step 1: 2-formyl-3-methylbutyronitrile

Isovaleronitrile (20 g, 0.24mol) was added dropwise to LDA (130 mL, 2 M in THF) at - 70 °C under argon atmosphere, and the reaction solution was stirred for 40 min after dropwise addition was completed. A solution of ethyl formate (22 mL) in THF (100 mL) was then added dropwise to the reaction solution at this temperature, and the resulting reaction solution was stirred at room temperature overnight after dropwise addition was completed. 4 N hydrochloric acid was added to the reaction solution until the pH was about 3, and ethyl acetate (200 mL) was added for extraction. The organic phase was dried over anhydrous sodium sulfate, filtered and concentrated by rotary evaporation to give **SZ-015256A1.**

Step 2: 4-isopropyl-1*H*-pyrazol-3-amine

**SZ-015256A1** obtained in the previous step, acetic acid (22 mL) and hydrazine hydrate (17 mL) were dissolved in ethanol (400 mL), and the reaction solution was refluxed overnight. Ethanol was removed by rotary evaporation, and the pH was adjusted to about 9 with saturated sodium bicarbonate. Dichloromethane (300 mL) was added for extraction, and the organic phase was dried over anhydrous sodium sulfate and concentrated by rotary evaporation to give **SZ-015256A2** (20 g).

Step 3: *N*-(4-isopropyl-1*H*-pyrazol-3-yl)acetimidamide

**SZ-015256A2** (20 g) and ethyl acetimidate hydrochloride (20 g) were dissolved in acetonitrile (200 mL) and acetic acid (10 g), and the reaction solution was stirred at room temperature overnight.Undissolved solids were filtered out, and the filtrate was concentrated by rotary evaporation. The residue was washed by flash silica gel column chromatography (MeOH: EA = 10% to 100%) to give **SZ-015256A3** (6 g, an oil). LCMS: $[M+H]^+$ 167.31.

Step 4: 8-isopropyl-2-methylpyrazolo[1,5-*a*][1,3,5]triazin-4(3*H*)-one

**SZ-015256A3** (6 g), 21% sodium ethoxide/ethanol (120 mL), and diethyl carbonate (30 mL) were mixed and stirred at 110 °C for 16 h. Ethanol was removed by rotary evaporation, and the residue was purified by flash silica gel column chromatography (PE:EA = 1:4; R*f* = 0.2) to give **SZ-015256A4** (2.5 g, a foamy solid). LCMS: $[M+H]^+$ 193.11.

Step 5: tert-butyl 4-((8-isopropyl-2-methylpyrazolo[1,5-*a*][1,3,5]triazin-4-yl)amino) piperidine-1-carboxylate

**SZ-015256A4** (1.7 g, 8.9 mmol), PyBOP (3.2 g, 10.6 mmol), DBU (1.0 g, 10.6 mmol) and 1-Boc-4-aminopiperidine (1.2 g, 10.6 mmol) were mixed in acetonitrile (20 mL), and the reaction solution was stirred at room temperature for 4 h. After the reaction was completed, the reaction solution was concentrated by rotary evaporation, and the residue was purified by flash silica gel column chromatography (PE:EA = 1:4; R*f* = 0.6) to give **SZ-015256A5** (600 mg, a foamy solid). LCMS: $[M+H]^+$ 375.25.

Step 6: 8-isopropyl-2-methyl-*N*-(piperidin-4-yl)pyrazolo[1,5-*a*][1,3,5]triazin-4-amine

The product obtained in the previous step was dissolved in dichloromethane (6 mL), and trifluoroacetic acid (5 mL) was slowly added dropwise. The reaction solution was stirred at room temperature for about 2 h, and then directly concentrated by rotary evaporation to give a crude product of **SZ-015256A6** (500 mg). LCMS: $[M+H]^+$ 275.26.

Step 7: (*S*)-1-(*tert*-butoxycarbonyl)pyrrolidin-3-yl 4-((8-isopropyl-2-methylpyrazolo [1,5-*a*][1,3,5]triazin-4-yl)amino)piperidine-1-carboxylate

*Tert-butyl* (*S*)-3-hydroxypyrrolidine-1-carboxylate (177 mg, 0.93 mmol), *p*-nitrophenyl chloroformate (205 mg, 0.51 mmol) and triethylamine (0.5 mL, 3.24 mmol) were mixed in acetonitrile (5 mL), and the reaction solution was stirred at 40 °C for 2 h. Then **SZ-015256A6** (200 mg, 0.72 mmol) was added, and the resulting reaction solution was stirred at room temperature for 2 h. The reaction solution was directly concentrated by rotary evaporation, and the residue was purified by flash silica gel column chromatography (PE:EA = 1:1; R*f* = 0.3) to give **SZ-015256A7** (158 mg, a foamy solid). LCMS: $[M+H]^+$ 488.34.

Step 8

**SZ-015256A7** (158 mg) was dissolved in dichloromethane (2 mL), and trifluoroacetic acid (2 mL) was slowly added dropwise. The reaction solution was stirred at room temperature for about 2 h, and then directly concentrated by rotary evaporation to give a crude product of **SZ-015256A8** (200 mg).LCMS: $[M+H]^+$ 390.31.

Step 9:

**SZ-015256A8** obtained in the previous step, *trans*-4-dimethylaminocrotonic acid hydrochloride (65 mg, 0.40 mmol), DIPEA (0.2 mL, 1.36 mmol) and HATU (188 mg, 0.49 mmol) were mixed in tetrahydrofuran (3 mL), and the reaction solution was stirred at room temperature for 1 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015256** (66 mg, a light yellow solid). LCMS: $[M+H]^+$ 499.09.

**SZ-015256:** $^1$H NMR (400 MHz, DMSO-$d_6$) 8.52 (d, *J* = 8.4 Hz, 1H), 8.03 (s, 1H), 6.74-6.63 (m, 2H), 5.23-5.18 (m, 1H), 4.30-4.28 (m, 1H), 4.02-3.79 (m, 5H), 3.69-3.40 (m, 3H), 3.13-3.16 (m, 1H), 2.98-2.95 (m, 2H), 2.80 (s, 6H), 2.41 (s, 3H), 2.12-2.09 (m, 2H), 1.86-1.84 (m, 2H), 1.70-1.64 (m, 2H), 1.29 (d, *J* = 7.2 Hz, 6H).

**Example 3 SZ-015264:** (*S*,*E*)-1-(4-(dimethylamino)but-2-enoyl)pyrrolidin-3-yl 4-((9-isopropyl-2-methyl-9*H*-purin-6-yl)amino)piperidine-1-carboxylate

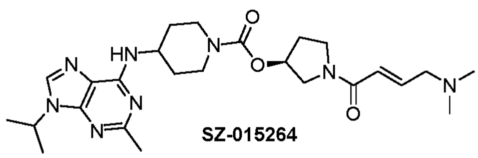

SZ-015264

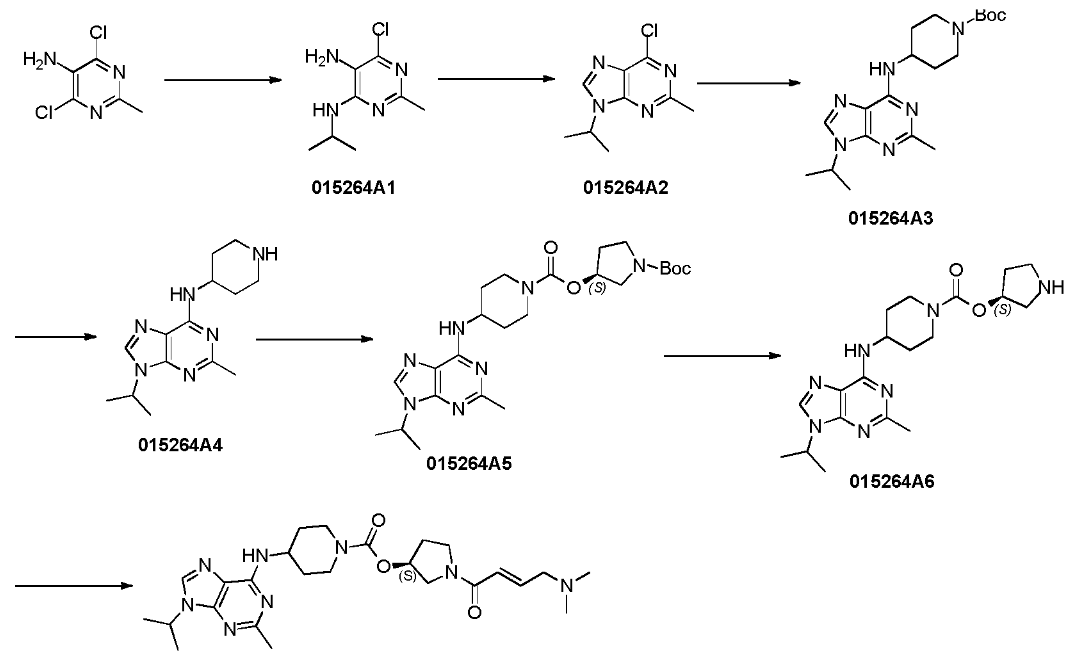

015264A1 015264A2 015264A3 015264A4 015264A5 015264A6

SZ-015264

Step 1: 6-chloro-$N^4$-isopropyl-2-methylpyrimidin-4,5-diamine

4,6-dichloro-2-methylpyrimidin-5-amine (1.0 g, 5.7 mmol), isopropylamine (3.4 g, 57 mmol) and isopropanol (10 mL) were added to a microwave flask, and the reaction solution was heated to 120 °C and reacted for 8 h under a microwave condition. The reaction solution was cooled to room temperature and distilled under reduced pressure to give the product **015264A1** (1 g, 87%, a light yellow solid). LCMS: $[M+H]^+$ 201.38.

Step 2: 6-chloro-9-isopropyl-2-methyl-9*H*-purine

**015264A1** (1.0 g, 5 mmol), trimethyl orthoformate (9 mL) and acetic acid (4 mL) were added to a microwave flask, and the reaction solution was heated to 100 °C and reacted for 3 h under a microwave condition. After the reaction was completed, the reaction solution was cooled to room temperature, and the solvent was removed by rotary evaporation. The residue was concentrated under reduced pressure to give a crude product, which was purified by flash silica gel column chromatography to give **015264A2** (1.0 g, 95%, a white solid). LCMS: $[M+H]^+$ 211.34.

Step 3: tert-butyl 4-((9-isopropyl-2-methyl-9*H*-purin-6-yl)amino)piperidine-1-carboxylate

Compound **015264A2** (1.0 g, 4.76 mmol) was dissolved in isopropanol (10 mL), and tert-butyl 4-aminopiperidine-1-carboxylate (1.05 g, 5.23 mmol) was added. The reaction solution was heated to 100 °C and stirred for 6 h, and then the reaction solution was cooled to room temperature and concentrated by rotary evaporation under reduced pressure to give a crude product of **015264A3,** which was purified by flash silica gel column chromatography to give **015264A3** (540 mg, 31%, a white solid). LCMS: $[M+H]^+$ 375.15.

Step 4: 9-isopropyl-2-methyl-*N*-(piperidin-4-yl)-9*H*-purin-6-amine

Compound **015264A3** (540 mg, 1.44 mmol) was dissolved in dichloromethane (9 mL), and trifluoroacetic acid (3 mL) was added. The reaction solution was stirred at room temperature for 1 h and concentrated by rotary evaporation under reduced pressure to give **015264A4 trifluoroacetate** (540 mg, 100%, a colorless oily liquid). LCMS: $[M+H]^+$ 275.12.

Step 5: (*S*)-1-(*tert*-butoxycarbonyl)pyrrolidin-3-yl 4-((9-isopropyl-2-methyl-9*H*-purin-6-yl)amino)piperidine-1-carboxylate

Compound **015264A4 trifluoroacetate** (540 mg, 1.44 mmol) was dissolved in acetonitrile (9 mL), and triethylamine (303 mg, 3 mmol) and tert-butyl (*S*)-3-(((4-nitrophenoxy)carbonyl)oxo)pyrrolidine-1-carboxylate (507 mg, 1.44 mmol) were added successively. The reaction solution was stirred at room temperature for 2 h and concentrated by

rotary evaporation under reduced pressure to give a crude product of **015264A5,** which was purified by flash silica gel column chromatography to give **015264A5** (240 mg, 34%, a white solid). LCMS: $[M+H]^+$ 488.34.

Step 6: (*S*)-pyrrolidin-3-yl-4-((9-isopropyl-2-methyl-9*H*-purin-6-yl)amino)piperidine-1-carboxylate

Compound **015264A5** (240 mg, 0.49 mmol) was dissolved in dichloromethane (6 mL), and trifluoroacetic acid (2 mL) was added. The reaction solution was stirred at room temperature for 0.5 h and concentrated by rotary evaporation under reduced pressure to give **015264A6 trifluoroacetate** (240 mg, 100%, a colorless oily liquid). LCMS: $[M+H]^+$ 388.16.

Step 7: (*S*,*E*)-1-(4-(dimethylamino)but-2-enoyl)pyrrolidin-3-yl 4-((9-isopropyl-2-methyl-9*H*-purin-6-yl)amino)piperidine-1-carboxylate

Compound **015264A5** trifluoroacetate (240 mg, 0.49 mmol) was dissolved in DMF (5 mL), and DIPEA (260 mg, 2 mmol), (*E*)-4-(dimethylamino)but-2-enoic acid hydrochloride (83 mg, 0.49 mmol) and HATU (228 mg, 0.6 mmol) were added successively. The reaction solution was stirred at room temperature for 0.5 h and concentrated by rotary evaporation under reduced pressure to give a crude product of **SZ-015264,** which was subjected to reverse phase column chromatography (acetonitrile/water with 0.1% formic acid) and lyophilized to give **SZ-015264** (5 mg, 2%, a white solid). LCMS: $[M+H]^+$ 499.14.

$^1$H NMR (400 MHz, DMSO-$d_6$) $\delta$ 8.15 (s, 1H), 7.43 (d, *J* = 8.5 Hz, 1H), 6.62 (ddd, *J* = 16.3, 10.4, 6.1 Hz, 1H), 6.36 (dd, *J* = 21.7, 15.4 Hz, 1H), 5.15 (d, *J* = 23.7 Hz, 1H), 4.70 (dt, *J* = 13.3, 6.6 Hz, 1H), 3.96 (s, 2H), 3.85 - 3.67 (m, 1H), 3.64 - 3.40 (m, 3H), 3.39-3.30 (m, 1H), 3.05-2.91 (m, 4H), 2.41 (s, 3H), 2.12 - 1.99 (m, 8H), 1.92-1.85 (m, 2H), 1.48 (d, *J* = 6.8 Hz, 6H), 1.52-1.50 (m, 2H).

**Example 4 SZ-015272:** (*S*,*E*)-1-(4-(dimethylamino)but-2-enoyl)pyrrolidin-3-yl 4-((1-isopropyl-6-methyl-1*H*-imidazo[4,5-*c*]pyridin-4-yl)amino)piperidine-1-carboxylate

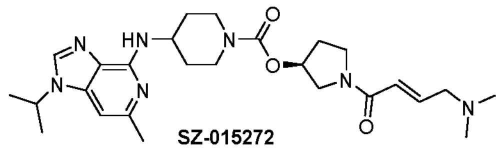

SZ-015272

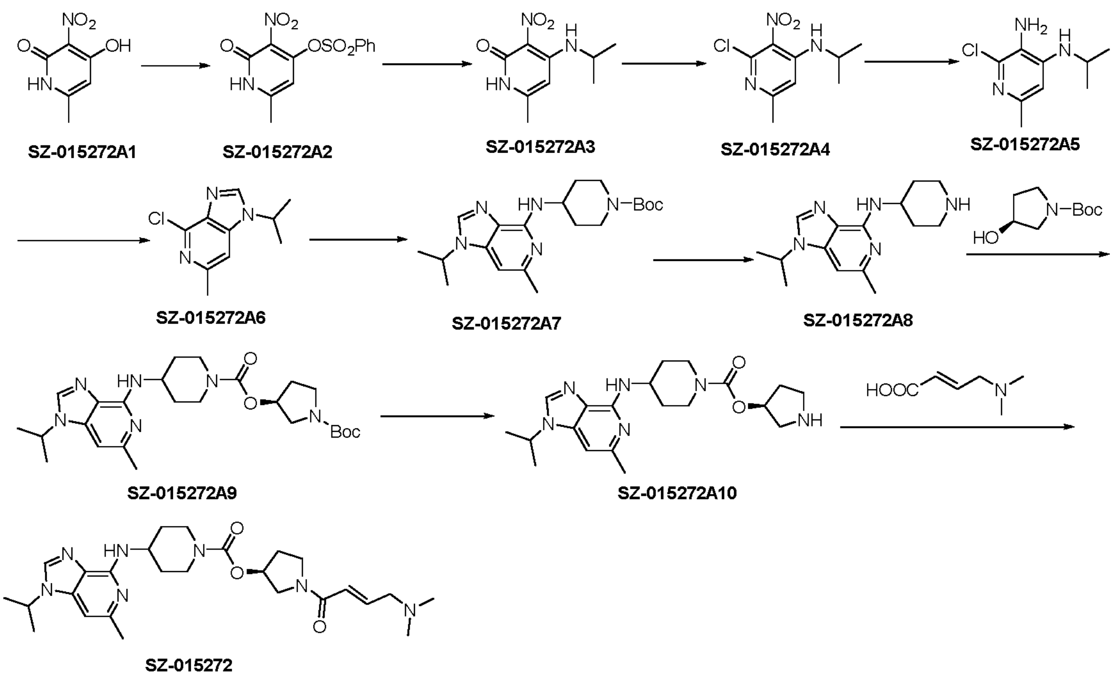

SZ-015272A1 SZ-015272A2 SZ-015272A3 SZ-015272A4 SZ-015272A5

SZ-015272A6 SZ-015272A7 SZ-015272A8

SZ-015272A9 SZ-015272A10

SZ-015272

Step 1: 6-methyl-3-nitro-2-oxo-1,2-dihydropyridin-4-*p*-methylbenzenesulfonate

THF (100 mL), **SZ-015272A1** (10.21 g), triethylamine (9.216 mL) and DMAP (368 mg) were added to a 500 mL reaction flask and stirred under reflux for 2 h, and then a solution of benzenesulfonyl chloride (10.60 g) in THF (50 mL) was added dropwise to the reaction solution about 30 min later. The resulting reaction system was further stirred under reflux for 4 h. After the reaction was completed, as detected by LCMS, the reaction solution was used in the next step without any treatment. LCMS: $[M+1]^+$ 310.97.

Step 2: 4-(isopropylamino)-6-methyl-3-nitropyridin-2(1*H*)-one

Triethylamine (9.216 mL) was added to the reaction solution in the step 1, and isopropylamine (5.11 mL) was added while stirring under reflux. The resulting reaction solution was further stirred under reflux for 2 h, and then the reaction solution was stirred overnight after the temperature was adjusted to 40 °C. After the reaction was completed, the reaction solution was filtered to give a residue, which was repeatedly washed with water and dichloromethane to give an off-white solid, and the off-white solid was dried with an oil pump to give **SZ-015272A3** (5.66 g). LCMS: $[M+1]^+$ 212.12.

Step 3: 2-chloro-*N*-isopropyl-6-methyl-3-nitropyridin-4-amine

**SZ-015272A3** (5.66 g), acetonitrile (10 mL), $POCl_3$ (2.5 mL) and DMF (0.5 mL) were added to a 100 mL reaction flask and stirred at 50 °C for 2 h. After the reaction was completed, a saturated $NaHCO_3$ solution was added to quench the reaction, and the reaction solution was adjusted to be alkaline and extracted with ethyl acetate. The organic phases were combined, and the solvent was removed by rotary evaporation. The residue was purified by silica gel column chromatography (ethyl acetate:petroleum ether = 1:2) to give **SZ-015272A4** (4.325 g). LCMS: $[M+1]^+$ 229.94.

Step 4: 2-chloro-*N*-isopropyl-6-methylpyridin-3,4-diamine

**SZ-015272A4** (3.3372 g), reduced iron powder (4.2 g), ammonium chloride (4.01 g), ethanol (55 mL) and water (11 mL) were added to a 250 mL reaction flask and stirred under reflux at 80 °C overnight. After the reaction was completed, the solvent was removed by rotary evaporation. The residue was diluted with ethanol, and the solid was removed by filtration. The filtrate was concentrated by rotary evaporation to give a crude product of **SZ-015272A5** (2.787 g), which was directly used in the next step without separation. LCMS: $[M+1]^+$ 200.08.

Step 5: 4-chloro-1-isopropyl-6-methyl-1*H*-imidazo[4,5-*c*]pyridine

**SZ-015272A5** (2.787 g) and triethyl orthoformate (100 mL) were added to a 250 mL reaction flask, DMF was added dropwise until the solution became clear, and 12 N concentrated hydrochloric acid (1.7 mL) was then added. The reaction solution was stirred at room temperature overnight under nitrogen atmosphere. After the reaction was completed, the reaction solution was concentrated by rotary evaporation, and the residue was separated by a silica gel column (ethyl acetate:petroleum ether = 1:2) to give **SZ-015272A6** (2.596 g). LCMS: $[M+1]^+$ 210.11.

Step 6: tert-butyl 4-((1-isopropyl-6-methyl-1*H*-imidazo[4,5-*c*]pyridin-4-yl)amino) piperidine-1-carboxylate

**SZ-015272A6** (836 mg), 1-Boc-4-aminopiperidine (2.4 g), BINAP (373.6 mg), $Pd_2(dba)_3$ (183.2 mg), sodium *tert*-butoxide (576 mg) and toluene (50 mL) were added to a 250 mL reaction flask, and refluxed at 110 °C overnight under nitrogen atmosphere. After the reaction was completed, the reaction solution was filtered through celite, washed with ethyl acetate and concentrated, and the residue was separated by a silica gel column (ethyl acetate:petroleum ether = 50%-100%) to give a crude product of **SZ-015272A7** (1.408 g), which was directly used in the next step. LCMS:$[M+1]^+$ 374.27.

Step 7: 1-isopropyl-6-methyl-*N*-(piperidin-4-yl)-1*H*-imidazo[4,5-*c*]pyridin-4-amine

**SZ-015272A7** (560 mg), dichloromethane (5 mL) and a solution of hydrogen chloride in methanol (4 M, 10 mL) were added to a 100 mL reaction flask and stirred at room temperature for 3 h. After the reaction was completed, the reaction solution was concentrated by rotary evaporation to give a crude product of **SZ-015272A8,** which was directly used in the next step without separation. LCMS: $[M+1]^+$ 274.08.

Step 8: (*S*)-1-(*tert*-butoxycarbonyl)pyrrolidin-3-yl 4-((1-isopropyl-6-methyl-1*H*-imidazo [4,5-*c*]pyridin-4-yl)amino)piperidine-1-carboxylate

(*S*)-1-*N*-*tert*-butoxycarbonyl-3-hydroxypyrrolidine (365.2 mg), acetonitrile (10 mL), triethylamine (1.5 mL) and *p*-nitrophenyl chloroformate (362.8 mg) were added to a 100 mL reaction flask and stirred at 40 °C for 2 h. The crude product in the previous step was dissolved in acetonitrile (10 mL), triethylamine was added to adjust the solution to be alkaline, and then the reaction solution of (*S*)-1-*N*-*tert*-butoxycarbonyl-3-hydroxypyrrolidine was added dropwise to the solution of **SZ-015272A8** in acetonitrile. The resulting reaction solution was stirred at room temperature for 4 h. After the reaction was completed, the solvent was removed by rotary evaporation, and the residue was separated by a short silica gel column (ethyl acetate:petroleum ether = 20%-100%) to give **SZ-015272A9** (597.6 mg). LCMS: $[M+1]^+$ 487.33.

Step 9: (*S*)-pyrrolidin-3-yl 4-((1-isopropyl-6-methyl-1*H*-imidazo[4,5-*c*]pyridin-4-yl)amino)piperidine-1-formic acid

**SZ-015272A9** (130 mg), dichloromethane (5 mL) and a solution of hydrogen chloride in methanol (4 M, 10 mL) were added to a 100 mL reaction flask and stirred at room temperature for 3 h. After the reaction was completed, the solvent was removed by rotary evaporation to give a crude product of **SZ-015272A10,** which was directly used in the next step without separation. LCMS: $[M+1]^+$ 387.02

Step 10: (*S*,*E*)-1-(4-(dimethylamino)but-2-enoyl)pyrrolidin-3-yl 4-((1-isopropyl-6-methyl-1*H*-imidazo[4,5-*c*]pyridin-4-yl)amino)piperidine-1-carboxylate

The crude product of **SZ-015272A10** in the previous step, (2*E*)-4-(dimethylamino)-2-butenoic acid hydrochloride (66.24 mg), DIPEA (0.3 mL), $T_3P$ (318 mg) and tetrahydrofuran (10 mL) were added to a 100 mL reaction flask and stirred at room temperature for 2 h. After the reaction was completed, the solvent was removed by rotary evaporation, and the residue was separated by a reverse phase column (water:acetonitrile) to give **SZ-015272** (23 mg). LCMS: $[M+1]^+$ 498.30.

$^1$H NMR (DMSO-$d_6$, 400 MHz): 8.10 (s, 1H), 6.69-6.61 (m, 1H), 6.67(s, 1H), 6.44-6.32(m, 2H), 5.18(d, *J* = 23.9 Hz, 1H), 4.60(hept, *J* = 7.0 Hz, 1H), 4.35-4.22 (m, 1H), 4.05-3.82 (m, 2H), 3.81-3.70 (m, 1H), 3.57-3.47 (m, 3H), 3.05 (d, *J*= 5.9 Hz, 2H), 2.95(br, 2H), 2.36(s, 3H), 2.17(s, 6H), 2.20-2.00 (m, 2H), 1.90-1.88 (m, 2H), 1.56-1.47 (m, 2H), 1.50 (d, *J* = 6.7 Hz, 6H).

**Example 5 SZ-015273:** (*S*,*E*)-1-(4-(dimethylamino)but-2-enoyl)pyrrolidin-3-yl 4-((3-isopropyl-6-methylimidazo[1,2-*b*]pyridazin-8-yl)amino)piperidin-1-carboxylate

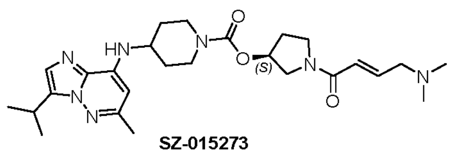

SZ-015273

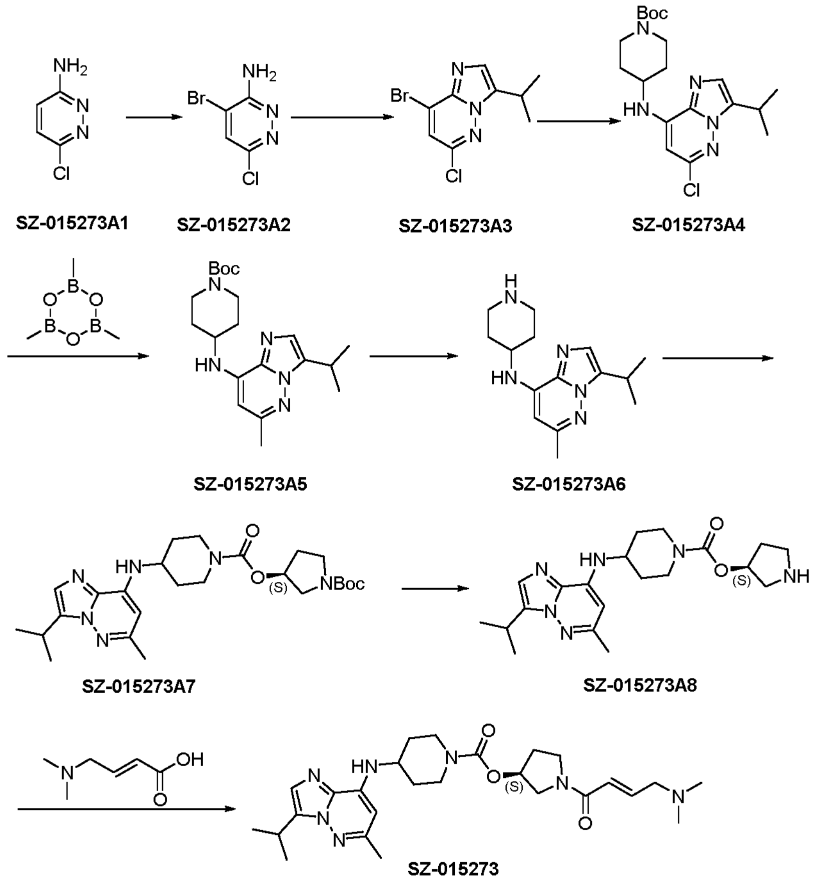

SZ-015273A1 SZ-015273A2 SZ-015273A3 SZ-015273A4

SZ-015273A5 SZ-015273A6

SZ-015273A7 SZ-015273A8

SZ-015273

Step 1: 4-bromo-6-chloropyridazin-3-amine

**SZ-015273A1** (25 g, 193 mmol) and sodium bicarbonate (32 g, 387 mmol) were dissolved in methanol (350 mL), and liquid bromine (31 g, 193 mmol) was slowly added dropwise under an ice-water bath. After the addition was completed, the reaction solution was stirred at room temperature overnight. The reaction solution was washed with saturated aqueous sodium bicarbonate solution (200 mL) and saturated sodium thiosulfate solution (100 mL), and extracted with ethyl acetate (300 mL). The organic phase was dried over sodium sulfate and concentrated by rotary evaporation to give **SZ-015273A2** (20 g, a brown solid). LCMS: $[M+H]^+$ 207.94.

Step 2: 8-bromo-6-chloro-3-isopropylimidazo[1,2-*b*]pyridazine

**SZ-015273A2** (12.7 g, 60 mmol) and 2-bromo-3-methylbutanal (10 g, 60 mmol) were dissolved in ethanol (100 mL), and the reaction solution was refluxed at 100 °C overnight. After the reaction was completed, the reaction solution was concentrated by rotary evaporation, and the residue was purified by flash silica gel column chromatography (PE:EA = 1:1; R*f* = 0.6) to give **SZ-015273A3** (1.6 g, a foamy solid). LCMS: $[M+H]^+$ 273.98.

Step 3: tert-butyl 4-((6-chloro-3-isopropylimidazo[1,2-*b*]pyridazin-8-yl)amino) piperidine-1-carboxylate

**SZ-015273A3** (1.5 g, 5.4 mmol), triethylamine (2.5 mL, 13.7 mmol) and tert-butyl 4-aminopiperidin-1-carboxylate (1.5 g, 7.1 mmol) were dissolved in dioxane (20 mL), and the reaction solution was stirred at 80 °C for 24 h.After the reaction was completed, the reaction solution was concentrated by rotary evaporation, and the residue was purified by flash silica gel column chromatography (PE:EA = 2:1; R*f* = 0.3) to give **SZ-015273A4** (1.3 g, a foamy solid). LCMS: $[M+H]^+$ 394.25.

Step 4: tert-butyl 4-((3-isopropyl-6-methylimidazo[1,2-*b*]pyridazin-8-yl)amino) piperidine-1-carboxylate

**SZ-015273A4** (1.1 g, 2.8 mmol), 2,4,6-trimethyl-1,3,5,2,4,6-trioxatriborinane (2.3 mL, 3.5 mol/L), pd(dppf)$Cl_2$.$CH_2Cl_2$ (200 mg) and potassium carbonate (1.1 g, 8.4 mmol) were mixed in dioxane (3 mL) and water (1

mL) under argon atmosphere, and then the reaction solution was heated to 100 °C and reacted for 3 h under a microwave condition. After the reaction was completed, the reaction solution was concentrated by rotary evaporation, and the residue was purified by flash silica gel column chromatography (PE:EA = 2:1; R*f* = 0.3) to give **SZ-015273A5** (200 mg, a foamy solid). LCMS: $[M+H]^+$ 374.41.

Step 5: 3-isopropyl-6-methyl-*N*-(piperidin-4-yl)imidazo[1,2-*b*]pyridazin-8-amine

**SZ-015273A5** (200 mg) was dissolved in dichloromethane (2 mL), and then TFA (1 mL) was added. The reaction solution was stirred at room temperature for 2 h. After the reaction was completed, the reaction solution was concentrated by rotary evaporation and directly used in the next step. $[M+H]^+$ 274.32.

Step 6: (*S*)-1-(*tert*-butoxycarbonyl)pyrrolidin-3-yl 4-((3-isopropyl-6-methylimidazo [1,2-*b*]pyridazin-8-yl)amino)piperidine-1-carboxylate

Tert-butyl (*S*)-3-hydroxypyrrolidine-1-carboxylate (100 mg, 0.54 mmol), *p*-nitrophenyl chloroformate (110 mg, 0.59 mmol) and triethylamine (0.2 mL) were mixed in acetonitrile (3 mL), and the reaction solution was stirred at 40 °C for 2 h. Then **SZ-015273A6** (150 mg, 0.41 mmol) was added, and the resulting reaction solution was stirred at room temperature for 2 h. The reaction solution was directly concentrated by rotary evaporation, and the residue was purified by flash silica gel column chromatography (PE:EA = 1:1; R*f* = 0.3) to give **SZ-015273A7** (80 mg, a foamy solid). LCMS: $[M+H]^+$ 487.55.

Step 7: (*S*)-pyrrolidin-3-yl 4-((3-isopropyl-6-methylimidazo [1,2-*b*]pyridazin-8-yl)amino)piperidine-1-carboxylate

**SZ-015273A7** (80 mg) was dissolved in dichloromethane (2 mL), and trifluoroacetic acid (1 mL) was slowly added dropwise. The reaction solution was stirred at room temperature for about 2 h, and then directly concentrated by rotary evaporation to give a crude product of **SZ-015273A8.LCMS:** $[M+H]^+$ 387.51.

Step 8:

**SZ-015273A8** obtained in the previous step, *trans*-4-dimethylaminocrotonic acid hydrochloride (42 mg, 0.25 mmol), DIPEA (0.2 mL, 1.36 mmol) and HATU (120 mg, 0.31 mmol) were mixed in DMF (3 mL), and the reaction solution was stirred at room temperature for 1 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015273** (14 mg, a light yellow solid). LCMS: $[M+H]^+$ 498.35.

**SZ-015273:** $^1$H NMR (400 MHz, DMSO-$d_6$) 7.22 (s, 1H), 6.97-6.95 (m, 1H), 6.68-6.51 (m, 2H), 6.09 (s, 1H), 5.22-5.16 (m, 1H), 4.08-3.96 (m, 2H), 3.85-3.48 (m, 8H), 2.96-2.91 (m, 2H), 2.53 (s, 6H), 2.38 (s, 3H), 2.21-2.03 (m, 2H), 1.86-1.84 (m, 2H), 1.52-1.50 (m, 2H), 1.29 (d, J = 7.2 Hz, 6H).

**Example 6 SZ-015268:** (*S,E*)-1-(4-(dimethylamino)but-2-enoyl)pyrrolidin-3-yl 4-((7-isopropyl-2-methylimidazo[2,1-*f*][1,2,4]triazin-4-yl)amino)piperidin-1-carboxylate

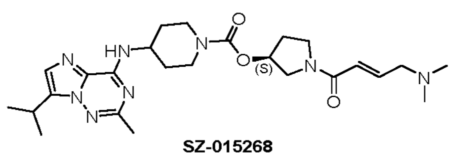

SZ-015268

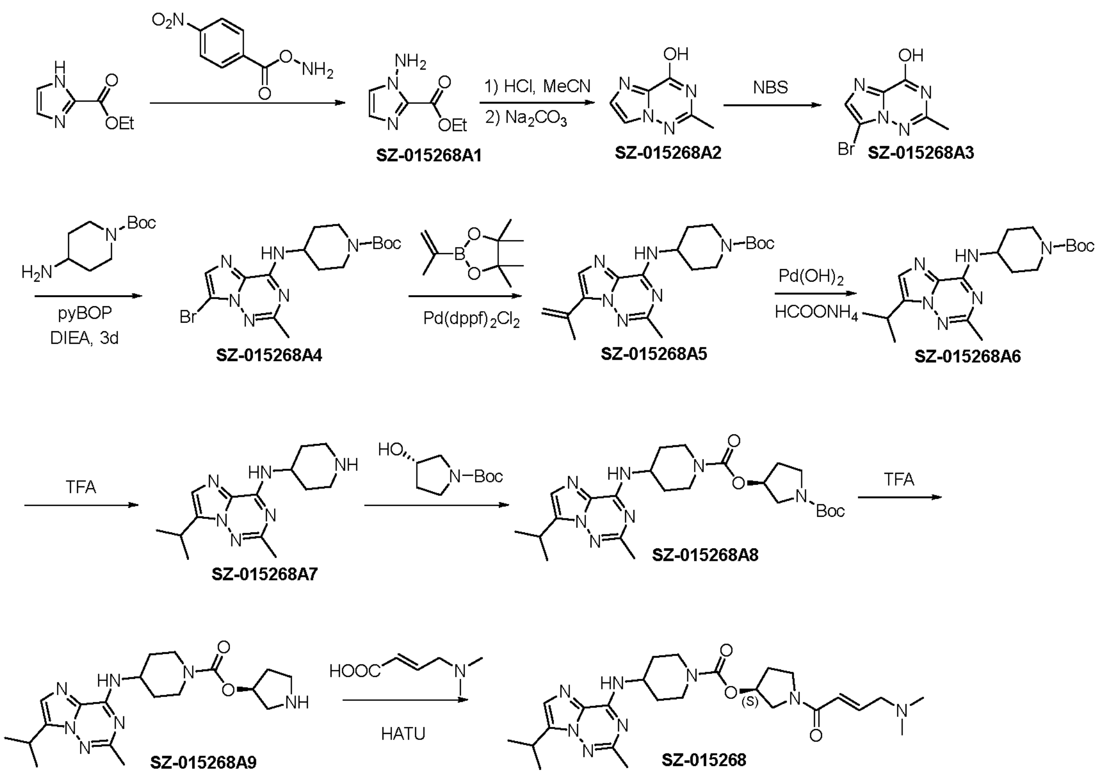


SZ-015268A1 SZ-015268A2 SZ-015268A3 SZ-015268A4 SZ-015268A5 SZ-015268A6 SZ-015268A7 SZ-015268A8 SZ-015268A9 SZ-015268

Step 1:

Ethyl imidazole-2-carboxylate (6 g) was dissolved in DMF (400 mL) in a reaction flask, and a solution of potassium *tert*-butoxide (5.282 g) in DMF (60 mL) was added dropwise to the reaction solution with stirring. The resulting reaction solution was stirred at room temperature for 1 h. *O-p*-nitrobenzoyl hydroxylamine (7.795 g) was dissolved in DMF (100 mL), and the solution was added dropwise to the reaction solution. The reaction solution changed from dark blue to brown and finally to orange as the dropwise addition proceeded. The reaction solution was stirred at room temperature overnight. After the reaction was completed, the reaction solution was concentrated, saturated sodium bicarbonate solution (400 mL) was added to quench the reaction, and dichloromethane (500 mL × 4) was added for extraction. The organic phases were combined, dried over anhydrous sodium sulfate and filtered, and the solvent was removed by rotary evaporation to give a crude product of **SZ-015268A1** (4.33 g). LCMS: $[M+1]^+$ 156.05.

Step 2:

Dry **SZ-015268A1** (4.33 g) and anhydrous acetonitrile (50 mL) were added to a three-necked flask and bubbled for 10 min with nitrogen introduced through a conduit. The reaction solution was cooled to 0 °C and stirred, and then dry hydrogen chloride gas was introduced for 20 min. The reaction solution was then stirred at 80 °C for 1 h. After the reaction was completed, the reaction solution was concentrated, and the remaining solid was slurried with diethyl ether and filtered to give a crude product as an intermediate, which was directly used in the next step without purification. The intermediate, 1,4-dioxane (40 mL), water (30 mL) and sodium carbonate (2.352 g) were added to a reaction flask and stirred at 100 °C for 1 h. After the reaction was completed, the solvent was removed under reduced pressure, and the remaining solid was diluted with acetonitrile and filtered to give a crude product of **SZ-015268A2** (5 g, a white solid) containing sodium chloride. LCMS: $[M+1]^+$ 150.98.

Step 3:

**SZ-015268A2** (2.1 g), NBS (3 g) and DMF (50 mL) were added to a reaction flask and stirred at 80 °C for 1 h. The reaction solution was concentrated by rotary evaporation under reduced pressure to remove the solvent, and the residue was diluted with dichloromethane and filtered to give a crude product of **SZ-015268A3** (2.182 g, a white solid) containing sodium chloride. LCMS: $[M+1]^+$ 230.89.

Step 4:

**SZ-015268A3** (2.182 g), 1-Boc-4-aminopiperidine (4 g), PyBOP (7.36 g), 1,2-dichloroethane (50 mL) and DIPEA (1.71 mL) were added to a reaction flask and stirred at room temperature for 3 d. After the reaction was completed, the reaction solution was filtered and washed with dichloromethane. The organic phase was concentrated by rotary evaporation to remove the solvent, and the resulting crude product was purified by flash silica gel column chromatography (EA/PE = 0%-40%) to give product **SZ-015268A4** (1.645 g). LCMS: $[M+1]^+$ 413.09.

Step 5:

**SZ-015268A4** (1.645 g), isopropenylboronic acid pinacol ester (1.3 g), $Pd(dppf)Cl_2$ (530 mg), potassium carbonate (1.6 g), 1,4-dioxane (40 mL) and water (7 mL) were added to a reaction flask and stirred at 80 °C for 3 h under nitrogen atmosphere. The solvent was removed by rotary evaporation, and the residue was diluted with ethyl acetate and filtered. The organic phase was concentrated by rotary evaporation to remove the solvent, and the resulting crude product was purified by flash silica gel column chromatography (EA/PE = 0%-30%) to give product **SZ-015268A5** (1.659 g). LCMS: $[M+1]^+$ 373.27.

Step 6:

**SZ-015268A5** (800 mg), $Pd(OH)_2$ (100 mg), ammonium formate (1 g) and methanol (10 mL) were added to a reaction flask and stirred at 80 °C for 1 h. The solvent was removed by rotary evaporation, and the residue was diluted with ethyl acetate and filtered. The organic phase was concentrated by rotary evaporation to remove the solvent, and the resulting crude product was purified by flash silica gel column chromatography (EA/PE = 0%-30%) to give product **SZ-015268A6** (597 mg). LCMS: $[M+1]^+$ 375.28.

Step 7:

**SZ-015268A6** (597 mg), dichloromethane (5 mL) and TFA (1 mL) were added to a reaction flask and stirred at room temperature for 1 h. After the reaction was completed, the reaction solution was concentrated by rotary evaporation to give a crude product of **SZ-015268A7,** which was directly used in the next step without separation. LCMS: $[M+1]^+$ 275.15.

Step 8:

(*S*)-1-*N*-*tert*-butoxycarbonyl-3-hydroxypyrrolidine (318 mg), acetonitrile (5 mL), triethylamine (1 mL) and *p*-nitrophenyl chloroformate (342 mg) were added successively to a reaction flask and stirred at 40 °C for 2 h. The crude product of **SZ-015268A7** in the previous step was dissolved in acetonitrile (5 mL), triethylamine was added to adjust the solution to be alkaline, and then the reaction solution of (*S*)-1-*N*-*tert*-butoxycarbonyl-3-hydroxypyrrolidine was added dropwise to the solution of **SZ-015268A7** in acetonitrile. The resulting reaction solution was stirred at room temperature for 4 h. After the reaction was completed, the solvent was removed by rotary evaporation, and the residue was purified by flash silica gel column chromatography (methanol:dichloromethane = 0%-5%) to give **SZ-015268A8** (422 mg). LCMS: $[M+1]^+$ 488.20

Step 9:

**SZ-015268A8** (150 mg), dichloromethane (5 mL) and TFA (1 mL) were added to a reaction flask and stirred at room temperature for 1 h. After the reaction was completed, the reaction solution was concentrated by rotary evaporation to give a crude product of **SZ-015268A9,** which was directly used in the next step without separation. LCMS: $[M+1]^+$ 388.25.

Step 10:

The crude product of **SZ-015268A9** in the previous step, (2*E*)-4-(dimethylamino)-2-butenoic acid hydrochloride (66 mg), DIPEA (0.5 mL), HATU (190 mg) and DMF (10 mL) were added to a reaction flask and stirred at room temperature for 2 h. After the reaction was completed, the solvent was removed by rotary evaporation, and the residue was separated by a reverse phase column (acetonitrile-water with 0.01% formic acid) to give **SZ-015268** (81 mg). LCMS: $[M+1]^+$ 499.22.
$^1H$ NMR (400 MHz, DMSO-$d_6$) $\delta$ 8.49 (d, *J*= 8.2 Hz, 1H), 7.33 (s, 1H), 6.73-6.49 (m, 2H), 5.20 (d, *J*= 20.3 Hz, 1H), 4.38-4.32 (m, 1H), 4.03=3.99 (m, 2H), 3.90-3.77 (m, 3H), 3.69-3.49 (m, 3H), 3.32-3.25 (m, 1H), 2.95-2.94 (m, 2H),

2.78 (s, 6H), 2.38 (s, 3H), 2.21-2.09 (m, 2H), 1.84-1.83 (m, 2H), 1.61-1.57 (m, 2H), 1.33 (d, *J*= 6.9 Hz, 6H).

Example 7 SZ-015274: (*S,E*)-1-(4-(dimethylamino)but-2-enoyl)tetrahydropyrrol-3-yl 4-((3-isopropyl-6-methyl-3*H*-imidazo[1,2-*a*]pyridin-8-yl)amino)piperidine-1-carboxylate

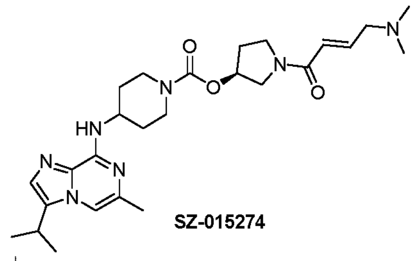

SZ-015274

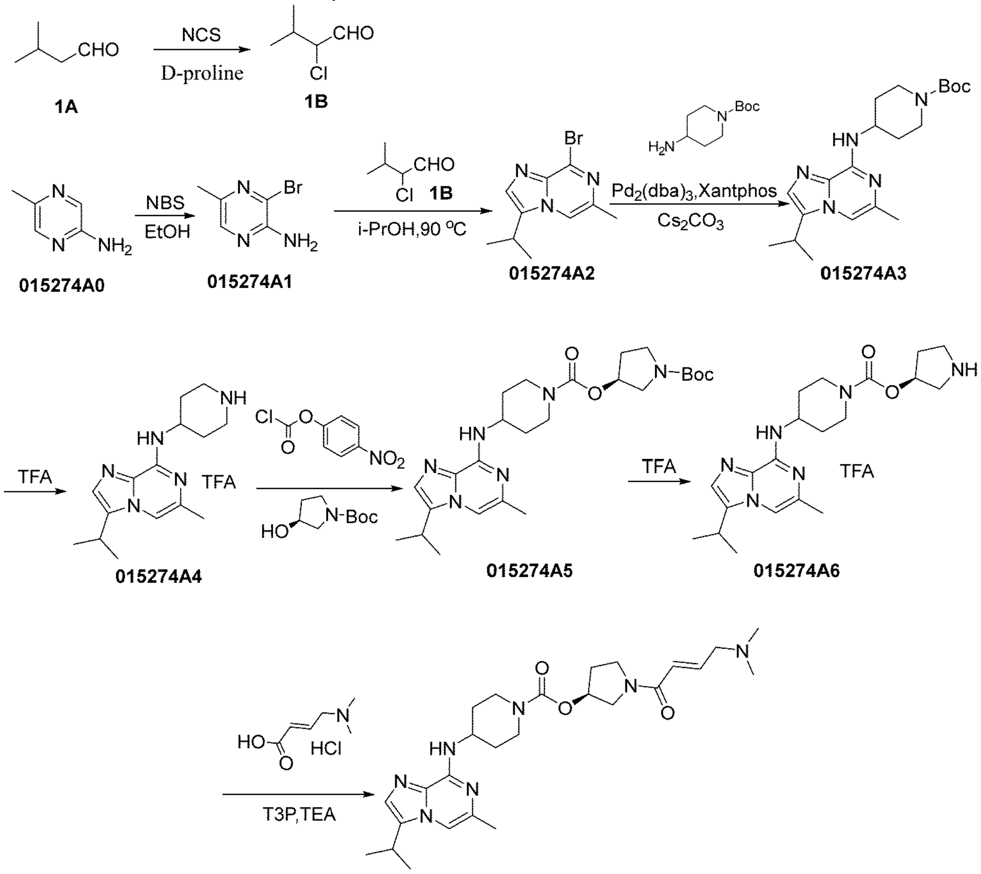


SZ-015274

Step 1:

NBS (18.6 g) was added in portions to a solution of compound **015274A0** (9.9 g, 90.8 mmol) in ethanol (110 mL) at 1-3 °C, and the resulting yellow suspension was stirred at 1-16 °C for 16 h. The resulting reaction solution was concentrated by rotary evaporation to remove the solvent, and water and ethyl acetate were added for liquid separation. After the organic phase was separated out, the aqueous phase was extracted with ethyl acetate (0.3 L × 3). The organic phases were combined, dried over anhydrous $MgSO_4$ and filtered, and the organic solvent was removed by rotary evaporation. The resulting oil was purified by column chromatography to give compound **015274A1** (4.1 g, a yellow solid). LCMS: $[M+H]^+$ 188.09/190.16

Step 2:

NCS (9.65 g, 71.8 mmol) was added in portions to a solution of compound 1A (5.6 g, 65 mmol) in dichloromethane

(110 mL) at 18 °C, and then D-proline (1.36 g, 11.8 mmol) was added in portions. The resulting reaction solution was stirred at 18 °C for 3 h. The reaction solution was diluted with petroleum ether and filtered through celite. The filtrates were combined, and the solvent was removed by rotary evaporation to give compound **1B** (7.86 g, a crude product, a yellow liquid), which was directly used in the next step.

Step 3:

A solution of compound **015274A1** (4.1 g, 21.6 mmol) and compound **1B** (7.86 g) in isopropanol (90 mL) was heated to 89 °C and stirred for 36 h. After the resulting reaction solution was concentrated by rotary evaporation to remove the organic solvent, the residue was dissolved in ethyl acetate, and the resulting solution was purified by column chromatography using a mixture of basic alumina and silica gel to give impure **015274A2** (1 g: LCMS for assisting in detection), which was directly used in the next step. LCMS: $[M+H]^+$ 254.11/256.16.

Step 4:

$Pd_2(dba)_3$ (110 mg, 0.39 mmol) and Xantphos (69 mg, 0.39 mmol) were added successively to a suspension of compound **015274A2** (1 g, 3.9 mmol), tert-butyl 4-aminopiperidin-1-carboxylate (1.1 g, 5.21 mmol) and cesium carbonate (3.9 g, 11.9 mmol) in 1,4-dioxane (16 mL). The resulting reaction solution was purged with nitrogen three times with stirring at room temperature, and then heated to 110 °C and further stirred for 16 h. The resulting dark brown reaction solution was cooled to room temperature, water was added to quench the reaction, and ethyl acetate (60 mL × 3) was added for extraction. The organic phases were combined, dried over anhydrous $MgSO_4$ and filtered, and the organic solvent was removed by rotary evaporation. The residue was purified by column chromatography to give compound **015274A3** (360 mg). LCMS: $[M+H]^+$ 374.26.

Step 5:

Trifluoroacetic acid (3 mL) was added in portions to compound **015274A3** (360 mg) at 21 °C. The resulting reaction solution was heated to 90 °C and stirred for 1 h. The reaction solution was concentrated by rotary evaporation to remove the solvent, and then the residue was dried with an oil pump for 3 min to give compound **015274A4** (190 mg), which was directly used in the next step. LCMS: $[M+H]^+$ 274.11.

Step 6:

Triethylamine (9 mL) was added in portions to a solution of p-nitrophenyl chloroformate (260 mg,1.3 mmol) and *tert-butyl* (*S*)-3-hydroxypyrrolidine-1-carboxylate (230 mg,1.3 mmol) in 1,4-dioxane (6 mL) at 21 °C. The resulting reaction solution was heated to 31 °C and stirred for 1 h. The reaction solution was added in portions to compound **015274A4** (190 mg) in the previous step. The resulting reaction solution was heated to 69 °C and further stirred for 16 h. The resulting yellow solution was cooled to room temperature, and water was added to quench the reaction. Solid sodium carbonate was added to the mixed solution until it was saturated, and ethyl acetate (30 mL × 3) was added for extraction. The organic phases were combined, dried over anhydrous $MgSO_4$ and filtered, and the organic solvent was removed by rotary evaporation. The residue was purified by column chromatography to give compound **015274A5** (190 mg). LCMS: $[M+H]^+$ 487.06.

Step 7:

Trifluoroacetic acid (3 mL) was added in portions to compound **015274A5** (190 mg) at 21 °C. The resulting reaction solution was stirred at 21 °C for 1 h. The reaction solution was concentrated by rotary evaporation to remove the organic solvent, and then the residue was dried with an oil pump for 3 min to give compound **015274A6** (160 mg), which was directly used in the next step. LCMS: $[M+H]^+$ 387.19.

Step 8:

A solution of $T_3P$ (6.39 mL, 6 mmol, 50 wt% in EtOAc) was added in portions to a suspension of compound **015274A6** (160 mg, 0.39 mmol), *trans*-4-dimethylaminocrotonic acid hydrochloride (160 mg, 1 mmol) and triethylamine (3.6 mL, 25.8 mmol) in acetonitrile (6 mL) at 21 °C. The resulting reaction solution was stirred at 18-21 °C for 16 h. Water was added to the reaction solution to quench the reaction, solid sodium carbonate was then added to the reaction solution until it was saturated, and ethyl acetate (60 mL × 3) was added for extraction. The organic phases were combined, dried over anhydrous $MgSO_4$ and filtered, and the organic solvent was removed by rotary evaporation. The residue was

purified by column chromatography to give impure **SZ-015274** (160 mg), which was then purified by preparative high performance liquid chromatography to give compound **SZ-015274** (31 mg, an off-white solid). LCMS: $[M+H]^+$ 498.31.

$^1H$ NMR (400 MHz, DMSO-$d_6$) $\delta$7.44 (s, 1H), 7.19 (s, 1H), 7.13 (d, J=8.0 Hz, 1H), 6.65-6.61 (m, 1H), 6.49-6.39 (m, 1H), 5.16 (d, J=24.0 Hz, 1H), 3.91-3.79 (m, 1H), 3.79-3.69 (m, 2H), 3.68-3.49 (m, 3H), 3.39-3.33 (m, 1H), 3.29-3.13 (m, 3H), 3.01-2.63 (m, 2H), 2.26 (d, *J=4.0* Hz, 6H), 2.21 (s, 3H), 2.16-1.89 (m, 2H), 1.86-1.76 (m, 2H), 1.58-1.39 (m, 2H), 1.27 (d, J=8.0 Hz, 6H).

**Example 8 SZ-015282:** (*R,E*)-1-(4-(dimethylamino)but-2-enoyl)pyrrolidin-3-yl 4-((8-isopropyl-2-methylpyrazolo[1,5-*a*][1,3,5]triazin-4-yl)amino)piperidine-1-carboxylate

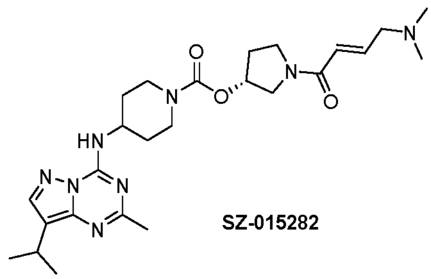

SZ-015282

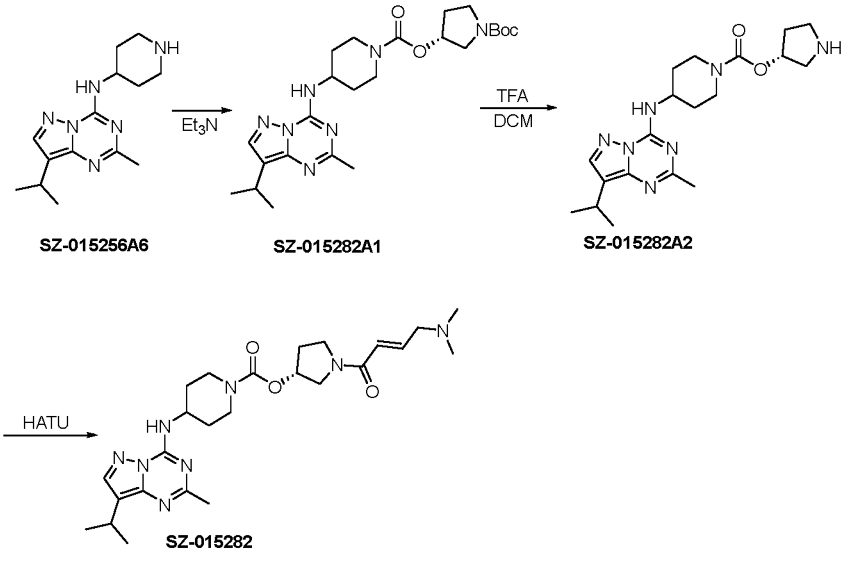


SZ-015256A6 SZ-015282A1 SZ-015282A2

SZ-015282

Step 1:

Tert-butyl (*R*)-3-hydroxypyrrolidine-1-carboxylate (219 mg, 0.8 mmol), *p*-nitrophenyl chloroformate (225 mg, 0.8 mmol) and triethylamine (0.6 mL) were mixed in acetonitrile (5 mL), and the reaction solution was stirred at 40 °C for 2 h. Then **SZ-015256A6** (200 mg, 0.8 mmol) was added, and the resulting reaction solution was stirred at room temperature for 2 h. The reaction solution was directly concentrated by rotary evaporation, and the residue was purified by flash silica gel column chromatography (PE:EA = 1:1; R*f* = 0.3) to give **SZ-015282A1** (200 mg, a foamy solid). LCMS: $[M+H]^+$ 488.37.

Step 2:

**SZ-015282A1** (110 mg) was dissolved in dichloromethane (5 mL), and trifluoroacetic acid (3 mL) was slowly added dropwise. The reaction solution was stirred at room temperature for about 2 h, and then directly concentrated by rotary evaporation to give a crude product of **SZ-015282A2** (200 mg).LCMS: $[M+H]^+$ 388.49.

Step 3:

**SZ-015282A2** obtained in the previous step, *trans*-4-dimethylaminocrotonic acid hydrochloride (37 mg, 0.22 mmol), DIPEA (0.3 mL) and HATU (111 mg, 0.29 mmol) were mixed in DMF (3 mL), and the reaction solution was stirred at room temperature for 1 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015282** (36 mg, a light yellow solid). LCMS: $[M+H]^+$ 499.53.

$^1$H NMR (400 MHz, DMSO-$d_6$) $\delta$8.54-8.52 (m, 1H), 8.01 (s, 1H), 6.68-6.63 (m, 1H), 6.47-6.39 (m, 1H), 5.22-5.16 (m, 1H), 4.29-4.01 (m, 4H), 3.90-3.39 (m, 3H), 3.15-3.06 (m, 3H), 2.96-2.92 (m, 2H), 2.41 (s, 3H), 2.23 (s, 6H), 2.10-2.02 (m, 2H), 1.85-1.83 (m, 2H), 1.67-1.66 (m, 2H), 1.29 (d, *J* = 7.2 Hz, 6H).

**Example 9 SZ-015294:** (*S*)-1-(but-2-ynoyl)pyrrolidin-3-yl 4-((8-isopropyl-2-methylpyrazolo[1,5-*a*][1,3,5]triazin-4-yl)amino)piperidine-1-carboxylate

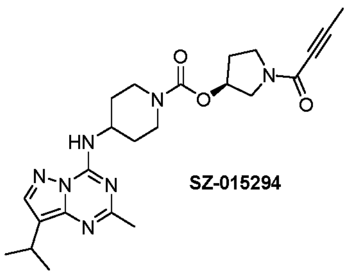

SZ-015294

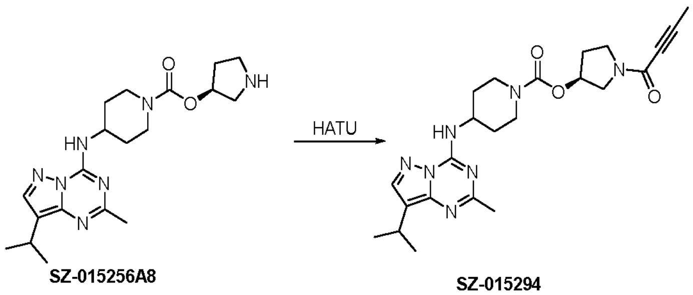


SZ-015256A8

SZ-015294

**SZ-015256A8** (156 mg, 0.41 mmol), 2-butynoic acid (34 mg, 0.41 mmol), HATU (236 mg, 0.49 mmol) and DIPEA (0.4 mL) were mixed in anhydrous DMF (3 mL) under argon atmosphere, and the reaction solution was stirred at room temperature for 2 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015294** (105 mg, a white solid). LCMS: $[M+H]^+$ 454.33.

$^1$H NMR (400 MHz, DMSO-$d_6$) $\delta$8.62-8.60 (m, 1H), 8.03 (s, 1H), 5.18-5.17 (m, 1H), 4.31-4.29 (m, 1H), 4.06-4.01 (m, 2H), 3.85-3.77 (m, 1H), 3.69-3.532 (m, 3H), 3.14-3.07 (m, 1H), 2.97-2.96 (m, 2H), 2.43 (s, 3H), 2.20-2.05 (m, 2H), 2.05 (s, 3H), 1.87-1.84 (m, 2H), 1.71-1.62 (m, 2H), 1.29 (d, *J* = 7.2 Hz, 6H).

Example 10 SZ-015284: (*R*)-1-(but-2- ynoyl)tetrahydropyrrol-3-yl 4-((3-isopropyl-5-methyl-3*H*-imidazo[4,5-*b*]pyridin-7-yl)amino)piperidine-1-carboxylate

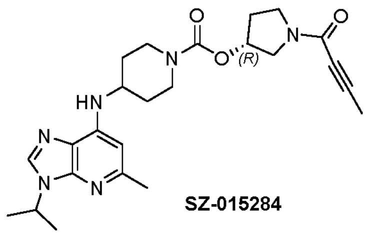

SZ-015284

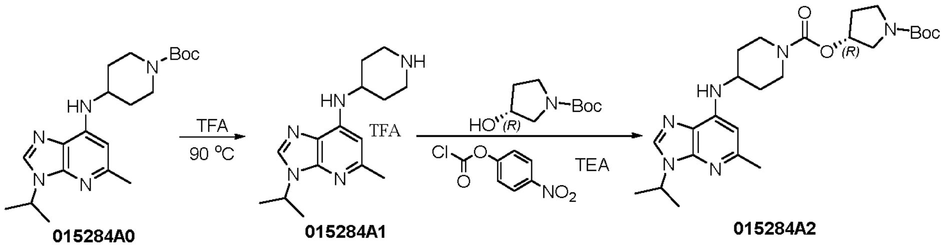


015284A0 015284A1 015284A2

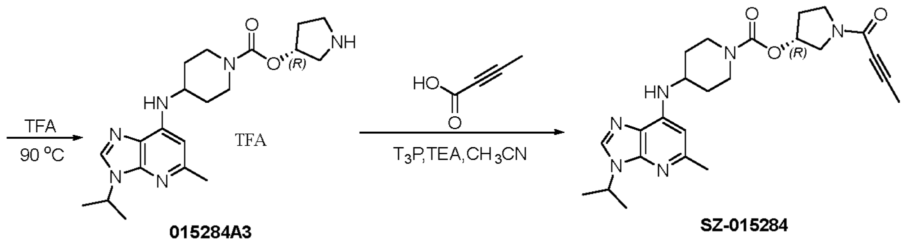


015284A3 SZ-015284

Step 1:

Trifluoroacetic acid (6 mL) was added to compound **015284A0 (015095A5)** (630 mg, 1.69 mmol), and the resulting reaction solution was heated to 90 °C and stirred for 1 h. The resulting reaction solution was concentrated by rotary evaporation to remove the solvent, and then the residue was dried for 3 min with an oil pump to give product **015284A1** (580 mg, a crude product, a tawny oil), which was directly used in the next step. LCMS: $[M+H]^+$ 274.03.

Step 2:

*P*-nitrophenyl chloroformate (1.26 g, 6 mmol) and *tert-butyl* (*R*)-3-hydroxypyrrolidine-1-carboxylate (1.26 g, 6.66 mmol) were dissolved in 1,4-dioxane (6 mL), and triethylamine (3.6 mL, 25.8 mmol) was added in portions to the reaction solution. The resulting reaction solution was heated to 31 °C and stirred for 3 h to give a light yellow suspension. The resulting reaction suspension was added in portions to a round-bottom flask containing compound **015284A1** (580 mg), and the resulting mixture was heated to 69 °C and stirred for 3 h. The resulting reaction solution was cooled to room temperature, and water was added to quench the reaction. Then solid sodium carbonate was added until the reaction solution was saturated, and ethyl acetate (60 mL × 3) was added for extraction. The organic phases were combined, dried over anhydrous $MgSO_4$ and filtered, and the organic solvent was removed by rotary evaporation. The resulting residue was purified by column chromatography to give compound **015284A2** (390 mg, 80 wt%, a light yellow oil). LCMS: $[M+H]^+$ 487.16.

Step 3:

Trifluoroacetic acid (6 mL) was added to compound **015284A2** (390 mg), and the resulting reaction solution was heated to 90 °C and stirred for 1 h. The resulting reaction solution was concentrated by rotary evaporation to remove the solvent, and then the residue was dried for 3 min with an oil pump to give product **015284A3** (360 mg, a crude product, a tawny oil), which was directly used in the next step. LCMS: $[M+H]^+$ 387.11.

Step 4:

Triethylamine (9 mL) was added in portions to a suspension of compound **015284A3** (360 mg) and 2-butynoic

acid (110 mg, 1.28 mmol) in acetonitrile (6 mL), and the resulting reaction solution was stirred at 16 °C. A solution of $T_3P$ (1.8 mL, 3 mmol, 50 wt% in EtOAc) was then added in portions to the above reaction solution. The resulting reaction solution was heated to 31 °C and stirred for 16 h. Water was added to the reaction solution to quench the reaction, solid sodium carbonate was then added to the reaction solution until it was saturated, and ethyl acetate (60 mL × 3) was added for extraction. The organic phases were combined, dried over anhydrous $MgSO_4$ and filtered, and the organic solvent was removed by rotary evaporation. The resulting residue was purified by column chromatography to give impure **SZ-015284,** which was then further purified by preparative high performance liquid chromatography to give compound **SZ-015284** (62.3 mg, a white solid). LCMS: $[M+H]^+$ 453.09.

$^1H$ NMR (400 MHz, $CD_3OD$) $\delta$ 8.09 (s, 1H), 6.38 (d, J=8.0 Hz, 1H), 6.27 (s, 1H), 5.13 (s, 1H), 4.86-4.69 (m, 1H), 3.96 (br, 3H), 3.79-3.69 (s, 1H), 3.63-3.46 (m, 2H), 3.39-3.33 (m, 1H), 2.94 (br, 2H), 2.37 (s, 3H), 2.18-2.06 (m, 2H), 2.01 (s, 3H), 1.9 (br, 2H), 1.48 (d, J=4.0 Hz, 6H), 1.39-1.31(m, 2H).

**Example 11 SZ-015289:** (*S*)-1-(but-2-ynoyl)tetrahydropyrrol-3-yl 4-((3-isopropyl-5-methyl-3*H*-imidazo[4,5-*b*]pyridin-7-yl)amino)piperidine-1-carboxylate

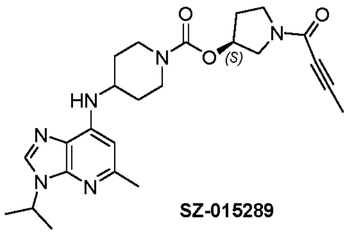

SZ-015289

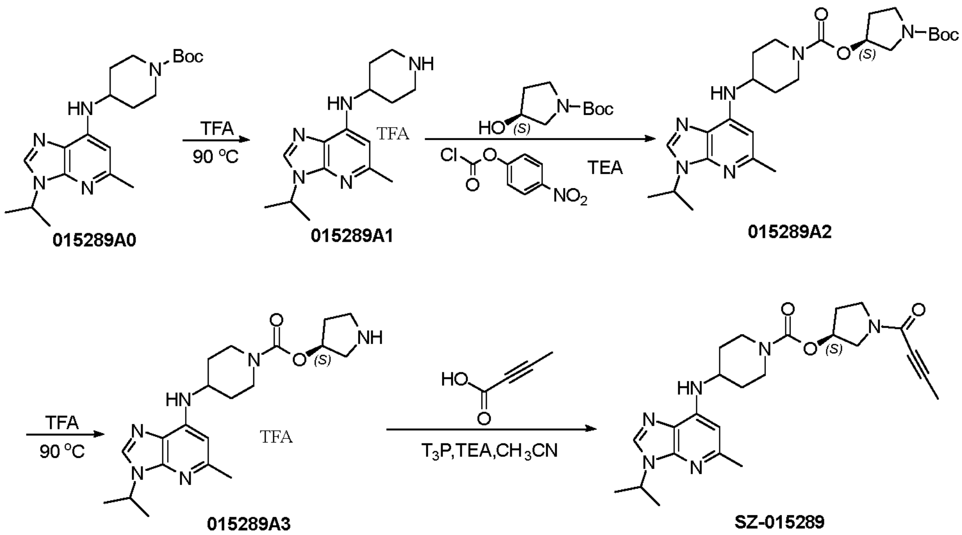


015289A0

015289A1

015289A2

015289A3

SZ-015289

Step 1:

Trifluoroacetic acid (9 mL) was added to compound **015289A0 (015095A5)** (1.6 g, 4.29 mmol), and the resulting reaction solution was heated to 90 °C and stirred for 1 h. The resulting reaction solution was concentrated by rotary evaporation to remove the solvent, and then the residue was dried for 3 min with an oil pump to give product **015289A1** (1.6 g, a crude product, a tawny oil), which was directly used in the next step. LCMS: $[M+H]^+$ 274.09.

Step 2:

*P*-nitrophenyl chloroformate (1.1 g, 5 mmol) and tert-butyl (*S*)-3-hydroxypyrrolidine-1-carboxylate (930 mg, 5 mmol) were dissolved in 1,4-dioxane (26 mL), and triethylamine (3.6 mL, 25.8 mmol) was added in portions to the reaction solution. The resulting reaction solution was heated to 31 °C and stirred for 3 h to give a light yellow suspension. The resulting reaction suspension was added in portions to a round-bottom flask containing compound **015289A1** (1.6

g), and the resulting mixture was heated to 69 °C and stirred for 3 h. The resulting reaction solution was cooled to room temperature, and water was added to quench the reaction. Then solid sodium carbonate was added until the reaction solution was saturated, and ethyl acetate (60 mL × 3) was added for extraction. The organic phases were combined, dried over anhydrous $MgSO_4$ and filtered, and the organic solvent was removed by rotary evaporation. The resulting residue was purified by column chromatography to give compound **015289A2** (1.9 g, 80 wt%, a light yellow oil). LCMS: $[M+H]^+$ 487.13.

Step 3:

Trifluoroacetic acid (9 mL) was added to compound **015289A2** (1.9 g), and the resulting reaction solution was heated to 90 °C and stirred for 1 h. The resulting reaction solution was concentrated by rotary evaporation to remove the solvent, and then the residue was dried for 3 min with an oil pump to give product **015289A3** (1.3 g, a crude product, a tawny oil), which was directly used in the next step. LCMS: $[M+H]^+$ 387.21.

Step 4:

Triethylamine (16 mL) was added to a suspension of compound **015289A3** (310 mg, 0.63 mmol) and 2-butynoic acid (110 mg, 1.28 mmol) in acetonitrile (9 mL), and the resulting reaction solution was stirred at 16 °C. A solution of $T_3P$ (1.8 mL, 3 mmol, 50 wt% in EtOAc) was then added in portions to the above reaction solution. The resulting reaction solution was heated to 31 °C and stirred for 16 h. Water was added to the reaction solution to quench the reaction, solid sodium carbonate was then added to the reaction solution until it was saturated, and ethyl acetate (60 mL × 3) was added for extraction. The organic phases were combined, dried over anhydrous $MgSO_4$ and filtered, and the organic solvent was removed by rotary evaporation. The resulting residue was purified by column chromatography to give impure **SZ-015289** (330 mg), which was then further purified by preparative high performance liquid chromatography to give compound **SZ-015289** (21.1 mg, a white solid). LCMS: $[M+H]^+$ 453.13.

$^1H$ NMR (400 MHz, $CD_3OD$) $\delta$ 8.11 (s, 1H), 6.39 (s, 1H), 6.39 (br, 1H), 5.23 (s, 1H), 4.91-4.86 (m, 1H), 4.04 (m, 2H), 3.86-3.71 (s, 3H), 3.69-3.52 (m, 2H), 3.23-3.06 (m, 2H), 2.49 (s, 3H), 2.23-2.11 (m, 2H), 2.08-2.05 (m, 2H), 2.03 (s, 3H), 1.56 (d, J=4.0 Hz, 6H), 1.53-1.39 (m, 2H).

**Example 12 SZ-015285:** (*S,E*)-1-(4-(dimethylamino)but-2-enoyl)pyrrolidin-3-yl 4-((3-ethyl-3*H*-imidazo[4,5-*b*]pyridin-7-yl)amino)piperidine-1-carboxylate

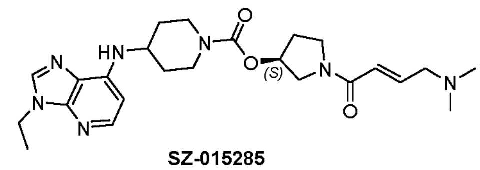

**SZ-015285**

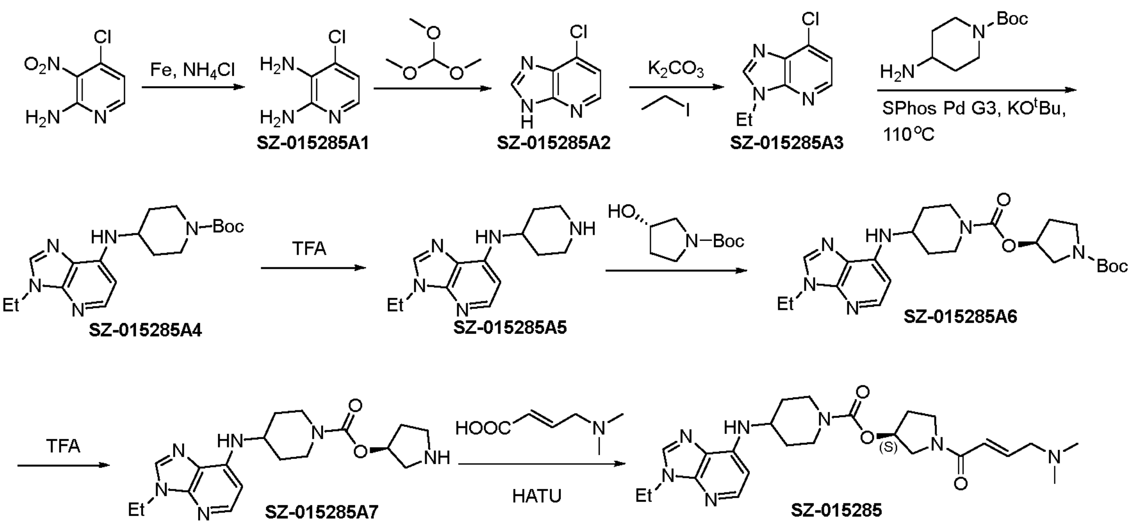


**SZ-015285A1** **SZ-015285A2** **SZ-015285A3**

**SZ-015285A4** **SZ-015285A5** **SZ-015285A6**

**SZ-015285A7** **SZ-015285**

Step 1:

2-amino-3-nitro-4-chloropyridine (3.471 g), iron powder (5.6 g), ammonium chloride (5.349 g), isopropanol (50 mL) and water (10 mL) were added to a reaction flask and stirred at 80 °C for 4 h. After the reaction was completed, the solvent was removed by rotary evaporation, and the residue was dissolved in ethyl acetate (100 mL). The resulting solution was stirred and filtered. The filtrate was collected, and the solvent was removed by rotary evaporation to give a crude product of **SZ-015285A1** (a colorless oil), which was directly used in the next step without purification. LCMS: $[M+1]^+$ 143.96.

Step 2:

The crude product of **SZ-015285A1** and triethyl orthoformate (100 mL) were added to a reaction flask, DMF was added dropwise until the suspended solids were completely dissolved, and then 12 N hydrochloric acid (2 mL) was added. The reaction solution was stirred at room temperature overnight. After the reaction was completed, the solvent was removed by rotary evaporation, and the residue was purified by flash silica gel column chromatography (EA/PE = 0%-40%) to give **productSZ-015285A2** (2.1 g). LCMS: $[M+1]^+$ 153.90.

Step 3:

**SZ-015285A2** (1 g), iodoethane (6.08 g), potassium carbonate (5.39 g) and DMSO (6 mL) were added to a reaction flask and stirred at room temperature for 12 h. After the reaction was completed, the solvent was removed by rotary evaporation, water (50 mL) was added, and ethyl acetate (50 mL × 3) was added for extraction. The organic phase was collected and dried over anhydrous sodium sulfate, and the solvent was removed by rotary evaporation. The resulting crude product was purified by flash silica gel column chromatography (EA/DCM = 0%-40%) to give product **SZ-015285A3** (423 mg, 36%). LCMS: $[M+1]^+$ 181.97.

Step 4:

**SZ-015285A3** (274.9 mg), 1-Boc-4-aminopiperidine (900 mg), potassium *tert*-butoxide (504 mg), SPhos Pd G3 (131.25 mg) and *tert*-amyl alcohol (20 mL) were added to a reaction flask and stirred at 110 °C for 6 h under nitrogen atmosphere. After the reaction was completed, the solvent was removed by rotary evaporation and the resulting crude product was purified by flash silica gel column chromatography (methanol/dichloromethane = 0%-5%) to give product **SZ-015285A4** (341 mg, 65%). LCMS: $[M+1]^+$ 346.20.

Step 5:

**SZ-015285A4** (542 mg), dichloromethane (5 mL) and TFA (1 mL) were added to a reaction flask and stirred at room temperature for 1 h. After the reaction was completed, the reaction solution was concentrated by rotary evaporation to give a crude product of **SZ-015285A5,** which was directly used in the next step without separation. LCMS: $[M+1]^+$ 245.96.

Step 6:

(*S*)-1-*N*-*tert*-butoxycarbonyl-3-hydroxypyrrolidine (294.9 mg), acetonitrile (10 mL), triethylamine (1 mL) andp-nitrophenyl chloroformate (302.3 mg) were added successively to a reaction flask and stirred at 40 °C for 2 h. The crude product of **SZ-015285A5** in the previous step was dissolved in acetonitrile (10 mL), triethylamine was added to adjust the solution to be alkaline, and then the reaction solution of (*S*)-1-*N*-*tert*-butoxycarbonyl-3-hydroxypyrrolidine was added dropwise to the solution of **SZ-015285A5** in acetonitrile. The resulting reaction solution was stirred at room temperature for 4 h. After the reaction was completed, the solvent was removed by rotary evaporation, and the residue was purified by flash silica gel column chromatography (methanol:dichloromethane = 0%-10%) to give **SZ-015285A6** (512.3 mg). LCMS: $[M+1]^+$ 459.15.

Step 7:

**SZ-015285A6** (160 mg), dichloromethane (5 mL) and TFA (1 mL) were added to a reaction flask and stirred at room temperature for 1 h. After the reaction was completed, the reaction solution was concentrated by rotary evaporation to give a crude product of **SZ-015285A7,** which was directly used in the next step without separation. LCMS: $[M+1]^+$ 359.25.

Step 8:

The crude product of **SZ-015285A7** in the previous step, (2*E*)-4-(dimethylamino)-2-butenoic acid hydrochloride (66.24 mg), DIPEA (0.3 mL), $T_3P$ (318 mg) and tetrahydrofuran (10 mL) were added to a reaction flask and stirred at room temperature for 2 h. After the reaction was completed, the solvent was removed by rotary evaporation, and the residue was separated by a reverse phase column (acetonitrile-10 mmol/L aqueous ammonium bicarbonate solution) to give **SZ-015285** (21 mg). LCMS: $[M+1]^+$ 470.19.

$^1$H NMR (400 MHz, DMSO-$d_6$) $\delta$ 8.13 (s, 1H), 7.90 (d, *J*= 5.5 Hz, 1H), 6.65-6.61 (m, 1H), 6.55 (d, *J* = 8.5 Hz, 1H), 6.44 - 6.35 (m, 2H), 5.18 (d, *J* = 23.9 Hz, 1H), 4.20 (q, *J* = 7.2 Hz, 2H), 3.99-3.97 (m, 0.3H), 3.83-3.73 (m, 0.3H), 3.65 - 3.50 (m, 2H), 3.05-3.04 (m, 2H), 2.99-2.89 (m, 2H), 2.16 (s, 6H), 2.16 - 1.92 (m, 4H), 1.46 - 1.40 (m, 5H).

**Example 13 SZ-015286:** (*S,E*)-1-(4-(dimethylamino)but-2-enoyl)pyrrolidin-3-yl 4-((3-isopropyl-3*H*-imidazo[4,5-*b*]pyridin-7-yl)amino)piperidine-1-carboxylate

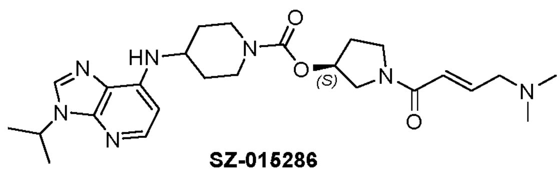

**SZ-015286**

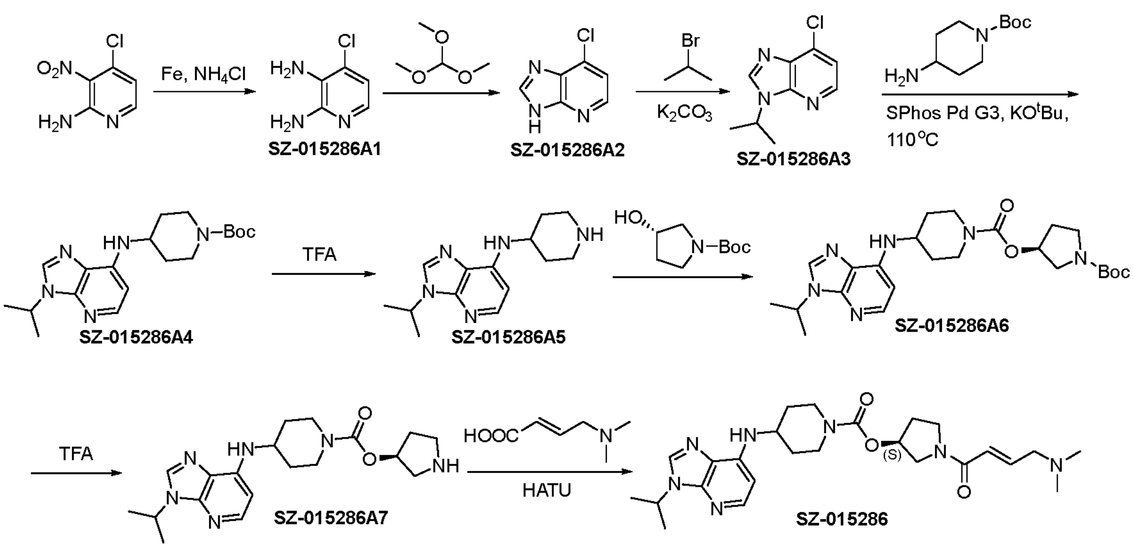


Step 1:

2-amino-3-nitro-4-chloropyridine (3.471 g), iron powder (5.6 g), ammonium chloride (5.349 g), isopropanol (50 mL) and water (10 mL) were added to a reaction flask and stirred at 80 °C for 4 h. After the reaction was completed, the solvent was removed by rotary evaporation, and the residue was dissolved in ethyl acetate (100 mL). The resulting solution was stirred and filtered. The filtrate was collected, and the solvent was removed by rotary evaporation to give a crude product of **SZ-015286A1** (a colorless oil), which was directly used in the next step without purification. LCMS: $[M+1]^+$ 142.96.

Step 2:

The crude product of **SZ-015286A1** and triethyl orthoformate (100 mL) were added to a reaction flask, DMF was added dropwise until the suspended solids were completely dissolved, and then 12 N hydrochloric acid (2 mL) was added. The reaction solution was stirred at room temperature overnight. After the reaction was completed, the solvent was removed by rotary evaporation, and the residue was purified by flash silica gel column chromatography (EA/PE = 0%-40%) to give product**SZ-015286A2** (2.1 g). LCMS: $[M+1]^+$ 153.90.

Step 3:

**SZ-015286A2** (1 g), 2-bromopropane (2.5 mL), potassium carbonate (2.8 g) and DMSO (10 mL) were added to a reaction flask and stirred at room temperature for 12 h. After the reaction was completed, the solvent was removed by rotary evaporation, water (50 mL) was added, and ethyl acetate (50 mL $\times$ 3) was added for extraction. The organic phase was collected and dried over anhydrous sodium sulfate, and the solvent was removed by rotary evaporation. The resulting crude product was purified by flash silica gel column chromatography (EA/DCM = 0%-40%) to give product **SZ-015286A3** (410 mg). LCMS: $[M+1]^+$ 196.04.

Step 4:

**SZ-015286A3** (1.44 g), 1-Boc-4-aminopiperidine (2.8 g), potassium *tert*-butoxide (1.57 g), SPhos Pd G3 (463 mg) and *tert*-amyl alcohol (40 mL) were added to a reaction flask and stirred at 110 °C for 6 h under nitrogen atmosphere. After the reaction was completed, the solvent was removed by rotary evaporation, and the resulting crude product was purified by flash silica gel column chromatography (methanol:dichloromethane = 0%-5%) to give **SZ-015286A4** (1.81 g, 69%). LCMS: $[M+1]^+$ 360.23.

Step 5:

**SZ-015286A4** (1.81 g), dichloromethane (10 mL) and TFA (3 mL) were added to a reaction flask and stirred at room temperature for 1 h. After the reaction was completed, the reaction solution was concentrated by rotary evaporation to give a crude product of **SZ-015286A5,** which was directly used in the next step without separation. LCMS: $[M+1]^+$ 260.11.

Step 6:

(*S*)-1-*N*-*tert*-butoxycarbonyl-3-hydroxypyrrolidine (973.7 mg), acetonitrile (10 mL), triethylamine (2 mL) and p-nitrophenyl chloroformate (1 g) were added successively to a reaction flask and stirred at 40 °C for 2 h. The crude product of **SZ-015286A5** in the previous step was dissolved in acetonitrile (10 mL), triethylamine was added to adjust the solution to be alkaline, and then the reaction solution of (*S*)-1-*N*-*tert*-butoxycarbonyl-3-hydroxypyrrolidine was added dropwise to the solution of **SZ-015286A5** in acetonitrile. The resulting reaction solution was stirred at room temperature for 4 h. After the reaction was completed, the solvent was removed by rotary evaporation, and the residue was purified by flash silica gel column chromatography (methanol:dichloromethane = 0%-10%) to give **SZ-015286A6** (2.18 g). LCMS: $[M+1]^+$ 473.22.

Step 7:

**SZ-015286A6** (300 mg), dichloromethane (5 mL) and TFA (1 mL) were added to a reaction flask and stirred at room temperature for 1 h. After the reaction was completed, the reaction solution was concentrated by rotary evaporation to give a crude product of **SZ-015286A7,** which was directly used in the next step without separation. LCMS: $[M+1]^+$ 372.97.

Step 8:

The crude product of **SZ-015286A7** in the previous step, (2*E*)-4-(dimethylamino)-2-butenoic acid hydrochloride (165 mg), DIPEA (0.3 mL), HATU (385 mg) and DMF (5 mL) were added to a reaction flask and stirred at room temperature for 2 h. After the reaction was completed, the solvent was removed by rotary evaporation, and the residue was separated by a reverse phase column (acetonitrile/0.1% ammonium hydroxide solution) to give **SZ-015286** (9 mg). LCMS: $[M+1]^+$ 484.28.

$^1$H NMR (400 MHz, DMSO-$d_6$) $\delta$ 8.20 (s, 1H), 7.89 (d, *J*= 5.4 Hz, 1H), 6.69-6.61 (m, 1H), 6.54 (d, *J*= 8.3 Hz, 1H), 6.49 - 6.33 (m, 2H), 5.18 (d, *J*= 23.5 Hz, 1H), 4.84-4.74 (m, 1H), 3.99 (br, 3H), 3.87 - 3.68 (m, 1H), 3.70 - 3.49 (m, 3H), 3.12 (d, *J*= 5.9 Hz, 2H), 2.96 (br, 2H), 2.21 (s, 6H), 2.16 - 1.83 (m, 4H), 1.53 (d, *J* = 6.7 Hz, 6H), 145-1.42 (m, 2H).

**Example 14 SZ-015306:** (*S*)-1-((*E*)-4-(dimethylamino)but-2-enoyl)pyrrolidin-3-yl 4-((5-(((*R*)-1-hydroxybutan-2-yl)amino)-3-isopropyl-3*H*-imidazo[4,5-*b*]pyridin-7-yl)amino)pip eridine-1-carboxylate

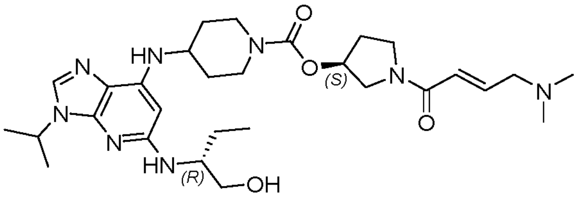

**SZ-015306**

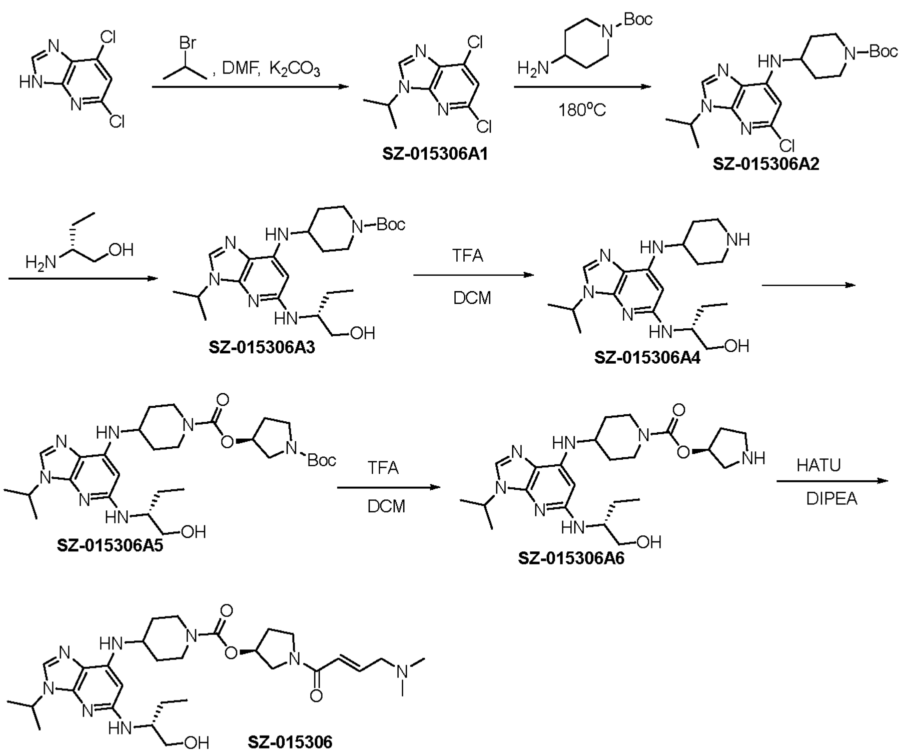


Step 1:

5,7-dichloro-1*H*-imidazo[4,5-*b*]pyridine (2.256 g), 2-bromopropane (3.37 mL), potassium carbonate (4.968 g) and DMF (30 mL) were added to a reaction flask and stirred at 60 °C for 12 h. The reaction solution was concentrated by rotary evaporation to remove the solvent, and the residue was diluted with dichloromethane and filtered. The organic phase was concentrated by rotary evaporation to remove the solvent, and the resulting crude product was purified by flash silica gel column chromatography (ethyl acetate/petroleum ether = 0%-30%) to give product **SZ-015306A1** (2.11 g). LCMS: $[M+1]^+$ 229.02.

Step 2:

**SZ-015306A1** (2.11 g) and 1-Boc-4-aminopiperidine (5 g) were added to a reaction flask and stirred at 180 °C for 2 h. After the reaction was completed, the remaining solid was purified by flash silica gel column chromatography (EA/PE = 0%-40%) to give product **SZ-015306A2** (1.025 g). LCMS: $[M+1]^+$ 394.15.

Step 3:

**SZ-015306A2** (492 mg), (R)-2-aminobutanol (223 mg), palladium acetate (14.6 mg), BINAP (40 mg), potassium *tert*-butoxide (280 mg) and toluene (20 mL) were added to a reaction flask, and the reaction solution was purged with nitrogen three times and stirred at 110 °C for 6 h. After the reaction was completed, the reaction solution was filtered and washed with dichloromethane The organic phase was concentrated by rotary evaporation to remove the solvent, and the resulting crude product was purified by flash silica gel column chromatography (methanol/dichloromethane = 0%-5%) to give product **SZ-015306A3** (374 mg). LCMS: $[M+1]^+$ 447.32.

Step 4:

**SZ-015306A3** (310 mg), dichloromethane (5 mL) and TFA (1 mL) were added to a reaction flask and stirred at room temperature for 1 h. After the reaction was completed, the reaction solution was concentrated by rotary evaporation to give a crude product of **SZ-015306A4,** which was directly used in the next step without separation. LCMS: $[M+1]^+$ 347.16.

Step 5:

(*S*)-1-*N*-*tert*-butoxycarbonyl-3-hydroxypyrrolidine (132 mg), acetonitrile (10 mL), triethylamine (1 mL) and *p*-nitrophenyl chloroformate (142 mg) were added successively to a reaction flask and stirred at 40 °C for 2 h. The crude product of **SZ-015306A4** in the previous step was dissolved in acetonitrile (5 mL), triethylamine was added to adjust the solution to be alkaline, and then the reaction solution of (*S*)-1-*N*-*tert*-butoxycarbonyl-3-hydroxypyrrolidine was added dropwise to the solution of **SZ-015306A4** in acetonitrile. The resulting reaction solution was stirred at room temperature for 4 h. After the reaction was completed, the solvent was removed by rotary evaporation, and the residue was purified by flash silica gel column chromatography (methanol/dichloromethane = 0%-5%) to give **SZ-015306A5** (199 mg). LCMS: $[M+1]^+$ 560.22.

Step 6:

**SZ-015306A5** (180 mg), dichloromethane (5 mL) and TFA (1 mL) were added to a reaction flask and stirred at room temperature for 1 h. After the reaction was completed, the reaction solution was concentrated by rotary evaporation to give a crude product of **SZ-015306A6,** which was directly used in the next step without separation. LCMS: $[M+1]^+$ 460.30.

Step 7:

The crude product of **SZ-015306A6** in the previous step, (2*E*)-4-(dimethylamino)-2-butenoic acid hydrochloride (66 mg), DIPEA (0.5 mL), HATU (152 mg) and DMF (5 mL) were added to a reaction flask and stirred at room temperature for 2 h. After the reaction was completed, the solvent was removed by rotary evaporation, and the residue was separated by a reverse phase column (acetonitrile-water with 0.1% formic acid) to give **SZ-015306** (12 mg). LCMS: $[M+1]^+$ 571.27.

$^1H$ NMR (400 MHz, DMSO-$d_6$) $\delta$ 9.95 (s, 1H), 6.73-6.57 (m, 2H), 5.84 (s, 1H), 5.18 (d, J = 20.4 Hz, 1H), 4.77-4.66 (m, 1H), 4.01-3.71 (m, 7H), 3.70-3.54 (m, 5H), 3.54-3.30 (m, 3H), 3.11 - 2.87 (m, 2H), 2.77 (s, 6H), 2.24-1.88 (m, 4H), 1.70-1.60 (m, 1H), 1.59-1.49 (d, *J* = 6.4 Hz, 6H), 1.49-1.33 (m, 3H), 0.9 (t, J = 7.4 Hz, 3H).

**Example 15 SZ-015303:** (*S,E*)-1-(4-(dimethylamino)but-2-enoyl)pyrrolidin-3-yl 4-((3-isopropyl-5-(isopropylamino)-3*H*-imidazo[4,5-*b*]pyridin-7-yl)amino)piperidine-1-carboxylate

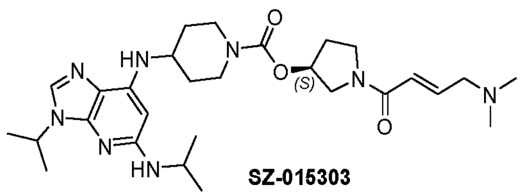

**SZ-015303**

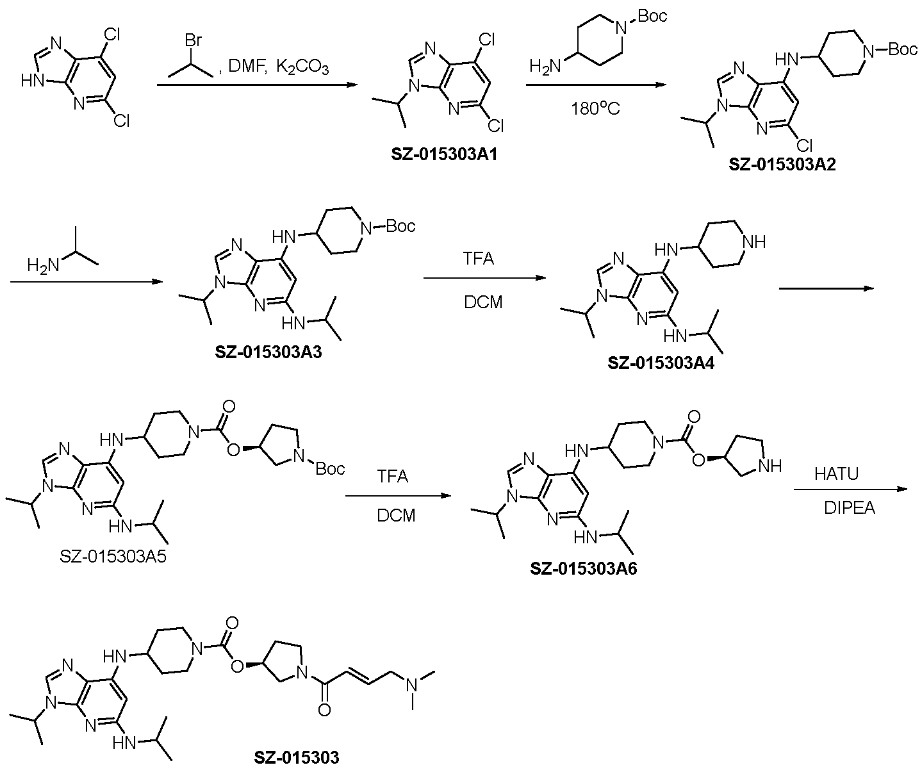


SZ-015303A1

SZ-015303A2

SZ-015303A3

SZ-015303A4

SZ-015303A5

SZ-015303A6

SZ-015303

Step 1:

5,7-dichloro-1*H*-imidazo[4,5-*b*]pyridine (2.256 g), 2-bromopropane (3.37 mL), potassium carbonate (4.968 g) and DMF (30 mL) were added to a reaction flask and stirred at 60 °C for 12 h. The reaction solution was concentrated by rotary evaporation to remove the solvent, and the residue was diluted with dichloromethane and filtered. The organic phase was concentrated by rotary evaporation to remove the solvent, and the resulting crude product was purified by flash silica gel column chromatography (ethyl acetate/petroleum ether = 0%-30%) to give product **SZ-015303A1** (2.11 g). LCMS: $[M+1]^+$ 229.02.

Step 2:

**SZ-015303A1** (2.11 g) and 1-Boc-4-aminopiperidine (5 g) were added to a reaction flask and stirred at 180 °C for 2 h. After the reaction was completed, the remaining solid was purified by flash silica gel column chromatography (EA/PE = 0%-40%) to give product **SZ-015303A2** (1.025 g). LCMS: $[M+1]^+$ 394.15.

Step 3:

**SZ-015303A2** (170 mg), isopropylamine (150 mg), palladium acetate (5 mg), BINAP (13.7 mg), potassium *tert*-butoxide (75 mg) and toluene (20 mL) were added to a reaction flask, and the reaction solution was purged with nitrogen three times and stirred at 110 °C for 6 h. After the reaction was completed, the reaction solution was filtered and washed with dichloromethane The organic phase was concentrated by rotary evaporation to remove the solvent, and the resulting crude product was purified by flash silica gel column chromatography (methanol/dichloromethane = 0%-5%) to give product **SZ-015303A3** (220 mg). LCMS: $[M+1]^+$ 417.34.

Step 4:

**SZ-015303A3** (220 mg), dichloromethane (5 mL) and TFA (1 mL) were added to a reaction flask and stirred at room temperature for 1 h. After the reaction was completed, the reaction solution was concentrated by rotary evaporation to give a crude product of **SZ-015303A4,** which was directly used in the next step without separation. LCMS: $[M+1]^+$ 317.05.

Step 5:

(*S*)-1-*N*-*tert*-butoxycarbonyl-3-hydroxypyrrolidine (112.3 mg), acetonitrile (10 mL), triethylamine (1 mL) andp-nitrophenyl chloroformate (120.93 mg) were added successively to a reaction flask and stirred at 40 °C for 2 h. The crude product of **SZ-015303A4** in the previous step was dissolved in acetonitrile (5 mL), triethylamine was added to adjust the solution to be alkaline, and then the reaction solution of (*S*)-1-*N*-*tert*-butoxycarbonyl-3-hydroxypyrrolidine was added dropwise to the solution of **SZ-015303A4** in acetonitrile. The resulting reaction solution was stirred at room temperature for 4 h. After the reaction was completed, the solvent was removed by rotary evaporation, and the residue was purified by flash silica gel column chromatography (methanol/dichloromethane = 0%-5%) to give **SZ-015303A5** (217 mg). LCMS: $[M+1]^+$ 530.22.

Step 6:

**SZ-015303A5** (217 mg), dichloromethane (5 mL) and TFA (1 mL) were added to a reaction flask and stirred at room temperature for 1 h. After the reaction was completed, the reaction solution was concentrated by rotary evaporation to give a crude product of **SZ-015303A6,** which was directly used in the next step without separation. LCMS: $[M+1]^+$ 430.15.

Step 7:

The crude product of **SZ-015303A6** in the previous step, (2*E*)-4-(dimethylamino)-2-butenoic acid hydrochloride (67 mg), DIPEA (0.5 mL), HATU (155 mg) and DMF (5 mL) were added to a reaction flask and stirred at room temperature for 2 h. After the reaction was completed, the solvent was removed by rotary evaporation, and the residue was separated by a reverse phase column (acetonitrile-water with 0.1% formic acid) to give **SZ-015303** (41 mg). LCMS: $[M+1]^+$ 541.02.

$^1$H NMR (400 MHz, DMSO-$d_6$) δ 9.66 (br, 1H), 6.84 - 6.56 (m, 3H), 5.78 (s, 1H), 5.21 (d, J = 19.6 Hz, 1H), 4.73 (br, 1H), 4.10 - 3.79 (m, 7H), 3.72 -3.51 (m, 4H), 3.04 (br, 1H), 2.80 (s, 6H), 2.32 - 1.82 (m, 4H), 1.57 (d, J = 6.4 Hz, 6H), 1.51 (d, J = 6.8 Hz, 1H), 1.45=1.42 (m, 2H), 1.20 (d, J = 6.1 Hz, 6H).

**Example 16 SZ-015307:** (*E*)-1-(4-(dimethylamino)but-2-enoyl)azetidin-3-yl 4-((3-isopropyl-5-methyl-3*H*-imidazo[4,5-*b*]pyridin-7-yl)amino)piperidine-1-carboxylate

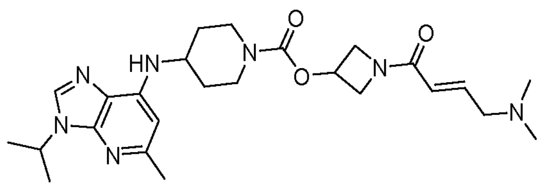

**SZ-015307**

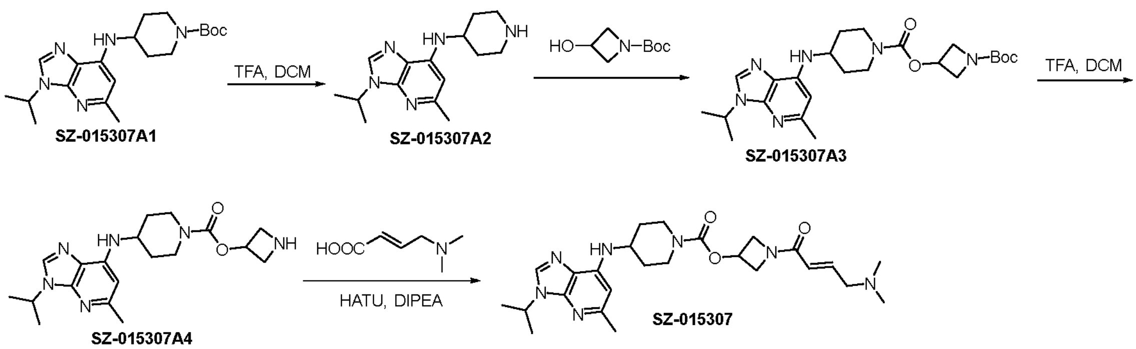


Step 1: 3-isopropyl-5-methyl-*N*-(piperidin-4-yl)-3*H*-imidazo[4,5-*b*]pyridin-7-amine

**SZ-015307A1 (015095A5)** (597 mg), dichloromethane (5 mL) and TFA (1 mL) were added to a reaction flask

and stirred at room temperature for 1 h. After the reaction was completed, the solvent was removed by rotary evaporation, and the resulting crude product of **SZ-015307A2** was directly used in the next step without separation. LCMS: $[M+H]^+$ 274.10.

Step 2: 1-(*tert*-butoxycarbonyl)azetidin-3-yl 4-((3-isopropyl-5-methyl-3*H*-imidazo [4,5-*b*]pyridin-7-yl)amino)piperidine-1-carboxylate

1-*N*-*tert*-butoxycarbonyl-3-hydroxyazetidine (260 mg), acetonitrile (5 mL), triethylamine (1 mL) and *p*-nitrophenyl chloroformate (303 mg) were added successively to a reaction flask and stirred at 40 °C for 2 h. The crude product of **SZ-015307A2** in the previous step was dissolved in acetonitrile (5 mL), triethylamine was added to adjust the solution to be alkaline, and then the reaction solution of (*S*)-1-*N*-*tert*-butoxycarbonyl-3-hydroxyazetidine was added dropwise to the solution of **SZ-015307A2** in acetonitrile. The resulting reaction solution was stirred at room temperature for 4 h. After the reaction was completed, the solvent was removed by rotary evaporation, and the residue was purified by flash silica gel column chromatography (methanol:dichloromethane = 0%-5%) to give **SZ-015307A3** (532 mg). LCMS: $[M+H]^+$ 472.28.

Step 3: azetidin-3-yl 4-((3-isopropyl-5-methyl-3*H*-imidazo[4,5-*b*]pyridin-7-yl)amino) piperidin-1-carboxylate

**SZ-015307A3** (389 mg), dichloromethane (5 mL) and TFA (1 mL) were added to a reaction flask and stirred at room temperature for 1 h. After the reaction was completed, the solvent was removed by rotary evaporation, and the resulting crude product of **SZ-015307A4** was directly used in the next step without separation. LCMS: $[M+H]^+$ 373.20.

Step 4:

The crude product of **SZ-015307A4,** (2E)-4-(dimethylamino)-2-butenoic acid hydrochloride (165 mg), DIPEA (0.5 mL), HATU (456 mg) and DMF (20 mL) were added to a reaction flask and stirred at room temperature for 2 h. After the reaction was completed, the solvent was removed by rotary evaporation, and the residue was separated by a reverse phase column (methanol/0.2% ammonium hydroxide solution) and lyophilized to give **SZ-015307** (17 mg, 23%, a white solid). LCMS: $[M+H]^+$ 484.25.

$^1$H NMR (400 MHz, DMSO-$d_6$) δ 8.13 (s, 1H), 6.61 (dt, *J*= 15.3, 6.2 Hz, 1H), 6.42 (d, *J* = 8.6 Hz, 1H), 6.30 (s, 1H), 6.13 (d, *J* = 15.4 Hz, 1H), 5.17 - 5.03 (m, 1H), 4.84 - 4.70 (m, 1H), 4.63 - 4.45 (m, 1H), 4.23 (dd, *J* = 10.9, 6.9 Hz, 1H), 4.15 (dd, *J* = 9.9, 3.6 Hz, 1H), 4.03 (br, 3H), 3.85 (dd, *J*= 11.1, 3.5 Hz, 1H), 3.05-2.95 (m, 4H), 2.40 (s, 3H), 2.17 (s, 6H), 1.99 - 1.86 (m, 2H), 1.54-1.48 (m, 8H).

**Example 17 SZ-015311:** (*S,E*)-1-(4-(dimethylamino)but-2-enoyl)pyrrolidin-3-yl 4-((3-isopropyl-5-((2-methoxyethyl)amino)-3*H*-imidazo[4,5-*b*]pyridin-7-yl)amino)piperidine-1-carboxylate

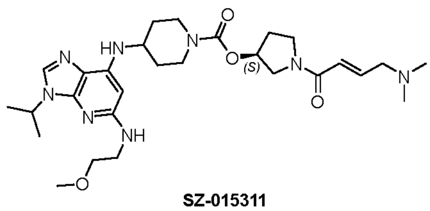

**SZ-015311**

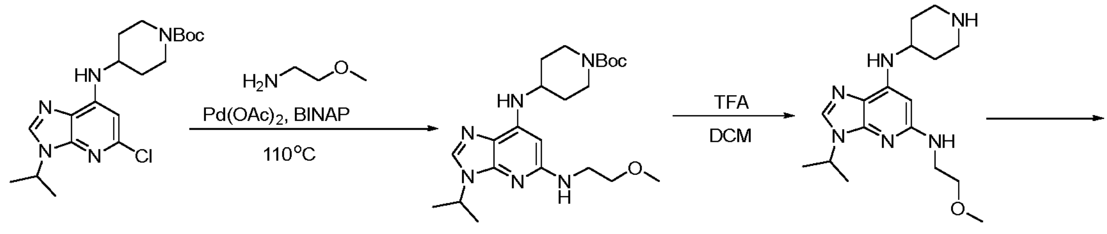


SZ-015311A1 SZ-015311A2 SZ-015311A3

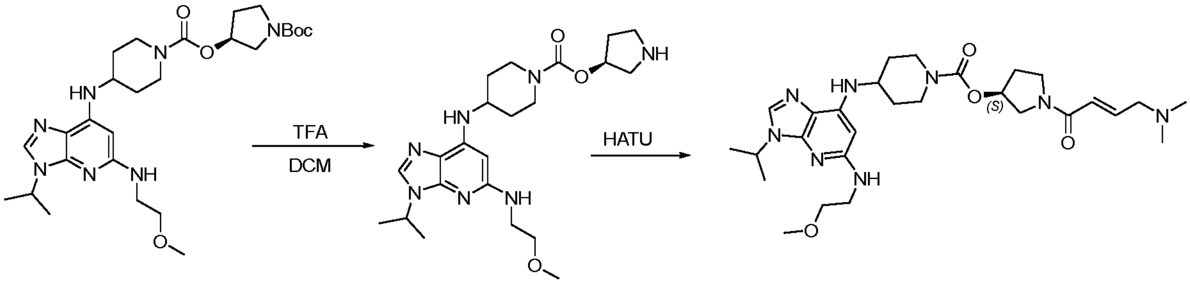


SZ-015311A4 SZ-015311A5 SZ-015311

Step 1:

**SZ-015311A1 (015306A2)** (300 mg, 0.76 mmol), 2-methoxyethylamine (190 mg, 2.5 mmol), potassium *tert*-butoxide (180 mg, 1.6 mmol), palladium acetate (50 mg) and BINAP (50 mg) were mixed in anhydrous toluene (10 mL) under argon atmosphere, and the reaction solution was stirred at 110 °C for 4 h. The reaction solution was directly concentrated by rotary evaporation, and the residue was purified by flash silica gel column chromatography (PE:EA = 1:2; R*f* = 0.3) to give **SZ-015311A2** (340 mg, a foamy solid). LCMS: $[M+H]^+$ 433.32.

Step 2:

**SZ-015311A2** (340 mg) was dissolved in dichloromethane (5 mL), and trifluoroacetic acid (32 mL) was slowly added dropwise. The reaction solution was stirred at room temperature for about 2 h, and then directly concentrated by rotary evaporation to give a crude product of **SZ-015311A3** (200 mg).LCMS: $[M+H]^+$ 333.31.

Step 3:

Tert-butyl (*S*)-3-hydroxypyrrolidine-1-carboxylate (147 mg, 0.78 mmol), *p*-nitrophenyl chloroformate (158 mg, 0.78 mmol) and triethylamine (0.5 mL) were mixed in acetonitrile (4 mL), and the reaction solution was stirred at 40 °C for 2 h. Then the crude product of **SZ-015311A3** was added, and the resulting reaction solution was stirred at room temperature overnight. The reaction solution was directly concentrated by rotary evaporation, and the residue was purified by flash silica gel column chromatography (PE:EA = 1:4; R*f* = 0.2) to give **SZ-015311A4** (220 mg, a foamy solid). LCMS: $[M+H]^+$546.21.

Step 4:

**SZ-015311A4** (220 mg) was dissolved in dichloromethane (4 mL), and trifluoroacetic acid (2 mL) was slowly added dropwise. The reaction solution was stirred at room temperature for about 2 h, and then directly concentrated by rotary evaporation to give a crude product of **SZ-015311A5** (200 mg).LCMS: $[M+H]^+$ 446.26.

Step 5:

**SZ-015311A5** obtained in the previous step, *trans*-4-dimethylaminocrotonic acid hydrochloride (55 mg, 0.33 mmol), $Et_3N$ (0.2 mL) and HATU (150 mg, 0.39 mmol) were mixed in DMF (2 mL), and the reaction solution was stirred at room temperature for 1 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015311** (26 mg, a light yellow solid). LCMS: $[M+H]^+$ 557.37.

$^1$H NMR (400 MHz, DMSO- d6) 7.76 (s, 1H), 6.69-6.61 (m, 1H), 6.41-6.34 (m, 1H), 5.98-5.95 (m, 2H), 5.59 (s, 1H), 5.20-5.14 (m, 1H), 4.65-4.58 (m, 1H), 3.98-3.91 (m, 2H), 3.84-3.72 (m, 2H), 3.64-3.46 (m, 5H), 3.43-3.40 (m, 2H), 3.29 (s, 3H), 3.06-3.05 (m, 2H), 2.93-2.92 (m, 2H), 2.17 (s, 6H), 2.09-1.90 (m, 4H), 1.50-1.48 (d, J =6.8 Hz, 6H), 1.43-1.37 (m, 2H).

**Example 18 SZ-015301:** (*S,E*)-*N*-(1-(4-(dimethylamino)but-2-enoyl)pyrrolidin-3-yl)-4-((3-isopropyl-5-methyl-3*H*-imidazo[4,5-*b*]pyridin-7-yl)amino)piperidine-1-carboxamide

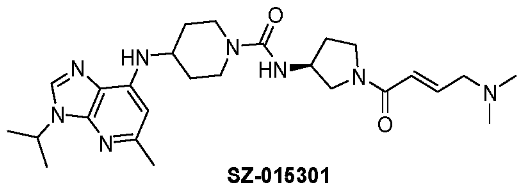

**SZ-015301**

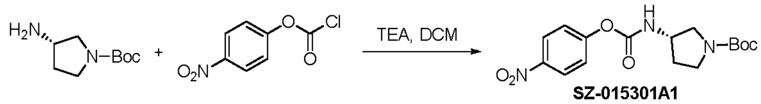


**SZ-015301A1**

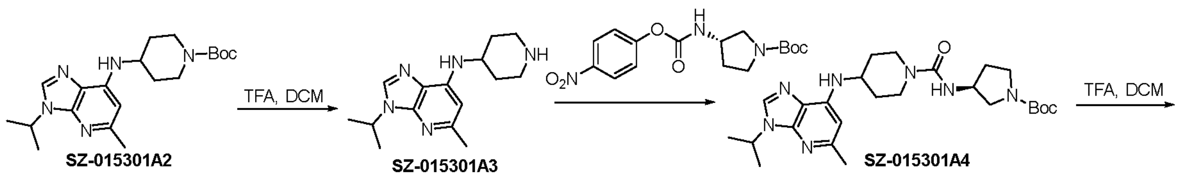


**SZ-015301A2** **SZ-015301A3** **SZ-015301A4**

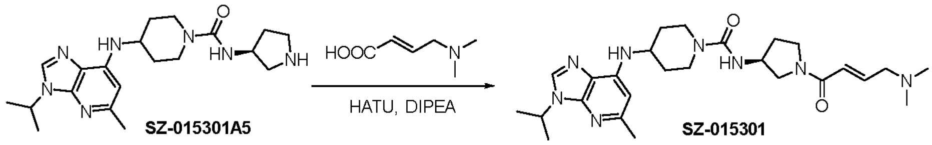


**SZ-015301A5** **SZ-015301**

Step 1:

(*S*)-1-*tert*-butoxycarbonyl-3-aminopyrrolidine (838 mg), dichloromethane (15 mL) and triethylamine (1.5 mL) were added to a reaction flask. *P*-nitrophenyl chloroformate (1.088 g) was added with stirring, and the resulting reaction solution was stirred at 60 °C for 12 h. After the reaction was completed, the solvent was removed by rotary evaporation, and the residue was purified by flash silica gel column chromatography (EA/PE = 50%-100%) to give product**SZ-015301A1** (319 mg, 22%, a light yellow oil). LCMS: $[M+1]^+$ 251.98, 295.96.

Step 2:

**SZ-015301A2 (015095A5)** (373 mg), dichloromethane (5 mL) and TFA (1 mL) were added to a reaction flask and stirred at room temperature for 1 h. After the reaction was completed, the solvent was removed by rotary evaporation, and the resulting crude product of **SZ-015301A3** was directly used in the next step without separation. LCMS: $[M+1]^+$ 274.10.

Step 3:

**SZ-015301A3** (crude product), **SZ-015301A1** (319 mg) and pyridine (10 mL) were added to a reaction flask and stirred at 100 °C for 12 h. After the reaction was completed, the solvent was removed by rotary evaporation, and the residue was purified by flash silica gel column chromatography (MeOH/DCM = 0%-10%) to give product**SZ-015301A4** (151 mg, 34 %, a light yellow oil). LCMS: $[M+1]^+$ 486.29.

Step 4:

**SZ-015301A4** (150 mg), dichloromethane (5 mL) and TFA (1 mL) were added to a reaction flask and stirred at room temperature for 1 h. After the reaction was completed, the reaction solution was concentrated by rotary evaporation to give a crude product of **SZ-015301A5,** which was directly used in the next step without separation. LCMS: $[M+1]^+$ 386.19.

Step 5:

The crude product of **SZ-015301A5,** (2E)-4-(dimethylamino)-2-butenoic acid hydrochloride (66.24 mg), DIPEA (0.3 mL), HATU (190 mg) and DMF (5 mL) were added to a reaction flask and stirred at room temperature for 2 h. After the reaction was completed, the solvent was removed by rotary evaporation, and the residue was separated by a reverse phase column (acetonitrile-0.1% aqueous ammonium hydroxide solution) and lyophilized to give **SZ-015301** (34 mg, 23%, a white solid). LCMS: $[M+1]^+$ 497.26.

$^1$H NMR (400 MHz, DMSO-$d_6$) δ 8.12 (s, 1H), 6.77-6.46 (m, 2H), 6.44-6.21 (m, 3H), 4.80-4.74 (m, 1H), 4.22-4.12 (m, 1H), 4.01 (d, J = 12.9 Hz, 3H), 3.78 (dd, J = 10.2, 6.7 Hz, 1H), 3.31-3.22 (m, 1H), 3.05 (d, J = 6.0 Hz, 2H), 2.82 (t, J = 12.6 Hz, 2H), 2.39 (s, 3H), 2.17 (s, 6H), 2.01 (m, 1H), 1.85 (m, 3H), 1.50 (d, J = 6.7 Hz, 6H), 1.41-1.28 (m, 2H).

**Example 19 SZ-015302:** (*S,E*)-*N*-(1-(4-(dimethylamino)but-2-enoyl)pyrrolidin-3-yl)-4-((3-isopropyl-5-methyl-3*H*-imidazo[4,5-*b*]pyridin-7-yl)amino)-*N*-methylpiperidine-1-carboxamide

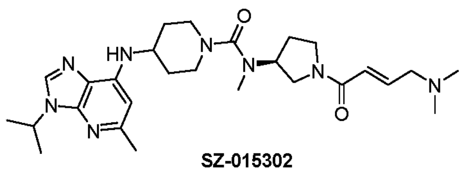

**SZ-015302**

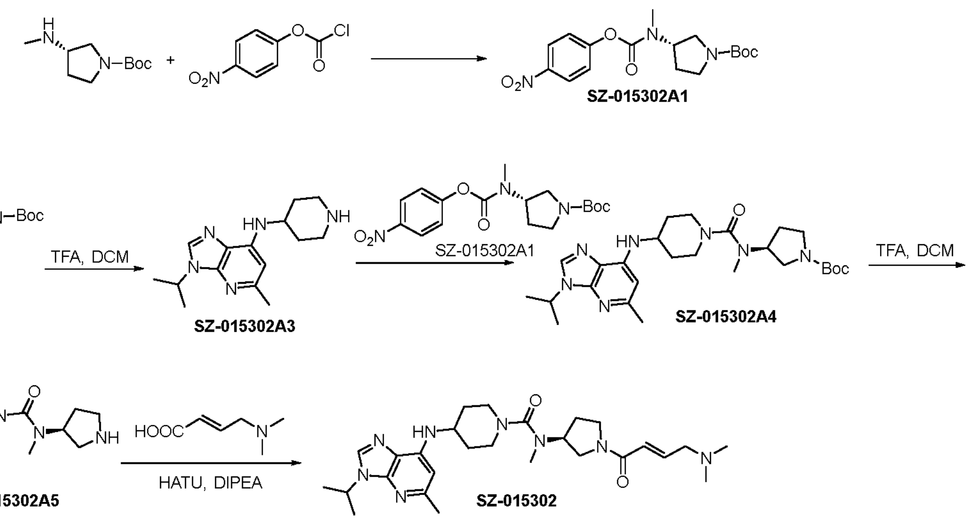


Step 1:

(*S*)-1-Boc-3-(methylamino)pyrrolidine (300 mg), dichloromethane (15 mL) and triethylamine (1.5 mL) were added to a reaction flask. *P*-nitrophenyl chloroformate (362 mg) was added with stirring, and the resulting reaction solution was stirred at 60 °C for 12 h. After the reaction was completed, the solvent was removed by rotary evaporation, and the residue was purified by flash silica gel column chromatography (EA/PE = 0%-50%) to give product**SZ-015302A1** (555 mg). LCMS: $[M+1]^+$ 266.03, 310.01.

Step 2:

**SZ-015302A2 (015095A5)** (522 mg), dichloromethane (5 mL) and TFA (1 mL) were added to a reaction flask and stirred at room temperature for 1 h. After the reaction was completed, the solvent was removed by rotary evaporation, and the resulting crude product of **SZ-015302A3** was directly used in the next step without separation. LCMS: $[M+1]^+$ 274.10.

Step 3:

**SZ-015302A3** (crude product), **SZ-015302A1** (555 mg) and pyridine (20 mL) were added to a reaction flask and stirred at 100 °C for 12 h. After the reaction was completed, the solvent was removed by rotary evaporation, and the residue was purified by flash silica gel column chromatography (MeOH/DCM = 0%-5%) to give **productSZ-015302A4** (219 mg). LCMS: $[M+1]^+$ 500.29.

Step 4:

**SZ-015302A4** (100 mg), dichloromethane (5 mL) and TFA (1 mL) were added to a reaction flask and stirred at room temperature for 1 h. After the reaction was completed, the reaction solution was concentrated by rotary evaporation to give a crude product of **SZ-015302A5,** which was directly used in the next step without separation. LCMS: $[M+1]^+$ 400.26.

Step 5:

The crude product of **SZ-015302A5** in the previous step, (2*E*)-4-(dimethylamino)-2-butenoic acid hydrochloride (41.25 mg), DIPEA (0.3 mL), HATU (114 mg) and DMF (5 mL) were added to a reaction flask and stirred at room temperature for 2 h. After the reaction was completed, the solvent was removed by rotary evaporation, and the residue was separated by a reverse phase column (acetonitrile/10 mmol/L aqueous ammonium acetate solution) and lyophilized to give **SZ-015302** (31 mg). LCMS: $[M+1]^+$ 511.28.

$^1$H NMR (400 MHz, DMSO-$d_6$) $\delta$ 8.22 (s, 1H), 6.66-6.60 (m, 1H), 6.43-6.36 (m, 2H), 6.29 (s, 1H), 4.79 - 4.75 (m, 1H), 4.31 - 4.13 (m, 1H), 4.04 (s, 1H), 3.81 - 3.60 (m, 4H), 3.54-3.43 (m, 1H), 3.25-3.23 (m, 1H), 3.09 (d, *J*= 6.1 Hz, 2H), 2.90-2.85 (m, 2H), 2.75 (d, *J* = 4.7 Hz, 3H), 2.40 (s, 3H), 2.20 (s, 6H), 2.15 - 1.85 (m, 4H), 1.51 (m, 8H).

**Example 20 SZ-015292:** (*S,E*)-N-(1-(4-(dimethylamino)but-2-enoyl)pyrrolidin-3-yl)-4-((9-isopropyl-2-methyl-9*H*-purin-6-yl)amino)piperidine-1-carboxamide

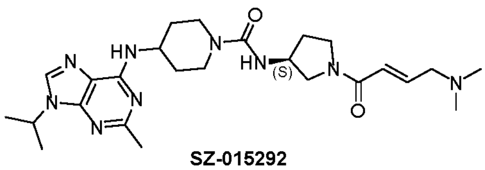

**SZ-015292**

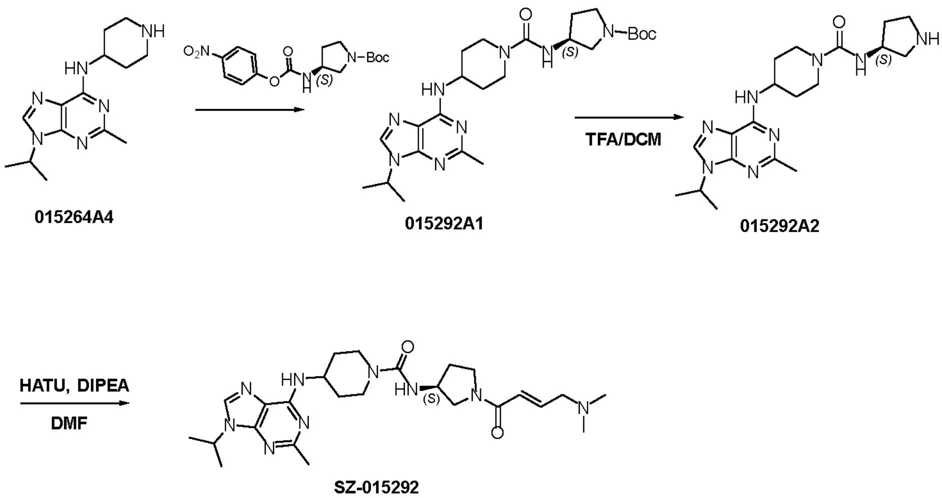


015264A4

015292A1

015292A2

SZ-015292

Step 1:

Compound **015264A4 trifluoroacetate** (750 mg, 2 mmol) was dissolved in pyridine (15 mL), and *tert-butyl* (*S*)-3-(((4-nitrophenoxy)carbonyl)amino)pyrrolidine-1-carboxylate (720 mg, 2 mmol) was added. The reaction solution was heated to 100 °C, stirred for 6 h, and concentrated by rotary evaporation under reduced pressure to give a crude product of **015292A1**, which was purified by flash silica gel column chromatography to give **015292A1** (500 mg, a white solid). LCMS: $[M+H]^+$ 487.5.

Step 2:

Compound **015292A1** (500 mg, 1 mmol) was dissolved in dichloromethane (6 mL), and trifluoroacetic acid (2 mL) was added. The reaction solution was stirred at room temperature for 0.5 h and concentrated by rotary evaporation under reduced pressure to give **015292A2 trifluoroacetate** (500 mg, 100%, a colorless oily liquid). LCMS: $[M+H]^+$ 387.4.

Step 3:

Compound **015292A2 trifluoroacetate** (500 mg, 1 mmol) was dissolved in DMF (5 mL), and DIPEA (390 mg, 3 mmol), (*E*)-4-(dimethylamino)but-2-enoic acid hydrochloride (166 mg, 1 mmol) and HATU (456 mg, 1.2 mmol) were added successively. The reaction solution was stirred at room temperature for 0.5 h and concentrated by rotary evaporation under reduced pressure to give a **crude product of SZ-015292,** which was subjected to reverse phase column chromatography (acetonitrile/water with 0.1% formic acid) and lyophilized to give **SZ-015292** (40 mg, a white solid). LCMS: $[M+H]^+$ 498.2.

$^1H$ NMR (400 MHz, DMSO-$d_6$) δ 9.60 (s, 1H), 8.41 (s, 1H), 6.75 - 6.45 (m, 3H), 4.75 (s, 1H), 4.35 - 3.85 (m, 7H), 3.72 - 3.49 (m, 6H), 2.77 (d, J = 4.4 Hz, 8H), 2.16 - 1.95 (m, 1H), 1.98 - 1.78 (m, 3H), 1.51 (d, J = 6.7 Hz, 8H).

**Example 21 SZ-015293:** (*S,E*)-1-(4-(dimethylamino)but-2-enoyl)pyrrolidin-3-yl 4-((9-isopropyl-2,8-dimethyl-9*H*-purin-6-yl)amino)piperidine-1-carboxylate

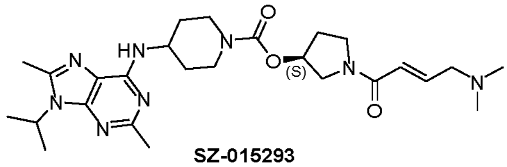

SZ-015293

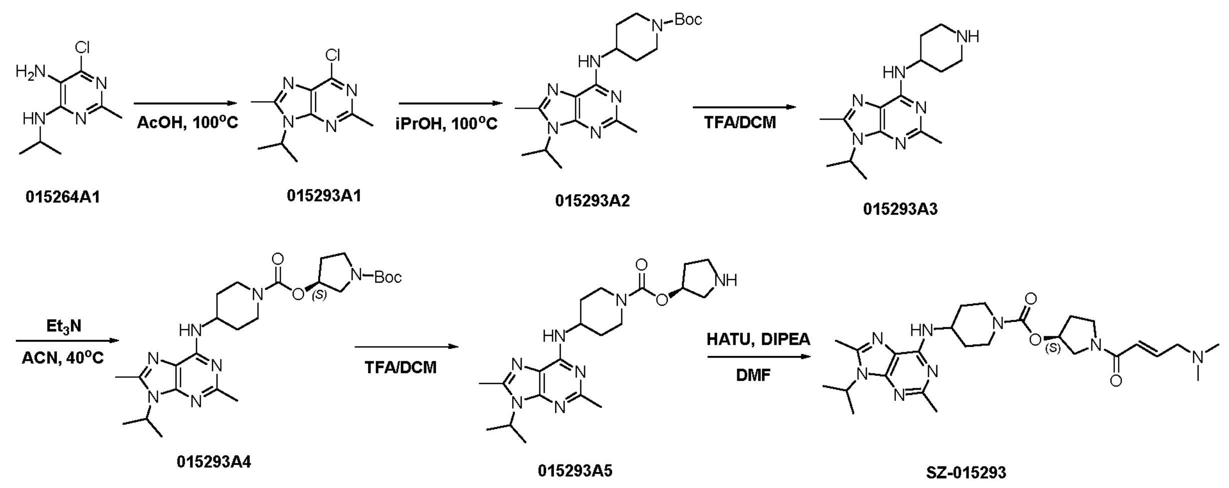


015264A1 015293A1 015293A2 015293A3

015293A4 015293A5 SZ-015293

Step 1:

**015264A1** (2.0 g, 10 mmol), trimethyl orthoacetate (9 mL) and acetic acid (4 mL) were added to a microwave flask, and the reaction solution was heated to 100 °C and reacted for 3 h under a microwave condition. After the reaction was completed, the reaction solution was cooled to room temperature, and the solvent was removed by rotary evaporation. The residue was concentrated under reduced pressure to give a crude product, which was purified by flash silica gel column chromatography to give **015293A1** (500 mg, a white solid). LCMS: $[M+H]^+$ 225.6.

Step 2:

Compound **015293A1** (500 mg, 2.23 mmol) was dissolved in isopropanol (10 mL), and *tert*-butyl-4-aminopiperidine-1-carboxylate (445 mg, 2.23 mmol) was added. The reaction solution was heated to 100 °C and stirred for 6 h, and then the reaction solution was cooled to room temperature and concentrated by rotary evaporation under reduced pressure to give a crude product of **015293A2,** which was purified by flash silica gel column chromatography to give **015293A2** (500 mg, a white solid). LCMS: $[M+H]^+$ 389.4.

Step 3:

Compound **015293A2** (500 mg, 1.29 mmol) was dissolved in dichloromethane (9 mL), and trifluoroacetic acid (3 mL) was added. The reaction solution was stirred at room temperature for 1 h and concentrated by rotary evaporation under reduced pressure to give **015293A3 trifluoroacetate** (500 mg, 100%, a colorless oily liquid). LCMS: $[M+H]^+$ 289.3.

Step 4:

Compound **015293A3 trifluoroacetate** (500 mg, 1.29 mmol) was dissolved in acetonitrile (9 mL), and triethylamine (393 mg, 3.9 mmol) and tert-butyl (*S*)-3-(((4-nitrophenoxy)carbonyl)oxo)pyrrolidine-1-carboxylate (507 mg, 1.44 mmol) were added successively. The reaction solution was stirred at room temperature for 2 h and concentrated by rotary evaporation under reduced pressure to give a **crude product of 015293A4,** which was purified by flash silica gel column chromatography to give **015293A4** (150 mg, a white solid). LCMS: $[M+H]^+$ 502.5.

Step 5:

Compound **015293A4** (150 mg, 0.3 mmol) was dissolved in dichloromethane (6 mL), and trifluoroacetic acid (2 mL) was added. The reaction solution was stirred at room temperature for 0.5 h and concentrated by rotary evaporation under reduced pressure to give **015293A5 trifluoroacetate** (150 mg, 100%, a colorless oily liquid). LCMS: $[M+H]^+$ 402.4.

Step 6:

Compound **015293A5 trifluoroacetate** (150 mg, 0.3 mmol) was dissolved in DMF (5 mL), and DIPEA (130 mg, 1 mmol), (*E*)-4-(dimethylamino)but-2-enoic acid hydrochloride (50 mg, 0.3 mmol) and HATU (137 mg, 0.36 mmol) were added successively. The reaction solution was stirred at room temperature for 0.5 h and concentrated by rotary

evaporation under reduced pressure to give a **crude product of SZ-015293,** which was subjected to reverse phase column chromatography (acetonitrile/water with 0.1% formic acid) and lyophilized to give **SZ-015293** (21.2 mg, a white solid). LCMS: $[M+H]^+$ 513.17.

$^1H$ NMR (400 MHz, DMSO-$d_6$) δ 7.26 (d, J = 8.1 Hz, 1H), 6.62 (ddd, J = 16.0, 10.5, 5.8 Hz, 1H), 6.37 (dd, J = 20.3, 15.5 Hz, 1H), 5.15 (d, J = 23.0 Hz, 1H), 4.65 (dt, J = 13.3, 6.6 Hz, 1H), 4.45-4.29 (m, 1H), 3.93 - 3.30 (m, 6H), 3.05 (d, J = 5.8 Hz, 2H), 2.95-2.90 (m, 2H), 2.51 (s, 3H), 2.49 (s, 3H), 2.15 (s, 6H), 2.02 (dd, J = 21.6, 8.3 Hz, 2H), 1.79 (s, 2H), 1.52-1.48 (t, J = 17.6 Hz, 8H).

**Example 22 SZ-015296:** (*S,E*)-1-(4-(dimethylamino)but-2-enoyl)pyrrolidin-3-yl 4-((9-ethyl-2-methyl-9*H*-purin-6-yl)amino)piperidine-1-carboxylate

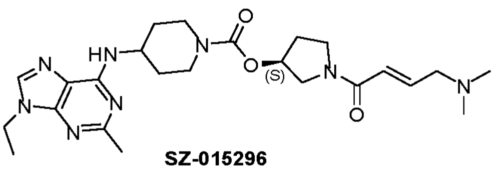

**SZ-015296**

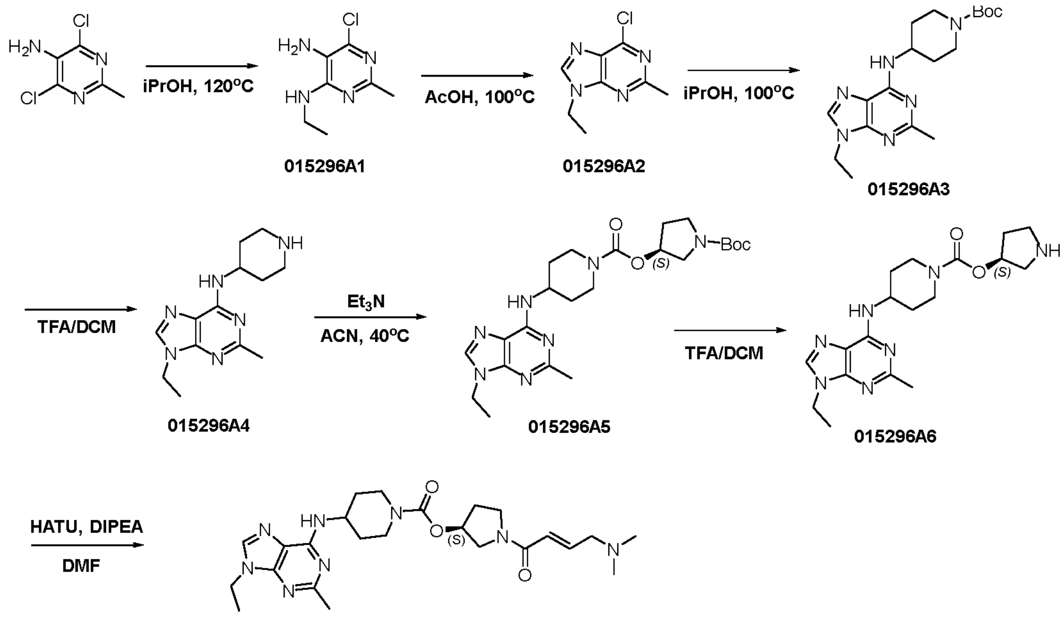


015296A1

015296A2

015296A3

015296A4

015296A5

015296A6

SZ-015296

Step 1:

4,6-dichloro-2-methylpyrimidin-5-amine (1.0 g, 5.7 mmol), ethylamine (2.6 g, 57 mmol) and isopropanol (10 mL) were added to a microwave flask, and the reaction solution was heated to 120 °C and reacted for 8 h under a microwave condition. The reaction solution was cooled to room temperature and distilled under reduced pressure to give the product **015296A1** (0.8 g, 75%, a light yellow solid). LCMS: $[M+H]^+$ 187.5.

Step 2:

**015296A1** (0.8 g, 4.3 mmol), trimethyl orthoformate (9 mL) and acetic acid (4 mL) were added to a microwave flask, and the reaction solution was heated to 100 °C and reacted for 3 h under a microwave condition. After the reaction was completed, the reaction solution was cooled to room temperature, and the solvent was removed by rotary evaporation. The residue was concentrated under reduced pressure to give a crude product, which was purified by flash silica gel column chromatography to give **015296A2** (0.8 g, 95%, a white solid). LCMS: $[M+H]^+$ 197.5.

Step 3:

Compound **015296A2** (0.8 g, 4.08 mmol) was dissolved in isopropanol (10 mL), and *tert*-butyl-4-aminopiperidine-1-carboxylate (0.82 g, 4.08 mmol) was added. The reaction solution was heated to 100 °C and stirred for 6 h, and then the reaction solution was cooled to room temperature and concentrated by rotary evaporation under reduced pressure to give a crude product of **015296A3,** which was purified by flash silica gel column chromatography to give **015296A3** (800 mg, 56%, a white solid). LCMS: $[M+H]^+$ 361.3.

Step 4:

Compound **015296A3** (800 mg, 2.22 mmol) was dissolved in dichloromethane (9 mL), and trifluoroacetic acid (3 mL) was added. The reaction solution was stirred at room temperature for 1 h and concentrated by rotary evaporation under reduced pressure to give **015296A4 trifluoroacetate** (800 mg, 100%, a colorless oily liquid). LCMS: $[M+H]^+$ 261.2.

Step 5:

Compound **015296A4 trifluoroacetate** (800 mg, 2.22 mmol) was dissolved in acetonitrile (9 mL), and triethylamine (672 mg, 6.66 mmol) and tert-butyl (*S*)-3-(((4-nitrophenoxy) carbonyl)oxo)pyrrolidine-1-carboxylate (782 mg, 2.22 mmol) were added successively. The reaction solution was stirred at room temperature for 2 h and concentrated by rotary evaporation under reduced pressure to give **a crude product of 015296A5,** which was purified by flash silica gel column chromatography to give **015296A5** (600 mg, 57%, a white solid). LCMS: $[M+H]^+$ 473.5.

Step 6:

Compound **015296A5** (400 mg, 0.85 mmol) was dissolved in dichloromethane (6 mL), and trifluoroacetic acid (2 mL) was added. The reaction solution was stirred at room temperature for 0.5 h and concentrated by rotary evaporation under reduced pressure to give **015296A6 trifluoroacetate** (400 mg, 100%, a colorless oily liquid). LCMS: $[M+H]^+$ 374.4.

Step 7:

Compound **015296A6 trifluoroacetate** (400 mg, 0.85 mmol) was dissolved in DMF (5 mL), and DIPEA (260 mg, 2 mmol), (*E*)-4-(dimethylamino)but-2-enoic acid hydrochloride (141 mg, 0.85 mmol) and HATU (388 mg, 1.02 mmol) were added successively. The reaction solution was stirred at room temperature for 0.5 h and concentrated by rotary evaporation under reduced pressure to give **a crude product of SZ-015296,** which was subjected to reverse phase column chromatography (acetonitrile/water with 0.1% formic acid) and lyophilized to give **SZ-015296** (240 mg, 58%, a white solid). LCMS: $[M+H]^+$ 485.23.

$^1$H NMR (400 MHz, DMSO-$d_6$) δ 9.60 (s, 1H), 8.37 (s, 1H), 6.80 - 6.50 (m, 2H), 5.18 (d, J = 19.6 Hz, 1H), 4.34 - 3.57 (m, 10H), 2.88 (s, 3H), 2.77 (d, J = 3.9 Hz, 6H), 2.57 (s, 3H), 2.24-190 (m, 4H), 1.62 - 1.49 (m, 2H), 1.40 (t, J = 7.3 Hz, 3H).

**Example 23 SZ-015291:** (*S,E*)-1-(4-(dimethylamino)but-2-enoyl)pyrrolidin-3-yl 4-((2-(3-hydroxyazetidin-1-yl)-9-isopropyl-9*H*-purin-6-yl)amino)piperidine-1-carboxylate

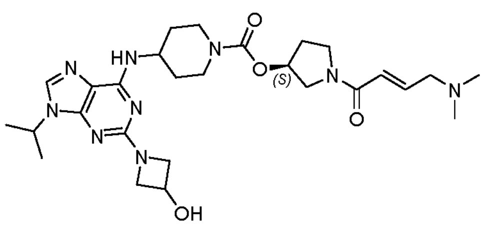

**SZ-015291**

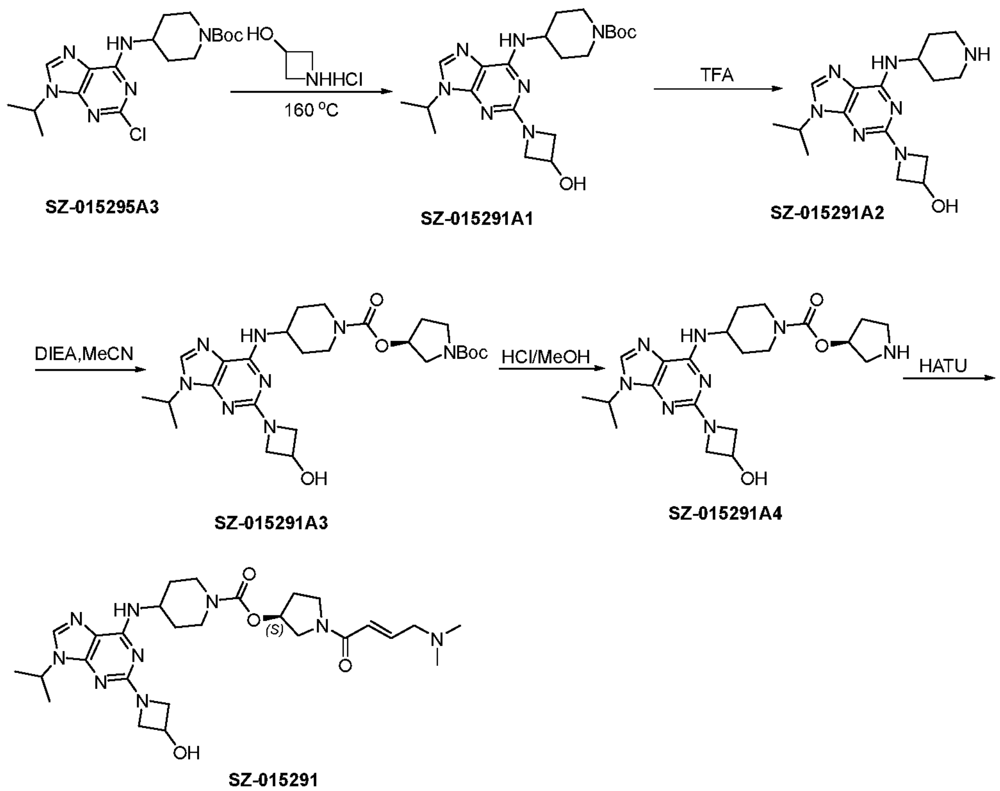


SZ-015295A3 SZ-015291A1 SZ-015291A2

SZ-015291A3 SZ-015291A4

SZ-015291

Step 1:

**SZ-015295A3** (2 g, 5.07 mmol), azetidin-3-ol hydrochloride (4 g, 10.14 mmol) and potassium carbonate (3 g, 22.05 mmol) were mixed in *N*-methylpyrrolidone (10 mL), and the reaction solution was heated to 160 °C and stirred for 8 h. The reaction solution was directly concentrated by rotary evaporation, and the residue was purified by flash silica gel column chromatography (PE:EA = 1:4; R*f* = 0.2) to give **SZ-015291A1** (300 mg, a foamy solid). LCMS: $[M+H]^+$ 432.44.

Step 2:

**SZ-015291A1** (250 mg, 0.58 mmol) was dissolved in dichloromethane (4 mL), and trifluoroacetic acid (2 mL) was then added. The reaction solution was stirred at room temperature overnight. After the reaction was completed, the reaction solution was concentrated by rotary evaporation to give a crude product of **SZ-015291A2.**

Step 3:

Tert-butyl (*S*)-3-hydroxypyrrolidine-1-carboxylate (108 mg, 0.58 mmol), *p*-nitrophenyl chloroformate (116 mg, 0.58 mmol) and triethylamine (0.2 mL) were mixed in acetonitrile (5 mL), and the reaction solution was stirred at 40 °C for 2 h. Then the crude product of **SZ-015291A2** was added, and the resulting reaction solution was stirred at room temperature overnight. The reaction solution was directly concentrated by rotary evaporation, and the residue was purified by flash silica gel column chromatography (PE:EA = 1:4; R*f* = 0.3) to give **SZ-015291A3** (200 mg, a foamy solid). LCMS: $[M+H]^+$ 545.35.

Step 4:

**SZ-015291A3** (100 mg) was dissolved in saturated methanolic hydrochloride solution, and the reaction solution was stirred at room temperature for about 2 h, and then directly concentrated by rotary evaporation to give a crude product of SZ-015291A4 (100 mg). LCMS: $[M+H]^+$ 444.39.

Step 5:

**SZ-015291A4** obtained in the previous step, *trans*-4-dimethylaminocrotonic acid hydrochloride (30 mg, 0.18

mmol), DIPEA (0.13 mL, 0.40 mmol) and HATU (83 mg, 0.21 mmol) were mixed in DMF (2 mL), and the reaction solution was stirred at room temperature for 1 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015291** (20 mg, a light yellow solid). LCMS: $[M+H]^+$ 556.31.

$^1H$ NMR (400 MHz, DMSO- d6) 7.89 (s, 1H), 7.27 (s, 1H), 6.68-6.65 (m, 2H), 5.57-5.56 (m, 1H), 5.22-5.17 (m, 1H), 4.60-4.47 (m, 2H), 4.16-4.13 (m, 2H), 3.98-3.86 (m, 2H), 3.80-3.50 (m, 8H), 2.91-2.90 (m, 2H), 2.71 (s, 6H), 2.19-2.03 (m, 2H), 1.87-1.80 (m, 2H), 1.48-1.46 (m, 8H).

**Example 24 SZ-015295:** (*S*)-1-((*E*)-4-(dimethylamino)but-2-enoyl)pyrrolidin-3-yl 4-((2-(((*R*)-1-hydroxybutan-2-yl)amino)-9-isopropyl-9*H*-purin-6-yl)amino)piperidine-1-carboxylate

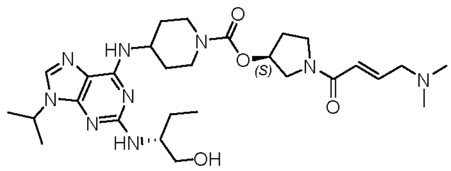

SZ-015295

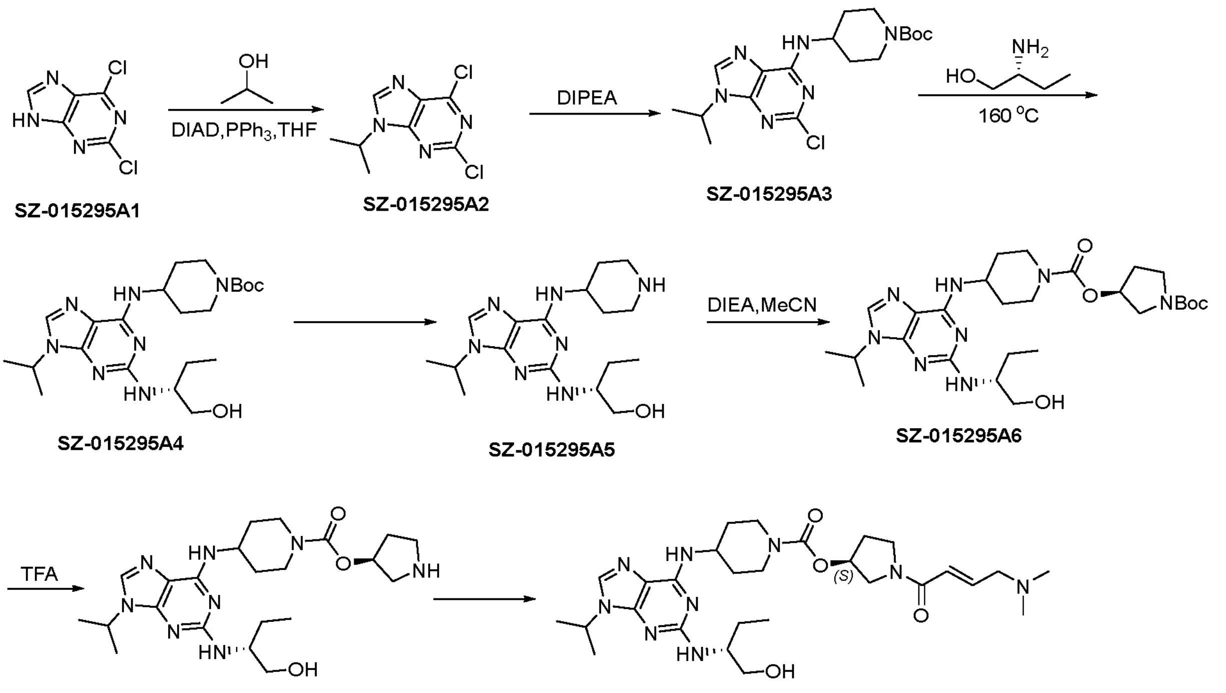


SZ-015295A1 SZ-015295A2 SZ-015295A3

SZ-015295A4 SZ-015295A5 SZ-015295A6

SZ-015295A7 SZ-015295

Step 1:

**SZ-015295A1**(10 g, 52 mmol), isopropanol (3.1 g, 52 mmol) and triphenylphosphine (13.8 g, 58 mmol) were mixed in tetrahydrofuran (150 mL), and then DIAD (11.7 g, 43 mmol) was added dropwise under an ice-water bath. The resulting reaction solution was stirred at room temperature overnight. The reaction solution was directly concentrated by rotary evaporation, and the residue was purified by flash silica gel column chromatography (PE:EA = 2:1; R*f* = 0.3) to give **SZ-015295A2** (6 g, a foamy solid). LCMS: $[M+H]^+$ 231.11.

Step 2:

**SZ-015295A2** (1 g, 4.2 mmol), tert-butyl 4-aminopiperidine-1-carboxylate (1.1 g, 5.5 mmol) and DIPEA (1.5

mL) were mixed in isopropanol (10 mL), and the reaction solution was stirred at 30 °C overnight. After the reaction was completed, the reaction solution was directly concentrated by rotary evaporation, and the residue was purified by flash silica gel column chromatography (PE:EA = 1:2; R*f* = 0.4) to give **SZ-015295A3** (1.1 g, a foamy solid). LCMS: $[M+H]^+$ 395.25.

Step 3:

**SZ-015295A3** (300 mg, 0.76 mmol) and (*R*)-2-aminobutan-1-ol (500 mg, 6.01 mmol) were mixed and then stirred at 160 °C for 8 h. After the reaction was completed, the reaction solution was dissolved in dichloromethane and purified by flash silica gel column chromatography (PE:EA = 1:4; R*f* = 0.2) to give **SZ-015295A4** (150 mg, a foamy solid). LCMS: $[M+H]^+$ 448.42.

Step 4:

**SZ-015295A4** (150 mg, 0.33 mmol) was dissolved in dichloromethane (4 mL), and trifluoroacetic acid (2 mL) was then added. The reaction solution was stirred at room temperature overnight. After the reaction was completed, the reaction solution was concentrated by rotary evaporation to give a crude product of **SZ-015295A5.**

Step 5:

Tert-butyl (*S*)-3-hydroxypyrrolidine-1-carboxylate (80 mg, 0.42 mmol), *p*-nitrophenyl chloroformate (92 mg, 0.45 mmol) and triethylamine (0.5 mL) were mixed in acetonitrile (5 mL), and the reaction solution was stirred at 40 °C for 2 h. Then the crude product of **SZ-015295A5** was added, and the resulting reaction solution was stirred at room temperature overnight. The reaction solution was directly concentrated by rotary evaporation, and the residue was purified by flash silica gel column chromatography (PE:EA = 1:4; R*f* = 0.2) to give **SZ-015295A6** (80 mg, a foamy solid). LCMS: $[M+H]^+$ 561.35.

Step 6:

**SZ-015295A6** (80 mg) was dissolved in dichloromethane (4 mL), and trifluoroacetic acid (2 mL) was slowly added dropwise. The reaction solution was stirred at room temperature for about 2 h, and then directly concentrated by rotary evaporation to give a crude product of **SZ-015295A7** (200 mg).LCMS: $[M+H]^+$ 461.38.

Step 7:

**SZ-015295A7** obtained in the previous step, *trans*-4-dimethylaminocrotonic acid hydrochloride (18.3 mg, 0.11 mmol), DIPEA (0.15 mL, 0.44 mmol) and HATU (50 mg, 0.13 mmol) were mixed in DMF (3 mL), and the reaction solution was stirred at room temperature for 1 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015295** (14 mg, a light yellow solid). LCMS: $[M+H]^+$ 572.35.

$^1$H NMR (400 MHz, DMSO- d6) 7.81 (s, 1H), 6.68-6.65 (m, 2H), 6.43-6.34 (m, 1H), 5.26-5.17 (m, 1H), 4.56-4.53 (m, 1H), 3.86-3.48 (m, 7H), 2.73-2.71 (m, 7H), 1.87-1.85 (m, 2H), 1.67-1.63 (m, 2H), 1.53-1.44 (m, 10H), 0.89 (t, J = 7.2 Hz, 3H).

**Example 25 SZ-015298:** (*S,E*)-1-(4-(dimethylamino)but-2-enoyl)pyrrolidin-3-yl 4-((9-isopropyl-2-(1*H*-pyrazol-4-yl)-9*H*-purin-6-yl)amino)piperidine-1-carboxylate

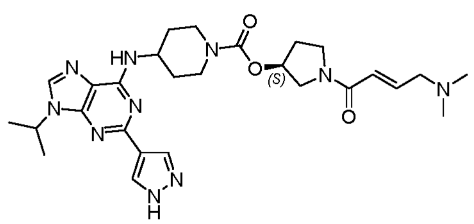

SZ-015298

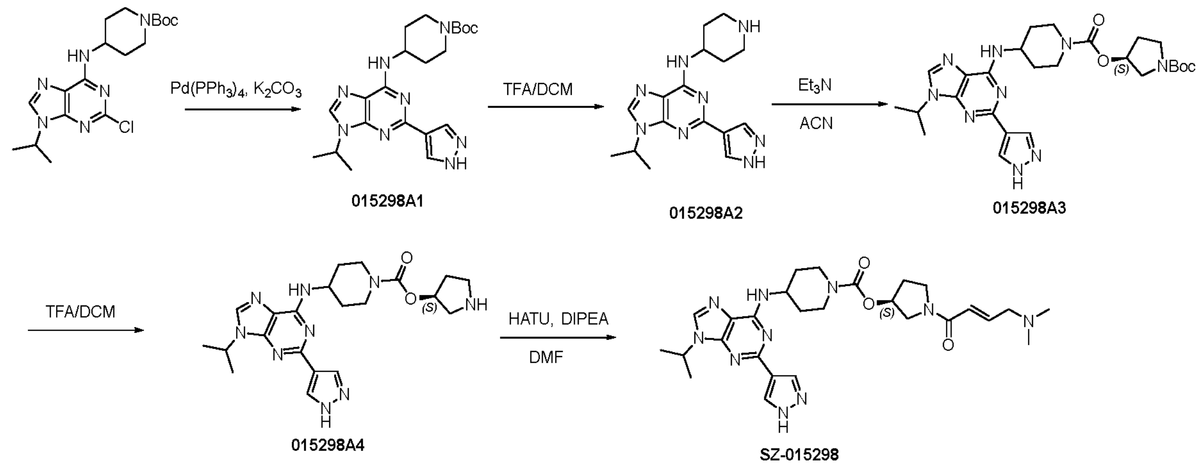


Step 1:

Tert-butyl 4-((2-chloro-9-isopropyl-9*H*-purin-6-yl)amino)piperidine-1-carboxylate (0.7 g, 1.78 mmol), 4-pyrazoleboronic acid pinacol ester (689 mg, 3.56 mmol), $Pd(PPh_3)_4$(102 mg, 5%) and potassium carbonate (737 mg, 5.34 mmol) were mixed in dioxane (6 mL) and water (2 mL) under argon atmosphere, and the reaction solution was stirred at 125 °C for 5 h under a microwave condition and then concentrated by rotary evaporation. The residue was purified by flash silica gel column chromatography to give **015298A1** (500 mg, 66%, a white solid). LCMS: $[M+H]^+$ 427.4.

Step 2:

Compound **015298A1** (500 mg, 1.17 mmol) was dissolved in dichloromethane (9 mL), and trifluoroacetic acid (3 mL) was added. The reaction solution was stirred at room temperature for 1 h and concentrated by rotary evaporation under reduced pressure to give **015298A2 trifluoroacetate** (500 mg, 100%, a colorless oily liquid). LCMS: $[M+H]^+$ 326.3.

Step 3:

Compound **015298A2 trifluoroacetate** (500 mg, 1.17 mmol) was dissolved in acetonitrile (9 mL), and triethylamine (355 mg, 3.51 mmol) and tert-butyl (*S*)-3-(((4-nitrophenoxy) carbonyl)oxo)pyrrolidine-1-carboxylate (412 mg, 1.17 mmol) were added successively. The reaction solution was stirred at room temperature for 2 h and concentrated by rotary evaporation under reduced pressure to give a crude product of **015298A3,** which was purified by flash silica gel column chromatography to give **015298A3** (330 mg, 53%, a white solid). LCMS: $[M+H]^+$ 540.5.

Step 4:

Compound **015298A3** (330 mg, 0.61 mmol) was dissolved in dichloromethane (6 mL), and trifluoroacetic acid (2 mL) was added. The reaction solution was stirred at room temperature for 0.5 h and concentrated by rotary evaporation under reduced pressure to give **015298A4 trifluoroacetate** (330 mg, 100%, a colorless oily liquid). LCMS: $[M+H]^+$ 440.4.

Step 5: (*S,E*)-1-(4-(dimethylamino)but-2-enoyl)pyrrolidin-3-yl 4-((9-isopropyl-2-(1*H*-pyrazol-4-yl)-9*H*-purin-6-yl)amino)piperidine-1-carboxylate

Compound **015298A4 trifluoroacetate** (330 mg, 0.61 mmol) was dissolved in DMF (5 mL), and DIPEA (260 mg, 2 mmol), (*E*)-4-(dimethylamino)but-2-enoic acid hydrochloride (101 mg, 0.61 mmol) and HATU (278 mg, 0.73 mmol) were added successively. The reaction solution was stirred at room temperature for 0.5 h and concentrated by rotary evaporation under reduced pressure to give a **crude product of SZ-015298,** which was subjected to reverse phase column chromatography (acetonitrile/water with 0.1% formic acid) and lyophilized to give **SZ-015298** (65 mg, 19 %, a white solid). LCMS: $[M+H]^+$ 551.25.

$^1H$ NMR (400 MHz, DMSO-$d_6$) δ 12.99 (s, 1H), 8.10 (d, J = 63.8 Hz, 3H), 7.52 (s, 1H), 6.62 (dtd, J = 10.4, 6.2, 4.3 Hz, 1H), 6.36 (dd, J = 21.2, 15.2 Hz, 1H), 5.16 (d, J = 23.2 Hz, 1H), 4.77 (dt, J = 13.4, 6.7 Hz, 1H), 4.38 (s, 1H), 4.00 (s, 2H), 3.85 - 3.67 (m, 1H), 3.67 - 3.45 (m, 3H), 3.00- 2.8 (m, 4H), 2.22 - 1.83 (m, 10H), 1.53 (d, J = 6.8 Hz, 8H).

**Example 26 SZ-015310:** (*S,E*)-1-(4-(dimethylamino)but-2-enoyl)pyrrolidin-3-yl 4-((2-ethyl-9-isopropyl-9*H*-purin-6-yl)amino)piperidine-1-carboxylate

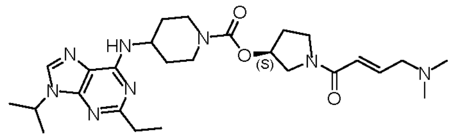

**SZ-015310**

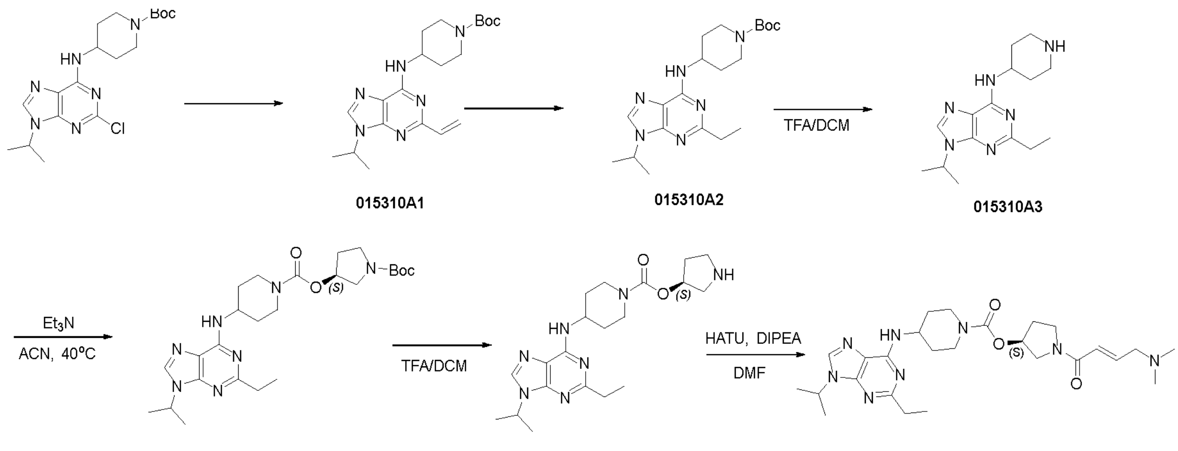


015310A1 015310A2 015310A3

015310A4 015310A5 SZ-015310

Step 1:

Tert-butyl 4-((2-chloro-9-isopropyl-9*H*-purin-6-yl)amino)piperidine-1-carboxylate (1.2 g, 3 mmol), vinylboronic acid pinacol ester (924 mg, 6 mmol), $Pd(PPh_3)_4$(173 mg, 5%) and potassium carbonate (1.24 g, 9 mmol) were mixed in dioxane (9 mL) and water (3 mL) under argon atmosphere, and the reaction solution was stirred at 125 °C for 5 h under a microwave condition and then concentrated by rotary evaporation. The residue was purified by flash silica gel column chromatography to give **015310A1** (800 mg, a white solid). LCMS: $[M+H]^+$ 387.4.

Step 2:

**015310A1** (800 mg, 2.07 mmol) was dissolved in methanol (10 mL), and ammonium formate (1304 mg, 20.7 mmol) and palladium hydroxide/carbon (80 mg) were added. The reaction solution was stirred at 60 °C for 3 h and filtered, and the organic phase was concentrated by rotary evaporation under reduced pressure to give a crude product, which was purified by flash silica gel column chromatography to give **015310A2** (600 mg, a white solid). LCMS: $[M+H]^+$ 389.4.

Step 3:

Compound **015310A2** (600 mg, 1.54 mmol) was dissolved in dichloromethane (9 mL), and trifluoroacetic acid (3 mL) was added. The reaction solution was stirred at room temperature for 1 h and concentrated by rotary evaporation under reduced pressure to give **015310A3 trifluoroacetate** (600 mg, 100%, a colorless oily liquid). LCMS: $[M+H]^+$ 289.3.

Step 4:

Compound **015310A3 trifluoroacetate** (600 mg, 1.54 mmol) was dissolved in acetonitrile (9 mL), and triethylamine (466 mg, 4.62 mmol) and tert-butyl (*S*)-3-(((4-nitrophenoxy) carbonyl)oxo)pyrrolidine-1-carboxylate (542 mg, 1.54 mmol) were added successively. The reaction solution was stirred at room temperature for 2 h and concentrated by rotary evaporation under reduced pressure to give a **crude product of 015310A4,** which was purified by flash silica gel

column chromatography to give **015310A4** (80 mg, a white solid). LCMS: [M+H]$^+$ 502.5.

Step 5:

Compound **015310A4** (80 mg, 0.16 mmol) was dissolved in dichloromethane (6 mL), and trifluoroacetic acid (2 mL) was added. The reaction solution was stirred at room temperature for 0.5 h and concentrated by rotary evaporation under reduced pressure to give **015310A5 trifluoroacetate** (80 mg, 100%, a colorless oily liquid). LCMS: [M+H]$^+$ 402.4.

Step 6:

Compound **015310A4 trifluoroacetate** (80 mg, 0.16 mmol) was dissolved in DMF (5 mL), and DIPEA (62 mg, 0.48 mmol), (*E*)-4-(dimethylamino)but-2-enoic acid hydrochloride (26.5 mg, 0.16 mmol) and HATU (73 mg, 0.192 mmol) were added successively. The reaction solution was stirred at room temperature for 0.5 h and concentrated by rotary evaporation under reduced pressure to give a **crude product of SZ-015310,** which was subjected to reverse phase column chromatography (acetonitrile/water with 0.1% formic acid) and lyophilized to give **SZ-015310** (40 mg, a white solid). LCMS: [M+H]$^+$ 513.34.

$^1$H NMR (400 MHz, DMSO-$d_6$) δ 8.16 (s, 1H), 7.45 (d, J = 6.8 Hz, 1H), 6.62 (d, J = 8.9 Hz, 2H), 5.17 (d, J = 20.6 Hz, 1H), 4.71 (dt, J = 13.4, 6.6 Hz, 1H), 4.05 - 3.42 (m, 9H), 2.91 (s, 2H), 2.65 (t, J = 8.7 Hz, 8H), 2.23 - 1.78 (m, 4H), 1.49 (d, J = 6.7 Hz, 8H), 1.23 (t, J = 7.5 Hz, 3H).

**Example 27 SZ-015308:** (*E*)-1-(4-(dimethylamino)but-2-enoyl)azetidin-3-yl 4-((9-isopropyl-2-methyl-9*H*-purin-6-yl)amino)piperidine-1-carboxylate

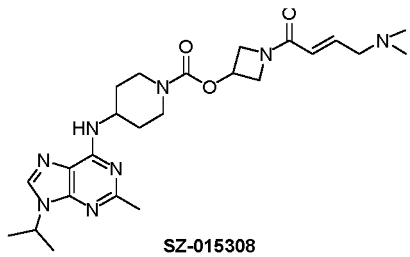

SZ-015308

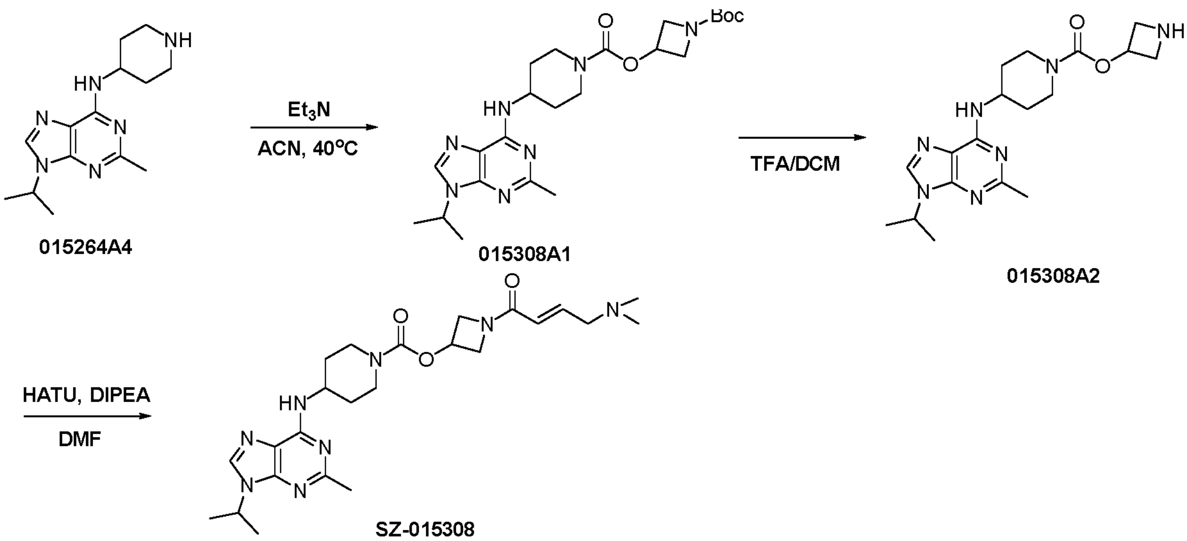


015264A4

015308A1

015308A2

SZ-015308

Step 1:

Compound **015264A4 trifluoroacetate** (374 mg, 1 mmol) was dissolved in acetonitrile (9 mL), and triethylamine (303 mg, 3 mmol) and tert-butyl 3-(((4-nitrophenoxy)carbonyl) oxo)azetidine-1-carboxylate (338 mg, 1 mmol) were added

successively. The reaction solution was stirred at room temperature for 2 h and concentrated by rotary evaporation under reduced pressure to give a **crude product of 015308A1,** which was purified by flash silica gel column chromatography to give **015308A1** (474 mg, a white solid). LCMS: $[M+H]^+$ 474.4.

Step 2:

Compound **015308A1** (474 mg, 1 mmol) was dissolved in dichloromethane (6 mL), and trifluoroacetic acid (2 mL) was added. The reaction solution was stirred at room temperature for 0.5 h and concentrated by rotary evaporation under reduced pressure to give **015308A2 trifluoroacetate** (474 mg, 100%, a colorless oily liquid). LCMS: $[M+H]^+$ 374.3.

Step 3:

Compound **015308A2 trifluoroacetate** (474 mg, 1 mmol) was dissolved in DMF (5 mL), and DIPEA (520 mg, 4 mmol), (*E*)-4-(dimethylamino)but-2-enoic acid hydrochloride (166 mg, 1 mmol) and HATU (456 mg, 1.2 mmol) were added successively. The reaction solution was stirred at room temperature for 0.5 h and concentrated by rotary evaporation under reduced pressure to give a **crude product of SZ-015308,** which was subjected to reverse phase column chromatography (acetonitrile/water with 0.1% formic acid) and lyophilized to give **SZ-015308** (180 mg, a white solid). LCMS: $[M+H]^+$ 485.11.

$^1$H NMR (400 MHz, DMSO-$d_6$) $\delta$ 8.17 (s, 1H), 7.49 (d, J = 7.8 Hz, 1H), 6.58 (m, 1H), 6.38 (d, J = 15.4 Hz, 1H), 5.15-5.10 (m, 1H), 4.69 (dd, J = 13.3, 6.9 Hz, 1H), 4.59 - 4.48 (m, 1H), 4.25 (dd, J = 11.4, 6.8 Hz, 1H), 4.17 (d, J = 6.4 Hz, 1H), 4.01 (s, 2H), 3.86 (d, J = 6.9 Hz, 3H), 3.02 (s, 1H), 2.96 - 2.83 (m, 1H), 2.76 (s, 7H), 2.42 (s, 3H), 1.86 (s, 2H), 1.48 (d, J = 6.7 Hz, 8H).

**Example 28 SZ-015317:** (*S*)-1-((*E*)-4-(dimethylamino)but-2-enoyl)pyrrolidin-3-yl 4-((6-(((*R*)-1-hydroxybutan-2-yl)amino)-3-isopropylimidazo[1,2-*b*]pyridazin-8-yl)amino) piperidine-1-carboxylate

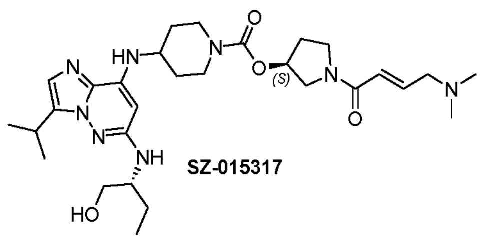

SZ-015317

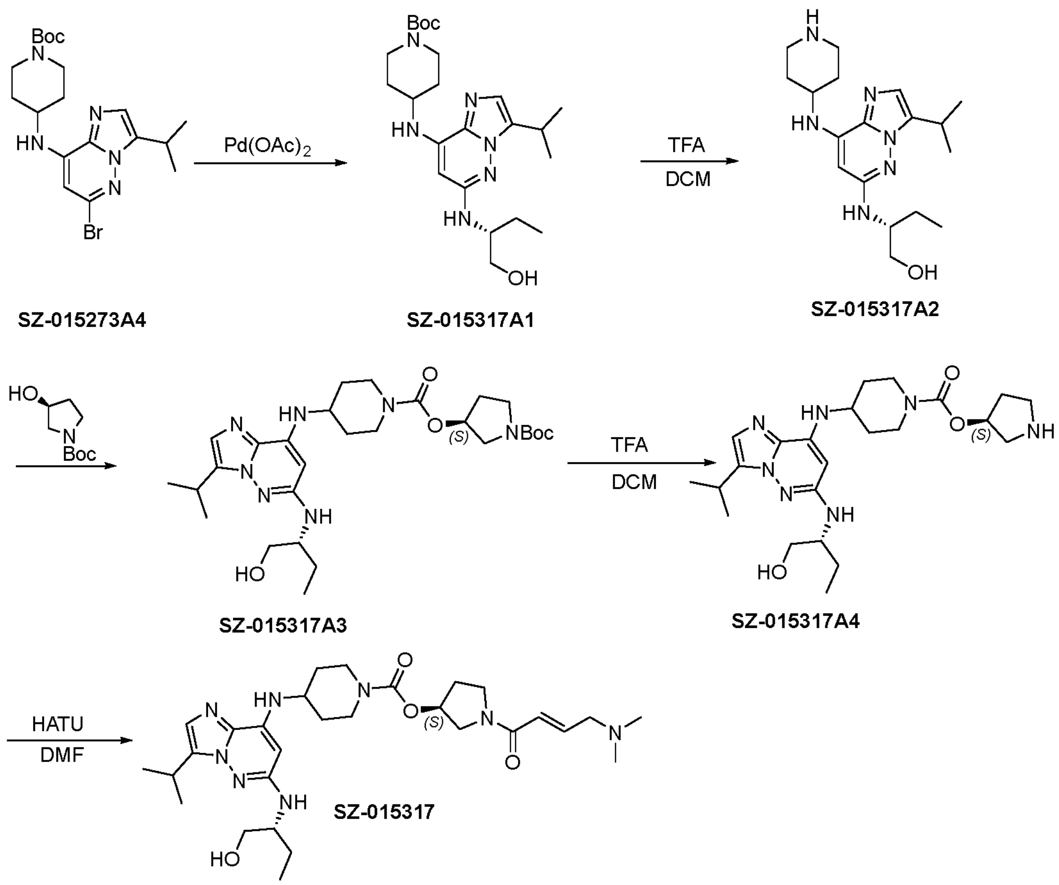


SZ-015273A4 SZ-015317A1 SZ-015317A2

SZ-015317A3 SZ-015317A4

SZ-015317

Step 1:

SZ-015273A4 (700 mg, 1.59 mmol), palladium acetate (200 mg), *R*-BINAP (300 mg, 0.48 mmol), (R)-2-aminobutanol (500 mg), and sodium *tert*-butoxide (500 mg, 5.2 mmol) were mixed in anhydrous toluene (30 mL) under argon atmosphere, and the reaction solution was stirred at 130 °C for 2 h. The reaction solution was directly concentrated by rotary evaporation, and the residue was purified by flash silica gel column chromatography (PE:EA = 1:2; R*f* = 0.2) to give SZ-015317A1 (600 mg, a foamy solid). LCMS: $[M+H]^+$ 447.35.

Step 2:

The product obtained in the previous step was dissolved in dichloromethane (10 mL), and trifluoroacetic acid (5 mL) was slowly added dropwise. The reaction solution was stirred at room temperature for about 1 h, and then directly concentrated by rotary evaporation to give a crude product of **SZ-015317A2** (500 mg). LCMS: $[M+H]^+$ 346.23.

Step 3:

Tert-butyl (*S*)-3-hydroxypyrrolidine-1-carboxylate (251 mg, 1.34 mmol), *p*-nitrophenyl chloroformate (269 mg, 1.34 mmol) and triethylamine (1 mL, 6.03 mmol) were mixed in acetonitrile (10 mL), and the reaction solution was stirred at 40 °C for 2 h. Then **SZ-015317A2** (500 mg) was added, and the resulting reaction solution was stirred at room temperature overnight. The reaction solution was directly concentrated by rotary evaporation, and the residue was purified by flash silica gel column chromatography (EA: 100%; R*f* = 0.3) to give **SZ-015317A3** (250 mg, a foamy solid). LCMS: $[M+H]^+$ 559.31.

Step 4:

**SZ-015317A3** (250 mg) was dissolved in dichloromethane (4 mL), and trifluoroacetic acid (2 mL) was slowly added dropwise. The reaction solution was stirred at room temperature for about 1 h, and then directly concentrated by rotary evaporation to give a crude product of **SZ-015317A4** (300 mg).LCMS: $[M+H]^+$ 459.29.

Step 5:

**SZ-015317A4** obtained in the previous step, *trans*-4-dimethylaminocrotonic acid hydrochloride (74 mg, 0.44 mmol), triethylamine (0.3 mL) and HATU (203 mg, 0.52 mmol) were mixed in DMF (3 mL), and the reaction solution was stirred at room temperature for 1 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015317** (70 mg, a light yellow solid). LCMS: $[M+H]^+$570.40.

**SZ-015317:** $^1H$ NMR (400 MHz, DMSO-$d_6$) 6.97 (s, 1H), 6.67-6.62 (m, 1H), 6.43-6.34 (m, 2H), 5.89-5.87 (d, J= 7.6 Hz, 1H), 5.64 (s, 1H), 5.21-5.15 (m, 1H), 4.62-4.59 (m, 1H), 4.01-3.99 (m, 2H), 3.81-3.41 (m, 8H), 3.19-3.16 (m, 1H), 3.05-3.04 (m, 2H), 2.93 (bs, 2H), 2.16 (s, 6H), 2.16-1.99 (m, 2H), 1.93-1.90 (m, 2H), 1.70-1.65 (m, 1H), 1.54-1.45 (m, 3H), 1.32 (d, J = 6.8 Hz, 6H), 0.92 (t, *J* = 7.6 Hz, 3H).

**Example 29 SZ-015319:** (*S,E*)-1-(4-(dimethylamino)but-2-enoyl)pyrrolidin-3-yl 4-((3,5-diisopropyl-3*H*-imidazo[4,5-*b*]pyridin-7-yl)amino)piperidine-1-carboxylate

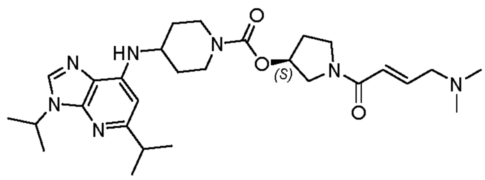

**SZ-015319**

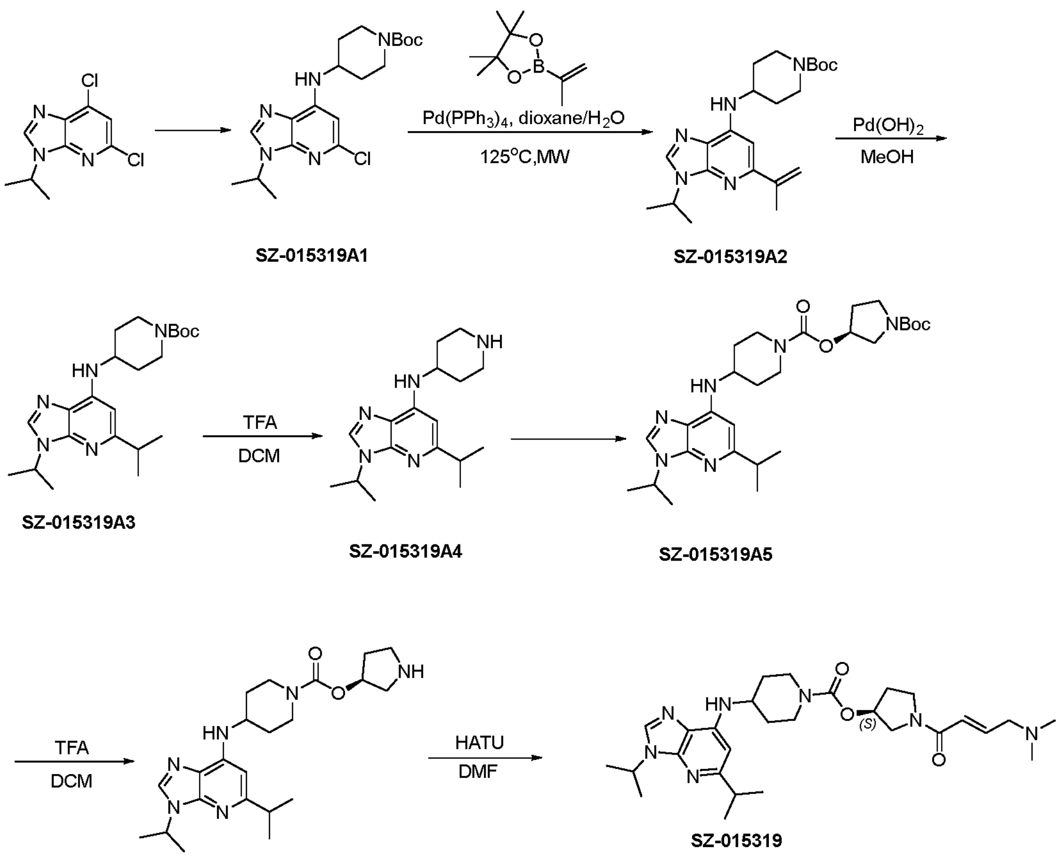


**SZ-015319A1** **SZ-015319A2** **SZ-015319A3** **SZ-015319A4** **SZ-015319A5** **SZ-015319A6** **SZ-015319**

Step 1:

5,7-dichloro-3-isopropyl-3*H*-imidazo[4,5-*b*]pyridine (2 g, 7.3 mmol, see US 20100280065 for synthesis) and 1-Boc-4-aminopiperidine (4.0 g) were mixed, and the reaction solution was stirred under melting at 160 °C for 7 h. After the reaction was completed, water (20 mL) and dichloromethane (20 mL) were added, and the pH was adjusted to about 8 with saturated sodium bicarbonate. The organic phase was directly concentrated by rotary evaporation, and the residue was purified by flash silica gel column chromatography (EA:PE = 2:1; R*f* = 0.3) to give **SZ-015319A1** (2.1 g, a foamy solid). LCMS: $[M+H]^+$ 394.35.

Step 2:

**SZ-015319A1** (500 mg, 1.27 mmol), isopropenylboronic acid pinacol ester (500 mg, 2.97 mmol), potassium carbonate (500 mg, 3.67 mmol) and tetrakis(triphenylphosphine) palladium (100 mg) were mixed in dioxane (6 mL) and water (2 mL) under argon atmosphere, and the reaction solution was stirred at 125 °C for 3 h under a microwave condition. After the reaction was completed, the reaction solution was directly concentrated by rotary evaporation, and the residue was purified by flash silica gel column chromatography (EA:PE = 2:1; R*f* = 0.2) to give **SZ-015319A2** (500 mg, a foamy solid). LCMS: $[M+H]^+$ 400.31.

Step 3:

**SZ-015319A2** (500 mg, 1.25 mmol) and palladium hydroxide (500 mg) were mixed in methanol (10 mL), and ammonium formate (500 mg) was then added. The reaction solution was stirred at 70 °C for 1 h. After the reaction was completed, the reaction solution was cooled and filtered. The filtrate was concentrated by rotary evaporation, and the residue was purified by flash silica gel column chromatography (EA:PE = 2:1; R*f*= 0.3) to give **SZ-015319A3** (500 mg, a foamy solid). LCMS: $[M+H]^+$ 402.31.

Step 4:

**SZ-015319A3** (500 mg, 1.25 mmol) and trifluoroacetic acid (5 mL) were mixed in dichloromethane (10 mL), and the reaction solution was stirred at room temperature for 1 h. After the reaction was completed, the reaction solution was directly concentrated by rotary evaporation without further treatment. LCMS: $[M+H]^+$ 302.29.

Step 5:

Tert-butyl (*S*)-3-hydroxypyrrolidine-1-carboxylate (233 mg, 1.25 mmol), *p*-nitrophenyl chloroformate (251 mg, 1.25 mmol) and triethylamine (0.7 mL) were mixed in acetonitrile (10 mL), and the reaction solution was stirred at 40 °C for 2 h. Then **SZ-015319A4** was added, and the resulting reaction solution was stirred at room temperature overnight. The reaction solution was directly concentrated by rotary evaporation, and the residue was purified by flash silica gel column chromatography (EA: 100%; R*f* = 0.2) to give **SZ-015319A5** (270 mg, a foamy solid). LCMS: $[M+H]^+$ 515.22.

Step 6:

**SZ-015319A5** (270 mg) was dissolved in dichloromethane (6 mL), and trifluoroacetic acid (3 mL) was slowly added dropwise. The reaction solution was stirred at room temperature for about 1 h, and then directly concentrated by rotary evaporation to give a crude product of **SZ-015319A6** (300 mg).LCMS: $[M+H]^+$ 415.29.

Step 7:

**SZ-015319A6** obtained in the previous step, *trans*-4-dimethylaminocrotonic acid hydrochloride (87 mg, 0.52 mmol), triethylamine (0.3 mL) and HATU (240 mg, 0.78 mmol) were mixed in DMF (4 mL), and the reaction solution was stirred at room temperature for 1 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015319** (72 mg, a light yellow solid). LCMS: $[M+H]^+$ 526.35.

**SZ-015319:** $^1H$ NMR (400 MHz, DMSO-$d_6$) 8.11 (s, 1H), 6.68-6.61 (m, 1H), 6.40-6.35 (m, 2H), 6.29 (s, 1H), 5.21-5.15 (m, 1H), 4.81-4.74 (m, 1H), 4.13-3.97 (m, 3H), 3.84-3.73 (m, 1H), 3.64-3.43 (m, 3H), 3.05 (d, J = 6.0 Hz, 2H), 2.99-2.89 (m, 3H), 2.17 (s, 6H), 2.11-1.94 (m, 4H), 1.53 (d, J = 6.4 Hz, 1H), 1.47-1.41 (m, 2H), 1.25 (d, J= 7.2 Hz, 6H).

**Example 30 SZ-015326:** (*S*)-1-((*E*)-4-(dimethylamino)but-2-enoyl)pyrrolidin-3-yl 4-((2-(((*R*)-1-hydroxybutan-2-yl)amino)-8-isopropylpyrazolo[1,5-*a*][1,3,5]triazin-4-yl) amino)piperidine-1-carboxylate

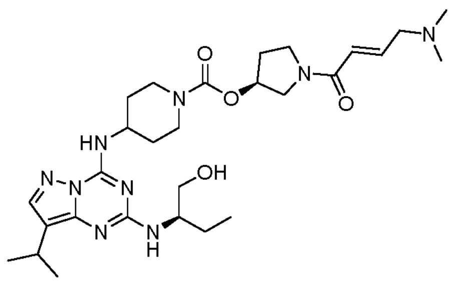

**SZ-015326**

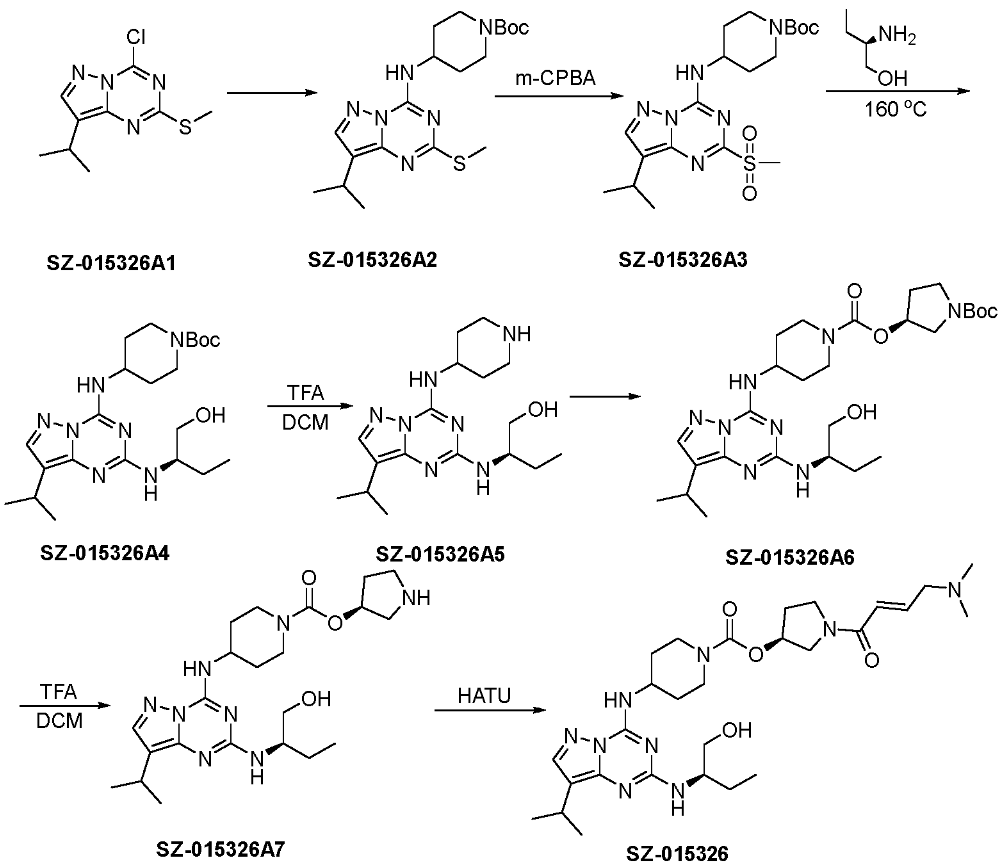


**SZ-015326A1** **SZ-015326A2** **SZ-015326A3**

**SZ-015326A4** **SZ-015326A5** **SZ-015326A6**

**SZ-015326A7** **SZ-015326**

Step 1:

SZ-015326A1 (3.6 g, 15.1 mmol, see WO2019197549 for synthesis) was dissolved in acetonitrile (50 mL), and 1-Boc-4-aminopiperidine (4.5 g, 22.5 mmol) and sodium bicarbonate (6.2 g, 45.3 mmol) were added successively. The reaction solution was stirred at 75 °C overnight. After the reaction was completed, water (50 mL) and ethyl acetate (100 mL) were added, and the organic phase was separated out and directly concentrated by rotary evaporation. The residue was purified by flash silica gel column chromatography (EA:PE = 1:7; R*f* = 0.4) to give **SZ-015326A2** (4.4 g, a white solid). LCMS: $[M+H]^+$ 407.32.

Step 2:

**SZ-015326A2** (4.4 g, 10.8 mmol) was dissolved in dichloromethane, and then *m*-CPBA (5.5 g, 32.4 mmol) was added. The reaction solution was stirred at room temperature for 2 h. After the reaction was completed, saturated sodium

thiosulfate (70 mL) and dichloromethane (50 mL) were added, and the organic phase was washed with saturated sodium chloride three times and then concentrated by rotary evaporation to give **SZ-015326A3** (5.5 g). LCMS: $[M+H]^+$ 439.31.

Step 3:

**SZ-015326A3** (1.0 g) was suspended in (*R*)-2-aminobutanol (2.0 g), and the suspension was stirred at 160 °C for 2 h. After the reaction was completed, the reaction solution was directly purified by flash silica gel column chromatography (EA:PE = 1:1; R*f*= 0.3) to give **SZ-015326A4** (600 mg, a white solid). LCMS: $[M+H]^+$ 448.31.

Step 4:

The product obtained in the previous step was dissolved in dichloromethane (8 mL), and trifluoroacetic acid (4 mL) was slowly added dropwise. The reaction solution was stirred at room temperature for about 1 h, and then directly concentrated by rotary evaporation to give a crude product of **SZ-015326A5.** LCMS: $[M+H]^+$ 348.29.

Step 5:

Tert-butyl (*S*)-3-hydroxypyrrolidine-1-carboxylate (251 mg, 1.34 mmol), *p*-nitrophenyl chloroformate (270 mg, 1.34 mmol) and triethylamine (1 mL, 6.03 mmol) were mixed in acetonitrile (5 mL), and the reaction solution was stirred at 40 °C for 2 h. Then SZ-015326A5 was added, and the resulting reaction solution was stirred at room temperature overnight. The reaction solution was directly concentrated by rotary evaporation, and the residue was purified by flash silica gel column chromatography (EA:PE = 2:1; R*f* = 0.3) to give SZ-015326A6 (390 mg, a foamy solid). LCMS: $[M+H]^+$ 561.24.

Step 6:

**SZ-015326A6** (390 mg) was dissolved in dichloromethane (10 mL), and trifluoroacetic acid (6 mL) was slowly added dropwise. The reaction solution was stirred at room temperature for about 1 h, and then directly concentrated by rotary evaporation to give a crude product of **SZ-015326A7** (300 mg).LCMS: $[M+H]^+$ 461.35.

Step 5:

**SZ-015326A7** obtained in the previous step, *trans*-4-dimethylaminocrotonic acid hydrochloride (118 mg, 0.71 mmol), triethylamine (0.4 mL) and HATU (325 mg, 0.85 mmol) were mixed in DMF (2 mL), and the reaction solution was stirred at room temperature for 1 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015326** (184 mg, a light yellow solid). LCMS: $[M+H]^+$ 572.41.

**SZ-015326:** $^1$H NMR (400 MHz, DMSO-*$d_6$*) 7.98 (s, 1H), 7.69 (s, 1H), 6.69-6.61 (m, 1H), 6.53-6.49 (m, 1H), 6.44-6.34 (m, 1H), 5.21-5.15 (m, 1H), 4.63 (bs, 1H), 4.18-3.99 (m, 3H), 3.84-3.73 (m, 2H), 3.64-3.37 (m, 5H), 3.07-3.05 (m, 2H), 2.95-2.82 (m, 3H), 2.17 (s, 6H), 2.14-1.99 (m, 2H), 1.86-1.83 (m, 2H), 1.69-1.43 (m, 4H), 1.25 (d, J = 6.4 Hz, 6H), 0.89 (t, J = 7.6 Hz, 3H).

**Example 31 SZ-015330:** (*S,E*)-1-(4-(dimethylamino)but-2-enoyl)pyrrolidin-3-yl 4-((8-isopropyl-2-(isopropylamino)pyrazolo[1,5-*a*][1,3,5]triazin-4-yl)amino)piperidine-1-carboxylate

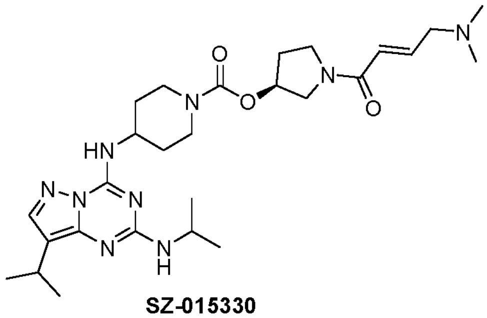

**SZ-015330**

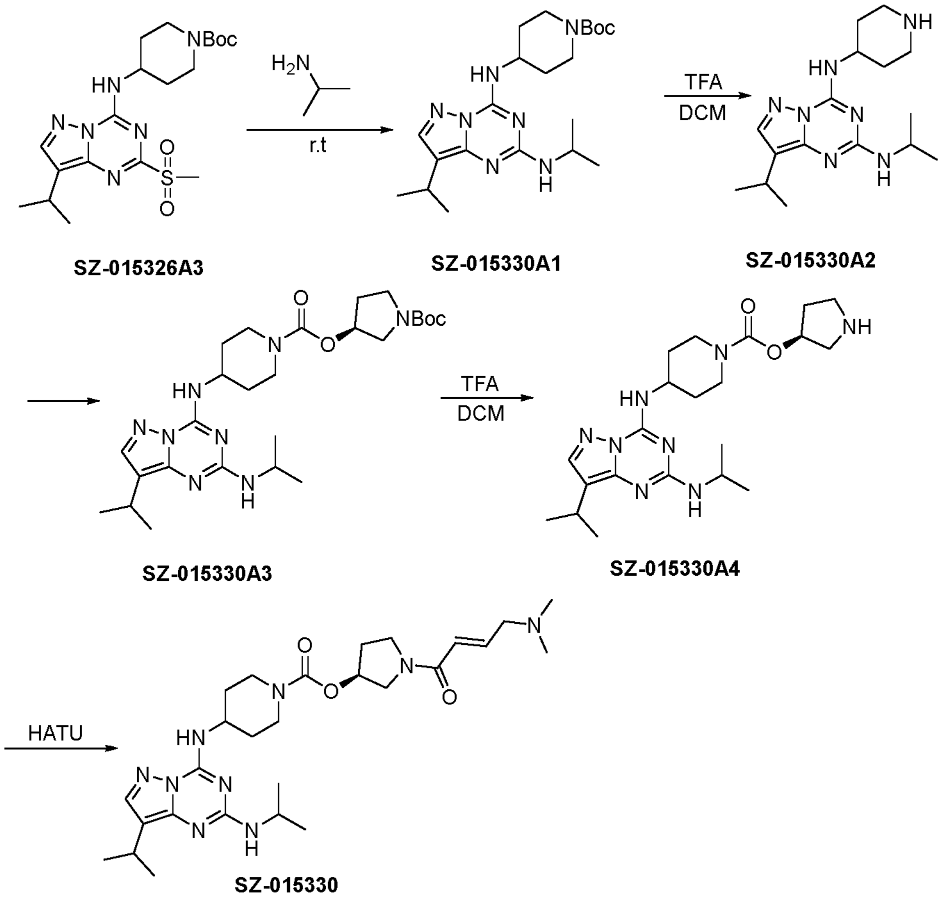


**SZ-015326A3** **SZ-015330A1** **SZ-015330A2**

**SZ-015330A3** **SZ-015330A4**

**SZ-015330**

Step 1:

**SZ-015326A3** (1.0 g) was suspended in isopropylamine (2.5 g) and stirred at room temperature overnight. After the reaction was completed, isopropylamine was directly removed by rotary evaporation, and the residue was purified by flash silica gel column chromatography (EA:PE = 1:3; R*f* = 0.3) to give **SZ-015330A1** (520 mg, a foamy solid). LCMS: $[M+H]^+$ 418.26.

Step 2:

The product obtained in the previous step was dissolved in dichloromethane (8 mL), and trifluoroacetic acid (6 mL) was slowly added dropwise. The reaction solution was stirred at room temperature for about 1 h, and then directly concentrated by rotary evaporation to give a crude product of **SZ-015330A2.** LCMS: $[M+H]^+$ 318.29.

Step 3:

Tert-butyl (*S*)-3-hydroxypyrrolidine-1-carboxylate (224 mg, 1.2 mmol), *p*-nitrophenyl chloroformate (241 mg,

1.2 mmol) and triethylamine (0.75 mL) were mixed in acetonitrile (10 mL), and the reaction solution was stirred at 40 °C for 2 h. Then **SZ-015330A2** was added, and the resulting reaction solution was stirred at room temperature overnight. The reaction solution was directly concentrated by rotary evaporation, and the residue was purified by flash silica gel column chromatography (EA:PE = 2:1; R*f*= 0.3) to give **SZ-015330A3** (100 mg, a foamy solid). LCMS: $[M+H]^+$ 531.40.

Step 4:

**SZ-015330A3** (100 mg) was dissolved in dichloromethane (4 mL), and trifluoroacetic acid (2 mL) was slowly added dropwise. The reaction solution was stirred at room temperature for about 1 h, and then directly concentrated by rotary evaporation to give a crude product of **SZ-015330A4** (300 mg).LCMS: $[M+H]^+$ 431.56.

Step 5:

**SZ-015330A4** obtained in the previous step, *trans*-4-dimethylaminocrotonic acid hydrochloride (33 mg, 0.2 mmol), triethylamine (0.2 mL) and HATU (90 mg, 0.23 mmol) were mixed in DMF (2 mL), and the reaction solution was stirred at room temperature for 1 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015330** (54 mg, a light yellow solid). LCMS: $[M+H]^+$ 542.41.

**SZ-015330:** $^1H$ NMR (400 MHz, DMSO- $d_6$) 7.95 (s, 1H), 7.69 (s, 1H), 6.70-6.61 (m, 1H), 6.53-6.49 (m, 1H), 6.44-6.34 (m, 1H), 5.21-5.15 (m, 1H), 4.15-3.99 (m, 4H), 3.84-3.73 (m, 1H), 3.64-3.37 (m, 3H), 3.07-3.05 (m, 2H), 2.95-2.82 (m, 3H), 2.17 (s, 6H), 2.14-1.99 (m, 2H), 1.86-1.83 (m, 2H), 1.69-1.43 (m, 2H), 1.25 (d, J = 6.4 Hz, 6H), 1.25 (d, J = 6.4 Hz, 6H).

**Example 32 SZ-015331:** (*S,E*)-1-(4-(dimethylamino)but-2-enoyl)pyrrolidin-3-yl 4-((2-(cyclopropylamino)-8-isopropylpyrazolo[1,5-*a*][1,3,5]triazin-4-yl)amino)piperidine-1-carboxylate

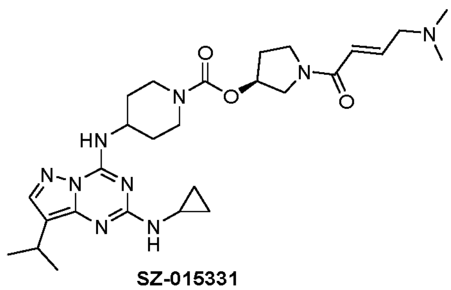

**SZ-015331**

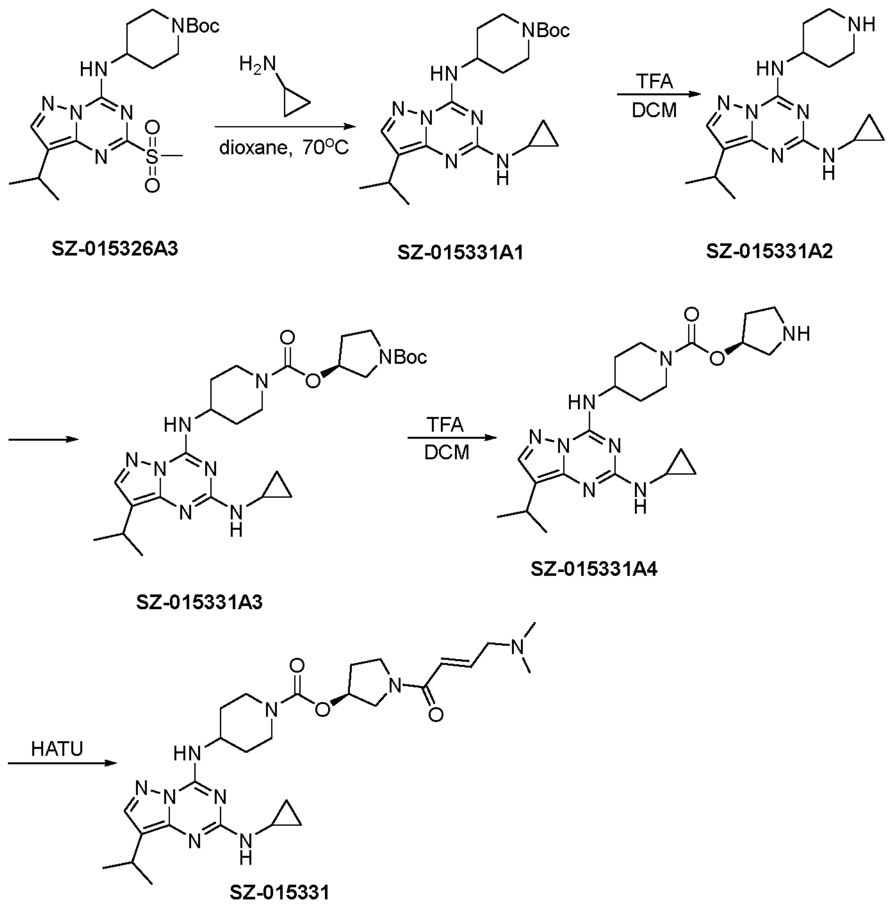


SZ-015326A3 SZ-015331A1 SZ-015331A2 SZ-015331A3 SZ-015331A4 SZ-015331

Step 1:

**SZ-015326A3** (1.0 g) was suspended in a solution of cyclopropylamine (1.0 g) in dioxane (20 mL), and the suspension was stirred at 70 °C overnight. After the reaction was completed, the solvent was directly removed by rotary evaporation, and the residue was purified by flash silica gel column chromatography (EA:PE = 1:3; R*f* = 0.6) to give **SZ-015331A1** (540 mg, a foamy solid). LCMS: $[M+H]^+$ 416.33.

Step 2:

The product obtained in the previous step was dissolved in dichloromethane (10 mL), and trifluoroacetic acid (6 mL) was slowly added dropwise. The reaction solution was stirred at room temperature for about 1 h, and then directly concentrated by rotary evaporation to give a crude product of **SZ-015331A2.** LCMS: $[M+H]^+$ 316.25.

Step 3:

Tert-butyl (*S*)-3-hydroxypyrrolidine-1-carboxylate (243 mg, 1.3 mmol), *p*-nitrophenyl chloroformate (261 mg, 1.3 mmol) and triethylamine (0.8 mL) were mixed in acetonitrile (10 mL), and the reaction solution was stirred at 40 °C for 2 h. Then **SZ-015331A2** was added, and the resulting reaction solution was stirred at room temperature overnight. The reaction solution was directly concentrated by rotary evaporation, and the residue was purified by flash silica gel column chromatography (EA:PE = 2:1; R*f* = 0.3) to give **SZ-015331A3** (300 mg, a foamy solid). LCMS: $[M+H]^+$ 529.32.

Step 4:

**SZ-015331A3** (300 mg) was dissolved in dichloromethane (4 mL), and trifluoroacetic acid (2 mL) was slowly added dropwise. The reaction solution was stirred at room temperature for about 1 h, and then directly concentrated by rotary evaporation to give a crude product of **SZ-015331A4** (300 mg).LCMS: $[M+H]^+$ 429.29.

Step 5:

**SZ-015331A4** obtained in the previous step, *trans*-4-dimethylaminocrotonic acid hydrochloride (93 mg, 0.6 mmol), triethylamine (0.4 mL) and HATU (214 mg, 0.6 mmol) were mixed in DMF (2 mL), and the reaction solution was stirred at room temperature for 1 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015331** (177 mg, a light yellow solid). LCMS: $[M+H]^+$ 540.41.

**SZ-015331:** $^1H$ NMR (400 MHz, DMSO- $d_6$) 8.03 (s, 1H), 7.71 (s, 1H), 7.09 (s, 1H), 6.70-6.61 (m, 1H), 6.43-6.34 (m, 1H), 5.21-5.15 (m, 1H), 4.14-4.02 (m, 3H), 3.84-3.73 (m, 1H), 3.64-3.34 (m, 3H), 3.07-3.05 (m, 2H), 2.98-2.75 (m, 4H), 2.17 (s, 6H), 2.14-1.99 (m, 2H), 1.86-1.83 (m, 2H), 1.66-1.63 (m, 2H), 1.25 (d, J = 6.4 Hz, 6H), 0.67-0.62 (m, 2H), 0.51-0.47 (m, 2H).

**Example 33** SZ-015332: (*S,E*)-1-(4-(dimethylamino)but-2-enoyl)pyrrolidin-3-yl 4-((2-isopropoxy-8-isopropylpyrazolo[1,5-*a*][1,3,5]triazin-4-yl)amino)piperidine-1-carboxylate

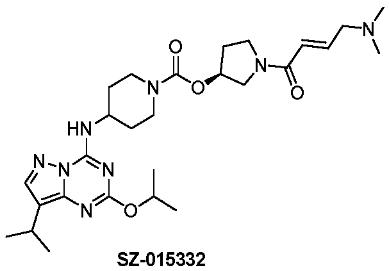

SZ-015332

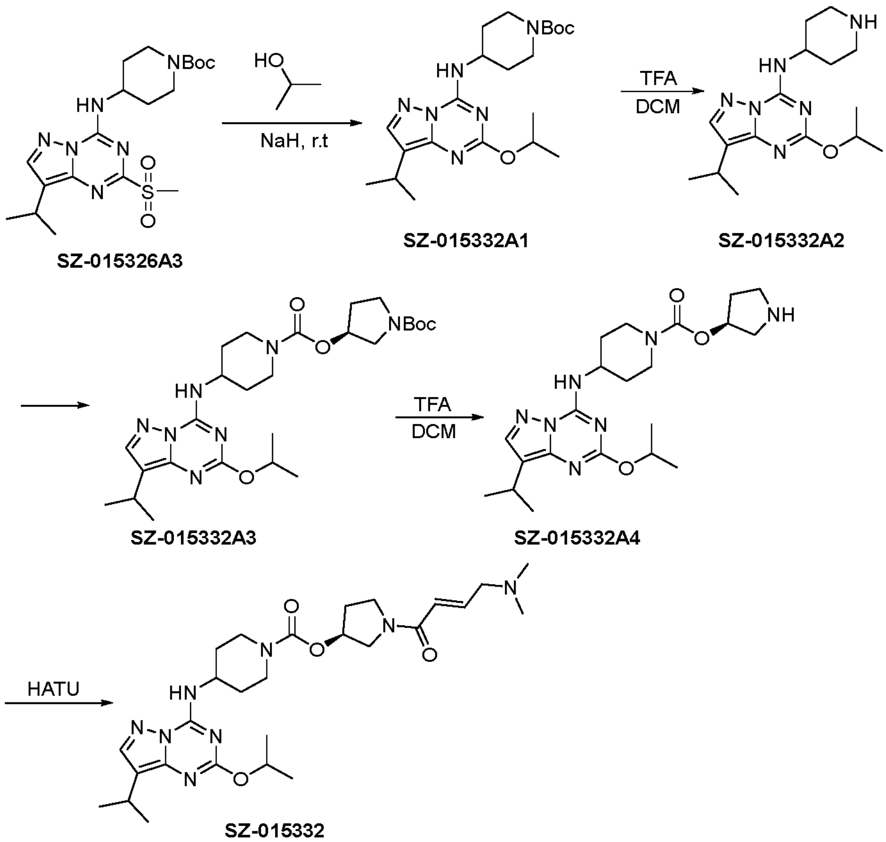


SZ-015326A3

SZ-015332A1

SZ-015332A2

SZ-015332A3

SZ-015332A4

SZ-015332

Step 1:

Sodium hydride (0.9 g, 22.8 mmol) was added to a solution of isopropanol (1.4 g, 22.8 mmol) in tetrahydrofuran (20 mL), and the reaction solution was stirred for 1 h. Then **SZ-015326A3** (1.0 g, 2.3 mmol) was added, and the resulting reaction solution was stirred at room temperature for 2 h. After the reaction was completed, the solvent was directly removed by rotary evaporation, and the residue was purified by flash silica gel column chromatography (EA:PE = 1:2; R*f* = 0.5) to give **SZ-015332A1** (270 mg, a foamy solid). LCMS: $[M+H]^+$ 419.31.

Step 2:

The product obtained in the previous step was dissolved in dichloromethane (5 mL), and trifluoroacetic acid (3 mL) was slowly added dropwise. The reaction solution was stirred at room temperature for about 1 h, and then directly concentrated by rotary evaporation to give a crude product of **SZ-015332A2.** LCMS: $[M+H]^+$ 319.32.

Step 3:

Tert-butyl (*S*)-3-hydroxypyrrolidine-1-carboxylate (120 mg, 0.64 mmol), *p*-nitrophenyl chloroformate (129 mg, 0.64 mmol) and triethylamine (0.4 mL) were mixed in acetonitrile (5 mL), and the reaction solution was stirred at 40 °C for 2 h. Then **SZ-015332A2** was added, and the resulting reaction solution was stirred at room temperature overnight. The reaction solution was directly concentrated by rotary evaporation, and the residue was purified by flash silica gel column chromatography (EA:PE = 1:1; R*f* = 0.3) to give **SZ-015332A3** (190 mg, a foamy solid). LCMS: $[M+H]^+$ 532.36.

Step 4:

**SZ-015332A3** (300 mg) was dissolved in dichloromethane (4 mL), and trifluoroacetic acid (2 mL) was slowly added dropwise. The reaction solution was stirred at room temperature for about 1 h, and then directly concentrated by rotary evaporation to give a crude product of **SZ-015332A4** (300 mg).LCMS: $[M+H]^+$ 432.32.

Step 5:

**SZ-015332A4** obtained in the previous step, *trans*-4-dimethylaminocrotonic acid hydrochloride (59 mg, 0.4 mmol), triethylamine (1 mL) and HATU (135 mg, 0.4 mmol) were mixed in DMF (2 mL), and the reaction solution was stirred at room temperature for 1 h. After the reaction was completed, the solvent was removed by rotary evaporation under reduced pressure to give a crude product. The crude product was subjected to reverse phase column chromatography (acetonitrile/water with 0.1% formic acid) and lyophilized to give **SZ-015332** (92 mg, a light yellow solid). LCMS: $[M+H]^+$ 543.41.

**SZ-015332:** $^1H$ NMR (400 MHz, DMSO- $d_6$) 8.57-8.54 (m, 1H), 7.91 (s, 1H), 6.69-6.61 (m, 1H), 6.43-6.34 (m, 1H), 5.21-5.15 (m, 1H), 4.20-3.95 (m, 3H), 3.84-3.73 (m, 1H), 3.64-3.34 (m, 3H), 3.31 (s, 1H), 3.07-2.92 (m, 5H), 2.17 (s, 6H), 2.14-1.99 (m, 2H), 1.86-1.83 (m,2H), 1.69-1.59 (m, 2H), 1.33 (d, J = 6.4 Hz, 6H), 1.29 (d, J = 6.8 Hz, 6H).

**Example 34 SZ-015342:** (*S,E*)-1-(4-(dimethylamino)but-2-enoyl)pyrrolidin-3-yl 4-((3,6-diisopropylimidazo[1,2-*a*] pyrazin-8-yl)amino)piperidine-1-carboxylate

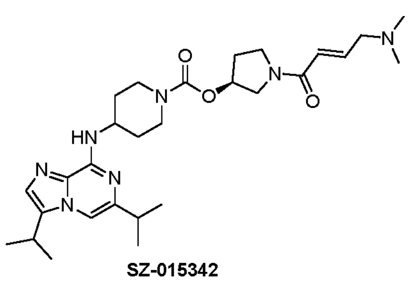

SZ-015342

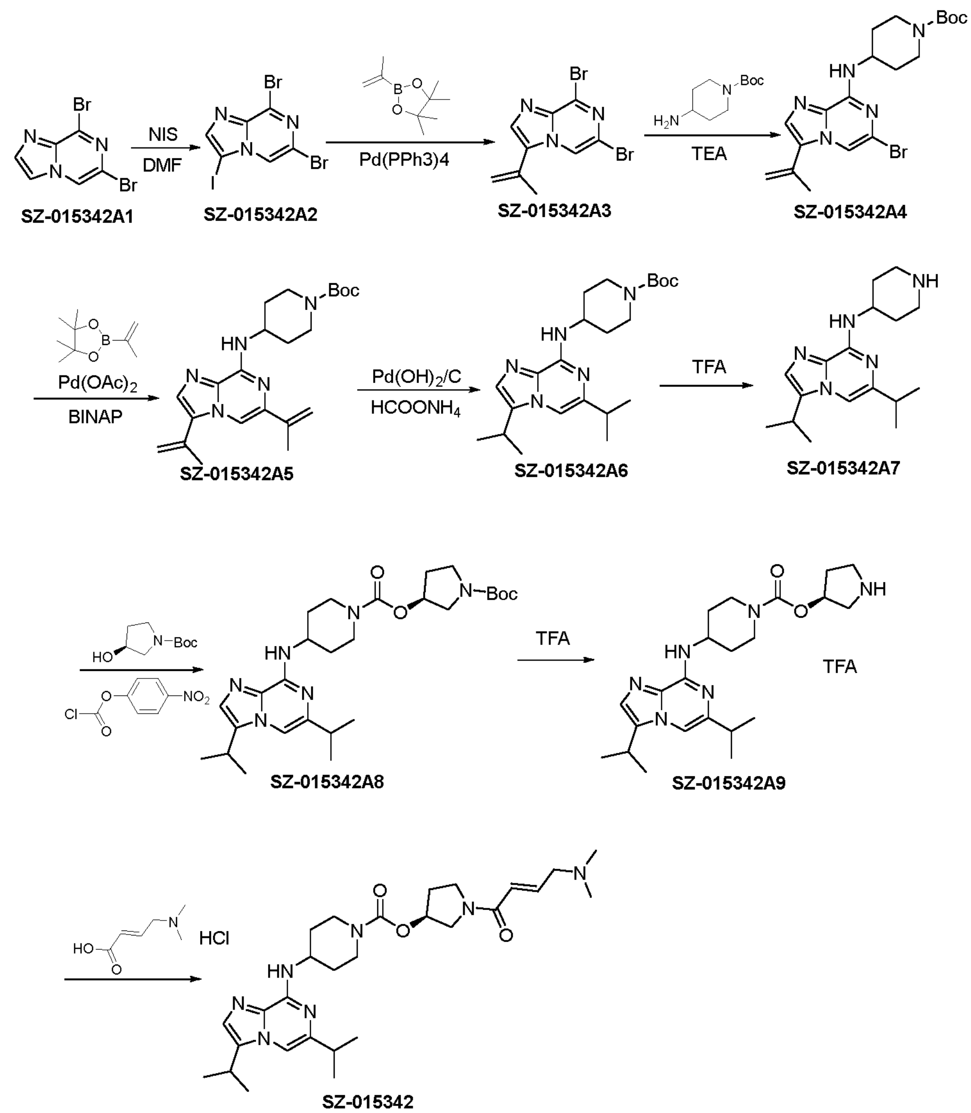


SZ-015342A1 SZ-015342A2 SZ-015342A3 SZ-015342A4 SZ-015342A5 SZ-015342A6 SZ-015342A7 SZ-015342A8 SZ-015342A9

SZ-015342

Step 1:

Compound NIS (13.1 g, 58 mmol) was added in portions to a solution of compound **015342A1** (15 g, 53.9 mmol) in red-yellow DMF (260 mL) at 26 °C. The resulting reaction solution was heated to 63 °C and stirred for 16 h. The resulting reactant was then concentrated by rotary evaporation to remove the solvent. Ethyl acetate (180 mL) and water (0.3 L) was added to the residue for liquid separation, and ethyl acetate (0.3 L × 3) was then added for extraction. The extracts were combined, dried over anhydrous $MgSO_4$ and filtered, and the solvent was removed by rotary evaporation. The residue was purified by column chromatography to give compound **015342A2** (5.6 g, a viscous liquid), which was solidified after being placed for a period of time. LCMS: $[M+H]^+$ 402.1/404.1.

Step 2:

Powder of compound $Pd(PPh_3)_4$ (1.26 g, 1.1 mmol) was added in portions to a suspension of compound **015342A2** (5.6 g, 13.9 mmol), isopropenylboronic acid pinacol ester (11.9 g, 69 mmol), potassium carbonate (1.96 g, 13.9 mmol), toluene (130 mL), ethanol (69 mL) and water (30 mL) at 26 °C. The resulting reaction solution was stirred and purged with nitrogen three times at room temperature, and then heated to 50 °C and further stirred for 16 h. The resulting reaction solution was concentrated by rotary evaporation to remove the solvent, and ethyl acetate and water were added to the residue for liquid separation, and ethyl acetate (0.3 L × 3) was then added for extraction. The extracts were combined, dried over anhydrous $MgSO_4$ and filtered, and the organic solvent was removed by rotary evaporation. The resulting residue was purified by column chromatography to give compound **015342A3** (3.6 g). LCMS: $[M+H]^+$ 316.1/318.1.

Step 3:

A mixture of compound **015342A3** (2.8 g, 8.9 mmol), *tert*-butyl 4-aminopiperidine-1-carboxylate (3 g, 15 mmol), triethylamine (3.6 mL, 63 mmol) and 1,4-dioxane (36 mL) was heated to 110 °C and stirred for 16 h. The resulting reaction solution was cooled to room temperature, water was added to quench the reaction, and then ethyl acetate (60 mL × 3) was added for extraction. The extracts were combined, dried over anhydrous $MgSO_4$ and filtered, and the solvent was removed by rotary evaporation. The residue was purified by column chromatography to give product **015342A4** (2.6 g, a yellow oil). LCMS: $[M+H]^+$ 435.9/437.9.

Step 4:

Compound $Pd(PPh_3)_4$ (0.69 g, 0.6 mmol) was added in portions to a suspension of compound **015342A4** (1.9 g, 4.3 mmol), isopropenylboronic acid pinacol ester (2.3 g, 13.3 mmol), sodium carbonate (1.39 g, 13 mmol), 1,4-dioxane (36 mL) and water (13 mL) at 29 °C. The resulting reaction solution was stirred and purged with nitrogen three times at 29 °C, and then heated to 110 °C and further stirred for 16 h. The resulting reaction solution was cooled to room temperature, water was added to quench the reaction, and then ethyl acetate (60 mL × 3) was added for extraction. The extracts were combined, dried over anhydrous $MgSO_4$ and filtered, and the organic solvent was removed by rotary evaporation. The resulting residue was purified by column chromatography to give product **015342A5** (1.6 g, a brown oil). LCMS: $[M+H]^+$ 398.1.

Step 5:

Compound **015342A5** (1.6 g) was added to a black suspension of $Pd(OH)_2/C$ (1 g) in methanol (130 mL), and then solid ammonium formate was added (9 g). The resulting reaction solution was heated to 90 °C and stirred for 3 h. The resulting reaction solution was cooled to room temperature and filtered through celite, and the filter cake was washed with methanol. The filtrates were combined and then concentrated by rotary evaporation, to remove the solvent, and ethyl acetate and water were added for liquid separation. The suspension was extracted with ethyl acetate (60 mL × 3). The filtrates were combined, dried over anhydrous $MgSO_4$ and filtered, and the solvent was removed by rotary evaporation. The residue was dried for 3 min with an oil pump to give product **015342A6** (1.36 g, a yellow oil). LCMS: $[M+H]^+$ 402.3.

Step 6:

A mixture of compound **015342A6** (1.36 g, 3.39 mmol) and trifluoroacetic acid (6 mL) was heated to 90 °C and stirred for 1 h. The resulting dark brown solution was concentrated by rotary evaporation to remove the solvent, and the residue was dried for 3 min with an oil pump to give product **015342A7** (1.36 g, a brown-yellow viscous oil). LCMS: $[M+H]^+$ 302.1.

Step 7:

The compound triethylamine (13 mL, 39 mmol) was added in portions to a solution of compounds p-nitrophenyl chloroformate (0.78 g, 3.9 mmol) and (*S*)-1-*N*-*tert*-butoxycarbonyl-3-hydroxypyrrolidine (0.78 g, 3.9 mmol) in 1,4-dioxane (16 mL) at 31 °C (the reaction system rapidly formed a light yellow suspension). The resulting reaction mixture was heated to 69 °C and stirred for 3 h (this suspension was directly used in the next reaction). Compound triethylamine (13 mL, 39 mmol) was added in portions to a solution of compound **015342A7** (1.3 g, 3.3 mmol) in 1,4-dioxane (6 mL) at 31 °C to basify the reaction system. Then, the suspension prepared above was added dropwise to the basic reaction system at 31 °C. The resulting reaction solution was heated to 69 °C and stirred for 16 h. The resulting reaction solution was cooled to room temperature, water was added to quench the reaction, and ethyl acetate (60 mL × 3) was added for extraction. The extracts were combined, dried over anhydrous $MgSO_4$ and filtered, and the solvent was removed by rotary evaporation. The residue was purified by column chromatography to give product **015342A8** (0.9 g, a yellow oil). LCMS: $[M+H]^+$ 515.3.

Step 8:

A mixture of compound **015342A8** (0.9 g) and trifluoroacetic acid (6 mL) was stirred at 31 °C for 1 h. The resulting dark brown solution was concentrated by rotary evaporation to remove the solvent, and the residue was dried for 3 min with an oil pump to give product **015342A9** (0.9 g, a brown-yellow viscous oil). LCMS: $[M+H]^+$ 415.3.

Step 9:

A solution of compound $T_3P$ in ethyl acetate (6.9 mL, 11 mmol, 50 wt%) was added dropwise to a solution of compound **015342A9** (0.9 g, 1.8 mmol), (2*E*)-4-(dimethylamino)-2-butenoic acid hydrochloride (1 g, 6 mmol) and triethylamine (16 mL, 115 mmol) in acetonitrile (16 mL) at 31 °C. The resulting reaction solution was stirred at 23-31 °C for 16 h. Water was added to the reaction solution to quench the reaction, and then solid sodium carbonate was added to basify and saturate the reaction solution. Then ethyl acetate (60 mL × 3) was added for extraction. The extracts were combined, dried over anhydrous $MgSO_4$ and filtered, and the solvent was removed by rotary evaporation. The residue was purified by column chromatography to give a crude product of **015342** (800 mg). This crude product was further purified by preparative chromatography to give compound **015342** (163 mg, a light yellow solid).

LCMS: $[M+H]^+$ 526.3. $^1H$ NMR ($CD_3OD$, 400 MHz): δ 7.37 (s, 1H), 7. 28 (s, 1H), 6. 81-6.71 (m, 2H), 5.33 (d, 1H, *J*=16.0 Hz), 4.36-4.28 (m, 1H), 4.19-3.98 (m, 2H), 3.93-3.49 (m, 6H), 3.29-3.16 (m, 3H), 2.86-2.83 (m, 1H), 2.85 (s, 6H), 2.33-2.26 (m, 1H), 2.26-2.13 (m, 3H), 1.63-1.53 (m, 2H), 1.40 (d, 6H, *J*= 4.0Hz), 1.32 (d, 6H, *J*= 4.0Hz).

**Example 35 SZ-015328:** (*S*,*E*)-1-(4-(dimethylamino)but-2-enoyl)pyrrolidin-3-yl 4-((7-isopropyl-2-(isopropylamino)imidazo[2,1-*f*][1,2,4]triazin-4-yl)amino)piperidine-1-carboxylate

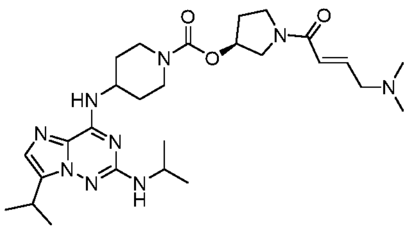

**SZ-015328**

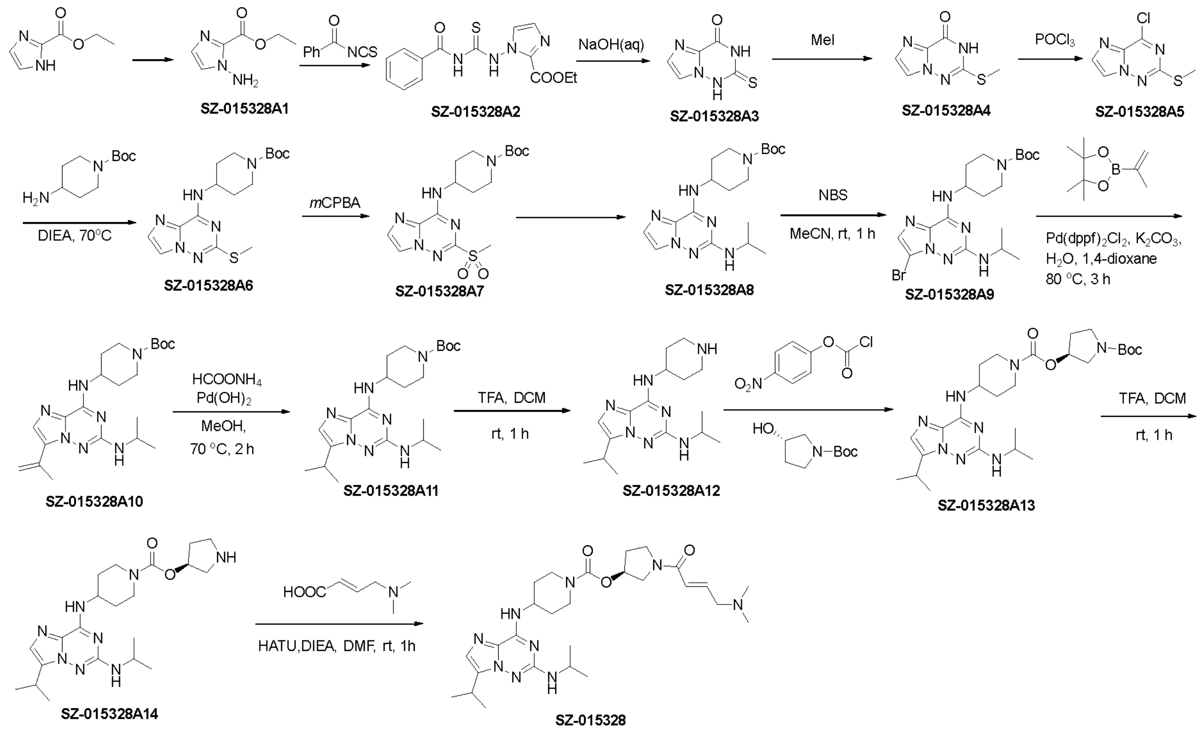


Step 1:

Ethyl imidazole-2-carboxylate (6 g) was dissolved in DMF (400 mL) in a reaction flask, and a solution of potassium *tert*-butoxide (5.282 g) in DMF (60 mL) was added dropwise to the reaction solution with stirring. The resulting reaction

solution was stirred at room temperature for 1 h. O-*p*-nitrobenzoyl hydroxylamine (7.795 g) was dissolved in DMF (100 mL), and the solution was added dropwise to the reaction solution. The reaction solution changed from dark blue to brown and finally to orange as the dropwise addition proceeded. The reaction solution was stirred at room temperature overnight. After the reaction was completed, the reaction solution was concentrated, saturated sodium bicarbonate solution (400 mL) was added to quench the reaction, and dichloromethane (500 mL × 4) was added for extraction. The organic phases were combined, dried over anhydrous sodium sulfate and filtered, and the solvent was removed by rotary evaporation to give a crude product of **SZ-015328A1** (4.33 g). LCMS: $[M+1]^+$ 156.05.

Step 2:

**SZ-015328A1** (6.7 g) and tetrahydrofuran (50 mL) were added to a reaction flask and stirred when the temperature was reduced to 0 °C, and benzoyl isothiocyanate (7.1 g) was added in portions. The reaction solution was stirred at room temperature overnight. After the reaction was completed, the solvent was removed by rotary evaporation to give a crude product of **SZ-015328A2,** which was directly used in the next step without purification. LCMS: $[M+1]^+$ 319.21.

Step 3:

The crude product **SZ-015328A2** in the previous step was added to a reaction flask, and aqueous NaOH solution (2 M, 100 mL) was added. The reaction solution was stirred at 80 °C for 1 h. After the reaction was completed, the reaction solution was adjusted to pH = 1 with 2 M aqueous HCl solution, and the precipitate was filtered and washed with DCM to give a crude product of **SZ-015328A3** (15 g) containing sodium chloride. LCMS: $[M+1]^+$ 169.02.

Step 4:

**SZ-015328A3** (15 g, crude product), NaOH (5 g), $H_2O$ (50 mL) and 1,4-dioxane (200 mL) were added to a reaction flask and stirred at room temperature. Methyl iodide was added in portions. The reaction was monitored by LCMS until it was completed, and then the addition of methyl iodide (4 g) was stopped. After the reaction was completed, the precipitated white solid was filtered out and dried to give a crude product of **SZ-015328A4** (4.1 g). LCMS: $[M+1]^+$ 183.01.

Step 5:

**SZ-015328A4** (4.1 g, crude product) and $POCl_3$ (200 mL) were added to a reaction flask and stirred at 105 °C for 24 h. After the reaction was completed, $POCl_3$ was removed by rotary evaporation, and the residue was purified by silica gel column chromatography (ethyl acetate:petroleum ether = 1:3-ethyl acetate:petroleum ether:triethylamine = 3:9:1) to give **SZ-015328A5** (2.73 g). LCMS: $[M+1]^+$ 200.94.

Step 6:

**SZ-015328A5** (2.73 g), 1-Boc-4-aminopiperidine (4.4 g), MeCN (50 mL) and TEA (5 mL) were added to a reaction flask and stirred at 90 °C for 3 h. After the reaction was completed, the reaction solution was concentrated, and the resulting crude product was purified by flash silica gel column chromatography (EA/PE = 0%-30%) to give product**SZ-015328A6** (2.77 g). LCMS: $[M+1]^+$ 365.11.

Step 7:

**SZ-015328A6** (2.77 g) and DCM (30 mL) were added to a reaction flask and stirred at 0 °C, and then *m*CPBA (4.6 g) was added. The reaction solution was warmed to room temperature and stirred for 2 h. Aqueous sodium thiosulfate solution was added to quench the reaction, and saturated potassium carbonate solution (100 mL) and dichloromethane (100 mL × 3) were added for extraction. The organic phases were combined and concentrated by rotary evaporation to remove the solvent, and the resulting crude product was purified by flash silica gel column chromatography (EA/DCM = 0%-50%) to give product **SZ-015328A7** (2.71 g). LCMS: 340.11.

Step 8:

**SZ-015328A7** (1.5 g), isopropylamine (4 mL) and NMP (15 mL) were added to an autoclave and stirred at 140 °C for 3 d. After the reaction was completed, Boc anhydride (5 mL) was added, and the reaction solution was stirred for

1 h. After the reaction was completed, the reaction solution was extracted with water (200 mL) and dichloromethane (100 mL × 3), and the organic phases were combined and concentrated by rotary evaporation to remove the solvent. The resulting crude product was purified by flash silica gel column chromatography (EA/PE = 0%-40%) to give product **SZ-015328A8** (869 mg). LCMS: $[M+1]^+$ 376.24.

Step 9:

**SZ-015328A8** (869 mg), NBS (329 mg) and MeCN (50 mL) were added to a reaction flask and stirred at room temperature for 30 min. The reaction solution was concentrated by rotary evaporation under reduced pressure to remove the solvent, and the resulting crude product was purified by flash silica gel column chromatography (EA/PE = 0%-20%) to give product**SZ-015328A9** (934 mg). LCMS: 455.61, 457.61.

Step 10:

**SZ-015328A9** (934 mg), isopropenylboronic acid pinacol ester (672 mg), $Pd(dppf)_2Cl_2$ (163 mg), potassium carbonate (690 mg), 1,4-dioxane (25 mL) and water (5 mL) were added to a reaction flask and stirred at 80 °C for 3 h under nitrogen atmosphere. The solvent was removed by rotary evaporation, and the residue was diluted with ethyl acetate and filtered. The organic phase was concentrated by rotary evaporation to remove the solvent, and the resulting crude product was purified by flash silica gel column chromatography (EA/PE = 0%-30%) to give product **SZ-015328A10** (1.02 g). LCMS: $[M+1]^+$ 416.20.

Step 11:

**SZ-015328A10** (1.02 g), $Pd(OH)_2$ (200 mg), ammonium formate (2 g) and methanol (50 mL) were added to a reaction flask and stirred at 80 °C for 1 h. The solvent was removed by rotary evaporation, and the residue was diluted with ethyl acetate and filtered. The organic phase was concentrated by rotary evaporation to remove the solvent, and the resulting crude product was purified by flash silica gel column chromatography (EA/PE = 0%-30%) to give product **SZ-015328A11** (920 mg). LCMS: $[M+1]^+$ 418.11.

Step 12:

**SZ-015328A11** (920 mg), dichloromethane (10 mL) and TFA (3 mL) were added to a reaction flask and stirred at room temperature for 1 h. After the reaction was completed, the reaction solution was concentrated by rotary evaporation to give a crude product of **SZ-015328A12,** which was directly used in the next step without separation. LCMS: $[M+1]^+$ 318.10.

Step 13:

*(S)*-1-*N*-*tert*-butoxycarbonyl-3-hydroxypyrrolidine (449 mg), acetonitrile (20 mL), triethylamine (5 mL) and p-nitrophenyl chloroformate (483 mg) were added successively to a reaction flask and stirred at 40 °C for 2 h. The crude product of **SZ-015328A12** in the previous step was dissolved in acetonitrile (5 mL), triethylamine was added to adjust the solution to be alkaline, and then the reaction solution was added dropwise to the solution of **SZ-015328A12** in acetonitrile. The resulting reaction solution was stirred at room temperature for 4 h. After the reaction was completed, the solvent was removed by rotary evaporation, and the residue was purified by flash silica gel column chromatography (methanol:dichloromethane = 0%-5%) to give **SZ-015328A13** (986 mg). LCMS: $[M+1]^+$ 531.13.

Step 14:

**SZ-015328A13** (986 mg), dichloromethane (10 mL) and TFA (3 mL) were added to a reaction flask and stirred at room temperature for 1 h. After the reaction was completed, the reaction solution was concentrated by rotary evaporation to give a crude product of **SZ-015328A14,** which was directly used in the next step without separation. LCMS: $[M+1]^+$ 431.18.

Step 15:

The crude product of **SZ-015328A14** in the previous step, (2*E*)-4-(dimethylamino)-2-butenoic acid hydrochloride (379 mg), DIEA (3 mL), HATU (798 mg) and DMF (10 mL) were added to a reaction flask and stirred at room temperature for 2 h. After the reaction was completed, the solvent was removed by rotary evaporation, and the residue was separated

by a reverse phase column (acetonitrile-water with 0.01% formic acid) to give **SZ-015328** (720 mg). LCMS: $[M+1]^+$ 542.15.

$^1H$ NMR (400 MHz, DMSO-$d_6$) δ 8.04 (d, J = 8.2 Hz, 1H), 7.11 (s, 1H), 6.70-6.54 (m, 2H), 6.12 (d, J = 7.1 Hz, 1H), 5.24-5.17 (m, 1H), 4.28-4.21 (m, 1H), 4.03 (br, 2H), 3.93-3.83 (m, 2H), 3.80-3.75 (m, 1H), 3.68-3.52 (m, 3H), 3.45-3.38 (m, 1H), 3.22-3.16 (m, 1H), 2.88 (br, 2H), 2.55-2.52 (m, 6H), 2.22-2.04 (m, 2H), 1.87-1.84 (m, 2H), 1.63-1.54 (m, 2H), 1.32 (d, J = 6.9 Hz, 6H), 1.19 (d, *J* = 6.5 Hz, 6H).

**Example 36 SZ-015338:** (*S,E*)-1-(4-(dimethylamino)but-2-enoyl)pyrrolidin-3-yl 4-((2-cyclopropyl-7-isopropylimidazo[2,1-*f*][1,2,4]triazin-4-yl)amino)piperidine-1-carboxylate

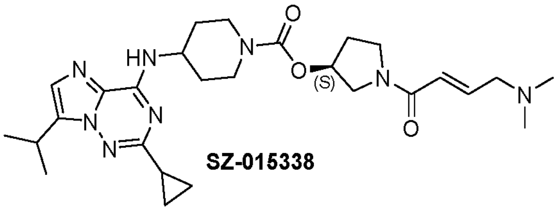

**SZ-015338**

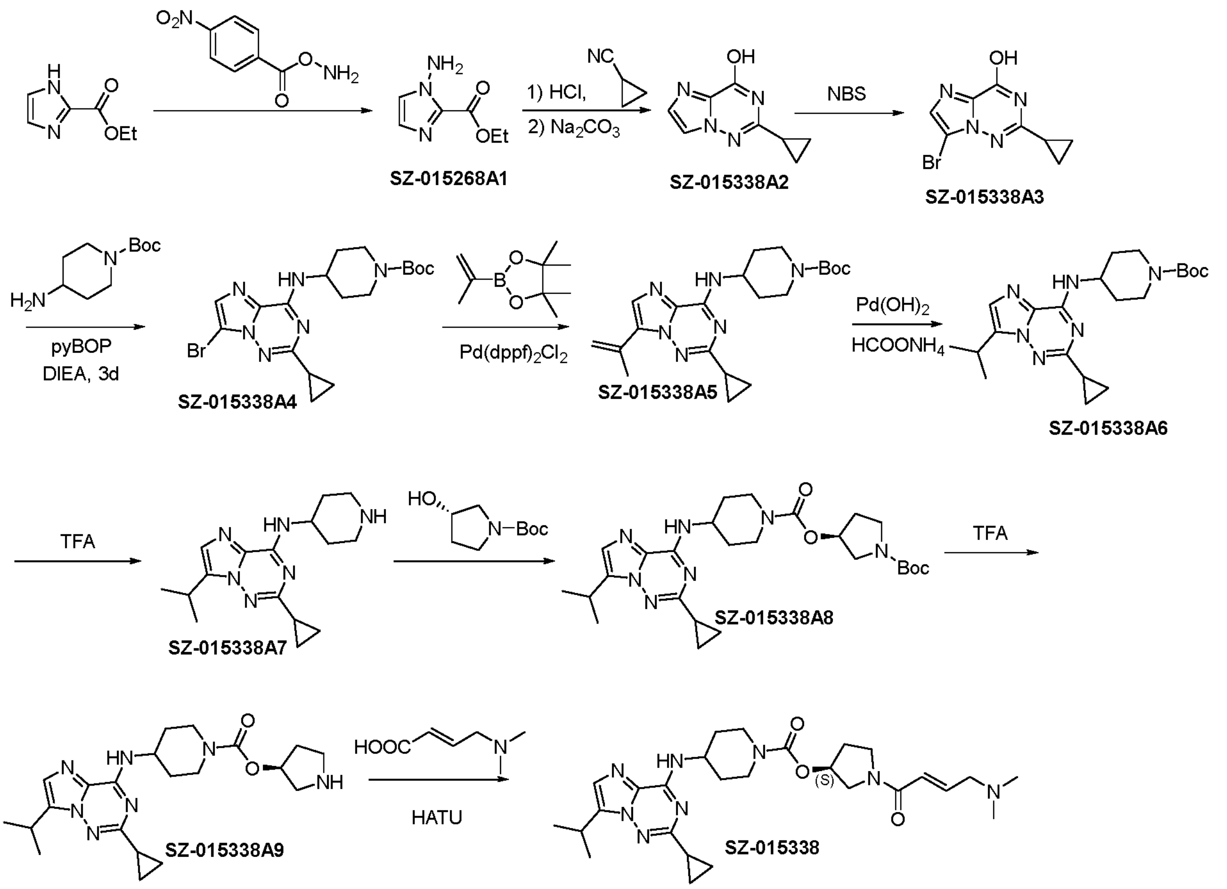


Step 2:

Dry **SZ-015268A1** (3.1 g) and anhydrous cyclopropanecarbonitrile (50 mL) were added to a three-necked flask and bubbled for 10 min with nitrogen introduced through a conduit. The reaction solution was cooled to 0 °C and stirred, and then dry hydrogen chloride gas was introduced for 20 min. The reaction solution was then stirred at 80 °C for 1 h. After the reaction was completed, the reaction solution was concentrated, and the remaining solid was slurried with diethyl ether and filtered to give a crude product as an intermediate, which was directly used in the next step without purification. The intermediate, 1,4-dioxane (40 mL), water (30 mL) and sodium bicarbonate (1.6 g) were added to a reaction flask and stirred at 100 °C for 1 h. After the reaction was completed, the solvent was removed under reduced

pressure, and the remaining solid was diluted with acetonitrile and filtered to give a crude product of **SZ-015338A2** (4 g, a white solid) containing sodium chloride. LCMS: $[M+1]^+$ 177.07.

Step 3:

**SZ-015338A2** (1.5 g, crude product), NBS (1.42 g) and DMF (50 mL) were added to a reaction flask and stirred at 80 °C for 1 h. The reaction solution was concentrated by rotary evaporation under reduced pressure to remove the solvent, and the residue was diluted with dichloromethane and filtered to give a crude product of **SZ-015338A3** (1.59 g, a white solid) containing sodium chloride. LCMS: 256.10.

Step 4:

**SZ-015338A3** (1.59 g), 1-Boc-4-aminopiperidine (2.6 g), PyBOP (5.2 g), DCE (20 mL) and DIEA (1.5 mL) were added to a reaction flask and stirred at room temperature for 12 h. After the reaction was completed, the reaction solution was filtered and washed with dichloromethane. The organic phase was concentrated by rotary evaporation to remove the solvent, and the resulting crude product was purified by flash silica gel column chromatography (EA/PE = 0%-40%) to give product **SZ-015338A4** (1.28 g). LCMS: 438.81.

Step 5:

**SZ-015338A4** (1.28 g), isopropenylboronic acid pinacol ester (1 g), $Pd(dppf)_2Cl_2$ (244 mg), potassium carbonate (1.24 g), 1,4-dioxane (36 mL) and water (6 mL) were added to a reaction flask and stirred at 80 °C for 3 h under nitrogen atmosphere. The solvent was removed by rotary evaporation, and the residue was diluted with ethyl acetate and filtered. The organic phase was concentrated by rotary evaporation to remove the solvent, and the resulting crude product was purified by flash silica gel column chromatography (EA/PE = 0%-30%) to give product **SZ-015338A5** (1.2 g). LCMS: $[M+1]^+$ 399.24.

Step 6:

**SZ-015338A5** (1.2 g), $Pd(OH)_2$ (160 mg), ammonium formate (1.2 g) and methanol (30 mL) were added to a reaction flask and stirred at 70 °C for 2 h. The solvent was removed by rotary evaporation, and the residue was diluted with ethyl acetate and filtered. The organic phase was concentrated by rotary evaporation to remove the solvent, and the resulting crude product was purified by flash silica gel column chromatography (EA/PE = 0%-30%) to give product **SZ-015338A6** (1.06 g). LCMS: 401.26.

Step 7:

**SZ-015338A6** (880 mg), dichloromethane (10 mL) and TFA (2 mL) were added to a reaction flask and stirred at room temperature for 1 h. After the reaction was completed, the reaction solution was concentrated by rotary evaporation to give a crude product of **SZ-015338A7,** which was directly used in the next step without separation. LCMS: $[M+1]^+$ 301.21.

Step 8:

(*S*-1-*N*-*tert*-butoxycarbonyl-3-hydroxypyrrolidine (449 mg), acetonitrile (10 mL), triethylamine (2 mL) and p-nitrophenyl chloroformate (483 mg) were added successively to a reaction flask and stirred at 40 °C for 2 h. The crude product of **SZ-015338A7** in the previous step was dissolved in acetonitrile (5 mL), triethylamine was added to adjust the solution to be alkaline, and then the reaction solution was added dropwise to the solution of **SZ-015338A7** in acetonitrile. The resulting reaction solution was stirred at room temperature overnight. After the reaction was completed, the solvent was removed by rotary evaporation, and the residue was purified by flash silica gel column chromatography (methanol:dichloromethane = 0%-5%) to give **SZ-015338A8** (793 mg). LCMS: $[M+1]^+$ 513.25.

Step 9:

**SZ-015338A8** (793 mg), dichloromethane (10 mL) and TFA (3 mL) were added to a reaction flask and stirred at room temperature for 1 h. After the reaction was completed, the reaction solution was concentrated by rotary evaporation to give a crude product of **SZ-015338A9,** which was directly used in the next step without separation. LCMS: $[M+1]^+$ 414.25.

Step 10:

The crude product of **SZ-015338A9** in the previous step, (2*E*)-4-(dimethylamino)-2-butenoic acid hydrochloride (305 mg), DIEA (1 mL), HATU (703 mg) and DMF (10 mL) were added to a reaction flask and stirred at room temperature for 2 h. After the reaction was completed, the solvent was removed by rotary evaporation, and the residue was separated by a reverse phase column (acetonitrile-water with 0.01% formic acid) to give **SZ-015338** (195.6 mg). LCMS: $[M+H]^+$ 525.27.

$^1H$ NMR (400 MHz, DMSO-$d_6$) $\delta$ 8.40 (d, *J*= 7.8 Hz, 1H), 7.3 (s, 1H), 6.70-6.57 (m, 2H), 5.21 (d, *J*= 20.3 Hz, 1H), 4.24-4.17 (m, 1H), 4.00 (br, 2H), 3.87 -3.75 (m, 2H), 3.68-3.55 (m, 5H), 3.02-2.88 (m, 2H), 2.63 (d, *J*= 5.6 Hz, 6H), 2.23-1.95 (m, 3H), 1.88-1.85 (m, 2H), 1.64 -1.53 (m, 2H), 1.34 (d, *J*= 6.9 Hz, 6H), 0.99-0.90 (m, 4H).

**Example 37 SZ-015339:** (*S,E*)-1-(4-(dimethylamino)but-2-enoyl)pyrrolidin-3-yl 4-((2,7-diisopropylimidazo[2,1-*f*][1,2,4]triazin-4-yl)amino)piperidine-1-carboxylate

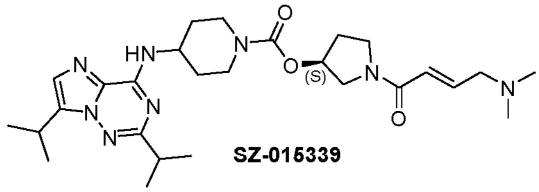

**SZ-015339**

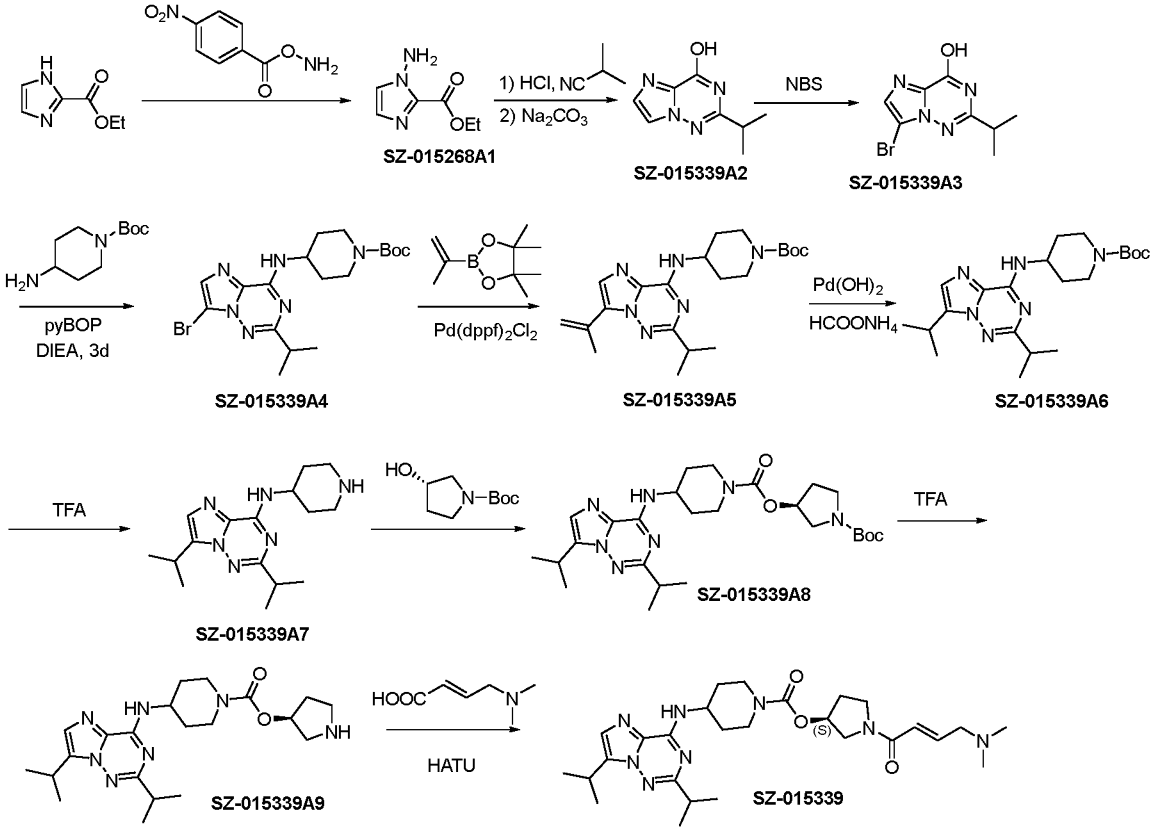


Step 2:

Dry **SZ-015339A1** (3.1 g) and anhydrous isobutyronitrile (50 mL) were added to a three-necked flask and bubbled for 10 min with nitrogen introduced through a conduit. The reaction solution was cooled to 0 °C and stirred, and then dry hydrogen chloride gas was introduced for 20 min. The reaction solution was then stirred at 80 °C for 1 h. After the reaction was completed, the reaction solution was concentrated, and the remaining solid was slurried with diethyl

ether and filtered to give a crude product as an intermediate, which was directly used in the next step without purification. The intermediate, 1,4-dioxane (40 mL), water (20 mL) and sodium bicarbonate (1.6 g) were added to a reaction flask and stirred at 100 °C for 1 h. After the reaction was completed, the solvent was removed under reduced pressure, and the remaining solid was diluted with acetonitrile and filtered to give a crude product of **SZ-015339A2** (4 g, a white solid) containing sodium chloride. LCMS: $[M+1]^+$ 179.09.

Step 3:

**SZ-015339A2** (1.5 g), NBS (1.42 g) and DMF (50 mL) were added to a reaction flask and stirred at 80 °C for 1 h. The reaction solution was concentrated by rotary evaporation under reduced pressure to remove the solvent, and the residue was diluted with dichloromethane and filtered to give a crude product of **SZ-015339A3** (1.4 g, a white solid) containing sodium chloride. LCMS: 258.42.

Step 4:

**SZ-015339A3** (1.43 g), 1-Boc-4-aminopiperidine (2.4 g), PyBOP (4.68 g), DCE (20 mL) and DIEA (1.5 mL) were added to a reaction flask and stirred at room temperature for 12 h. After the reaction was completed, the reaction solution was filtered and washed with dichloromethane. The organic phase was concentrated by rotary evaporation to remove the solvent, and the resulting crude product was purified by flash silica gel column chromatography (EA/PE = 0%-40%) to give product **SZ-015339A4** (1.43 g). LCMS: 440.60.

Step 5:

**SZ-015339A4** (1.27 g), isopropenylboronic acid pinacol ester (1 g), $Pd(dppf)_2Cl_2$ (244 mg), potassium carbonate (1.24 g), 1,4-dioxane (36 mL) and water (6 mL) were added to a reaction flask and stirred at 80 °C for 3 h under nitrogen atmosphere. The solvent was removed by rotary evaporation, and the residue was diluted with ethyl acetate and filtered. The organic phase was concentrated by rotary evaporation to remove the solvent, and the resulting crude product was purified by flash silica gel column chromatography (EA/PE = 0%-30%) to give product **SZ-015339A5** (1.29 g). LCMS: $[M+1]^+$ 401.26.

Step 6:

**SZ-015339A5** (1.2 g), $Pd(OH)_2$ (160 mg), ammonium formate (1.2 g) and methanol (30 mL) were added to a reaction flask and stirred at 80 °C for 1 h. The solvent was removed by rotary evaporation, and the residue was diluted with ethyl acetate and filtered. The organic phase was concentrated by rotary evaporation to remove the solvent, and the resulting crude product was purified by flash silica gel column chromatography (EA/PE = 0%-30%) to give product **SZ-015339A6** (888 mg). LCMS: $[M+1]^+$ 403.27.

Step 7:

**SZ-015339A6** (984 mg), dichloromethane (10 mL) and TFA (2 mL) were added to a reaction flask and stirred at room temperature for 1 h. After the reaction was completed, the reaction solution was concentrated by rotary evaporation to give a crude product of **SZ-015339A7,** which was directly used in the next step without separation. LCMS: $[M+1]^+$ 303.22.

Step 8:

(*S*)-1-*N*-*tert*-butoxycarbonyl-3-hydroxypyrrolidine (505 mg), acetonitrile (10 mL), triethylamine (2 mL) and p-nitrophenyl chloroformate (544 mg) were added successively to a reaction flask and stirred at 40 °C for 2 h. The crude product of **SZ-015339A7** in the previous step was dissolved in acetonitrile (5 mL), triethylamine was added to adjust the solution to be alkaline, and then the reaction solution was added dropwise to the solution of **SZ-015339A7** in acetonitrile. The resulting reaction solution was stirred at room temperature overnight. After the reaction was completed, the solvent was removed by rotary evaporation, and the residue was purified by flash silica gel column chromatography (methanol:dichloromethane = 0%-5%) to give **SZ-015339A8** (987 mg). LCMS: $[M+1]^+$ 516.24.

Step 9:

**SZ-015339A8** (987 mg), dichloromethane (10 mL) and TFA (3 mL) were added to a reaction flask and stirred

at room temperature for 1 h. After the reaction was completed, the reaction solution was concentrated by rotary evaporation to give a crude product of **SZ-015339A9,** which was directly used in the next step without separation. LCMS: $[M+1]^+$ 416.10.

Step 10:

The crude product of **SZ-015339A9** in the previous step, (2*E*)-4-(dimethylamino)-2-butenoic acid hydrochloride (378 mg), DIEA (2 mL), HATU (874 mg) and DMF (10 mL) were added to a reaction flask and stirred at room temperature for 2 h. After the reaction was completed, the solvent was removed by rotary evaporation, and the residue was separated by a reverse phase column (acetonitrile-water with 0.01% formic acid) to give **SZ-015339** (440 mg). LCMS: $[M+1]^+$ 527.28.

$^1$H NMR (400 MHz, DMSO-$d_6$) δ 8.40 (d, *J* = 7.7 Hz, 1H), 7.33 (s, 1H), 6.74- 6.60 (m, 2H), 5.21 (d, *J* = 19.3 Hz, 1H), 4.34-4.26 (m, 1H), 4.02 (br, 2H), 3.90-3.76 (m, 3H), 3.69-3.60 (m, 2H), 3.56-3.27 (m, 2H), 3.02-2.86 (m, 3H), 2.79 (s, 6H), 2.28-2.02 (m, 2H), 1.93-1.90 (m, 2H), 1.67-1.54 (m, 2H), 1.34 (d, *J* = 6.9 Hz, 6H), 1.28 (d, *J* = 6.8 Hz, 6H).

**Example 38 SZ-015346:** (*S,E*)-1-(4-(dimethylamino)but-2-enoyl)pyrrolidin-3-yl 4-((2-isopropoxy-7-isopropylimidazo[2,1-*f*][1,2,4]triazin-4-yl)amino)piperidine-1-carboxylate

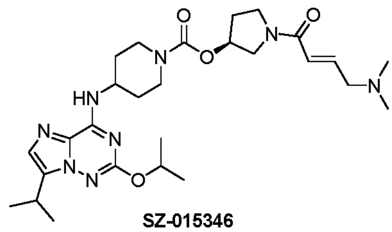

SZ-015346

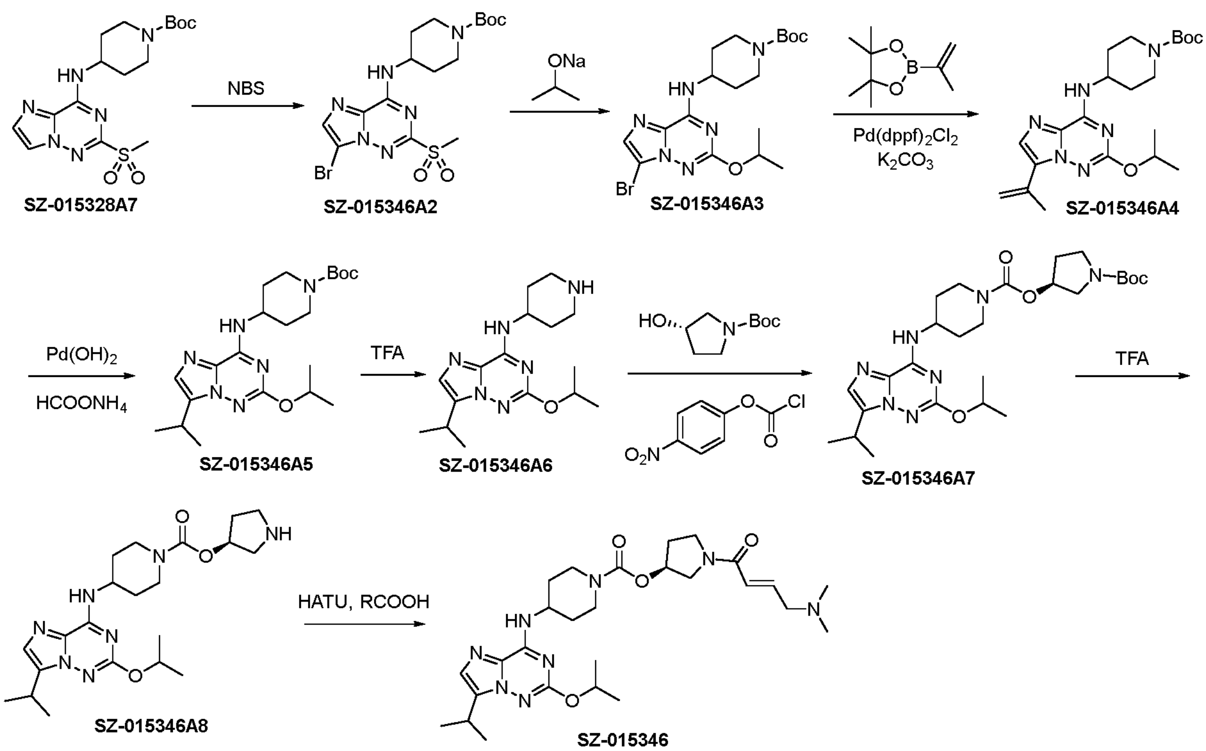


Step 1:

**SZ-015328A7** (792 mg), NBS (711 mg) and MeCN (30 mL) were added to a reaction flask and stirred at 80 °C for 1 h. After the reaction was completed, the solvent was removed by rotary evaporation, and the resulting crude product was purified by flash silica gel column chromatography (EA/PE = 0%-40%) to give product**SZ-015346A2** (1.3 g). LCMS:

374.81, 376.77, 420.80, 418.83.

Step 2:

Isopropanol (100 mL) was added to a reaction flask, and NaH (500 mg) was added with stirring at 0 °C. The reaction solution was stirred at room temperature for 30 min, and then **SZ-015346A2** (1.3 g) was added. The resulting reaction solution was stirred at 80 °C for 1 h. Water (400 mL) was added to the reaction solution to quench the reaction, and dichloromethane (200 mL × 3) was added for extraction. The organic phases were combined and concentrated by rotary evaporation under reduced pressure to remove the solvent, and the resulting crude product was purified by flash silica gel column chromatography (EA/PE = 0%-30%) to give product **SZ-015346A3** (808 mg). LCMS: 418.81, 420.82

Step 3:

**SZ-015346A3** (808 mg), isopropenylboronic acid pinacol ester (504 mg), Pd(dppf)$_2$Cl$_2$ (163 mg), potassium carbonate (552 mg), 1,4-dioxane (20 mL) and water (5 mL) were added to a reaction flask and stirred at 80 °C for 3 h under nitrogen atmosphere. The solvent was removed by rotary evaporation, and the residue was diluted with ethyl acetate and filtered. The organic phase was concentrated by rotary evaporation to remove the solvent, and the resulting crude product was purified by flash silica gel column chromatography (EA/PE = 0%-30%) to give product **SZ-015346A4** (900 mg). LCMS: $[M+1]^+$ 417.11.

Step 4:

**SZ-015346A4** (900 mg), $Pd(OH)_2$ (200 mg), ammonium formate (3 g) and methanol (30 mL) were added to a reaction flask and stirred at 80 °C for 1 h. The solvent was removed by rotary evaporation, and the residue was diluted with ethyl acetate and filtered. The organic phase was concentrated by rotary evaporation to remove the solvent, and the resulting crude product was purified by flash silica gel column chromatography (EA/PE = 0%-30%) to give product **SZ-015346A5** (438 mg). LCMS: $[M+1]^+$ 419.11.

Step 5:

**SZ-015346A5** (438 mg), dichloromethane (10 mL) and TFA (3 mL) were added to a reaction flask and stirred at room temperature for 1 h. After the reaction was completed, the reaction solution was concentrated by rotary evaporation to give a crude product of **SZ-015346A6,** which was directly used in the next step without separation. LCMS: $[M+1]^+$ 319.10.

Step 6:

(*S*)-1-*N*-*tert*-butoxycarbonyl-3-hydroxypyrrolidine (280 mg), acetonitrile (20 mL), triethylamine (5 mL) and *p*-nitrophenyl chloroformate (302 mg) were added successively to a reaction flask and stirred at 40 °C for 2 h. The crude product of **SZ-015346A6** in the previous step was dissolved in acetonitrile (5 mL), triethylamine was added to adjust the solution to be alkaline, and then the reaction solution was added dropwise to the solution of **SZ-015346A6** in acetonitrile. The resulting reaction solution was stirred at room temperature for 4 h. After the reaction was completed, the solvent was removed by rotary evaporation, and the residue was purified by flash silica gel column chromatography (methanol:dichloromethane = 0%-5%) to give **SZ-015346A7** (688 mg). LCMS: $[M+1]^+$ 532.24.

Step 7:

**SZ-015346A7** (688 mg), dichloromethane (10 mL) and TFA (3 mL) were added to a reaction flask and stirred at room temperature for 1 h. After the reaction was completed, the reaction solution was concentrated by rotary evaporation to give a crude product of **SZ-015346A8,** which was directly used in the next step without separation. LCMS: $[M+1]^+$ 432.10.

Step 8:

The crude product of **SZ-015346A8** in the previous step, (2*E*)-4-(dimethylamino)-2-butenoic acid hydrochloride (330 mg), DIEA (3 mL), HATU (760 mg) and DMF (20 mL) were added to a reaction flask and stirred at room temperature for 2 h. After the reaction was completed, the solvent was removed by rotary evaporation, and the residue was separated by a reverse phase column (acetonitrile-water with 0.01% formic acid) to give **SZ-015346** (324 mg). LCMS: $[M+1]^+$ 543.35.

$^{1}$H NMR (400 MHz, DMSO-$d_6$) δ 8.55 (d, J = 8.1 Hz, 1H), 7.24 (s, 1H), 6.66-6.59 (m, 1H), 6.41-6.32 (m, 1H), 5.21 (d, *J*= 19.3 Hz, 1H), 5.10-5.01 (m, 1H), 4.28-4.20 (m, 1H), 3.98 (br, 2H), 3.82-3.70 (m, 1H), 3.62-3.34 (m, 3H), 3.24-3.17 (m, 1H), 3.05 (d, J = 6.1 Hz, 2H), 2.91 (br, 2H), 2.16 (s, 6H), 2.15-1.97 (m, 2H), 1.82-1.80 (m, 2H), 1.61-1.52 (m, 2H), 1.33-1.30 (m, 12H).

**Example 39 SZ-015297:** (*S,E*)-1-(4-(dimethylamino)but-2-enoyl)pyrrolidin-3-yl-4-((2-(hydroxymethyl)-9-isopropyl-9*H*-purin-6-yl)amino)piperidine-1-carboxylate

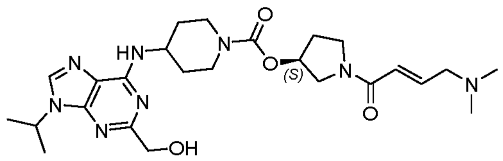

**SZ-015297**

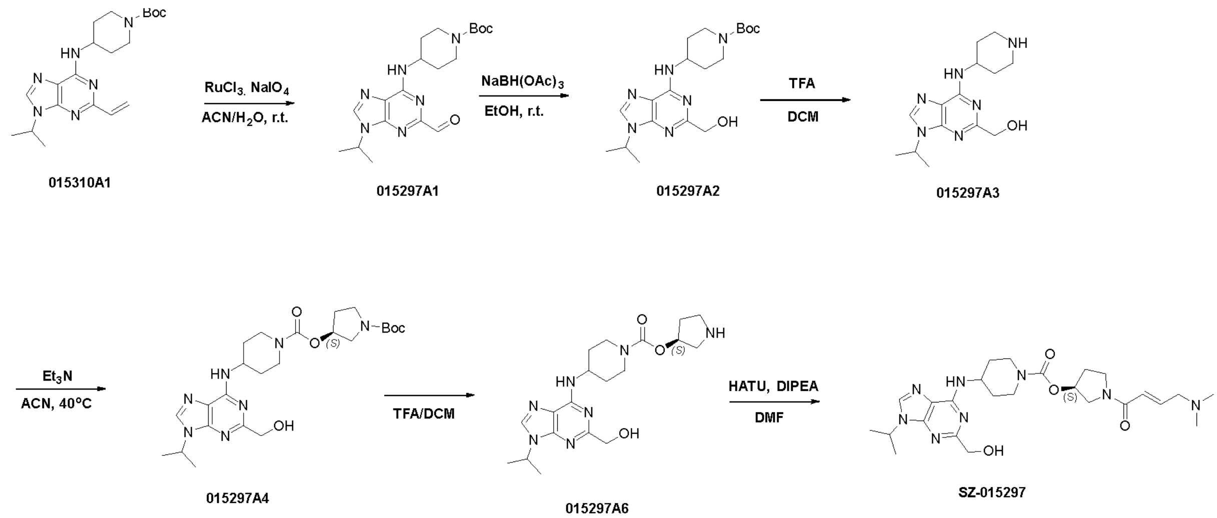

015310A1
015297A1
015297A2
015297A3
015297A4
015297A6
SZ-015297

Step 1:

**015310A1** (1.9 g, 5 mmol) was dissolved in acetonitrile (50 mL) and water (10 mL), and ruthenium trichloride (82 mg, 5 mol%) and $NaIO_4$ (3.2 g, 15 mmol) were added. The reaction solution was stirred at room temperature for 12 h, and then water (100 mL) was added and ethyl acetate (30 mL × 3) was added for extraction. The organic phase was dried over sodium sulfate and concentrated by rotary evaporation under reduced pressure to give a crude product, which was purified by flash silica gel column chromatography to give **015297A1**(1.2 g, a white solid). LCMS: $[M+H]^+$ 389.3.

Step 2:

**015297A1**(1.2 g, 3 mmol) was dissolved in ethanol (40 mL), and $NaBH(OAc)_3$ (1.27 g, 6 mmol) was added. The reaction solution was stirred at room temperature for 2 h and concentrated by rotary evaporation under reduced pressure to give a crude product, which was purified by flash silica gel column chromatography to give **015297A2** (380 mg, a white solid). LCMS: $[M+H]^+$ 391.4.

Step 3:

Compound **015297A2** (340 mg, 0.85 mmol) was dissolved in dichloromethane (9 mL), and trifluoroacetic acid (3 mL) was added. The reaction solution was stirred at room temperature for 1 h and concentrated by rotary evaporation under reduced pressure to give **015297A3 trifluoroacetate** (340 mg, 100%, a colorless oily liquid). LCMS: $[M+H]^+$ 291.3.

Step 4:

Compound **015297A3 trifluoroacetate** (340 mg, 0.85 mmol) was dissolved in acetonitrile (9 mL), and triethylamine (258 mg, 2.55 mmol) and tert-butyl (*S*)-3-(((4-nitrophenoxy) carbonyl)oxo)pyrrolidine-1-carboxylate (300 mg, 0.85 mmol) were added successively. The reaction solution was stirred at room temperature for 2 h and concentrated by rotary evaporation under reduced pressure to give **a crude product of 015297A4,** which was purified by flash silica gel column chromatography to give **015297A4** (340 mg, a white solid). LCMS: $[M+H]^+$ 504.5.

Step 5:

Compound **015297A4** (340 mg, 0.68 mmol) was dissolved in dichloromethane (6 mL), and trifluoroacetic acid (2 mL) was added. The reaction solution was stirred at room temperature for 0.5 h and concentrated by rotary evaporation under reduced pressure to give **015297A5 trifluoroacetate** (340 mg, 100%, a colorless oily liquid). LCMS: $[M+H]^+$ 404.4.

Step 6:

Compound **015297A5 trifluoroacetate** (340 mg, 0.68 mmol) was dissolved in DMF (5 mL), and DIPEA (260 mg, 2 mmol), (*E*)-4-(dimethylamino)but-2-enoic acid hydrochloride (112.5 mg, 0.68 mmol) and HATU (310 mg, 0.82 mmol) were added successively. The reaction solution was stirred at room temperature for 0.5 h and concentrated by rotary evaporation under reduced pressure to give **a crude product of SZ-015297,** which was subjected to reverse phase column chromatography (acetonitrile/water with 0.1% formic acid) and lyophilized to give **SZ-015297** (70 mg, a white solid). LCMS: $[M+H]^+$ 515.13.

$^1H$ NMR (400 MHz, DMSO-$d_6$) $\delta$ 8.22 (s, 1H), 7.61 (d, J = 6.4 Hz, 1H), 6.62 (ddd, J = 16.3, 10.5, 6.1 Hz, 1H), 6.50-6.31 (m, 1H), 5.15 (d, J = 23.4 Hz, 1H), 4.74 (dt, J = 13.2, 6.6 Hz, 1H), 4.41 (s, 3H), 4.04-3.27 (m, 6H), 3.15 (d, J = 5.3 Hz, 2H), 2.88 (t, J = 26.8 Hz, 3H), 2.34-1.75 (m, 10H), 1.60-1.40 (m, 8H).

**Efficacy Example 1: Activity Assay**

$IC_{50}$ of test compounds inhibiting CDK7 was detected using the mobility shift assay, and effective compounds were evaluated and screened. The initial concentration of compound was 10 μM, and the compound was 3-fold diluted to obtain 10 concentrations. Duplicate wells were set for the assay, and the pre-incubation time of compound and enzyme was 60 min.

1. Preparation of compounds

Compound powders were dissolved in 100% DMSO to prepare 10 mM stock solution, which was 3-fold diluted with DMSO, with 0.5 mM as the initial concentration, to obtain 10 concentration gradients of compound solutions.

2. Reaction process of kinase

(1) Compound at each concentration was 8.3-fold diluted with $ddH_2O$, and then 2 μL of each compound solution and positive control were added to a 384-well plate.
(2) 5 μL of a kinase working solution (CDK7/Cyclin H/MAT1) was added to the compound wells and positive control wells to make the final concentration of CDK7 be 3 nM.
(3) The plate was pre-incubated at room temperature for 60 min.
(4) 5 μL of substrate ((5-FAM)-YSPTSPSYSPTSPSYSPTSPSKKKK-NH2) solution was added to each well to initiate the reaction; the concentration of ATP is 2 mM, and the concentration of peptide substrate was 2 μM.
(5) The 384-well plate was incubated at 27 °C for 36 min.
(6) 4 μL of 80 mM EDTA was added to terminate the reaction.
(7) The conversion rates were read using a Caliper EZ Reader II.

3. The results are shown in Table 1 below

Table 1

| Compound | $IC_{50}$ (nM) |
|---|---|
| **SZ-015200** (Example 1 in WO2019099298) | 54.06 |
| **SZ-015201** (Example 3 in WO2019099298) | 134.00 |
| **CT7001** | 33.20 |
| **SZ-015256** | 54.29 |
| **SZ-015095** | 128.30 |
| **SZ-015272** | 278.20 |
| **SZ-015273** | 44.46 |
| **SZ-0015268** | 23.56 |
| **SZ-015274** | 197.16 |

**Efficacy Example 2: Inhibition of Compounds against Cell Proliferation**

**1. Preparation of compounds**

The compounds were each prepared with 100% DMSO into 10 mM mother liquor.

**2. Test method**

(1) Day 0: cell plating

HCC70 cells and OVCAR3 cells were digested and counted. Cells were diluted to an appropriate concentration according to cell density. 100 μL of cells were added to each well of a 96-well plate. Medium wells served as blank control (Min). The cells were incubated overnight in an incubator at 37 °C/5% $CO_2$.

(2) Day 1: treatment of compounds

A 200-fold stock solution of a compound was prepared with DMSO, and the compound was diluted with growth medium to a 3-fold stock solution, i.e., 3 μL of each of 200-fold stock solutions of compound at different concentrations was added to 197 μL of medium. 50 μL of diluted compound was added to each well and the mixture was incubated at 37 °C/5% $CO_2$ for 72 h.

(3) Day 4: detection

The 96-well plate was equilibrated to room temperature before detection. 40 μL of CellTiter-Glo solution was added to each well. The mixture was mixed for 2 min using a horizontal shaker to induce cell lysis. The mixture was incubated at room temperature for 60 min to stabilize fluorescence signals. Fluorescence values were read on ENVISION.

**3. Data analysis**

(1) Data was analyzed by GraphPad Prism 5
(2)

$$\%\ \text{inhibition rate} = (\text{maximum signal} - \text{compound signal})/(\text{maximum signal} - \text{minimum signal}) \times 100$$

(3) Maximum signal: signal of DMSO well

(4) Minimum signal: signal of medium well

**4. The results are shown in Table 2 below**

Table 2

| Compound No. | HCC70 $IC_{50}$ (nM) | OVCAR-3 $IC_{50}$ (nM) |
|---|---|---|
| **SZ-015200** (i.e., compound of Example 1 in WO2019099298A1) | 34.14 | 32.00 |
| **SZ-015201** (compound of Example 3 in WO2019099298A1) | 25.47 | 22.56 |
| CT7001 | 310.20 | 467.00 |
| **SZ-015256** | 174.70 | 75.10 |
| **SZ-015095** | 479.30 | 137.50 |
| **SZ-015264** | 181.00 | 233.70 |
| **SZ-015272** | 947.90 | 460.2 |
| **SZ-015273** | 291.20 | 31.85 |
| **SZ-015268** | 57.55 | 29.92 |
| **SZ-015274** | 186.6 | 150.9 |
| **SZ-015282** | 82.36 | 92.14 |
| **SZ-015294** | 50.19 | 72.07 |
| **SZ-015284** | 97.44 | - |
| **SZ-015289** | 116.9 | - |
| **SZ-015285** | 1049 | 620 |
| **SZ-015286** | 569.6 | 166.9 |
| **SZ-015303** | 128.8 | 114.3 |
| **SZ-015307** | 77.95 | 138.8 |
| **SZ-015308** | 92.06 | - |
| **SZ-015295** | 110.3 | 62.81 |
| **SZ-015298** | 405.4 | - |
| **SZ-015310** | 29.49 | 58.4 |
| **SZ-015306** | 205.2 | 280.7 |
| **SZ-015311** | 95.01 | 99.6 |
| **SZ-015297** | 252.5 | 167.6 |
| **SZ-015317** | 237.4 | 428.6 |
| **SZ-015319** | 50.53 | 72.46 |
| **SZ-015326** | 50.26 | 33.23 |
| **SZ-015330** | 60.99 | 43.96 |
| **SZ-015331** | 70.10 | 36.73 |
| **SZ-015332** | 51.51 | 44.68 |
| **SZ-015342** | 418.9 | 204 |
| **SZ-015338** | 48.69 | 29.7 |
| **SZ-015339** | 54.68 | 64.28 |

(continued)

| Compound No. | HCC70 $IC_{50}$ (nM) | OVCAR-3 $IC_{50}$ (nM) |
|---|---|---|
| **SZ-015328** | 252.3 | 110.4 |
| **SZ-015346** | 139.5 | 48.77 |
| Note: "-" represents not tested. | | |

**Efficacy Example 3: Inhibition of Compounds against Cell Proliferation**

The effect of SZ-015200, SZ-015256 and SZ-015268 on cell proliferation after incubation with HCC70, OVCAR-3, HCT116 and HCC1806 cells for 3 days was detected according to the method described in Efficacy Example 2, and the results are shown in Table 3.

Table 3. Cell proliferation inhibition rates

| Compound No. | HCC70 $IC_{50}$ (nM) | OVCAR-3 $IC_{50}$ (nM) | HCT116 $IC_{50}$ (nM) | HCC1806 $IC_{50}$ (nM) |
|---|---|---|---|---|
| **SZ-015200** (i.e., compound of Example 1 in WO2019099298A1) | 68.61 | 101.50 | 24.11 | 65.43 |
| **SZ-015256** | 50.85 | 45.31 | 25.26 | 44.47 |
| **SZ-015268** | 33.00 | 80.56 | 12.53 | 61.55 |

**Efficacy Example 4: *In Vivo* Pharmacokinetic Properties after Intragastric Administration of Compounds in Mice**

Male ICR mice were divided into different groups with 3 mice in each group, and single intragastric administration was conducted for each group. The compounds were each formulated with 10% DMSO/10% Solutol/80% (20% Captisol) to reach an appropriate concentration and then administered intragastrically at 20 mg/kg. Blood was taken 5 min, 15 min, 30 min, 1 h, 2 h, 4 h, 6 h, 8 h and 24 h after administration, and then subjected to EDTA-K2 anticoagulation. The concentration of the compound in the plasma was quantitatively detected by an LC-MS/MS method. Pharmacokinetic parameters of each compound were calculated by Phoenix WinNonlin 7.0, and the results are summarized in Table 4 below.

Table 4. Pharmacokinetic parameters of each group after intragastric administration of compounds in mice

| | **Dosage** mg/kg | $T_{1/2}$ h | $T_{max}$ h | $C_{max}$ ng/mL | $AUC_{(0-t)}$ h*ng/mL | $AUC_{(0-\infty)}$ h*ng/mL |
|---|---|---|---|---|---|---|
| **SZ-015200** | **20.00** | 1.97 | 0.67 | 1209.14 | 2926.85 | 3121.49 |
| **SZ-015256** | **20.00** | 1.48 | 0.50 | 3379.92 | 6258.34 | 6375.00 |
| **SZ-015268** | **20.00** | 1.39 | 0.42 | 1544.63 | 3095.46 | 3149.75 |

The results show that compared with the control compound SZ-015200, the $C_{max}$ and the AUC of SZ-015256 and SZ-015268 in mice after single intragastric administration were not lower than those of SZ-015200, with those of SZ-015256 being significantly higher.

**Efficacy Example 5: *In Vivo* Pharmacokinetic Properties after Intragastric Administration of Compounds in Rats**

Male SD rats were divided into different groups with 3 rats in each group, and single intragastric administration was conducted for each group. The compounds were each formulated with 10% DMSO/10% Solutol/80% (20% Captisol) to reach an appropriate concentration and then administered intragastrically at 40 mg/kg. Blood was taken 5 min, 15 min, 30 min, 1 h, 2 h, 4 h, 6 h, 8 h and 24 h after administration, and then subjected to EDTA-K2 anticoagulation. The concentration of the compound in the plasma was quantitatively detected by an LC-MS/MS method. Pharmacokinetic parameters of each compound were calculated by Phoenix WinNonlin 7.0, and the results are summarized in Table 5 below.

Table 5. Pharmacokinetic parameters of each group after intragastric administration of compounds in rats

| | **Dosage** mg/kg | $T_{1/2}$ h | $T_{max}$ h | $C_{max}$ ng/mL | $AUC_{(0-t)}$ h*ng/mL | $AUC_{(0-\infty)}$ h*ng/mL |
|---|---|---|---|---|---|---|
| **SZ-015200** | **40.00** | 4.11 | 3.33 | 322.98 | 1988.83 | 2554.45 |
| **SZ-015256** | **40.00** | 2.99 | 4.67 | 783.01 | 7828.87 | 7879.45 |
| **SZ-015268** | **40.00** | 3.92 | 4.00 | 542.53 | 4449.20 | 4513.87 |

The results show that compared with the control compound SZ-015200, the $C_{max}$ and the AUC of SZ-015256 and SZ-015268 in rats after single intragastric administration were significantly higher, which indicates that SZ-015256 and SZ-015268 have better pharmacokinetic properties.

**Efficacy Example 6: *In Vivo* Efficacy of Compounds in Subcutaneous Xenograft Tumor Model of Human Colon Cancer Cells HCT116**

**Test method**

**Cell culturing**

HCT116 tumor cells used in this test were purchased from ATCC. HCT116 tumor cells were cultured in McCoy's 5a medium containing inactivated 10% fetal bovine serum, 100 U/mL penicillin, 100 μg/mL streptomycin and 2 mM glutamine in an incubator at 37 °C/5% $CO_2$. The cells were divided and transferred to other bottles every 3 to 4 days for passaging when the culture bottle was full of cells. The tumor cells in the logarithmic growth phase were used for tumor seeding *in vivo.*

**Seeding and grouping of tumor cells**

HCT116 tumor cells resuspended in serum-free McCoy's 5a culture solution were seeded subcutaneously into the right costal part of male BALB/c nude mice at $5 \times 10^6$/0.1 mL. When the tumor grew to 101 $mm^3$, animals with uniform tumor volume were selected and divided into groups with 8 animals in each group for administration, and the specific administration regimens are shown in Table 6 below.

**Table 6. Administration regimens**

| **Group** | **Cases of animals** | **Treatment** | **Dosage (mg/kg)*** | **Administration volume (μL/g)** | **Route of administration** | **Course of treatment** |
|---|---|---|---|---|---|---|
| 1 | 8 | Vehicle (control) group | - | 10 | p.o. | QD × 21 days |
| 2 | 8 | SZ-015200 | 25 | 10 | p.o. | QD × 21 days |
| 3 | 8 | SZ-015256 | 25 | 10 | p.o. | QD × 21 days |
| 4 | 8 | SZ-015256 | 15 | 10 | p.o. | QD × 21 days |
| 5 | 8 | SZ-015268 | 25 | 10 | p.o. | QD × 21 days |
| Note: *: dosage refers to the amount of the pure drug | | | | | | |

**Test indexes**

Tumor volume: the long and short diameters of the tumor were measured twice every week using a vernier caliper for calculating the tumor volume, and the calculation formula was: volume = 0.5 × long diameter × short $diameter^2$.

Reaction of animals after the administration: the mice were weighed while measuring tumor volume. The relationship between the change in the body weight of mice and the time of administration was recorded. At the same time, the survival and health of the mice, such as animal activity, feeding and other general states during the administration were observed.

**Drug evaluation indexes**

Relative increase ratio of tumor volume T/C (%)

$$T/C\ (\%) = \text{mean value of relative tumor volume of treatment group/mean value of relative tumor volume of control group} \times 100$$

Tumor growth inhibition rate (TGI, %)

$$\text{Tumor growth inhibition rate (TGI, \%)} = (1 - T/C) \times 100$$

**Statistical analysis**

Statistical analysis between groups was performed for tumor volume using One-Way ANOVA test, and a significant difference was considered to be present when $p < 0.05$.

**Test results**

**Change in body weight of animal**

The body weight of the experimental animal was used as a reference index for indirectly measuring the toxicity of the drug. Relative body weight changes are shown in FIG. 1. The results show that the body weight of animals in each administration group was reduced by a certain degree compared with that in vehicle control group, but there were no significant differences in body weight of animals between administration groups. The experimental animals in all groups were in good state in terms of activity, feeding and other general states, and no obvious clinical abnormalities were found. It is indicated that the animal can tolerate an administration dose of 25 mg/kg.

**Tumor growth curves**

The tumor growth curves are shown in FIG. 2.

**Calculation of anti-tumor efficacy evaluation index TGI**

TGI data are summarized in Table 7.

Table 7. Inhibition of compounds against tumor growth in xenograft model of HCT116 human colon cancer

| Group | Treatment | Tumor volume ($mm^3$, day 21 after grouping)[a] | % proliferation percentage of tumor volume (Day 21 after grouping)[a] | Tumor inhibition rate (%)[c] | *P* value[b] (Dunnett T3) |
|---|---|---|---|---|---|
| **1** | **Solvent control** | 1,062±86 | 1,061±65 | - | - |
| **2** | **SZ-015200 25 mg/kg QDx21** | 766±71 | 764±58 | 27.9 | 0.210 |
| **3** | **SZ-015256 25 mg/kg QDx21** | 201±43 | 192±38 | 81.9 | <0.001 |
| **4** | **SZ-015256 15 mg/kg QDx21** | 402±42 | 407±40 | 61.6 | 0.001 |

(continued)

| Group | Treatment | Tumor volume (mm³, day 21 after grouping)[a] | % proliferation percentage of tumor volume (Day 21 after grouping)[a] | Tumor inhibition rate (%)[c] | *P* value[b] (Dunnett T3) |
|---|---|---|---|---|---|
| **5** | **SZ-015268 25 mg/kg QDx21** | 172±41 | 167±39 | 84.3 | <0.001 |

Note: a. mean value±standard error, b. vs. solvent control group;
c. tumor growth inhibition rate (TGI, %) = (1 - T/C) × 100, where T/C (%) = mean value of relative tumor volume of treatment group/mean value of relative tumor volume of control group × 100

The above results show that: compound SZ-015256 shows significant anti-tumor effect in both 25 mg/kg and 15 mg/kg treatment groups of this compound, compound SZ-015268 shows significant anti-tumor effect in 25 mg/kg treatment group of this compound, and compound SZ-015200 shows slight anti-tumor tendency in 25 mg/kg treatment group of this compound. Compared with the control compound SZ-015200, SZ-015256 and SZ-015268 have stronger effect on inhibiting tumor proliferation and have extremely significant anti-tumor advantage.

## Claims

1. A compound of formula I:

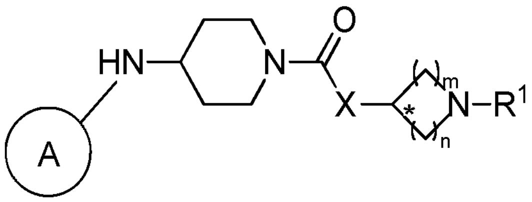

**I**

or a tautomer, a stereoisomer or an isotopic derivative thereof, or a pharmaceutically acceptable salt of any of the foregoing, or a crystalline form or a solvate of any of the foregoing;
wherein, ring A is

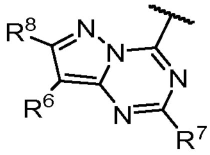 , 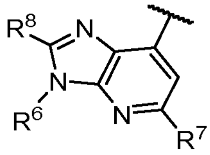 , 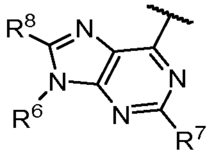 ,

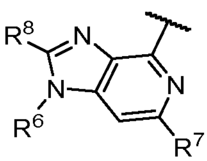 , 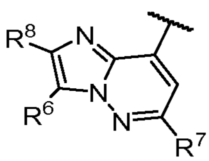 , 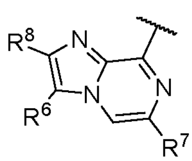 or 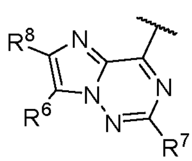 ;

X is O or $NR^a$;
$R^a$ is hydrogen, methyl or ethyl;
m is 1 or 2;
n is 1, 2 or 3;
$R^1$ is

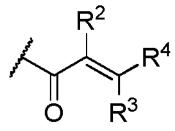 or 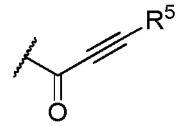 ;

$R^2$ is hydrogen or $C_{1-6}$ alkyl;
$R^3$ is hydrogen or $C_{1-6}$ alkyl;
$R^4$ is hydrogen, halogen, $C_{1-6}$ alkyl or $-CH_2-NR^{4a}R^{4b}$;
$R^{4a}$ and $R^{4b}$ are each independently hydrogen or $C_{1-6}$ alkyl; or, $R^{4a}$ and $R^{4b}$, together with a nitrogen atom attached thereto, form a substituted or unsubstituted 4-6 membered heterocycloalkyl, wherein the 4-6 membered heterocycloalkyl has 0, 1 or 2 additional heteroatoms independently selected from N, O and S, and the substituted 4-6 membered heterocycloalkyl means that the 4-6 membered heterocycloalkyl is substituted with 1, 2, 3 or 4$R^b$;
each $R^b$ is independently halogen, hydroxy or $C_{1-4}$ alkyl;
$R^5$ is hydrogen or $C_{1-6}$ alkyl;
each $R^6$ is independently hydrogen, cyclopropyl or $C_{1-6}$ alkyl;
each $R^7$ is independently hydrogen, a substituted or unsubstituted $C_{1-6}$ alkyl, a substituted or unsubstituted phenyl, a substituted or unsubstituted 5-6 membered heteroaryl, a substituted or unsubstituted $C_{3-6}$ cycloalkyl, $-OR^{7a}$ or $-NR^{7b}R^{7c}$, wherein the substituted $C_{1-6}$ alkyl, the substituted $C_{3-6}$ cycloalkyl, the substituted phenyl and the substituted 5-6 membered heteroaryl mean that the $C_{1-6}$ alkyl, the $C_{3-6}$ cycloalkyl, the phenyl and the 5-6 membered heteroaryl are each independently substituted with 1, 2, 3 or 4 $R^{7d}$;
each $R^{7d}$ is independently hydroxy, halogen, $C_{1-4}$ alkyl, $-NR^{a1}R^{a2}$ or $C_{1-4}$ alkoxy;
$R^{7a}$ is hydrogen, a substituted or unsubstituted $C_{1-6}$ alkyl, a substituted or unsubstituted $C_{3-6}$ cycloalkyl, a substituted or unsubstituted 4-6 membered heterocycloalkyl, or a substituted or unsubstituted 5-6 membered heteroaryl, wherein the substituted $C_{1-6}$ alkyl, the substituted $C_{3-6}$ cycloalkyl, the substituted 4-6 membered heterocycloalkyl and the substituted 5-6 membered heteroaryl mean that the $C_{1-6}$ alkyl, the $C_{3-6}$ cycloalkyl, the 4-6 membered heterocycloalkyl and the 5-6 membered heteroaryl are each independently substituted with 1, 2, 3 or 4 $R^c$;
each $R^c$ is independently hydroxy, halogen, $C_{1-4}$ alkyl, $-NR^{c1}R^{c2}$ or $C_{1-4}$ alkoxy;
$R^{7b}$ and $R^{7c}$ are each independently hydrogen, a substituted or unsubstituted $C_{1-4}$ alkyl, a substituted or unsubstituted $C_{3-6}$ cycloalkyl, a substituted or unsubstituted 4-6 membered heterocycloalkyl, or a substituted or unsubstituted 5-6 membered heteroaryl, wherein the substituted $C_{1-4}$ alkyl, the substituted $C_{3-6}$ cycloalkyl, the substituted 4-6 membered heterocycloalkyl and the substituted 5-6 membered heteroaryl mean that the $C_{1-4}$ alkyl, the $C_{3-6}$ cycloalkyl, the 4-6 membered heterocycloalkyl and the 5-6 membered heteroaryl are each independently substituted with 1, 2, 3 or 4 $R^d$;
or, $R^{7b}$ and $R^{7c}$, together with a nitrogen atom attached thereto, form a substituted or unsubstituted 4-6 membered heterocycloalkyl, wherein the 4-6 membered heterocycloalkyl has 0, 1 or 2 additional heteroatoms independently selected from N, O and S, and the substituted 4-6 membered heterocycloalkyl means that the 4-6 membered heterocycloalkyl is substituted with 1, 2, 3 or 4 $R^e$;
each $R^d$ is independently hydroxy, halogen, $C_{1-4}$ alkyl, $-NR^{d1}R^{d2}$ or $C_{1-4}$ alkoxy;
each $R^e$ is independently hydroxy, halogen, $C_{1-4}$ alkyl, $-NR^{e1}R^{e2}$ or $C_{1-4}$ alkoxy;
each $R^{a1}$, each $R^{a2}$ , each $R^{c1}$, each $R^{c2}$ , each $R^{d1}$ , each $R^{d2}$ , each $R^{e1}$ and each $R^{e2}$ are each independently hydrogen or $C_{1-4}$ alkyl;
each $R^8$ is independently hydrogen or $C_{1-4}$ alkyl;
when a carbon atom marked by * has chirality, the carbon atom is in S configuration, R configuration, or a mixture thereof;
the number of heteroatoms in the 4-6 membered heterocycloalkyl and the number of heteroatoms in the 5-6 membered heteroaryl are independently 1, 2 or 3, and each heteroatom is independently selected from N, O and S.

**2.** The compound or the tautomer, the stereoisomer or the isotopic derivative thereof, or the pharmaceutically acceptable salt of any of the foregoing, or the crystalline form or the solvate of any of the foregoing according to claim 1, wherein each $C_{1-4}$ alkyl is independently methyl, ethyl, *n*-propyl, isopropyl, *n*-butyl, isobutyl, *sec*-butyl or *tert*-butyl;

and/or, each halogen is independently fluorine, chlorine, bromine or iodine;
and/or, each $C_{1-6}$ alkyl is independently methyl, ethyl, *n*-propyl, isopropyl, *n*-butyl, isobutyl, *sec*-butyl or *tert*-butyl;
and/or, each $C_{3-6}$ cycloalkyl is independently cyclopropyl, cyclobutyl, cyclopentyl or cyclohexyl;
and/or, each $C_{1-4}$ alkoxy is independently methoxy, ethoxy, *n*-propoxy, isopropoxy, *n*-butoxy, isobutoxy, *sec*-butoxy or *tert*-butoxy.

**3.** The compound or the tautomer, the stereoisomer or the isotopic derivative thereof, or the pharmaceutically acceptable salt of any of the foregoing, or the crystalline form or the solvate of any of the foregoing according to claim 1, wherein,

ring A is

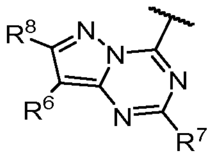, 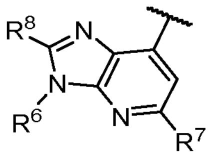, 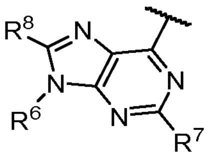, 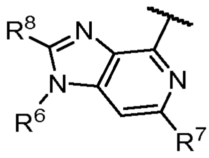 or

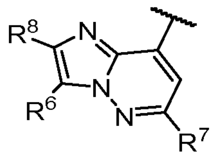;

X is O or $NR^a$;
$R^a$ is hydrogen, methyl or ethyl;
m is 1 or 2;
n is 1, 2 or 3;
$R^1$ is

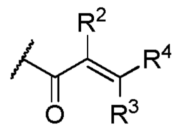 or 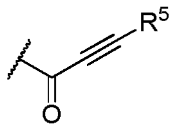;

$R^2$ is hydrogen or $C_{1-6}$ alkyl;
$R^3$ is hydrogen or $C_{1-6}$ alkyl;
$R^4$ is hydrogen, halogen, $C_{1-6}$ alkyl or $-CH_2-NR^{4a}R^{4b}$;
$R^{4a}$ and $R^{4b}$ are each independently hydrogen or $C_{1-6}$ alkyl; or, $R^{4a}$ and $R^{4b}$, together with a nitrogen atom attached thereto, form a substituted or unsubstituted 4-6 membered heterocycloalkyl, wherein the 4-6 membered heterocycloalkyl has 0, 1 or 2 additional heteroatoms independently selected from N, O and S, and the substituted 4-6 membered heterocycloalkyl means that the 4-6 membered heterocycloalkyl is substituted with 1, 2, 3 or 4$R^b$;
each $R^b$ is independently halogen, hydroxy or $C_{1-4}$ alkyl;
$R^5$ is hydrogen or $C_{1-6}$ alkyl;
each $R^6$ is independently hydrogen, cyclopropyl or $C_{1-6}$ alkyl;
each $R^7$ is independently hydrogen, $C_{1-6}$ alkyl, phenyl, 5-6 membered heteroaryl, $-OR^{7a}$ or $-NR^{7b}R^{7c}$, wherein the number of heteroatoms in the 5-6 membered heteroaryl is 1, 2 or 3, and each heteroatom is independently selected from N, O and S;
$R^{7a}$ is hydrogen or a substituted or unsubstituted $C_{1-6}$ alkyl, wherein the substituted $C_{1-6}$ alkyl means that the $C_{1-6}$ alkyl is substituted with 1, 2, 3 or 4 $R^c$;
each $R^c$ is independently hydroxy, halogen, $C_{1-4}$ alkyl or $C_{1-4}$ alkoxy;
$R^{7b}$ and $R^{7c}$ are each independently hydrogen or a substituted or unsubstituted $C_{1-4}$ alkyl, wherein the substituted $C_{1-4}$ alkyl means that the $C_{1-4}$ alkyl is substituted with 1, 2, 3 or 4 $R^d$;
or, $R^{7b}$ and $R^{7c}$, together with a nitrogen atom attached thereto, form a substituted or unsubstituted 4-6 membered heterocycloalkyl, wherein the 4-6 membered heterocycloalkyl has 0, 1 or 2 additional heteroatoms independently selected from N, O and S, and the substituted 4-6 membered heterocycloalkyl means that the 4-6 membered heterocycloalkyl is substituted with 1, 2, 3 or 4 $R^e$;
each $R^d$ is independently hydroxy, halogen, $C_{1-4}$ alkyl or $C_{1-4}$ alkoxy;
each $R^e$ is independently hydroxy, halogen, $C_{1-4}$ alkyl or $C_{1-4}$ alkoxy;
each $R^8$ is independently hydrogen or $C_{1-4}$ alkyl;

when a carbon atom marked by * has chirality, the carbon atom is in S configuration, R configuration, or a mixture thereof.

**4.** The compound or the tautomer, the stereoisomer or the isotopic derivative thereof, or the pharmaceutically acceptable salt of any of the foregoing, or the crystalline form or the solvate of any of the foregoing according to claim 1, wherein, when $R^{4a}$ is $C_{1-6}$ alkyl, the $C_{1-6}$ alkyl is methyl;

and/or, when $R^{4b}$ is $C_{1-6}$ alkyl, the $C_{1-6}$ alkyl is methyl;
and/or, when $R^5$ is $C_{1-6}$ alkyl, the $C_{1-6}$ alkyl is methyl;
and/or, when $R^6$ is $C_{1-6}$ alkyl, the $C_{1-6}$ alkyl is methyl, ethyl, *n*-propyl or isopropyl;
and/or, when $R^7$ is a substituted or unsubstituted $C_{1-6}$ alkyl, the $C_{1-6}$ alkyl is methyl, ethyl or isopropyl;
and/or, when $R^7$ is a substituted or unsubstituted 5-6 membered heteroaryl, the 5-6 membered heteroaryl is pyrazolyl, e.g.,

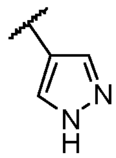 ;

and/or, when $R^7$ is a substituted or unsubstituted $C_{3-6}$ cycloalkyl, the $C_{3-6}$ cycloalkyl is cyclopropyl;
and/or, when $R^{7d}$ is $C_{1-4}$ alkoxy, the $C_{1-4}$ alkoxy is methoxy;
and/or, when $R^{7a}$ is a substituted or unsubstituted $C_{1-6}$ alkyl, the $C_{1-6}$ alkyl is isopropyl;
and/or, when $R^{7a}$ is a substituted or unsubstituted $C_{3-6}$ cycloalkyl, the $C_{3-6}$ cycloalkyl is cyclopropyl;
and/or, when $R^{7b}$ and $R^{7c}$ are each independently a substituted or unsubstituted $C_{1-4}$ alkyl, the $C_{1-4}$ alkyl is ethyl, isopropyl or *sec*-butyl;
and/or, when $R^{7b}$ and $R^{7c}$ are each independently a substituted or unsubstituted $C_{3-6}$ cycloalkyl, the $C_{3-6}$ cycloalkyl is cyclopropyl or cyclopentyl;
and/or, when $R^{7b}$ and $R^{7c}$ are each independently a substituted or unsubstituted 5-6 membered heteroaryl, the 5-6 membered heteroaryl is pyrazolyl, e.g.,

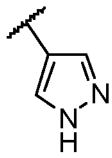 or 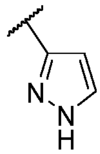 ;

and/or, when $R^{7b}$ and $R^{7c}$, together with a nitrogen atom attached thereto, form a substituted or unsubstituted 4-6 membered heterocycloalkyl, the 4-6 membered heterocycloalkyl is azetidinyl;
and/or, when $R^c$ is $C_{1-4}$ alkoxy, the $C_{1-4}$ alkoxy is methoxy;
and/or, when $R^d$ is $C_{1-4}$ alkoxy, the $C_{1-4}$ alkoxy is methoxy;
and/or, when $R^e$ is $C_{1-4}$ alkoxy, the $C_{1-4}$ alkoxy is methoxy;
and/or, when each $R^{a1}$, each $R^{a2}$, each $R^{c1}$, each $R^{c2}$, each $R^{d1}$, each $R^{d2}$, each $R^{e1}$ and each $R^{e2}$ are each independently $C_{1-4}$ alkyl, the $C_{1-4}$ alkyl is methyl;
and/or, when $R^8$ is $C_{1-4}$ alkyl, the $C_{1-4}$ alkyl is methyl.

**5.** The compound or the tautomer, the stereoisomer or the isotopic derivative thereof, or the pharmaceutically acceptable salt of any of the foregoing, or the crystalline form or the solvate of any of the foregoing according to claim 1, wherein, when $R^{7a}$ is a substituted or unsubstituted $C_{1-6}$ alkyl, the substituted or unsubstituted $C_{1-6}$ alkyl is isopropyl;

and/or, when $R^{7a}$ is a substituted or unsubstituted $C_{3-6}$ cycloalkyl, the substituted or unsubstituted $C_{3-6}$ cycloalkyl is cyclopropyl;
and/or, when $R^{7b}$ and $R^{7c}$ are each independently a substituted or unsubstituted $C_{1-4}$ alkyl, the substituted or unsubstituted $C_{1-4}$ alkyl is ethyl, isopropyl,

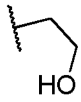, 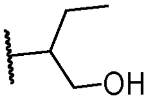, 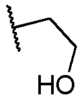,

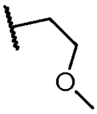 or 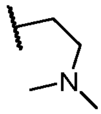;

and/or, when $R^{7b}$ and $R^{7c}$ are each independently a substituted or unsubstituted $C_{3-6}$ cycloalkyl, the substituted or unsubstituted $C_{3-6}$ cycloalkyl is cyclopropyl or

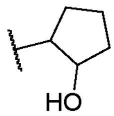;

and/or, when $R^{7b}$ and $R^{7c}$ are each independently a substituted or unsubstituted 5-6 membered heteroaryl, the substituted or unsubstituted 5-6 membered heteroaryl is

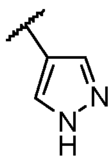 or 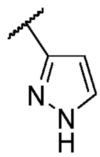;

and/or, when $R^{7b}$ and $R^{7c}$, together with a nitrogen atom attached thereto, form a substituted or unsubstituted 4-6 membered heterocycloalkyl, the substituted or unsubstituted 4-6 membered heterocycloalkyl is

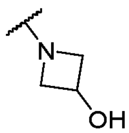;

and/or, when $R^c$ is $-NR^{c1}R^{c2}$, the $-NR^{c1}R^{c2}$ is $-N(CH_3)_2$;
and/or, when $R^d$ is $-NR^{d1}R^{d2}$, the $-NR^{d1}R^{d2}$ is $-N(CH_3)_2$;
and/or, when $R^e$ is $-NR^{e1}R^{e2}$, the $-NR^{e1}R^{e2}$ is $-N(CH_3)_2$;
and/or, when $R^{7d}$ is $-NR^{a1}R^{a2}$, the $-NR^{a1}R^{a2}$ is $-N(CH_3)_2$.

**6.** The compound or the tautomer, the stereoisomer or the isotopic derivative thereof, or the pharmaceutically acceptable salt of any of the foregoing, or the crystalline form or the solvate of any of the foregoing according to claim 1, wherein when $R^1$ is

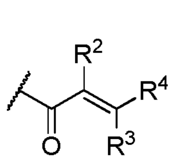,

the

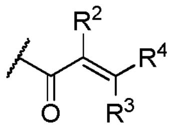 is 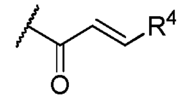 ;

and/or, when $R^4$ is $-CH_2-NR^{4a}R^{4b}$, the $-CH_2-NR^{4a}R^{4b}$ is $-CH_2-N(CH_3)_2$;
and/or, when $R^7$ is a substituted or unsubstituted $C_{1-6}$ alkyl, the substituted or unsubstituted $C_{1-6}$ alkyl is methyl, ethyl, isopropyl or $-CH_2OH$;
and/or, when $R^7$ is a substituted or unsubstituted 5-6 membered heteroaryl, the substituted or unsubstituted 5-6 membered heteroaryl is

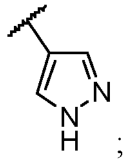 ;

and/or, when $R^7$ is a substituted or unsubstituted $C_{3-6}$ cycloalkyl, the substituted or unsubstituted $C_{3-6}$ cycloalkyl is cyclopropyl;
and/or, when $R^7$ is $-OR^{7a}$, the $-OR^{7a}$ is

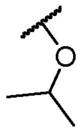 or 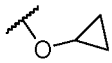 ;

and/or, when $R^7$ is $-NR^{7b}R^{7c}$ , the $-NR^{7b}R^{7c}$ is

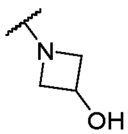 , 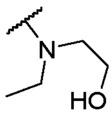 , 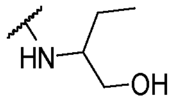 ,

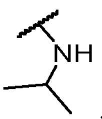 , 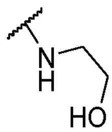 , 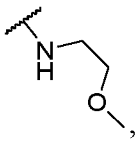 , 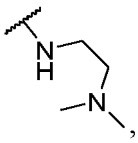 , 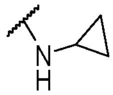 , 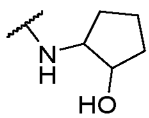 , 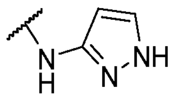

or

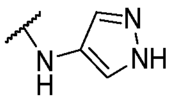 .

7. The compound or the tautomer, the stereoisomer or the isotopic derivative thereof, or the pharmaceutically acceptable salt of any of the foregoing, or the crystalline form or the solvate of any of the foregoing according to claim 1, wherein $R^a$ is hydrogen;

and/or, n is 1;
and/or, $R^2$ is hydrogen;
and/or, $R^3$ is hydrogen;
and/or, $R^4$ is $-CH_2-NR^{4a}R^{4b}$;

and/or, $R^{4a}$ is $C_{1-6}$ alkyl;
and/or, $R^{4b}$ is $C_{1-6}$ alkyl;
and/or, $R^5$ is $C_{1-6}$ alkyl;
and/or, each $R^7$ is independently hydrogen, a substituted or unsubstituted $C_{1-6}$ alkyl, a substituted or unsubstituted 5-6 membered heteroaryl, a substituted or unsubstituted $C_{3-6}$ cycloalkyl, $-OR^{7a}$ or $-NR^{7b}R^{7c}$, wherein the substituted $C_{1-6}$ alkyl and the substituted 5-6 membered heteroaryl mean that the $C_{1-6}$ alkyl, the $C_{3-6}$ cycloalkyl and the 5-6 membered heteroaryl are each independently substituted with 1, 2, 3 or 4 $R^{7d}$;
and/or, each $R^{7d}$ is independently hydroxy, $-NR^{a1}R^{a2}$ or $C_{1-4}$ alkoxy;
and/or, $R^{a1}$ and $R^{a2}$ are $C_{1-4}$ alkyl;
and/or, $R^{7a}$ is hydrogen, a substituted or unsubstituted $C_{1-6}$ alkyl, or a substituted or unsubstituted $C_{3-6}$ cycloalkyl, wherein the substituted $C_{1-6}$ alkyl and the substituted $C_{3-6}$ cycloalkyl mean that the $C_{1-6}$ alkyl and the $C_{3-6}$ cycloalkyl are each independently substituted with 1, 2, 3 or 4 $R^c$;
and/or, $R^c$ is hydroxy, $-NR^{c1}R^{c2}$ or $C_{1-4}$ alkoxy;
and/or, $R^{c1}$ and $R^{c2}$ are $C_{1-4}$ alkyl;
and/or, $R^{7b}$ and $R^{7c}$ are each independently hydrogen, a substituted or unsubstituted $C_{1-4}$ alkyl, a substituted or unsubstituted $C_{3-6}$ cycloalkyl, or a substituted or unsubstituted 5-6 membered heteroaryl, wherein the substituted $C_{1-4}$ alkyl, the substituted $C_{3-6}$ cycloalkyl and the substituted 5-6 membered heteroaryl mean that the $C_{1-4}$ alkyl, the $C_{3-6}$ cycloalkyl and the 5-6 membered heteroaryl are each independently substituted with 1, 2, 3 or 4 $R^d$;
and/or, $R^d$ is hydroxy, $-NR^{d1}R^{d2}$ or $C_{1-4}$ alkoxy;
and/or, $R^{d1}$ and $R^{d2}$ are $C_{1-4}$ alkyl.

**8.** The compound or the tautomer, the stereoisomer or the isotopic derivative thereof, or the pharmaceutically acceptable salt of any of the foregoing, or the crystalline form or the solvate of any of the foregoing according to claim 1, wherein the compound is selected from any of the following structures:

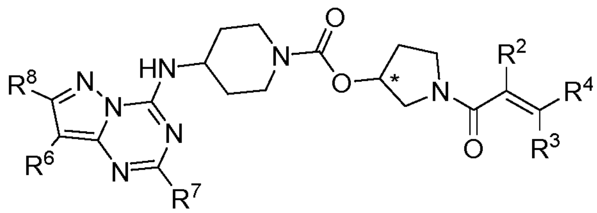
,
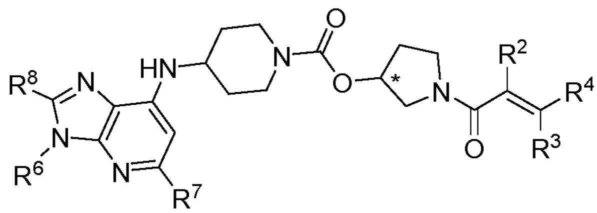
,
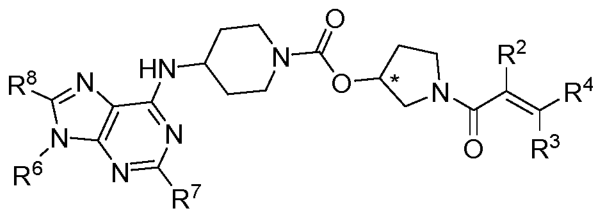
,
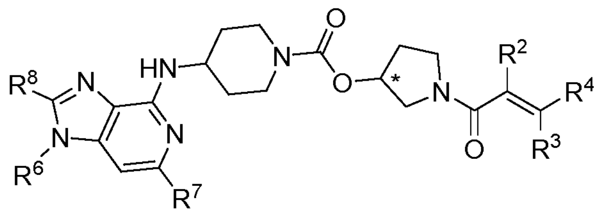
,
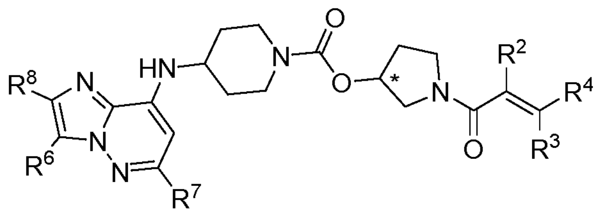
,
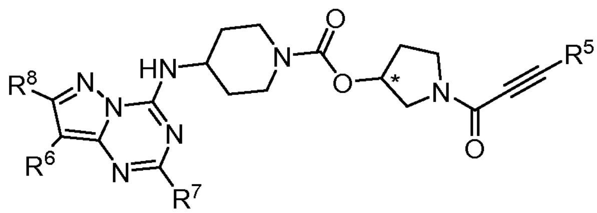
,

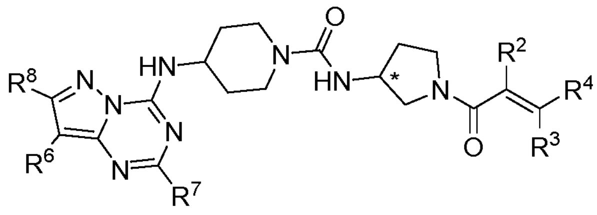
,
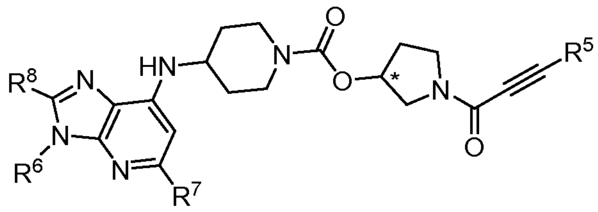
,

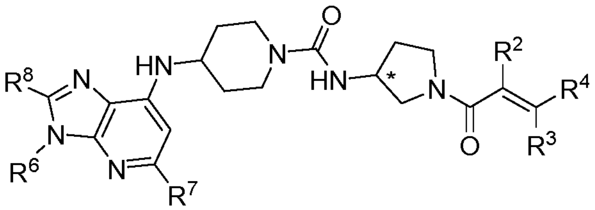
,
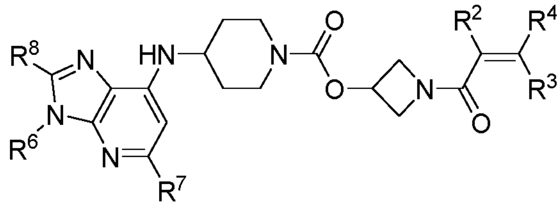
,

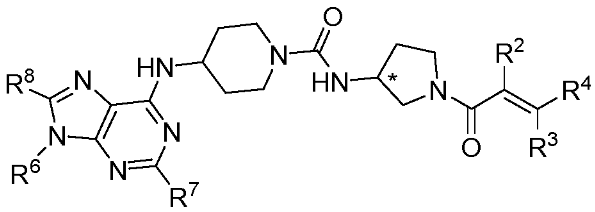
,
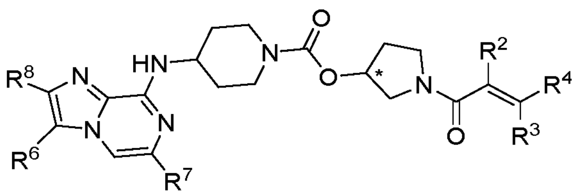
,

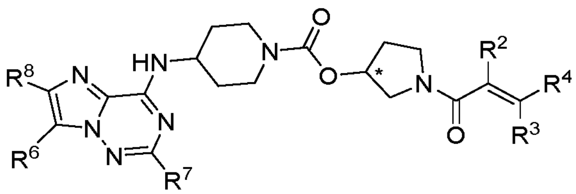
,
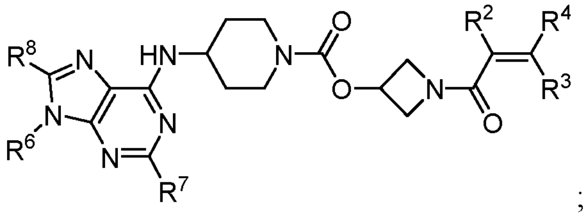
;

wherein $R^2$, $R^3$, $R^4$, $R^5$, $R^6$, $R^7$, $R^8$ and * are defined as in any of claims 1-7.

**9.** The compound or the tautomer, the stereoisomer or the isotopic derivative thereof, or the pharmaceutically acceptable salt of any of the foregoing, or the crystalline form or the solvate of any of the foregoing according to any of claims 1-8, wherein $R^6$ is $C_{1-6}$ alkyl;

and/or, $R^7$ is $C_{1-6}$ alkyl;
and/or, $R^8$ is hydrogen.

**10.** The compound or the tautomer, the stereoisomer or the isotopic derivative thereof, or the pharmaceutically acceptable salt of any of the foregoing, or the crystalline form or the solvate of any of the foregoing according to claim 1, wherein the compound is any of the following structures:

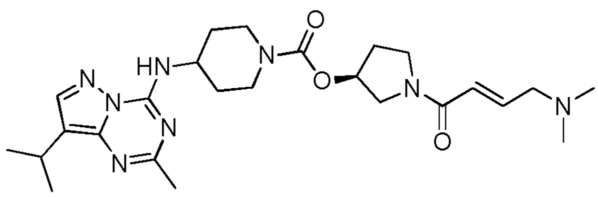
,
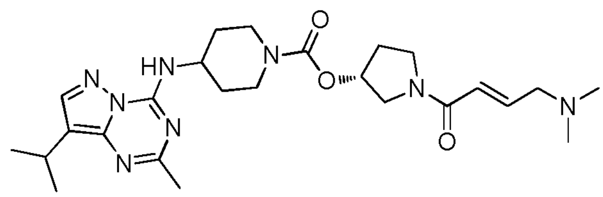
,

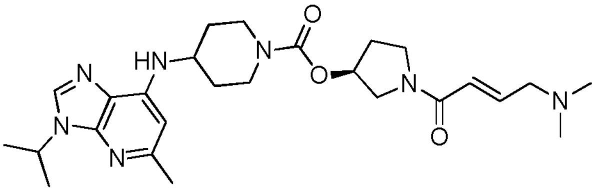
,
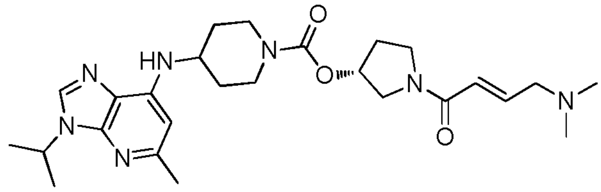
,

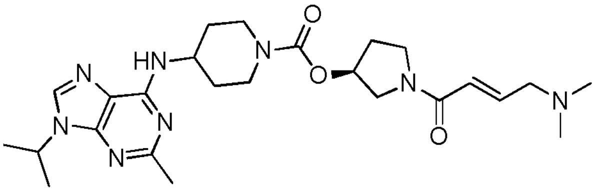
,
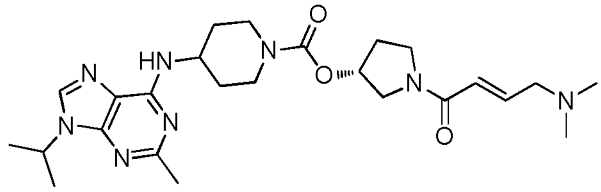
,

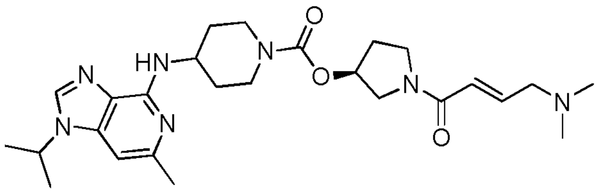
,
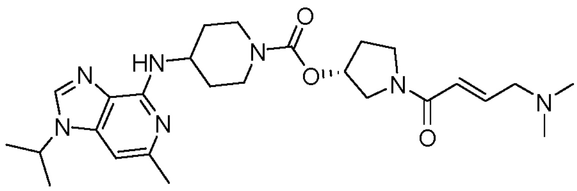
,

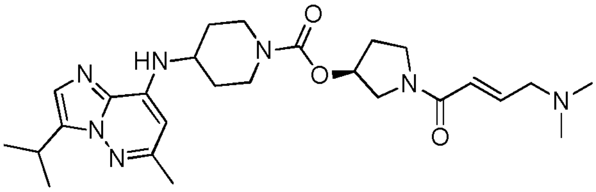
,
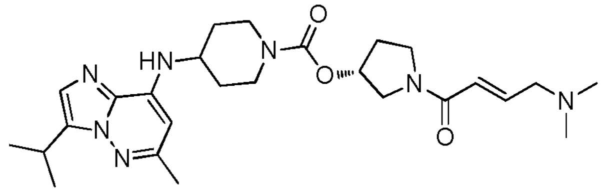
,

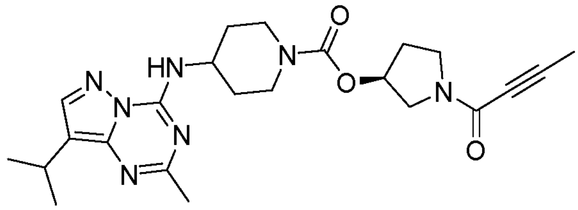
,
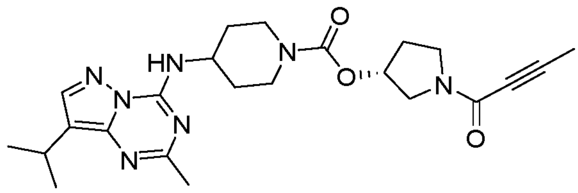
,

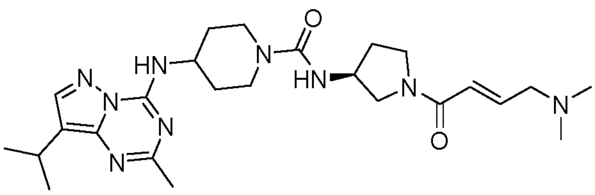
,
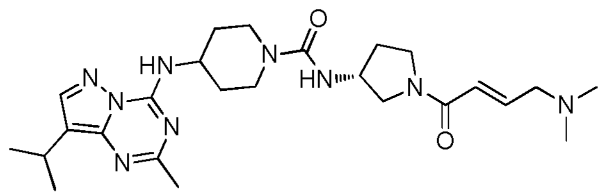
,

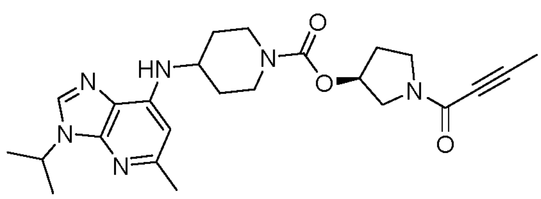
,
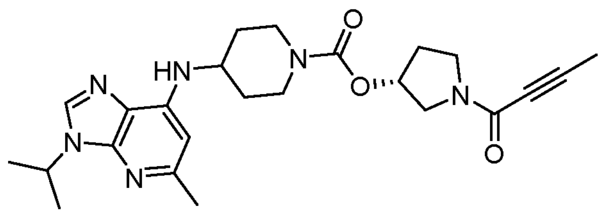
,

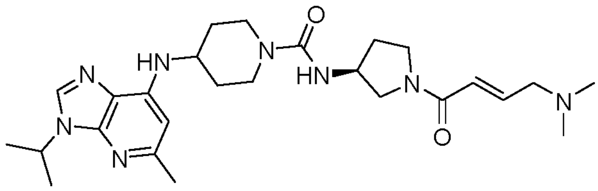,

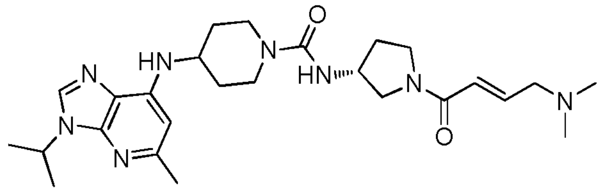,

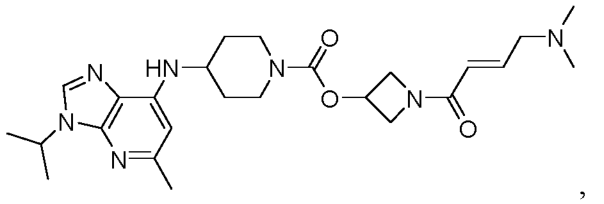,

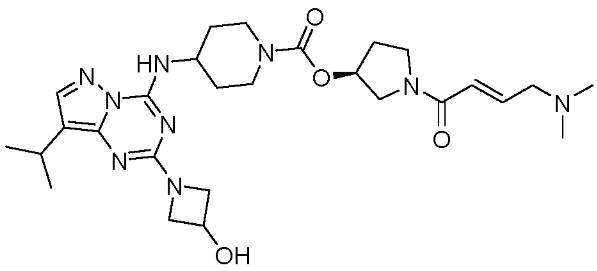,

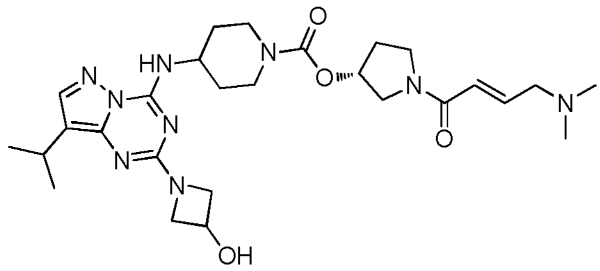,

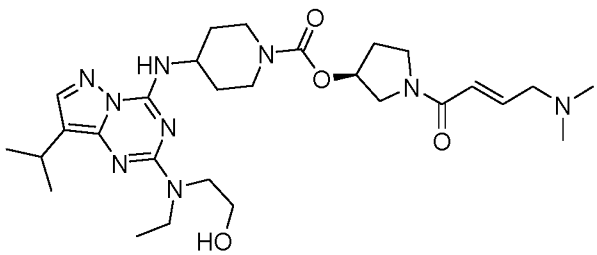,

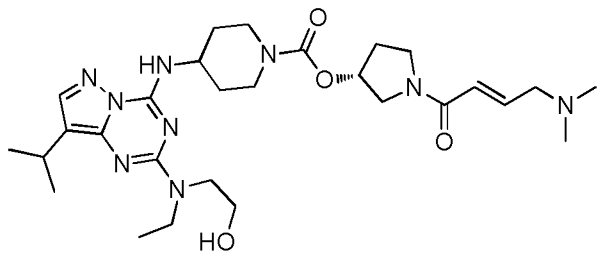,

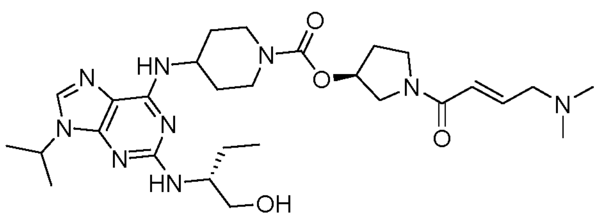,

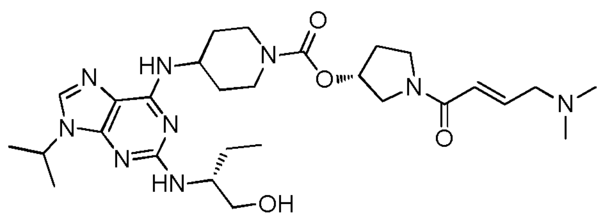,

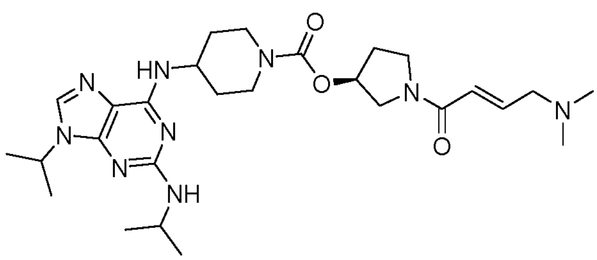,

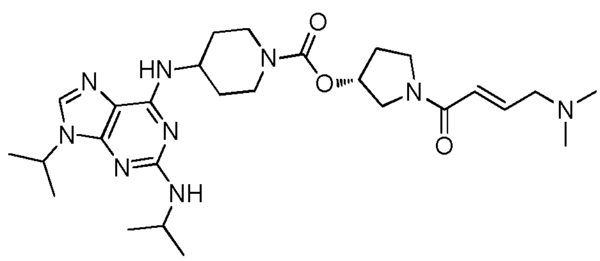,

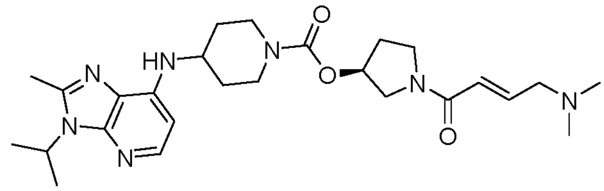,

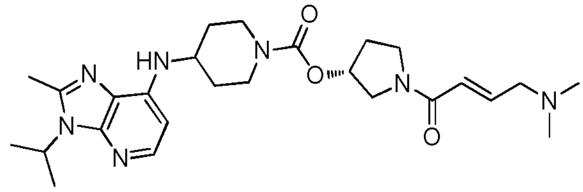,

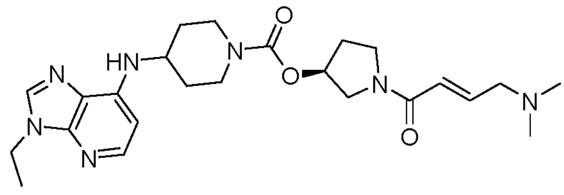,

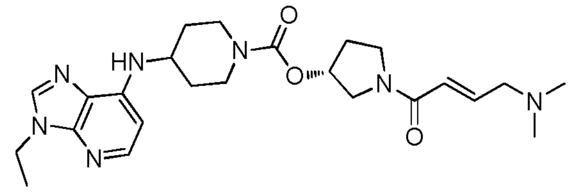,

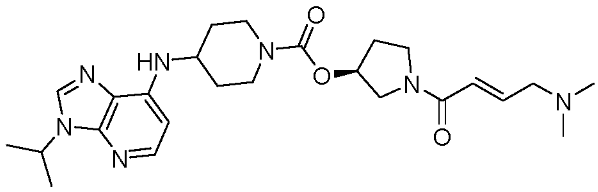,

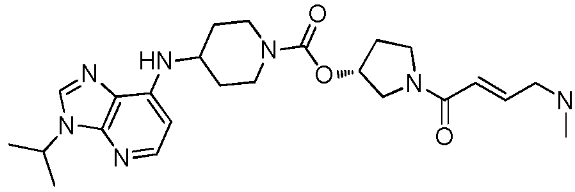,

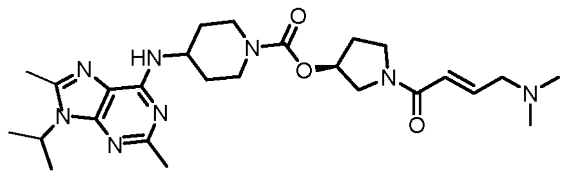,

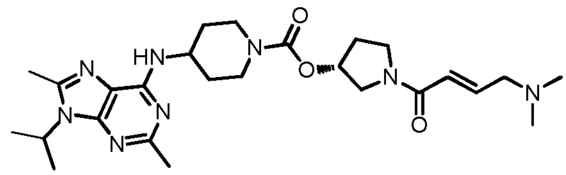,

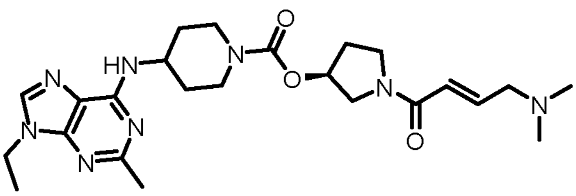,

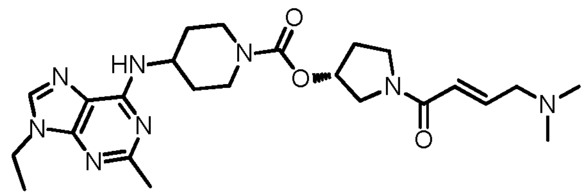,

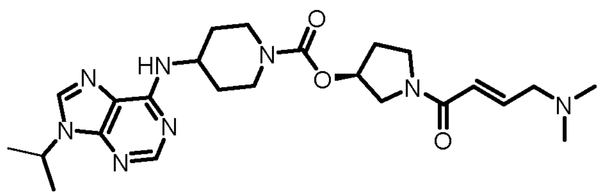,

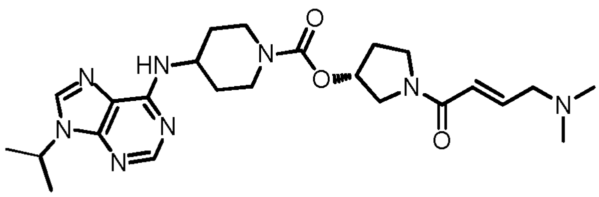,

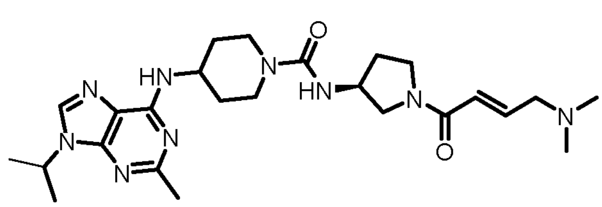,

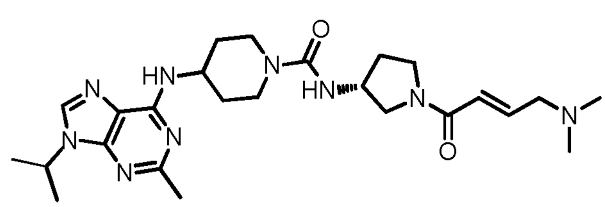,

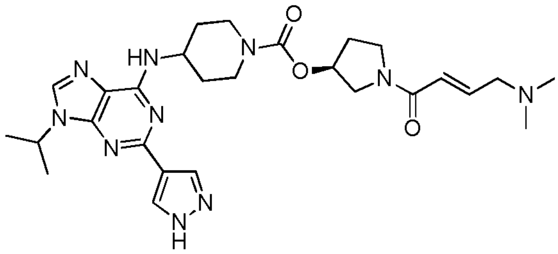

,

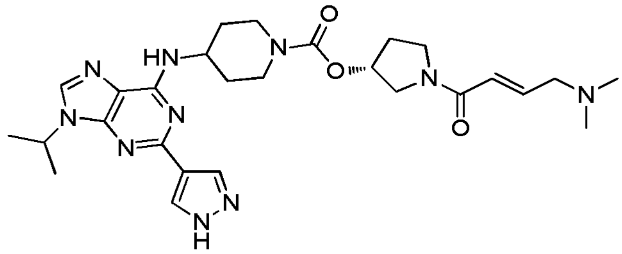

,

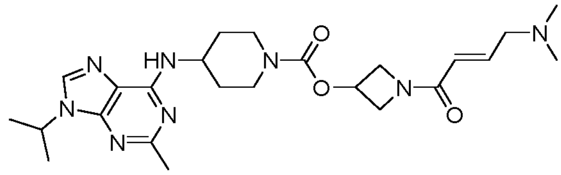

,

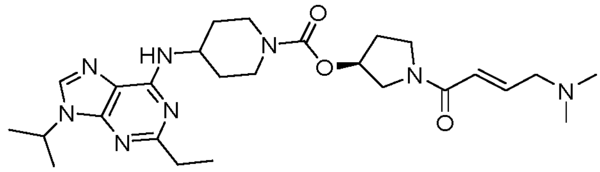

,

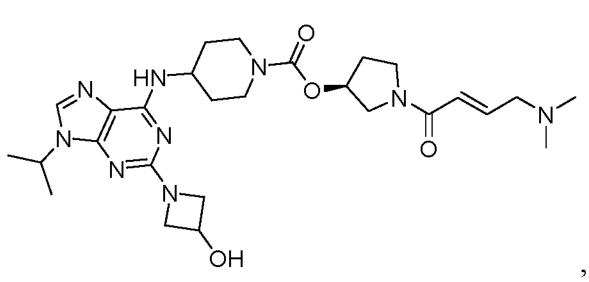

,

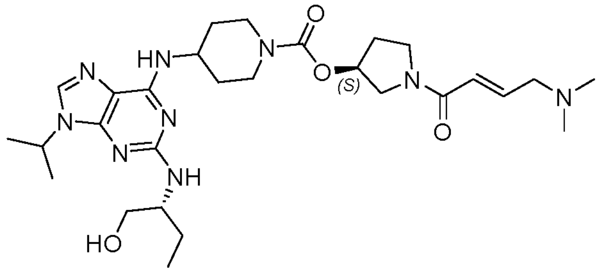

,

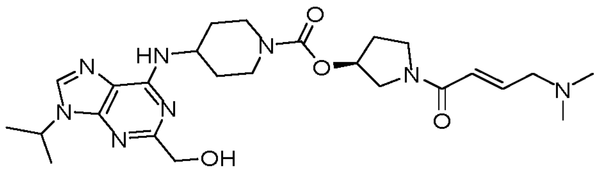

,

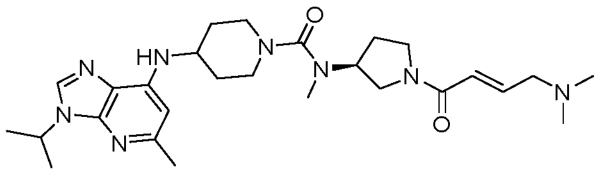

,

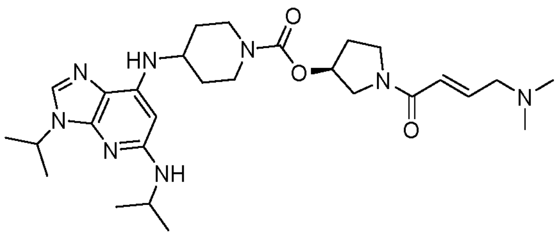

,

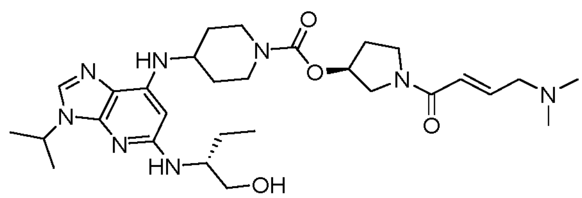

,

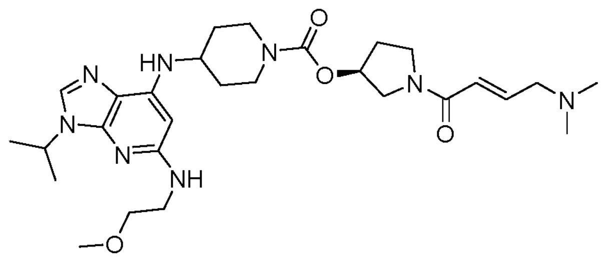

,

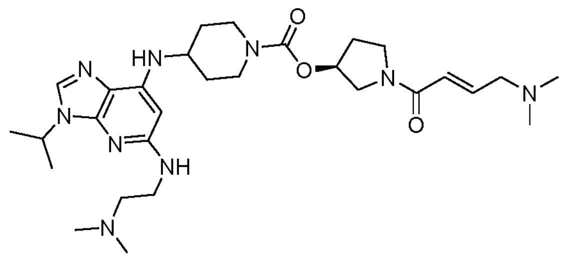

,

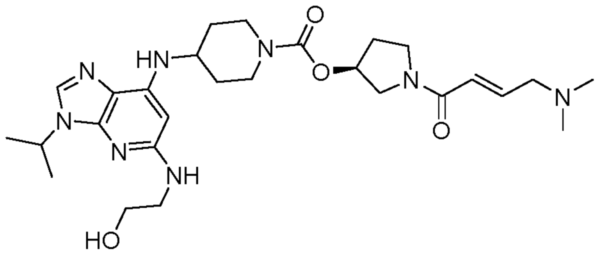

,

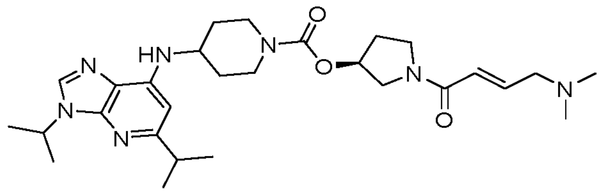

,

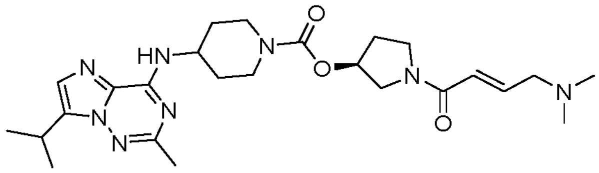

,

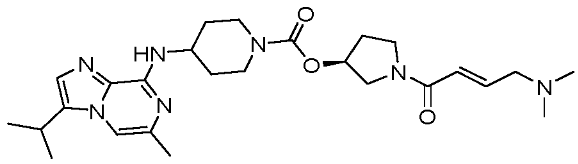

,

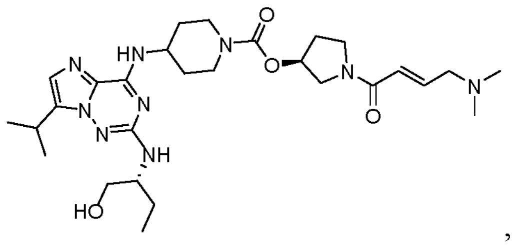

,

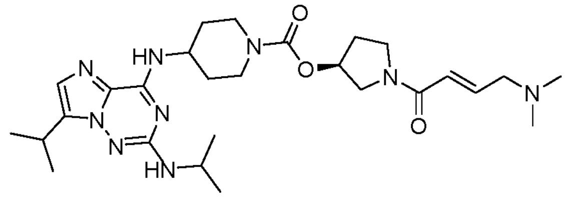

,

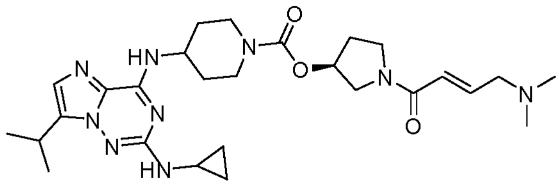

,

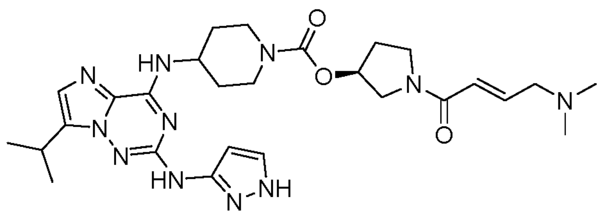

,

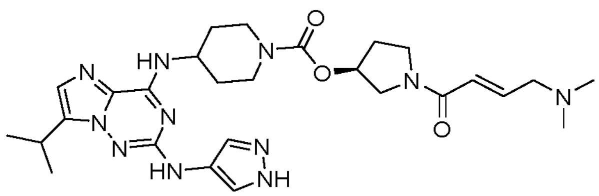

,

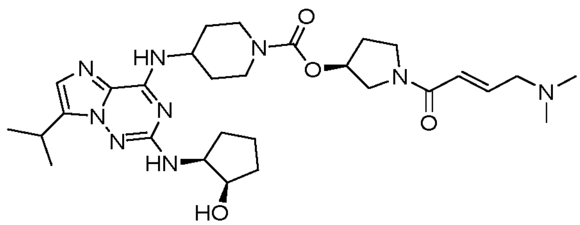

,

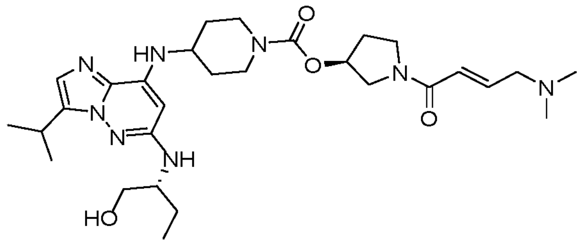

,

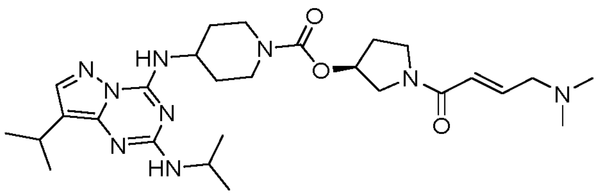

,

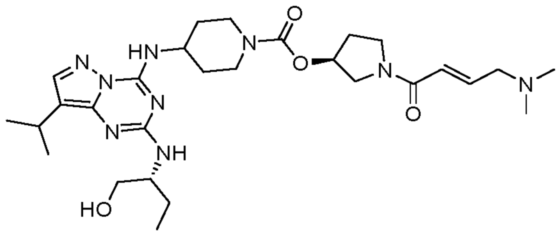
,
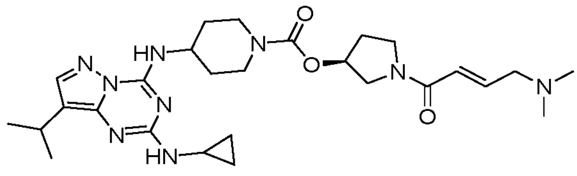
,
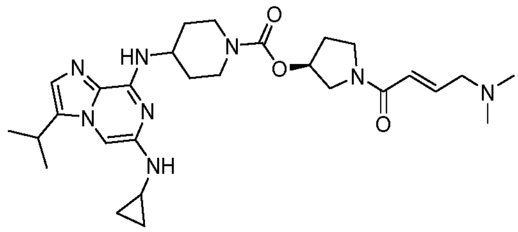
,
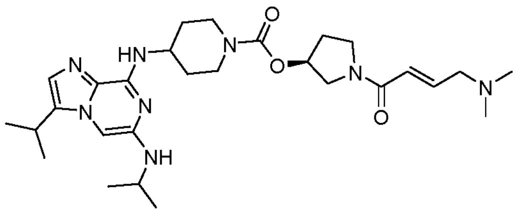
,
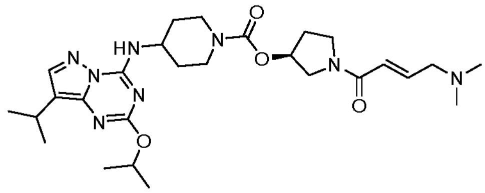
,
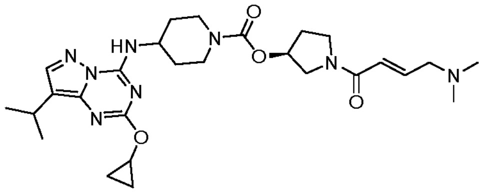
,
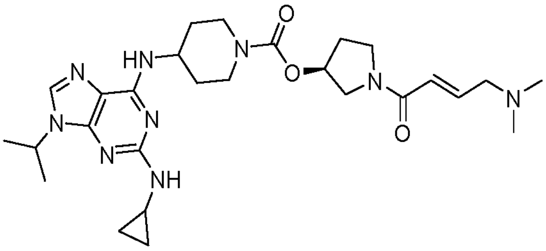
,
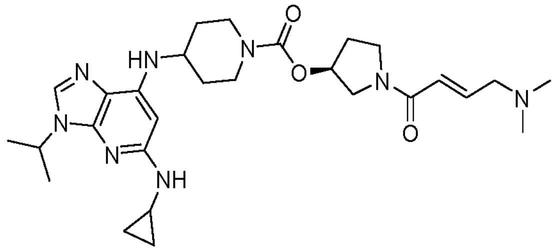
,
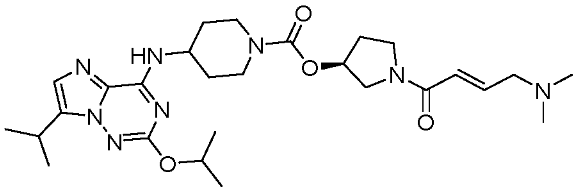
,
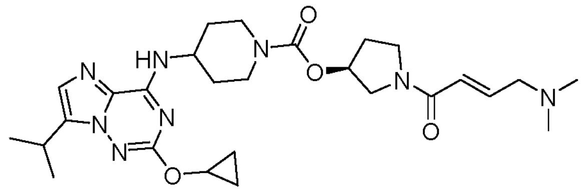
,
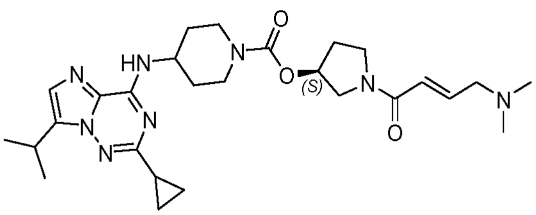
,
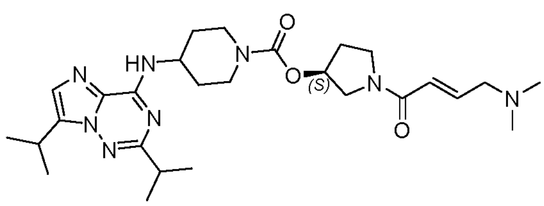
,

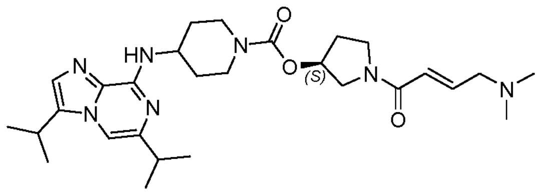

.

**11.** A method for preparing the compound of formula I according to claim 1, comprising: subjecting a compound of formula II and

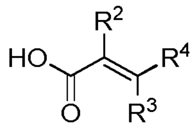 or 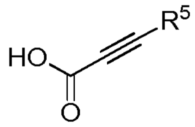

to a condensation reaction shown below in an organic solvent in the presence of a condensing agent and a base to obtain the compound of formula I, wherein ring A, X, m, n, $R^1$, $R^2$, $R^3$, $R^4$, $R^5$ and * are defined as in any of claims 1-10;

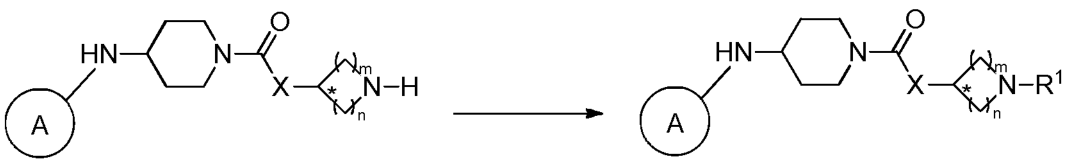

II I

.

**12.** A compound or a tautomer, a stereoisomer or an isotopic derivative thereof, or a pharmaceutically acceptable salt of any of the foregoing, or a crystalline form or a solvate of any of the foregoing, wherein the compound is selected from any of the following structures:

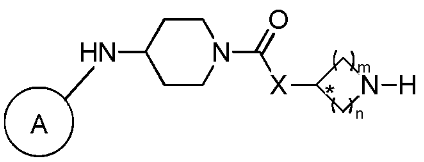

II

,

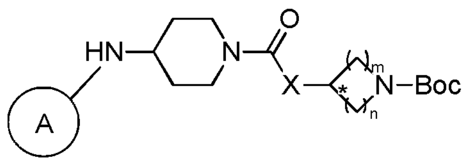

III

,

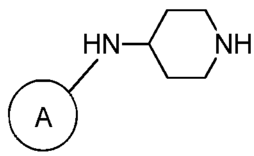

IV-1

,

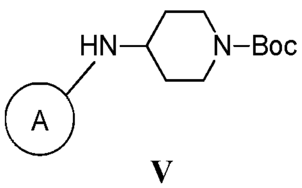

V

;

wherein ring A, X, m, n and * are defined as in any of claims 1-10.

**13.** A pharmaceutical composition, comprising:

(i) the compound of formula I or the tautomer, the stereoisomer or the isotopic derivative thereof, or the pharmaceutically acceptable salt of any of the foregoing, or the crystalline form or the solvate of any of the foregoing according to any of claims 1-10; and
(ii) at least one pharmaceutical adjuvant.

**14.** Use of the compound or the tautomer, the stereoisomer or the isotopic derivative thereof, or the pharmaceutically acceptable salt of any of the foregoing, or the crystalline form or the solvate of any of the foregoing according to any of claims 1-10 in preparing a medicament.

**15.** The use according to claim 14, wherein the medicament is a medicament for preventing and/or treating a CDK7-mediated disease.

**16.** The use according to claim 15, wherein the CDK7-mediated disease is a tumor, such as breast cancer, ovarian cancer, small cell lung cancer, acute myeloid leukemia, acute lymphocytic leukemia, bladder cancer, colon cancer, prostate cancer, epithelial sarcoma and soft tissue sarcoma.

**17.** The use according to claim 14, wherein the medicament is a medicament for preventing and/or treating a tumor, such as breast cancer, ovarian cancer, small cell lung cancer, acute myeloid leukemia, acute lymphocytic leukemia, bladder cancer, colon cancer, prostate cancer, epithelial sarcoma and soft tissue sarcoma.

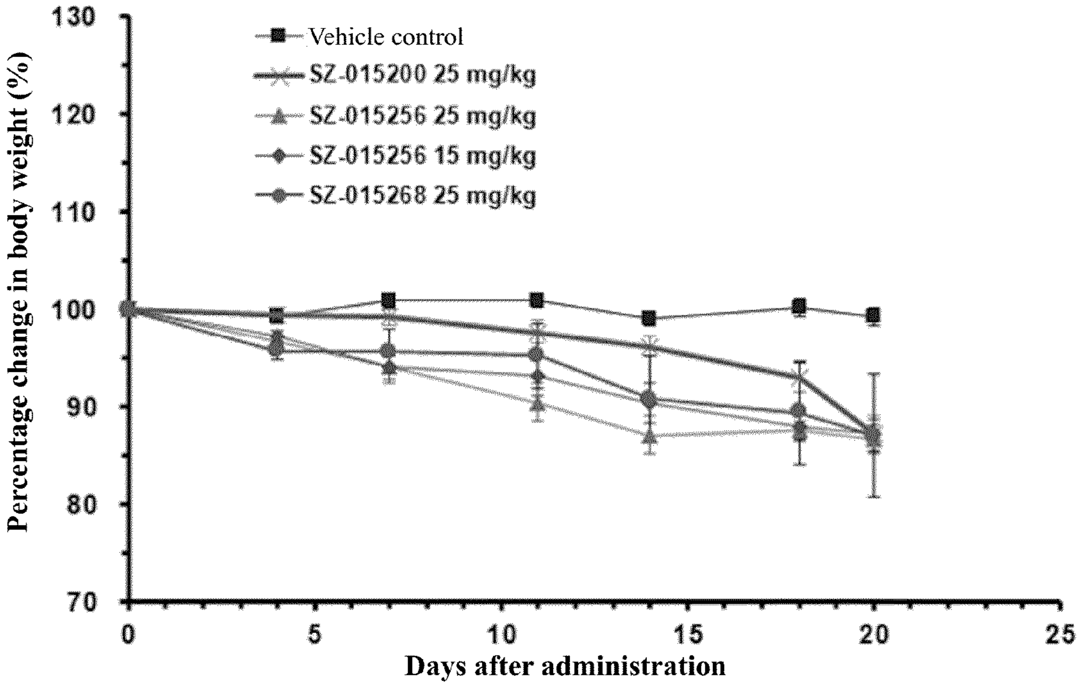
Vehicle control
SZ-015200 25 mg/kg
SZ-015256 25 mg/kg
SZ-015256 15 mg/kg
SZ-015268 25 mg/kg
Percentage change in body weight (%)
130
120
110
100
90
80
70
0
5
10
15
20
25
Days after administration

FIG. 1

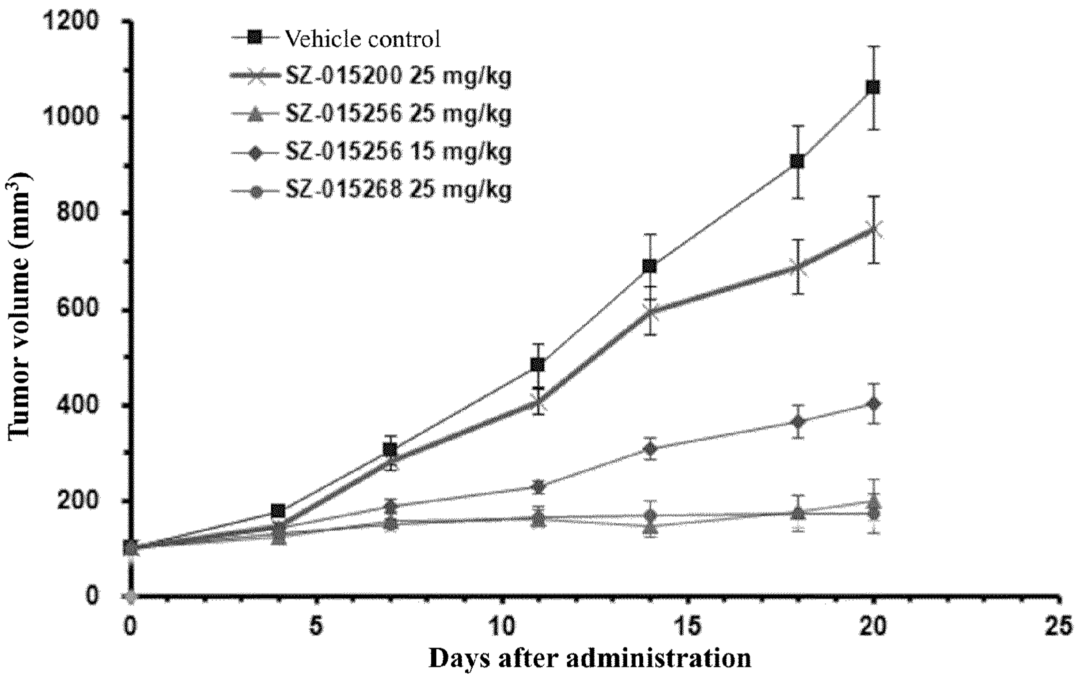
Vehicle control
SZ-015200 25 mg/kg
SZ-015256 25 mg/kg
SZ-015256 15 mg/kg
SZ-015268 25 mg/kg
Tumor volume (mm3)
1200
1000
800
600
400
200
0
0
5
10
15
20
25
Days after administration

FIG. 2

## INTERNATIONAL SEARCH REPORT

International application No.
**PCT/CN2020/137618**

**A. CLASSIFICATION OF SUBJECT MATTER**

C07D 487/04(2006.01)i; A61P 35/00(2006.01)i; A61K 31/41(2006.01)i

According to International Patent Classification (IPC) or to both national classification and IPC

**B. FIELDS SEARCHED**

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

DWPI, SIPOABS, WOTXT, EPTXT, USTXT, CNTXT, CATXT, GBTXT, JPTXT, KRABS, CNABS, CNKI, STNext, ISI Web of Science: 细胞周期蛋白依赖性激酶, 哌啶, 甲酸, 吡咯烷, 苏州信诺维医药, cyclin dependent kinase, CDK, piperidine, carboxylate, pyrrolidin, SUZOU SINOVENT

**C. DOCUMENTS CONSIDERED TO BE RELEVANT**

| Category* | Citation of document, with indication, where appropriate, of the relevant passages | Relevant to claim No. |
|---|---|---|
| Y | WO 2019099298 A1 (ELI LILLY AND CO.) 23 May 2019 (2019-05-23) abstract, claims 2-16, 18-23 | 1-17 |
| Y | CN 107530329 A (AURIGENE DISCOVERY TECHNOLOGIES LTD.) 02 January 2018 (2018-01-02) abstract, claim 17 | 1-17 |
| A | CN 1880317 A (SCHERING CORP. et al.) 20 December 2006 (2006-12-20) abstract, claims 1-12 | 1-17 |
| A | WO 2006005548 A1 (F. HOFFMANN-LA ROCHE AG.) 19 January 2006 (2006-01-19) abstract, claims 1-37 | 1-17 |
| A | WO 2008130570 A1 (SCHERING CORP. et al.) 30 October 2008 (2008-10-30) abstract, claims 1-39 | 1-17 |
| A | WO 2015154022 A1 (SYROS PHARMACEUTICALS, INC.) 08 October 2015 (2015-10-08) abstract, claims 1-47 | 1-17 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 February 2021** | **17 March 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/137618**

| Patent document cited in search report | Publication date (day/month/year) | Patent family member(s) | Publication date (day/month/year) |
|---|---|---|---|
| WO 2019099298 A1 | 23 May 2019 | EP 3710446 A1 | 23 September 2020 |
| | | CR 20200184 A | 01 June 2020 |
| | | US 2020017513 A1 | 16 January 2020 |
| | | CN 111344292 A | 26 June 2020 |
| | | US 2019144456 A1 | 16 May 2019 |
| | | KR 20200070323 A | 17 June 2020 |
| | | TW 201930305 A | 01 August 2019 |
| | | EC SP20025899 A | 30 June 2020 |
| | | CL 2020001218 A1 | 21 August 2020 |
| | | US 10787460 B2 | 29 September 2020 |
| | | SG CN 11202003677U A | 28 May 2020 |
| | | AU 2018369508 A1 | 07 May 2020 |
| | | US 10472370 B2 | 12 November 2019 |
| | | JP 6633254 B2 | 22 January 2020 |
| | | BR 112020008253 A2 | 17 November 2020 |
| | | JP 2019537616 A | 26 December 2019 |
| | | IL 274540 D0 | 30 June 2020 |
| | | CA 3080910 A1 | 23 May 2019 |
| CN 107530329 A | 02 January 2018 | US 2018258092 A9 | 13 September 2018 |
| | | WO 2016142855 A3 | 03 November 2016 |
| | | JP 2018507877 A | 22 March 2018 |
| | | WO 2016142855 A2 | 15 September 2016 |
| | | JP 6789962 B2 | 25 November 2020 |
| | | CA 2978170 A1 | 15 September 2016 |
| | | EP 3268000 A4 | 22 August 2018 |
| | | US 2018057497 A1 | 01 March 2018 |
| | | EP 3268000 A2 | 17 January 2018 |
| CN 1880317 A | 20 December 2006 | ZA 200501855 A | 29 March 2006 |
| | | CN 1735614 A | 15 February 2006 |
| | | CN 100376580 C | 26 March 2008 |
| | | CN 1880317 B | 10 October 2012 |
| | | ZA 200501855 B | 29 March 2006 |
| WO 2006005548 A1 | 19 January 2006 | KR 20070028539 A | 12 March 2007 |
| | | EP 1771420 B1 | 14 October 2009 |
| | | JP 2008505955 A | 28 February 2008 |
| | | AR 050427 A1 | 25 October 2006 |
| | | ES 2330765 T3 | 15 December 2009 |
| | | AT 445604 T | 15 October 2009 |
| | | US 7423051 B2 | 09 September 2008 |
| | | MX 2007000574 A | 07 March 2007 |
| | | BR PI0513366 A | 06 May 2008 |
| | | US 2006014708 A1 | 19 January 2006 |
| | | CN 1980893 A | 13 June 2007 |
| | | RU 2385866 C2 | 10 April 2010 |
| | | CN 1980893 B | 19 January 2011 |
| | | KR 100853973 B1 | 25 August 2008 |
| | | EP 1771420 A1 | 11 April 2007 |
| | | CA 2573604 A1 | 19 January 2006 |
| | | AU 2005261899 A1 | 19 January 2006 |

Form PCT/ISA/210 (patent family annex) (January 2015)

**INTERNATIONAL SEARCH REPORT**
**Information on patent family members**

International application No.
**PCT/CN2020/137618**

| Patent document cited in search report | | | Publication date (day/month/year) | Patent family member(s) | | | Publication date (day/month/year) |
|---|---|---|---|---|---|---|---|
| | | | | TW | 200612929 | A | 01 May 2006 |
| | | | | RU | 2007105558 | A | 20 August 2008 |
| | | | | DE | 602005017152 | D1 | 26 November 2009 |
| WO | 2008130570 | A1 | 30 October 2008 | US | 2008050384 | A1 | 28 February 2008 |
| | | | | PE | 20090156 | A1 | 26 February 2009 |
| | | | | TW | 200904443 | A | 01 February 2009 |
| | | | | CA | 2685278 | A1 | 30 October 2008 |
| | | | | US | 8673924 | B2 | 18 March 2014 |
| | | | | AR | 070299 | A1 | 31 March 2010 |
| WO | 2015154022 | A1 | 08 October 2015 | US | 2019330218 | A1 | 31 October 2019 |
| | | | | EP | 3129371 | A1 | 15 February 2017 |
| | | | | AU | 2015240518 | A1 | 20 October 2016 |
| | | | | US | 2020010473 | A1 | 09 January 2020 |
| | | | | AU | 2019222848 | A1 | 19 September 2019 |
| | | | | EP | 3129371 | B1 | 29 July 2020 |
| | | | | US | 10336760 | B2 | 02 July 2019 |
| | | | | US | 2017174692 | A1 | 22 June 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201911329611X
- CN 202010312893
- CN 202010882490
- WO 2019099298 A1
- US 20100280065 A
- WO 2019197549 A
- WO 2019099298 A

### Non-patent literature cited in the description


- **ASGHAR U ; WITKIEWICZ A K ; TURNER N C et al.** The history and future of targeting cyclin-dependent kinases in cancer therapy. *Nat Rev Drug Discov,* 2015, vol. 2, 130-146
- **YEE A ; NICHOLS MA ; WU L ; HALL FL ; KOBAYASHI R ; XIONG Y.** Molecular cloning of CDK7-associated human MAT1, a cyclin-dependent kinase-activating kinase (CAK) assembly factor. *Cancer Res.,* 1995, vol. 55, 6058-6062
- **KELSO TW ; BAUMGART K ; EICKHOFF J ; ALBERT T ; ANTRECHT C ; LEMCKE S et al.** Cyclin-dependent kinase 7 controls mRNA synthesis by affecting stability of preinitiation complexes, leading to altered gene expression, cell cycle progression, and survival of tumor cells. *Mol Cell Biol.,* 2014, vol. 34, 3675-3688
- **CHIPUMURO E ; MARCO E ; CHRISTENSEN CL ; KWIATKOWSKI N ; ZHANG T ; HATHEWAY CM et al.** CDK7 inhibition suppresses super-enhancer-linked oncogenic transcription in MYCN-driven cancer. *Cell.,* 2014, vol. 159, 1126-39
- **WANG Y ; ZHANG T ; KWIATKOWSKI N ; ABRAHAM BJ ; LEE TI ; XIE S et al.** CDK7dependent transcriptional addiction in triple-negative breast cancer. *Cell,* 2015, vol. 163, 174-86
- **CHRISTENSEN CL ; KWIATKOWSKI N ; ABRAHAM BJ ; CARRETERO J ; AL-SHAHROUR F ; ZHANG T et al.** Targeting transcriptional addictions in small cell lung cancer with a covalent CDK7 inhibitor. *Cancer Cell,* 2014, vol. 26, 909-22
- **BERGE et al.** Pharmaceutical Salts. *Journal of Pharmaceutical Science,* 1977, vol. 66, 1-19
- Properties, Selection, and Use. Handbook of Pharmaceutical Salts. Wiley, 2002
- *Pharmacopoeia of The People's Republic of China,* 2015
- **RAYMOND C ROWE.** *Handbook of Pharmaceutical Excipients,* 2009